(19) Europäisches Patentamt
European Patent Office
Office européen des brevets

(11) EP 4 332 086 B1

(12) EUROPEAN PATENT SPECIFICATION

(45) Date of publication and mention of the grant of the patent:
**08.04.2026 Bulletin 2026/15**

(21) Application number: **22795877.4**

(22) Date of filing: **28.04.2022**

(51) International Patent Classification (IPC):
***C07C 219/16*** *(2006.01)* ***C07C 323/25*** *(2006.01)*
***A61K 9/127*** *(2025.01)* ***A61K 9/51*** *(2006.01)*
***A61K 31/7088*** *(2006.01)* ***A61K 47/18*** *(2017.01)*
***A61K 47/24*** *(2006.01)* ***A61K 47/28*** *(2006.01)*
***A61K 48/00*** *(2006.01)* ***C12N 15/88*** *(2006.01)*

(52) Cooperative Patent Classification (CPC):
(C-Sets available)
**C07C 219/16; A61K 9/127; A61K 9/5123; A61K 31/7088; C07C 323/25; C12N 15/88;**
C07C 2603/74 (Cont.)

(86) International application number:
**PCT/JP2022/019220**

(87) International publication number:
**WO 2022/230964 (03.11.2022 Gazette 2022/44)**

(54) **COMPOUND OR SALT THEREOF, LIPID PARTICLES, AND PHARMACEUTICAL COMPOSITION**

VERBINDUNG ODER SALZ DAVON, LIPIDPARTIKEL UND PHARMAZEUTISCHE ZUSAMMENSETZUNG

COMPOSÉ OU SEL DE CELUI-CI, PARTICULES LIPIDIQUES ET COMPOSITION PHARMACEUTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.04.2021 JP 2021075525**

(43) Date of publication of application:
**06.03.2024 Bulletin 2024/10**

(73) Proprietor: **FUJIFILM Corporation**
**Tokyo 106-8620 (JP)**

(72) Inventors:
- **TANABE Shintaro**
  **Ashigarakami-gun, Kanagawa 258-8577 (JP)**
- **NITABARU Tatsuya**
  **Ashigarakami-gun, Kanagawa 258-8577 (JP)**
- **YAMAMOTO Masahiko**
  **Ashigarakami-gun, Kanagawa 258-8577 (JP)**
- **FUKUNAGA Hirofumi**
  **Ashigarakami-gun, Kanagawa 258-8577 (JP)**
- **NAKAMURA Naoto**
  **Ashigarakami-gun, Kanagawa 258-8577 (JP)**
- **KASAGI Noriyuki**
  **Ashigarakami-gun, Kanagawa 258-8577 (JP)**
- **ENDO Taisuke**
  **Ashigarakami-gun, Kanagawa 258-8577 (JP)**
- **SUZUKI Keiko**
  **Ashigarakami-gun, Kanagawa 258-8577 (JP)**

(74) Representative: **Hoffmann Eitle**
**Patent- und Rechtsanwälte PartmbB**
**Arabellastraße 30**
**81925 München (DE)**

(56) References cited:
**WO-A1-2018/232120**
**WO-A1-2019/235635**
**WO-A1-2019/235635**
**WO-A1-2020/219876**
**WO-A1-2020/246581**
**WO-A1-2021/095876**
**WO-A1-2021/117770**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61K 31/7088, A61K 2300/00**

## Description

Technical Field

The present invention relates to a compound or a salt thereof, and lipid particles and a pharmaceutical composition using the compound or a salt thereof.

Background Art

Nucleic acid drugs have a clear mechanism of action on diseases, have few side effects, and are regarded as promising next-generation medicines. For example, a nucleic acid drug using small interfering RNA (siRNA) can inhibit the expression of a target gene in a cell in a sequence-specific manner. As a result, the nucleic acid drug can relieve or treat the diseases and symptoms caused by the abnormal expression of a specific gene or gene group. In order to express the function of these nucleic acids, the nucleic acid drugs need to be delivered into cells.

One of the methods for efficiently delivering nucleic acids into cells is a method using a viral vector such as a retrovirus or an adenovirus. The method using a viral vector brings a high gene transfer efficiency. However, the size of genes to be transferred by this method is limited, and there is a concern over immunogenicity and safety of this method. On the other hand, in a case where lipid particles are used for gene transfer, any gene can be transferred without limitation, and the above problems can be solved. Therefore, the lipid particles are being developed vigorously.

As a compound to be incorporated into the lipid particles, Patent Document 1 discloses a compound having an ester group, an acetal group, or the like as a linking group that links an aliphatic group to an amino group. In addition, Patent Document 2 describes a compound having an alkylenediamine structure such as an ethylenediamine structure, and describes that lipid particles containing this compound exhibit a high nucleic acid encapsulation rate and excellent delivery of nucleic acids

Prior Art Documents

Patent Documents

Patent Document 1: WO2010/054401A
Patent Document 2: WO2019/235635A

## SUMMARY OF THE INVENTION

### OBJECT TO BE SOLVED BY THE INVENTION

The lipid particles that can function as vectors and the compounds that constitute the lipid particles are further sought for, and there is a demand for the development of compounds that can achieve excellent delivery of nucleic acids.

The present invention has been made under such circumstances, and an object thereof is to provide a compound or a salt thereof constituting lipid particles that can achieve a high nucleic acid encapsulation rate and excellent delivery of nucleic acids, and to provide lipid particles and pharmaceutical composition using the compound or a salt thereof, which can achieve a high nucleic acid encapsulation rate and excellent delivery of nucleic acids.

### MEANS FOR SOLVING THE OBJECT

In order to achieve the above object, the inventors of the present invention have conducted intensive studies. As a result, the inventors have found that lipid particles prepared using a compound represented by Formula (1) or a salt thereof exhibit a high nucleic acid encapsulation rate and excellent deliver of nucleic acids. Based on the finding, the inventors have accomplished the present invention. According to the present invention, the following inventions are provided.

<1> A compound represented by Formula (1) or a salt thereof.

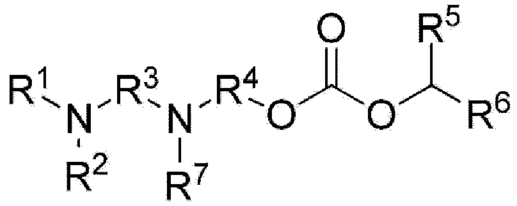

(1)

In the formula,

$R^1$ and $R^2$ each independently represent a hydrocarbon group having 1 to 18 carbon atoms, and $R^3$ represents a hydrocarbon group having 2 to 8 carbon atoms, where the hydrocarbon groups represented by $R^1$, $R^2$, and $R^3$ may be substituted with one or more substituents selected from -OH, -COOH, $-NR^{51}R^{52}$, $-OC(O)O-R^{53}$, $-C(O)O-R^{54}$, $-OC(O)-R^{55}$, and $-O-R^{56}$,

$R^4$ represents a hydrocarbon group having 1 to 8 carbon atoms,

$R^5$ and $R^6$ each independently represent a hydrocarbon group having 1 to 8 carbon atoms or $-R^8-L^1-R^9$, excluding a case that both $R^5$ and $R^6$ are hydrocarbon groups having 1 to 8 carbon atoms,

$R^7$ represents $-R^{10}-L^2-R^{11}-L^3-R^{12}$,

$R^{51}$ and $R^{52}$ each independently represent a hydrocarbon group having 1 to 8 carbon atoms,

$R^{53}$, $R^{54}$, $R^{55}$, and $R^{56}$ each independently represent a hydrocarbon group having 1 to 24 carbon atoms,

the hydrocarbon groups represented by $R^{53}$, $R^{54}$, $R^{55}$, and $R^{56}$ may be substituted with an aryl group having 6 to 20 carbon atoms or $-S-R^{58}$,

the above-described aryl group having 6 to 20 carbon atoms may be substituted with -OH, -COOH, $-NR^{51}R^{52}$, $-OC(O)O-R^{51}$, $-C(O)O-R^{54}$, $-OC(O)-R^{55}$, $-O-R^{56}$, or -(hydrocarbon group having 1 to 12 carbon atoms)-$R^{57}$,

$R^{58}$ represents a hydrocarbon group having 1 to 12 carbon atoms, and

$R^{57}$ represents -OH, -COOH, $-NR^{61}R^{62}$, $-OC(O)O-R^{63}$, $-C(O)O-R^{64}$, $-OC(O)-R^{65}$, or $-O-R^{66}$.

$R^{61}$ and $R^{62}$ each independently represent a hydrocarbon group having 1 to 8 carbon atoms,

$R^{63}$, $R^{64}$, $R^{65}$, and $R^{66}$ each independently represent a hydrocarbon group having 1 to 24 carbon atoms,

the hydrocarbon groups represented by $R^{63}$, $R^{64}$, $R^{65}$, and $R^{66}$ may be substituted with an aryl group having 6 to 20 carbon atoms or $-S-R^{68}$,

the above-described aryl group having 6 to 20 carbon atoms may be substituted with -OH, -COOH, $-NR^{61}R^{62}$, $-OC(O)O-R^{63}$, $-C(O)O-R^{64}$, $-OC(O)-R^{65}$, $-O-R^{66}$, or -(hydrocarbon group having 1 to 12 carbon atoms)-$R^{67}$,

$R^{68}$ represents a hydrocarbon group having 1 to 12 carbon atoms, and

$L^1$, $L^2$, and $L^3$ each independently represent -OC(O)O-, -C(O)O-, -OC(O)-, or -O-.

$R^8$ represents a hydrocarbon group having 1 to 12 carbon atoms,

$R^9$ represents a hydrocarbon group having 1 to 24 carbon atoms,

$R^{10}$ represents a hydrocarbon group having 1 to 8 carbon atoms,

$R^{11}$ represents a hydrocarbon group having 1 to 24 carbon atoms,

$R^{12}$ represents a hydrocarbon group having 1 to 24 carbon atoms,

the hydrocarbon groups represented by $R^9$ and $R^{12}$ may be substituted with an aryl group, $-OC(O)O-R^{53}$, $-C(O)O-R^{54}$, $-OC(O)-R^{55}$, or $-S-R^{58}$, where definitions of $R^{53}$, $R^{54}$, $R^{55}$ and $R^{58}$ are as described above, and

the hydrocarbon group represented by $R^{11}$ may be substituted with $-OC(O)O-R^{53}$, $-C(O)O-R^{54}$, or $-OC(O)-R^{55}$, where the definitions of $R^{53}$, $R^{54}$, and $R^{55}$ are as described above.

<2> The compound or a salt thereof according to <1>, in which $R^1$ and $R^2$ each independently represent a hydrocarbon group having 1 to 3 carbon atoms, and $R^3$ represents a hydrocarbon group having 2 to 4 carbon atoms, where the hydrocarbon groups represented by $R^1$, $R^2$, and $R^3$ may be substituted with -OH,

$R^4$ represents a hydrocarbon group having 1 to 8 carbon atoms,

$R^5$ and $R^6$ each independently represent a hydrocarbon group having 1 to 8 carbon atoms or $-R^8-L^1-R^9$, excluding a case that both $R^5$ and $R^6$ are hydrocarbon groups having 1 to 8 carbon atoms,

$R^7$ represents $-R^{10}-L^2-R^{11}-L^3-R^{12}$, and

$L^1$ and $L^3$ each independently represent -C(O)O- or -OC(O)-.

$L^2$ represents -OC(O)O-, -C(O)O-, or -OC(O)-.

$R^8$ represents a hydrocarbon group having 1 to 8 carbon atoms,

$R^9$ represents a hydrocarbon group having 1 to 16 carbon atoms,

$R^{10}$ represents a hydrocarbon group having 1 to 8 carbon atoms,

$R^{11}$ represents a hydrocarbon group having 1 to 9 carbon atoms,

$R^{12}$ represents a hydrocarbon group having 1 to 16 carbon atoms,

the hydrocarbon groups represented by $R^9$ and $R^{12}$ may be substituted with an aryl group or -S-$R^{58}$,
$R^{58}$ represents a hydrocarbon group having 1 to 8 carbon atoms,
the hydrocarbon group represented by $R^{11}$ may be substituted with -C(O)O-$R^{55}$ or -OC(O)-$R^{56}$,
$R^{55}$ and $R^{56}$ each independently represent a hydrocarbon group having 1 to 16 carbon atoms, and
the hydrocarbon groups represented by $R^{55}$ and $R^{56}$ may be substituted with an aryl group having 6 to 20 carbon atoms or -S-$R^{58}$, and definition of $R^{58}$ is as described above.

<3> The compound or a salt thereof according to <1>, which is a compound represented by Formula (1-1) or a salt thereof.

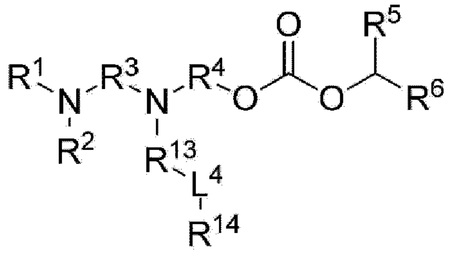

(1 − 1)

In the formula,
$R^1$ and $R^2$ each independently represent a hydrocarbon group having 1 to 18 carbon atoms, and $R^3$ represents a hydrocarbon group having 2 to 8 carbon atoms, where the hydrocarbon groups represented by $R^1$, $R^2$, and $R^3$ may be substituted with -OH, -COOH, -N$R^{51}R^{52}$, -OC(O)O-$R^{13}$, -C(O)O-$R^{54}$, -OC(O)-$R^{55}$, or -O-$R^{56}$,
$R^4$ represents a hydrocarbon group having 1 to 8 carbon atoms,
$R^5$ and $R^6$ each independently represent a hydrocarbon group having 1 to 8 carbon atoms or -$R^8$-$L^1$-$R^9$, excluding a case that both $R^5$ and $R^6$ are hydrocarbon groups having 1 to 8 carbon atoms,
$L^1$ represents -OC(O)O-, -C(O)O-, -OC(O)-, or -O-,
$R^8$ represents a hydrocarbon group having 1 to 12 carbon atoms,
$R^9$ represents a hydrocarbon group having 1 to 24 carbon atoms, where the hydrocarbon group represented by $R^9$ may be substituted with an aryl group, -OC(O)O-$R^{53}$, -C(O)O-$R^{54}$, -OC(O)-$R^{55}$, or -S-$R^{58}$,
$R^{51}$ and $R^{52}$ each independently represent a hydrocarbon group having 1 to 8 carbon atoms,
$R^{53}$, $R^{54}$, $R^{55}$, and $R^{56}$ each independently represent a hydrocarbon group having 1 to 24 carbon atoms,
the hydrocarbon groups represented by $R^{53}$, $R^{54}$, $R^{55}$, and $R^{56}$ may be substituted with an aryl group having 6 to 20 carbon atoms or -S-$R^{58}$,
the above-described aryl group having 6 to 20 carbon atoms may be substituted with -OH, -COOH, -N$R^{51}R^{52}$, -OC(O)O-$R^{13}$, -C(O)O-$R^{54}$, -OC(O)-$R^{55}$, -O-$R^{56}$, or -(hydrocarbon group having 1 to 12 carbon atoms)-$R^{57}$,
$R^{58}$ represents a hydrocarbon group having 1 to 12 carbon atoms, and
$R^{57}$ represents -OH, -COOH, -N$R^{51}R^{52}$, -OC(O)O-$R^{53}$, -C(O)O-$R^{54}$, -OC(O)-$R^{55}$, or -O-$R^{56}$.
$R^{13}$ represents a hydrocarbon group having 1 to 8 carbon atoms,
$R^{14}$ represents -$R^{15}$-$L^5$-$R^{16}$, where $R^{15}$ represents a hydrocarbon group having 1 to 24 carbon atoms, $L^5$ represents -OC(O)O-, -C(O)O-, -OC(O)-, or -O-, and $R^{16}$ represents a hydrocarbon group having 1 to 24 carbon atoms,
the hydrocarbon group having 1 to 24 carbon atoms represented by $R^{15}$ may be substituted with -OC(O)O-$R^{53}$, -C(O)O-$R^{54}$, or -OC(O)-$R^{55}$, where definitions of $R^{53}$, $R^{54}$, and $R^{55}$ are as described above, and
the hydrocarbon group having 1 to 24 carbon atoms represented by $R^{16}$ may be substituted with an aryl group having 6 to 20 carbon atoms, -OC(O)O-$R^{53}$, -C(O)O-$R^{54}$, -OC(O)-$R^{55}$ or -S-$R^{58}$, where the definitions of $R^{53}$, $R^{54}$, $R^{55}$, and $R^{58}$ are as described above.

<4> The compound and a salt thereof according to <3>, in which in Formula (1-1),

$R^1$ and $R^2$ each independently represent a hydrocarbon group having 1 to 3 carbon atoms, and $R^3$ represents a hydrocarbon group having 2 to 4 carbon atoms, where the hydrocarbon groups represented by $R^1$, $R^2$, and $R^3$ may be substituted with -OH,
$R^4$ represents a hydrocarbon group having 1 to 8 carbon atoms,
$R^5$ and $R^6$ each independently represent a hydrocarbon group having 1 to 8 carbon atoms or -$R^8$-$L^1$-$R^9$, excluding a case that both $R^5$ and $R^6$ are hydrocarbon groups having 1 to 8 carbon atoms,
$L^1$ represents -C(O)O- or -OC(O)-,

$R^8$ represents a hydrocarbon group having 1 to 8 carbon atoms,
$R^9$ represents a hydrocarbon group having 1 to 18 carbon atoms, and the hydrocarbon group represented by $R^9$ may be substituted with an aryl group having 6 to 20 carbon atoms or -S-$R^{58}$,
$R^{58}$ represents a hydrocarbon group having 1 to 8 carbon atoms,
$R^{13}$ represents a hydrocarbon group having 1 to 8 carbon atoms,
$R^{14}$ represents -$R^{15}$-$L^5$-$R^{16}$, where $R^{15}$ represents a hydrocarbon group having 1 to 18 carbon atoms, $L^5$ represents -OC(O)O-, and $R^{16}$ represents a hydrocarbon group having 1 to 18 carbon atoms,
the hydrocarbon group having 1 to 18 carbon atoms represented by $R^{15}$ may be substituted with -C(O)O-$R^{55}$ or -OC(O)-$R^{56}$,
$R^{55}$ and $R^{56}$ each independently represent a hydrocarbon group having 1 to 16 carbon atoms, and the hydrocarbon groups represented by $R^{55}$ and $R^{56}$ may be substituted with an aryl group having 6 to 20 carbon atoms or -S-$R^{58}$, where the definition of $R^{58}$ is as described above, and
the hydrocarbon group having 1 to 18 carbon atoms represented by $R^{16}$ may be substituted with an aryl group or -S-$R^{58}$, where the definition of $R^{58}$ is as described above.

<5> The compound or a salt thereof according to <1>, which is a compound represented by Formula (1-2) or a salt thereof.

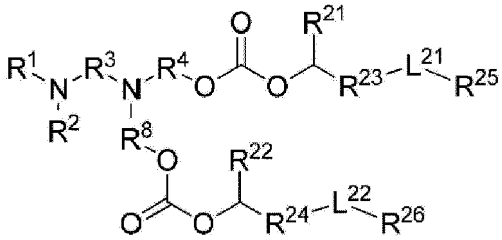

(1－2)

In the formula,
$R^1$ and $R^2$ each independently represent a hydrocarbon group having 1 to 18 carbon atoms, and $R^3$ represents a hydrocarbon group having 2 to 8 carbon atoms, where the hydrocarbon groups represented by $R^1$, $R^2$, and $R^3$ may be substituted with -OH, -COOH, -$NR^{51}R^{52}$, -OC(O)O-$R^{53}$, -C(O)O-$R^{54}$, -OC(O)-$R^{55}$, or -O-$R^{56}$,
$R^4$ and $R^8$ each independently represent a hydrocarbon group having 1 to 8 carbon atoms,
$R^{21}$ and $R^{22}$ each independently represent a hydrocarbon group having 1 to 18 carbon atoms,
$R^{23}$ and $R^{24}$ each independently represent a hydrocarbon group having 1 to 12 carbon atoms,
$R^{25}$ and $R^{26}$ each independently represent a hydrocarbon group having 1 to 24 carbon atoms,
$L^{21}$ and $L^{22}$ each independently represent -OC(O)O-, -C(O)O-, -OC(O)-, or -O-,
the hydrocarbon groups represented by $R^{25}$ and $R^{26}$ may be substituted with an aryl group having 6 to 20 carbon atoms, -OC(O)O-$R^{53}$, -C(O)O-$R^{54}$, -OC(O)-$R^{55}$, or -S-$R^{58}$,
$R^{51}$ and $R^{52}$ each independently represent a hydrocarbon group having 1 to 8 carbon atoms,
$R^{53}$, $R^{54}$, $R^{55}$, and $R^{56}$ each independently represent a hydrocarbon group having 1 to 18 carbon atoms,
the above-described aryl group having 6 to 20 carbon atoms may be substituted with -OH, -COOH, -$NR^{51}R^{52}$, -OC(O)O-$R^{53}$, -C(O)O-$R^{54}$, -OC(O)-$R^{55}$, -O-$R^{56}$, or -(hydrocarbon group having 1 to 12 carbon atoms)-$R^{57}$, and
$R^{57}$ represents -OH, -COOH, -$NR^{51}R^{52}$, -OC(O)O-R $^{51}$, -C(O)O-$R^{54}$, -OC(O)-$R^{55}$, or -O-$R^{56}$.
$R^{58}$ represents a hydrocarbon group having 1 to 12 carbon atoms.

<6> The compound and a salt thereof according to <5>, in which in Formula (1-2),

$R^1$ and $R^2$ each independently represent a hydrocarbon group having 1 to 3 carbon atoms, where the hydrocarbon groups represented by $R^1$ and $R^2$ may be substituted with -OH,
$R^3$ represents a hydrocarbon group having 2 to 4 carbon atoms,
$R^4$ and $R^8$ each independently represent a hydrocarbon group having 1 to 8 carbon atoms,
$R^{21}$ and $R^{22}$ each independently represent a hydrocarbon group having 1 to 8 carbon atoms,
$R^{23}$ and $R^{24}$ each independently represent a hydrocarbon group having 1 to 8 carbon atoms,
$R^{25}$ and $R^{26}$ each independently represent a hydrocarbon group having 1 to 16 carbon atoms, and
$L^{21}$ and $L^{22}$ each independently represent -C(O)O- or -OC(O)-.

<7> The compound and a salt thereof according to <5>, in which in Formula (1-2),

$R^1$ and $R^2$ each independently represent a hydrocarbon group having 1 to 3 carbon atoms,
$R^3$ represents a hydrocarbon group having 2 to 4 carbon atoms,
$R^4$ and $R^8$ each independently represent a hydrocarbon group having 1 to 8 carbon atoms,
$R^{21}$ and $R^{22}$ each independently represent a hydrocarbon group having 1 to 6 carbon atoms,
$R^{23}$ and $R^{24}$ each independently represent a hydrocarbon group having 1 to 8 carbon atoms,
$R^{25}$ and $R^{26}$ each independently represent a hydrocarbon group having 1 to 12 carbon atoms, and
$L^{21}$ and $L^{22}$ each independently represent -C(O)O- or -OC(O)-.

<8> The compound or a salt thereof according to <1>, which is a compound represented by Formula (1-3) or a salt thereof.

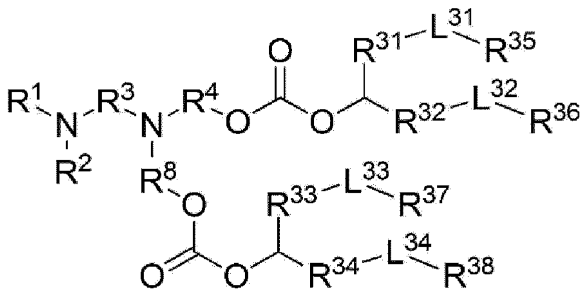

(1-3)

In the formula,
$R^1$ and $R^2$ each independently represent a hydrocarbon group having 1 to 18 carbon atoms, and $R^3$ represents a hydrocarbon group having 2 to 8 carbon atoms, where the hydrocarbon groups represented by $R^1$, $R^2$, and $R^3$ may be substituted with -OH, -COOH, -$NR^{51}R^{52}$, -OC(O)O-$R^{53}$, -C(O)O-$R^{54}$, -OC(O)-$R^{55}$, or -O-$R^{56}$,
$R^4$ and $R^8$ each independently represent a hydrocarbon group having 1 to 8 carbon atoms,
$R^{31}$, $R^{32}$, $R^{33}$, and $R^{34}$ each independently represent a hydrocarbon group having 1 to 12 carbon atoms,
$R^{35}$, $R^{36}$, $R^{37}$, and $R^{38}$ each independently represent a hydrocarbon group having 1 to 24 carbon atoms,
$L^{31}$, $L^{32}$, $L^{33}$, and $L^{34}$ each independently represent -OC(O)O-, -C(O)O-, -OC(O)-, or -O-,
the hydrocarbon groups represented by $R^{35}$, $R^{36}$, $R^{37}$, and $R^{38}$ may be substituted with an aryl group having 6 to 20 carbon atoms, -OC(O)O-$R^{53}$, -C(O)O-$R^{54}$, -OC(O)-$R^{55}$, or -S-$R^{58}$,
$R^{51}$ and $R^{52}$ each independently represent a hydrocarbon group having 1 to 8 carbon atoms,
$R^{53}$, $R^{54}$, $R^{55}$, and $R^{56}$ each independently represent a hydrocarbon group having 1 to 18 carbon atoms,
the above-described aryl group having 6 to 20 carbon atoms may be substituted with -OH, -COOH, -$NR^{51}R^{52}$, -OC(O)O-$R^{53}$, -C(O)O-$R^{54}$, -OC(O)-$R^{55}$, -O-$R^{56}$, or -(hydrocarbon group having 1 to 12 carbon atoms)-$R^{57}$, and $R^{57}$ represents -OH, -COOH, -$NR^{51}R^{52}$, -OC(O)O-$R^{53}$, -C(O)O-$R^{54}$, -OC(O)-$R^{55}$, or -O-$R^{56}$.
$R^{58}$ represents a hydrocarbon group having 1 to 12 carbon atoms.

<9> The compound and a salt thereof according to <8>, in which in Formula (1-3),

$R^1$ and $R^2$ each independently represent a hydrocarbon group having 1 to 3 carbon atoms, where the hydrocarbon groups represented by $R^1$ and $R^2$ may be substituted with -OH,
$R^3$ represents a hydrocarbon group having 2 to 4 carbon atoms,
$R^4$ and $R^8$ each independently represent a hydrocarbon group having 1 to 8 carbon atoms,
$R^{31}$, $R^{32}$, $R^{33}$, and $R^{34}$ each independently represent a hydrocarbon group having 1 to 8 carbon atoms,
$R^{35}$, $R^{36}$, $R^{37}$, and $R^{38}$ each independently represent a hydrocarbon group having 1 to 16 carbon atoms,
$L^{31}$, $L^{32}$, $L^{33}$, and $L^{34}$ each independently represent -C(O)O- or -OC(O)-,
the hydrocarbon groups represented by $R^{35}$, $R^{36}$, $R^{37}$, and $R^{38}$ may be substituted with an aryl group having 6 to 20 carbon atoms or -S-$R^{58}$, and
$R^{58}$ represents a hydrocarbon group having 1 to 8 carbon atoms.

<10> The compound and a salt thereof according to <8>, in which in Formula (1-3),

$R^1$ and $R^2$ each independently represent a hydrocarbon group having 1 to 3 carbon atoms,
$R^3$ represents a hydrocarbon group having 2 to 4 carbon atoms,
$R^4$ and $R^8$ each independently represent a hydrocarbon group having 1 to 8 carbon atoms,
$R^{31}$, $R^{32}$, $R^{33}$, and $R^{34}$ each independently represent a hydrocarbon group having 1 to 3 carbon atoms,

$R^{35}$, $R^{36}$, $R^{37}$, and $R^{38}$ each independently represent a hydrocarbon group having 1 to 12 carbon atoms,
$L^{31}$, $L^{32}$, $L^{33}$, and $L^{34}$ each independently represent -C(O)O- or -OC(O)-,
the hydrocarbon groups represented by $R^{35}$, $R^{36}$, $R^{37}$, and $R^{38}$ may be substituted with -S-$R^{58}$, and
$R^{58}$ represents a hydrocarbon group having 1 to 8 carbon atoms.

<11> The compound and a salt thereof according to <8>, in which in Formula (1-3),

$R^1$ and $R^2$ each independently represent a hydrocarbon group having 1 to 3 carbon atoms,
$R^3$ represents a hydrocarbon group having 2 to 4 carbon atoms,
$R^4$ and $R^8$ each independently represent a hydrocarbon group having 1 to 8 carbon atoms,
$R^{31}$, $R^{32}$, $R^{33}$, and $R^{34}$ each independently represent a hydrocarbon group having 1 to 3 carbon atoms,
$R^{35}$, $R^{36}$, $R^{37}$, and $R^{38}$ each independently represent a hydrocarbon group having 1 to 12 carbon atoms substituted with -S-$R^{58}$, where $R^{58}$ represents a hydrocarbon group having 1 to 8 carbon atoms, and
$L^{31}$, $L^{32}$, $L^{33}$, and $L^{34}$ each independently represent -C(O)O- or -OC(O)-.

<12> The compound and a salt thereof according to <8>, in which in Formula (1-3),

$R^1$ and $R^2$ each independently represent a hydrocarbon group having 1 to 3 carbon atoms,
$R^3$ represents a hydrocarbon group having 2 to 4 carbon atoms,
$R^4$ and $R^8$ each independently represent a hydrocarbon group having 1 to 8 carbon atoms,
$R^{31}$, $R^{32}$, $R^{33}$, and $R^{34}$ each independently represent a hydrocarbon group having 1 to 3 carbon atoms,
$R^{35}$, $R^{36}$, $R^{37}$, and $R^{38}$ each independently represent a hydrocarbon group having 1 to 12 carbon atoms, and
$L^{31}$, $L^{32}$, $L^{33}$, and $L^{34}$ each independently represent -C(O)O- or -OC(O)-.

<13> Compounds shown in the following formulae, or salts thereof,

Bis(2-butyloctyl) 16-(3-(diethylamino)propyl)-10,22-dihexyl-12,20-dioxo-11,13,19,21-tetraoxa-16-azahentriaco ntanedioate:

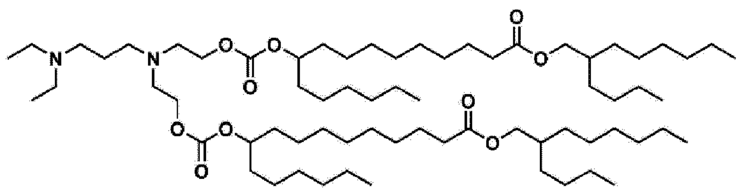

Bis(2-butyloctyl)

11-(2-(diethylamino)ethyl)-5,17-dihexyl-7,15-dioxo-6,8,14,16-tetraoxa-11-azahenicosanedioat e:

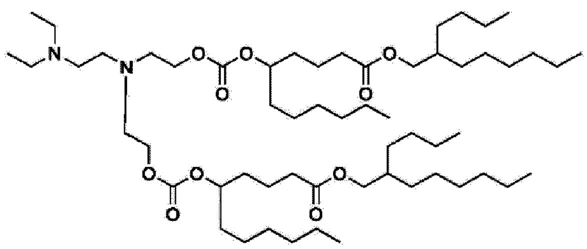

Bis(2-pentylheptyl)

11-(2-(diethylamino)ethyl)-5,17-dihexyl-7,15-dioxo-6,8,14,16-tetraoxa-11-azahenicosanedioat e:

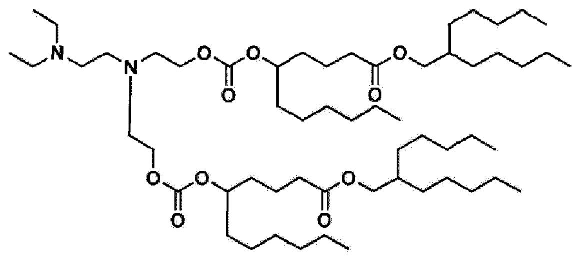

Bis(2-pentylheptyl)

11-(2-(dimethylamino)ethyl)-5,17-dihexyl-7,15-dioxo-6,8,14,16-tetraoxa-11-azahenicosanedio ate:

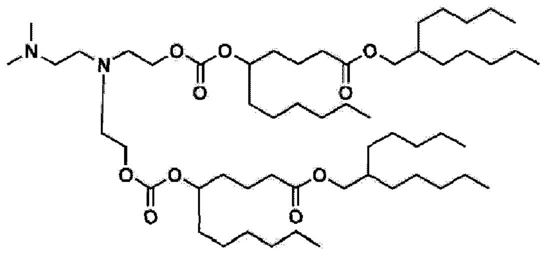

Bis(2-pentylheptyl)

11-(3-(diethylamino)propyl)-5,17-dihexyl-7,15-dioxo-6,8,14,16-tetraoxa-11-azahenicosanedio ate:

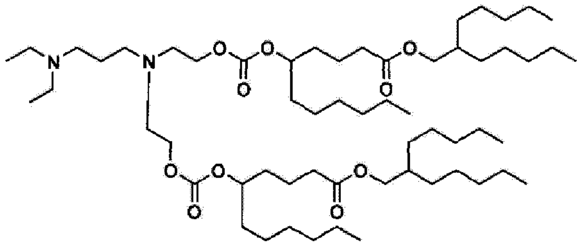

Bis(2-pentylheptyl)

11-(4-(diethylamino)butyl)-5,17-dihexyl-7,15-dioxo-6,8,14,16-tetraoxa-11-azahenicosanedioat e:

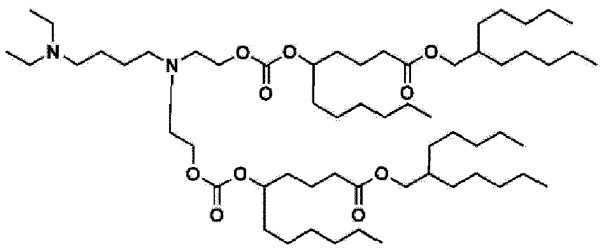

Bis(2-pentylheptyl)

12-(2-(diethylamino)ethyl)-5,19-dihexyl-7,17-dioxo-6,8,16,18-tetraoxa-12-azatricosanedioate:

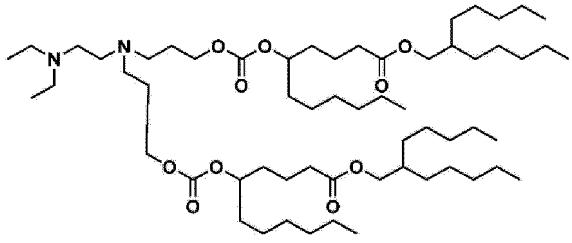

Bis(2-pentylheptyl) 13-(2-(diethylamino)ethyl)-5,21-dihexyl-7,19-dioxo-6,8,18,20-tetraoxa-13-azapentacosanedio ate:

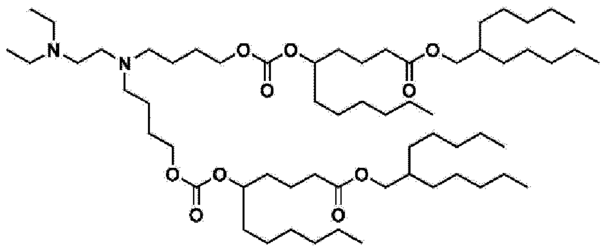

Bis(2-pentylheptyl) 10-(2-(diethylamino)ethyl)-4,16-dihexyl-6,14-dioxo-5,7,13,15-tetraoxa-10-azanonadecanedioa te:

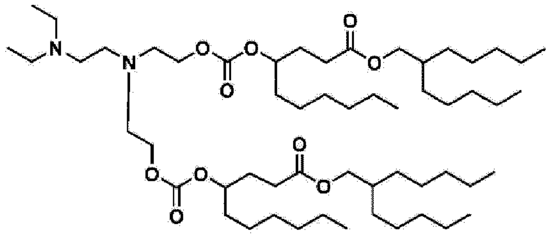

Bis(2-pentylheptyl) 11-(2-(diethylamino)ethyl)-5,17-dimethyl-7,15-dioxo-6,8,14,16-tetraoxa-11-azahenicosanedio ate:

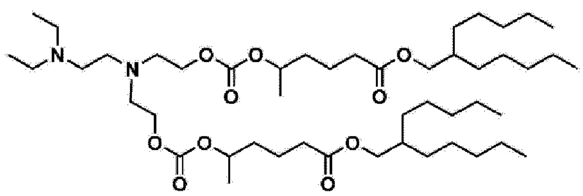

Bis(2-pentylheptyl) 11-(2-(diethylamino)ethyl)-5,17-diethyl-7,15-dioxo-6,8,14,16-tetraoxa-11-azahenicosanedioat e:

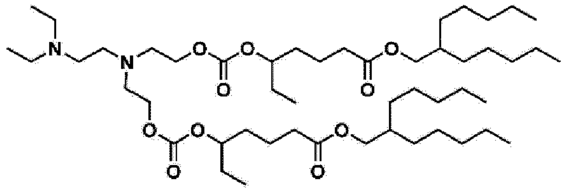

Bis(2-pentylheptyl) 11-(2-(diethylamino)ethyl)-7,15-dioxo-5,17-dipropyl-6,8,14,16-tetraoxa-11-azahenicosanedioa te:

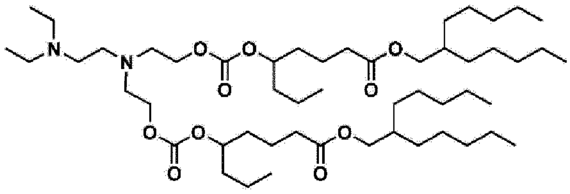

Bis(2-pentylheptyl) 5,17-dibutyl-11-(2-(diethylamino)ethyl)-7,15-dioxo-6,8,14,16-tetraoxa-11-azahenicosanedioat e:

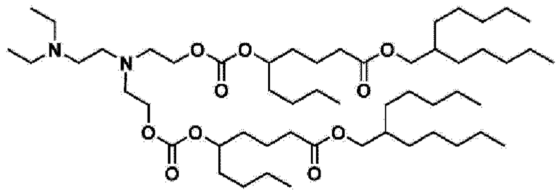

Bis(2-pentylheptyl) 11-(2-(diethylamino)ethyl)-7,15-dioxo-5,17-dipentyl-6,8,14,16-tetraoxa-11-azahenicosanedioa te:

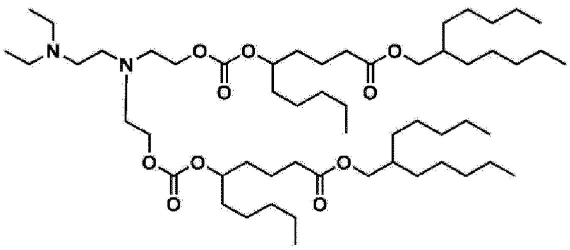

Bis(2-pentylheptyl) 11-(2-(diethylamino)ethyl)-5,17-diheptyl-7,15-dioxo-6,8,14,16-tetraoxa-11-azahenicosanedioa te:

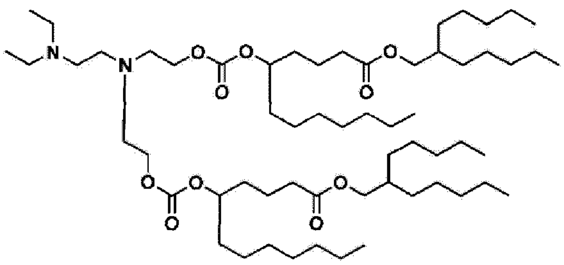

Bis(2-((3r,5r,7r)-adamantane-1-yl)ethyl)

11-(2-(diethylamino)ethyl)-5,17-dihexyl-7,15-dioxo-6,8,14,16-tetraoxa-11-azahenicosanedioat e:

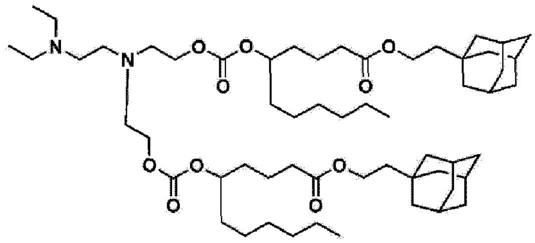

Bis(2-pentylheptyl)

11-(3-(diethylamino)propyl)-7,15-dioxo-5,17-dipropyl-6,8,14,16-tetraoxa-11-azahenicosanedi oate:

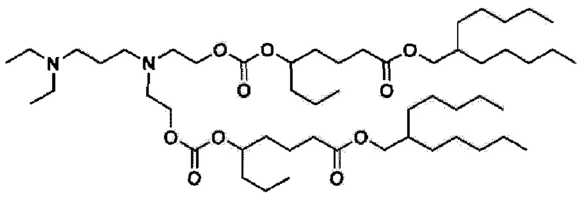

Diheptyl

11-(2-(diethylamino)ethyl)-5,17-bis(4-(heptyloxy)-4-oxobutyl)-7,15-dioxo-6,8,14,16-tetraoxa-11-azahenicosanedioate:

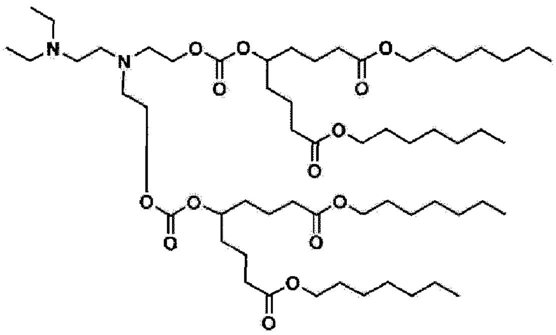

Dihexyl

11-(2-(diethylamino)ethyl)-5,17-bis(4-(hexyloxy)-4-oxobutyl)-7,15-dioxo-6,8,14,16-tetraoxa-11-azahenicosanedioate:

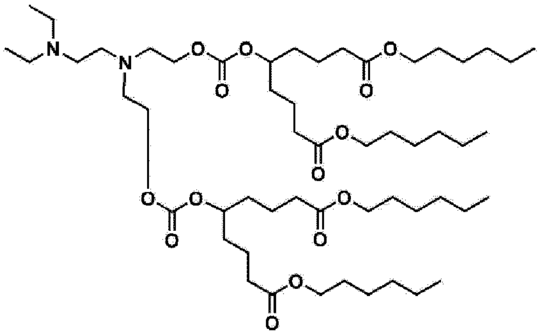

Dioctyl

11-(2-(diethylamino)ethyl)-5,17-bis(4-(octyloxy)-4-oxobutyl)-7, 15-dioxo-6,8,14,16-tetraoxa-1 1-azahenicosanedioate:

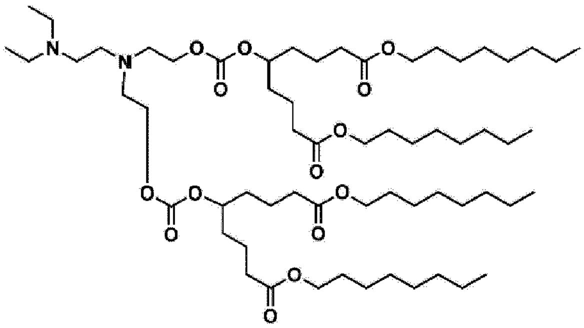

Dinonyl

11-(2-(diethylamino)ethyl)-5,17-bis(4-(nonyloxy)-4-oxobutyl)-7,15-dioxo-6,8,14,16-tetraoxa-11-azahenicosanedioate:

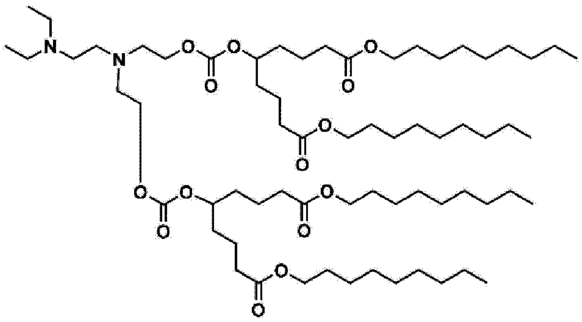

1-Heptyl 21-hexyl

11-(2-(diethylamino)ethyl)-5-(4-(heptyloxy)-4-oxobutyl)-17-(4-(hexyloxy)-4-oxobutyl)-7,15-d ioxo-6,8,14,16-tetraoxa-11-azahenicosanedioate:

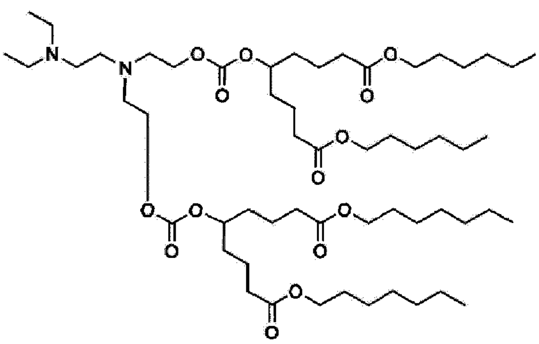

Diheptyl

11-(3-(diethylamino)propyl)-5,17-bis(4-(heptyloxy)-4-oxobutyl)-7,15-dioxo-6,8,14,16-tetraox a-11-azahenicosanedioate:

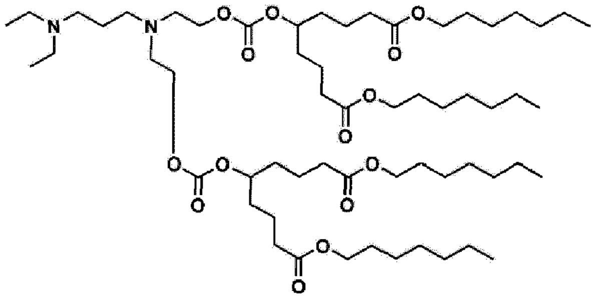

Diheptyl

10-(2-(diethylamino)ethyl)-4,16-bis(3-(heptyloxy)-3-oxopropyl)-6,14-dioxo-5,7,13,15-tetraox a-10-azanonadecanedioate:

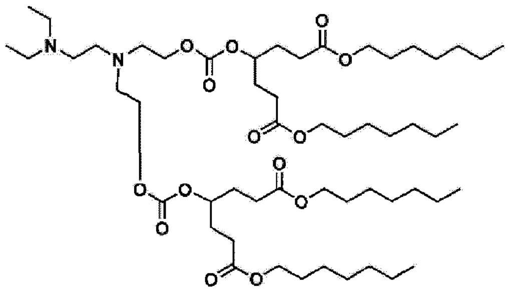

1-Hexyl 21-octyl

11-(2-(diethylamino)ethyl)-5-(4-(hexyloxy)-4-oxobutyl)-17-(4-(octyloxy)-4-oxobutyl)-7,15-di oxo-6,8,14,16-tetraoxa-11-azahenicosanedioate:

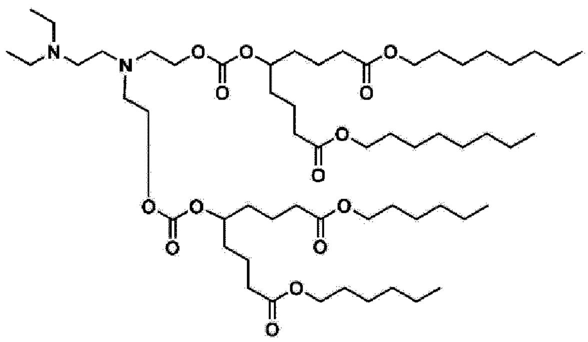

Bis(2-(hexylthio)ethyl)

11-(2-(diethylamino)ethyl)-5,17-bis(4-(2-(hexylthio)ethoxy)-4-oxobutyl)-7,15-dioxo-6,8,14,16 -tetraoxa-11-azahenicosanedioate:

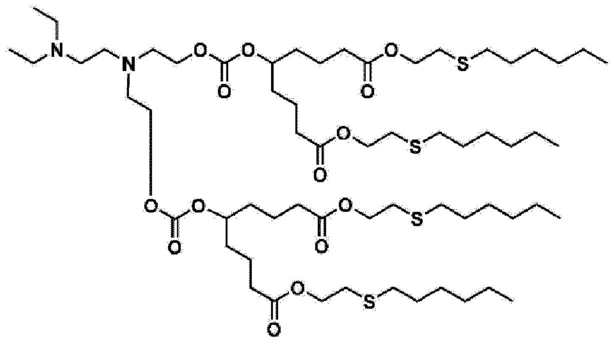

Bis(8-(methylthio)octyl)

11-(2-(diethylamino)ethyl)-5,17-bis(4-((8-(methylthio)octyl)oxy)-4-oxobutyl)-7,15-dioxo-6,8, 14,16-tetraoxa-11-azahenicosanedioate:

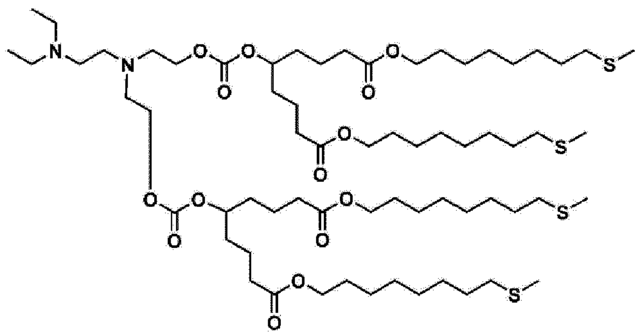

.

<14> Lipid particles containing the compound or a salt thereof according to any one of <1> to <13>, and a lipid.
<15> The lipid particles according to <14>, in which the lipid is at least one kind of lipid selected from the group consisting of a sterol and a lipid having a nonionic hydrophilic polymer chain.
<16> The lipid particles according to <14> or <15>, further containing a neutral lipid.
<17> The lipid particles according to any one of <14> to <16>, further containing a nucleic acid.
<18> The lipid particles according to <17>, in which the nucleic acid includes a nucleic acid having 50 or more bases.
<19> A pharmaceutical composition contains the lipid particles according to any one of <14> to <18> as an active ingredient.

By using the compound according to an aspect of the present invention, it is possible to manufacture lipid particles and a pharmaceutical composition that can achieve a high nucleic acid encapsulation rate and excellent delivery of nucleic acids. The lipid particles and the pharmaceutical composition according to an aspect of the present invention can achieve a high nucleic acid encapsulation rate and excellent delivery of nucleic acids.

## DESCRIPTION OF THE PREFERRED EMBODIMENTS

Hereinafter, the present invention is specifically described.
In the present specification, "to" shows a range including numerical values described before and after "to" as a minimum value and a maximum value, respectively.

<Compound according to embodiment of present invention>

The compound according to the embodiment of the present invention is represented by Formula (1).

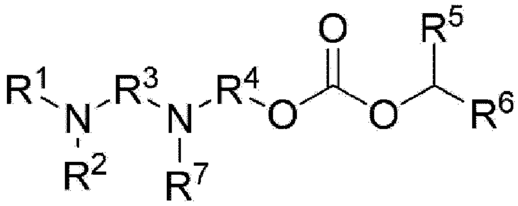

(1)

In the formula,

$R^1$ and $R^2$ each independently represent a hydrocarbon group having 1 to 18 carbon atoms, and $R^3$ represents a hydrocarbon group having 2 to 8 carbon atoms, where the hydrocarbon groups represented by $R^1$, $R^2$, and $R^3$ may be substituted with one or more substituents selected from -OH, -COOH, $-NR^{51}R^{52}$, $-OC(O)O-R^{53}$, $-C(O)O-R^{54}$, $-OC(O)-R^{55}$, and $-O-R^{56}$,
$R^4$ represents a hydrocarbon group having 1 to 8 carbon atoms,
$R^5$ and $R^6$ each independently represent a hydrocarbon group having 1 to 8 carbon atoms or $-R^8-L^1-R^9$, excluding a case that both $R^5$ and $R^6$ are hydrocarbon groups having 1 to 8 carbon atoms,
$R^7$ represents $-R^{10}-L^2-R^{11}-L^3-R^{12}$,
$R^{51}$ and $R^{52}$ each independently represent a hydrocarbon group having 1 to 8 carbon atoms,
$R^{53}$, $R^{54}$, $R^{55}$, and $R^{56}$ each independently represent a hydrocarbon group having 1 to 24 carbon atoms,
the hydrocarbon groups represented by $R^{53}$, $R^{54}$, $R^{55}$, and $R^{56}$ may be substituted with an aryl group having 6 to 20 carbon atoms or $-S-R^{58}$,
the above-described aryl group having 6 to 20 carbon atoms may be substituted with -OH, -COOH, $-NR^{51}R^{52}$, $-OC(O)O-R^{51}$, $-C(O)O-R^{54}$, $-OC(O)-R^{55}$, $-O-R^{56}$, or -(hydrocarbon group having 1 to 12 carbon atoms)-$R^{57}$,
$R^{58}$ represents a hydrocarbon group having 1 to 12 carbon atoms, and
$R^{57}$ represents -OH, -COOH, $-NR^{61}R^{62}$, $-OC(O)O-R^{63}$, $-C(O)O-R^{64}$, $-OC(O)-R^{65}$, or $-O-R^{66}$.
$R^{61}$ and $R^{62}$ each independently represent a hydrocarbon group having 1 to 8 carbon atoms,
$R^{63}$, $R^{64}$, $R^{65}$, and $R^{66}$ each independently represent a hydrocarbon group having 1 to 24 carbon atoms,
the hydrocarbon groups represented by $R^{63}$, $R^{64}$, $R^{65}$, and $R^{66}$ may be substituted with an aryl group having 6 to 20 carbon atoms or $-S-R^{68}$,
the above-described aryl group having 6 to 20 carbon atoms may be substituted with -OH, -COOH, $-NR^{61}R^{62}$, $-OC(O)O-R^{63}$, $-C(O)O-R^{64}$, $-OC(O)-R^{65}$, $-O-R^{66}$, or -(hydrocarbon group having 1 to 12 carbon atoms)-$R^{67}$,
$R^{68}$ represents a hydrocarbon group having 1 to 12 carbon atoms, and
$L^1$, $L^2$, and $L^3$ each independently represent -OC(O)O-, -C(O)O-, -OC(O)-, or -O-.
$R^8$ represents a hydrocarbon group having 1 to 12 carbon atoms,
$R^9$ represents a hydrocarbon group having 1 to 24 carbon atoms,
$R^{10}$ represents a hydrocarbon group having 1 to 8 carbon atoms,
$R^{11}$ represents a hydrocarbon group having 1 to 24 carbon atoms,
$R^{12}$ represents a hydrocarbon group having 1 to 24 carbon atoms,
the hydrocarbon groups represented by $R^9$ and $R^{12}$ may be substituted with an aryl group, $-OC(O)O-R^{53}$, $-C(O)O-R^{54}$, $-OC(O)-R^{55}$, or $-S-R^{58}$, where definitions of $R^{53}$, $R^{54}$, $R^{55}$, and $R^{58}$ are as described above, and
the hydrocarbon group represented by $R^{11}$ may be substituted with $-OC(O)O-R^{53}$, $-C(O)O-R^{54}$, or $-OC(O)-R^{55}$, where the definitions of $R^{53}$, $R^{54}$, and $R^{55}$ are as described above.

A hydrocarbon group having 1 to 24 carbon atoms, a hydrocarbon group having 1 to 18 carbon atoms, a hydrocarbon group having 1 to 12 carbon atoms, a hydrocarbon group having 2 to 8 carbon atoms, and a hydrocarbon group having 1 to 8 carbon atoms are each preferably an alkyl group, an alkenyl group, or an alkynyl group.

The alkyl group may be linear or branched, or may be chainlike or cyclic. Specifically, examples of the alkyl group include a methyl group, an ethyl group, a propyl group, an isopropyl group, a cyclopropyl group, a butyl group, an isobutyl group, a tert-butyl group, a cyclobutyl group, a pentyl group, a cyclopentyl group, a hexyl group, a cyclohexyl group, a heptyl group, an octyl group, a nonyl group, a decyl group, an undecyl group, a dodecyl group, a tridecyl group, a trimethyldodecyl group (preferably a 3,7,11-trimethyldodecyl group), a tetradecyl group, a pentadecyl group, a hexadecyl group, a tetramethylhexadecyl group (preferably a 3,7,11,15-tetramethylhexadecyl group), a heptadecyl group, an octadecyl group, a 2-butylhexyl group, a 2-butyloctyl group, a 1-pentylhexyl group, a 2-pentylheptyl group, a 3-pentyloctyl group, a 1-hexylheptyl group, a 1-hexylnonyl group, a 2-hexyloctyl group, a 2-hexyldecyl group, a 3-hexylnonyl group, a 1-heptyloctyl group, a 2-heptylnonyl group, a 2-heptylundecyl group, a 3-heptyldecyl group, a 1-octylnonyl group, a 2-octyldecyl group, a 2-octyldodecyl group, a 3-octylundecyl group, a 2-nonylundecyl group, a 3-nonyldodecyl group, a 2-decyldodecyl group, a 2-decyltetradecyl group, a 3-decyltridecyl group, a 2-(4,4-dimethylpentan-2-yl)-5,7,7-trimethyloctyl

group, and the like.

The alkenyl group may be linear or branched, or may be chainlike or cyclic. Specifically, examples of the alkenyl group include an allyl group, a prenyl group, a pentenyl group, a hexenyl group, a heptenyl group, an octenyl group, a nonenyl group (preferably a (Z)-2-nonenyl group or an (E)-2-nonenyl group), a decenyl group, an undecenyl group, a dodecenyl group, a dodecadienyl group, a tridecenyl group (preferably a (Z)-tridec-8-enyl group), a tetradecenyl group (preferably a tetradec-9-enyl group), a pentadecenyl group (preferably a (Z)-pentadec-8-enyl group), a hexadecenyl group (preferably a (Z)-hexadec-9-enyl group), a hexadecadienyl group, a heptadecenyl group (preferably a (Z)-heptadec-8-enyl group), a heptadecadienyl group (preferably a (8Z,11Z)-heptadeca-8,11-dienyl group), an octadecenyl group (preferably a (Z)-octadec-9-enyl group), an octadecadienyl group (preferably a (9Z,12Z)-octadeca-9,12-dienyl group), and the like.

The alkynyl group may be linear or branched, or may be chainlike or cyclic. Specifically, examples of alkynyl group include a propargyl group, a butynyl group, a pentynyl group, a hexynyl group, a heptynyl group, an octynyl group, a nonynyl group, a decynyl group, an undecynyl group, a dodecynyl group, a tetradecynyl group, a pentadecynyl group, a hexadecynyl group, a heptadecynyl group, an octadecynyl group, and the like.

All of the above alkenyl groups preferably have one double bond or two double bonds. All of the above alkynyl groups preferably have one triple bond or two triple bonds.

The hydrocarbon group having 1 to 12 carbon atoms in -(hydrocarbon group having 1 to 12 carbon atoms)-$R^{67}$ is preferably an alkylene group having 1 to 12 carbon atoms or an alkenylene group having 2 to 12 carbon atoms. The alkylene group having 1 to 12 carbon atoms and the alkenylene group having 2 to 12 carbon atoms may be linear or branched, or may be chainlike or cyclic.

Specifically, examples thereof include a methylene group, an ethylene group, a trimethylene group, a tetramethylene group, a pentamethylene group, a hexamethylene group, a heptamethylene group, an octamethylene group, a nonamethylene group, a decamethylene group, an undecamethylene group, and the like.

The aryl group preferably has 6 to 20 carbon atoms, more preferably has 6 to 18 carbon atoms, and even more preferably 6 to 10 carbon atoms. Specifically, examples of the aryl group include a phenyl group, a naphthyl group, an anthracenyl group, a phenanthrenyl group, and the like.

$R^1$ and $R^2$ each independently preferably represent a hydrocarbon group having 1 to 12 carbon atoms, more preferably represent a hydrocarbon group having 1 to 6 carbon atoms, and even more preferably represent a hydrocarbon group having 1 to 3 carbon atoms.

$R^3$ preferably represents a hydrocarbon group having 2 to 6 carbon atoms and more preferably represents a hydrocarbon group having 2 to 4 carbon atoms.

The hydrocarbon groups represented by $R^1$, $R^2$, and $R^3$ may be preferably substituted with -OH.

$L^1$ and $L^3$ each independently preferably represent -C(O)O- or -OC(O)-.

$L^2$ preferably represents -OC(O)O-, -C(O)O-, or -OC(O)-.

$R^8$ preferably represents a hydrocarbon group having 1 to 10 carbon atoms and more preferably represents a hydrocarbon group having 1 to 8 carbon atoms.

$R^9$ preferably represents a hydrocarbon group having 1 to 20 carbon atoms and more preferably represents a hydrocarbon group having 1 to 16 carbon atoms.

$R^{11}$ preferably represents a hydrocarbon group having 1 to 16 carbon atoms and more preferably represents a hydrocarbon group having 1 to 9 carbon atoms.

$R^{12}$ preferably represents a hydrocarbon group having 1 to 20 carbon atoms and more preferably represents a hydrocarbon group having 1 to 16 carbon atoms.

The hydrocarbon groups represented by $R^9$ and $R^{12}$ may be preferably substituted with an aryl group or -S-$R^{58}$. Here, $R^{58}$ preferably represents a hydrocarbon group having 1 to 8 carbon atoms.

The hydrocarbon group represented by $R^{11}$ may be preferably substituted with -C(O)O-$R^{55}$ or -OC(O)-$R^{56}$, where $R^{55}$ and $R^{56}$ each independently represent a hydrocarbon group having 1 to 16 carbon atoms.

The hydrocarbon groups represented by $R^{55}$ and $R^{56}$ may be preferably substituted with an aryl group having 6 to 20 carbon atoms or -S-$R^{58}$, and the definition of $R^{58}$ is as described above.

The compound represented by Formula (1) is preferably a compound represented by Formula (1-1) as a first example.

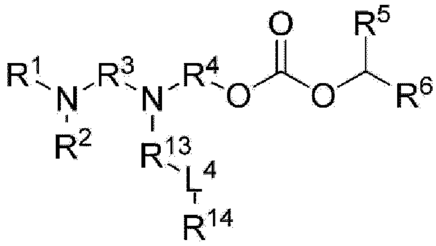

(1 − 1)

In the formula,

$R^1$ and $R^2$ each independently represent a hydrocarbon group having 1 to 18 carbon atoms, and $R^3$ represents a hydrocarbon group having 2 to 8 carbon atoms, where the hydrocarbon groups represented by $R^1$, $R^2$, and $R^3$ may be substituted with -OH, -COOH, -$NR^{51}R^{52}$, -OC(O)O-$R^{53}$, -C(O)O-$R^{54}$, -OC(O)-$R^{55}$, or -O-$R^{56}$,
$R^4$ represents a hydrocarbon group having 1 to 8 carbon atoms,
$R^5$ and $R^6$ each independently represent a hydrocarbon group having 1 to 8 carbon atoms or -$R^8$-$L^1$-$R^9$, excluding a case that both $R^5$ and $R^6$ are hydrocarbon groups having 1 to 8 carbon atoms,
$L^1$ represents -OC(O)O-, -C(O)O-, -OC(O)-, or -O-,
$R^8$ represents a hydrocarbon group having 1 to 12 carbon atoms,
$R^9$ represents a hydrocarbon group having 1 to 24 carbon atoms, where the hydrocarbon group represented by $R^9$ may be substituted with an aryl group, -OC(O)O-$R^{53}$, -C(O)O-$R^{54}$, -OC(O)-$R^{55}$, or -S-$R^{58}$,
$R^{51}$ and $R^{52}$ each independently represent a hydrocarbon group having 1 to 8 carbon atoms,
$R^{53}$, $R^{54}$, $R^{55}$, and $R^{56}$ each independently represent a hydrocarbon group having 1 to 24 carbon atoms,
the hydrocarbon groups represented by $R^{53}$, $R^{54}$, $R^{55}$, and $R^{56}$ may be substituted with an aryl group having 6 to 20 carbon atoms or -S-$R^{58}$,
the above-described aryl group having 6 to 20 carbon atoms may be substituted with -OH, -COOH, -$NR^{51}R^{52}$, -OC(O)O-$R^{53}$, -C(O)O-$R^{54}$, -OC(O)-$R^{55}$, -O-$R^{56}$, or -(hydrocarbon group having 1 to 12 carbon atoms)-$R^{57}$,
$R^{58}$ represents a hydrocarbon group having 1 to 12 carbon atoms, and
$R^{57}$ represents -OH, -COOH, -$NR^{51}R^{52}$, -OC(O)O-R $^{51}$, -C(O)O-$R^{54}$, -OC(O)-$R^{55}$, or -O-$R^{56}$.
$R^{13}$ represents a hydrocarbon group having 1 to 8 carbon atoms,
$R^{14}$ represents -$R^{15}$-$L^5$-$R^{16}$, where $R^{15}$ represents a hydrocarbon group having 1 to 24 carbon atoms, $L^5$ represents -OC(O)O-, -C(O)O-, -OC(O)-, or -O-, and $R^{16}$ represents a hydrocarbon group having 1 to 24 carbon atoms,
the hydrocarbon group having 1 to 24 carbon atoms represented by $R^{15}$ may be substituted with -OC(O)O-$R^{53}$, -C(O)O-$R^{54}$, or -OC(O)-$R^{55}$, where definitions of $R^{53}$, $R^{54}$, and $R^{55}$ are as described above, and
the hydrocarbon group having 1 to 24 carbon atoms represented by $R^{16}$ may be substituted with an aryl group having 6 to 20 carbon atoms, -OC(O)O-$R^{53}$, -C(O)O-$R^{54}$, -OC(O)-$R^{55}$ or -S-$R^{58}$, where the definitions of $R^{53}$, $R^{54}$, $R^{55}$, and $R^{58}$ are as described above.

In Formula (1-1), $R^1$ and $R^2$ each independently preferably represent a hydrocarbon group having 1 to 12 carbon atoms, more preferably represent a hydrocarbon group having 1 to 6 carbon atoms, and even more preferably represent a hydrocarbon group having 1 to 3 carbon atoms.
$R^3$ preferably represents a hydrocarbon group having 2 to 6 carbon atoms and more preferably represents a hydrocarbon group having 2 to 4 carbon atoms.
The hydrocarbon groups represented by $R^1$, $R^2$, and $R^3$ may be preferably substituted with -OH.
$L^1$ preferably represents -C(O)O- or -OC(O)-.
$R^8$ preferably represents a hydrocarbon group having 1 to 10 carbon atoms and more preferably represents a hydrocarbon group having 1 to 8 carbon atoms.
$R^9$ preferably represents a hydrocarbon group having 1 to 18 carbon atoms, and the hydrocarbon group represented by $R^9$ may be substituted with an aryl group having 6 to 20 carbon atoms or -S-$R^{58}$.
$R^{14}$ preferably represents -$R^{15}$-$L^5$-$R^{16}$, where $R^{15}$ represents a hydrocarbon group having 1 to 18 carbon atoms, $L^5$ represents -OC(O)O-, and $R^{16}$ represents a hydrocarbon group having 1 to 18 carbon atoms.
The hydrocarbon group having 1 to 18 carbon atoms represented by $R^{15}$ may be preferably substituted with -C(O)O-$R^{55}$ or -OC(O)-$R^{56}$. $R^{55}$ and $R^{56}$ each independently represent a hydrocarbon group having 1 to 16 carbon atoms, and the hydrocarbon groups represented by $R^{55}$ and $R^{56}$ may be substituted with an aryl group having 6 to 20 carbon atoms or -S-$R^{58}$, where the definition of $R^{58}$ is as described above.
The hydrocarbon group having 1 to 18 carbon atoms represented by $R^{16}$ may be preferably substituted with an aryl group or -S-$R^{58}$, where the definition of $R^{58}$ is as described above.
The compound represented by Formula (1) is preferably a compound represented by Formula (1-2) as a second

example.

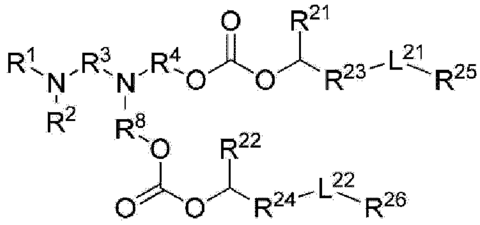

(1-2)

In the formula,

$R^1$ and $R^2$ each independently represent a hydrocarbon group having 1 to 18 carbon atoms, and $R^3$ represents a hydrocarbon group having 2 to 8 carbon atoms, where the hydrocarbon groups represented by $R^1$, $R^2$, and $R^3$ may be substituted with -OH, -COOH, -$NR^{51}R^{52,}$ -OC(O)O-$R^{53}$, -C(O)O-$R^{54}$, -OC(O)-$R^{55}$, or -O-$R^{56}$,
$R^4$ and $R^8$ each independently represent a hydrocarbon group having 1 to 8 carbon atoms,
$R^{21}$ and $R^{22}$ each independently represent a hydrocarbon group having 1 to 18 carbon atoms,
$R^{23}$ and $R^{24}$ each independently represent a hydrocarbon group having 1 to 12 carbon atoms,
$R^{25}$ and $R^{26}$ each independently represent a hydrocarbon group having 1 to 24 carbon atoms,
$L^{21}$ and $L^{22}$ each independently represent -OC(O)O-, -C(O)O-, -OC(O)-, or -O-,
the hydrocarbon groups represented by $R^{25}$ and $R^{26}$ may be substituted with an aryl group having 6 to 20 carbon atoms, -OC(O)O-$R^{53}$, -C(O)O-$R^{54}$, -OC(O)-$R^{55}$, or -S-$R^{58}$,
$R^{51}$ and $R^{52}$ each independently represent a hydrocarbon group having 1 to 8 carbon atoms,
$R^{53}$, $R^{54}$, $R^{55}$, and $R^{56}$ each independently represent a hydrocarbon group having 1 to 18 carbon atoms,
the above-described aryl group having 6 to 20 carbon atoms may be substituted with -OH, -COOH, -$NR^{51}R^{52}$, -OC(O)O-R $^{51}$, -C(O)O-$R^{54}$, -OC(O)-$R^{55}$, -O-$R^{56}$, or -(hydrocarbon group having 1 to 12 carbon atoms)-$R^{57}$, and
$R^{57}$ represents -OH, -COOH, -$NR^{51}R^{52}$, -OC(O)O-R $^{51}$, -C(O)O-$R^{54}$, -OC(O)-$R^{55}$, or -O-$R^{56}$.

$R^{58}$ represents a hydrocarbon group having 1 to 12 carbon atoms.

In Formula (1-2), $R^1$ and $R^2$ each independently preferably represent a hydrocarbon group having 1 to 12 carbon atoms, more preferably represent a hydrocarbon group having 1 to 6 carbon atoms, and even more preferably represent a hydrocarbon group having 1 to 3 carbon atoms. The hydrocarbon groups represented by $R^1$ and $R^2$ may be preferably substituted with -OH, but has more preferably a hydrocarbon group having no substituent.

$R^3$ preferably represents a hydrocarbon group having 2 to 6 carbon atoms and more preferably represents a hydrocarbon group having 2 to 4 carbon atoms.

$R^{21}$ and $R^{22}$ each independently preferably represent a hydrocarbon group having 1 to 12 carbon atoms, more preferably represent a hydrocarbon group having 1 to 8 carbon atoms, and even more preferably represent a hydrocarbon group having 1 to 6 carbon atoms.

$R^{23}$ and $R^{24}$ each independently preferably represent a hydrocarbon group having 1 to 10 carbon atoms and more preferably represent a hydrocarbon group having 1 to 8 carbon atoms.

$R^{25}$ and $R^{26}$ each independently preferably represent a hydrocarbon group having 1 to 20 carbon atoms, more preferably represent a hydrocarbon group having 1 to 16 carbon atoms, and even more preferably represent a hydrocarbon group having 1 to 12 carbon atoms.

$L^{21}$ and $L^{22}$ each independently preferably represent -C(O)O- or -OC(O)-.

The compound represented by Formula (1) is preferably a compound represented by Formula (1-3) as a third example.

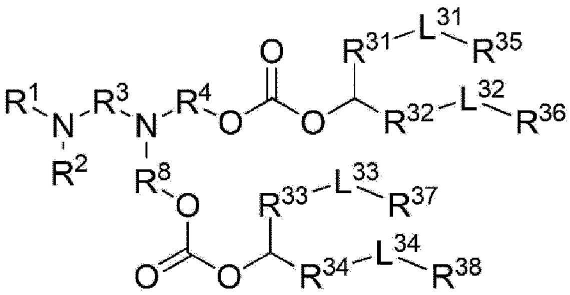

(1-3)

In the formula,

$R^1$ and $R^2$ each independently represent a hydrocarbon group having 1 to 18 carbon atoms, and $R^3$ represents a hydrocarbon group having 2 to 8 carbon atoms, where the hydrocarbon groups represented by $R^1$, $R^2$, and $R^3$ may be substituted with -OH, -COOH, $-NR^{51}R^{52}$, $-OC(O)O-R^{53}$, $-C(O)O-R^{54}$, $-OC(O)-R^{55}$, or $-O-R^{56}$,
$R^4$ and $R^8$ each independently represent a hydrocarbon group having 1 to 8 carbon atoms,
$R^{31}$, $R^{32}$, $R^{33}$, and $R^{34}$ each independently represent a hydrocarbon group having 1 to 12 carbon atoms,
$R^{35}$, $R^{36}$, $R^{37}$, and $R^{38}$ each independently represent a hydrocarbon group having 1 to 24 carbon atoms,
$L^{31}$, $L^{32}$, $L^{33}$, and $L^{34}$ each independently represent -OC(O)O-, -C(O)O-, -OC(O)-, or -O-,
the hydrocarbon groups represented by $R^{35}$, $R^{36}$, $R^{37}$, and $R^{38}$ may be substituted with an aryl group having 6 to 20 carbon atoms, $-OC(O)O-R^{53}$, $-C(O)O-R^{54}$, $-OC(O)-R^{55}$, or $-S-R^{58}$,
$R^{51}$ and $R^{52}$ each independently represent a hydrocarbon group having 1 to 8 carbon atoms,
$R^{53}$, $R^{54}$, $R^{55}$, and $R^{56}$ each independently represent a hydrocarbon group having 1 to 18 carbon atoms,
the above-described aryl group having 6 to 20 carbon atoms may be substituted with -OH, -COOH, $-NR^{51}R^{52}$, $-OC(O)O-R^{53}$, $-C(O)O-R^{54}$, $-OC(O)-R^{55}$, $-O-R^{56}$, or -(hydrocarbon group having 1 to 12 carbon atoms)-$R^{57}$, and
$R^{57}$ represents -OH, -COOH, $-NR^{51}R^{52}$, $-OC(O)O-R^{53}$, $-C(O)O-R^{54}$, $-OC(O)-R^{55}$, or $-O-R^{56}$.

$R^{58}$ represents a hydrocarbon group having 1 to 12 carbon atoms.

In Formula (1-3), $R^1$ and $R^2$ each independently preferably represent a hydrocarbon group having 1 to 12 carbon atoms, more preferably represent a hydrocarbon group having 1 to 6 carbon atoms, and even more preferably represent a hydrocarbon group having 1 to 3 carbon atoms. The hydrocarbon groups represented by $R^1$ and $R^2$ may be preferably substituted with -OH, but has more preferably a hydrocarbon group having no substituent.

$R^3$ preferably represents a hydrocarbon group having 2 to 6 carbon atoms and more preferably represents a hydrocarbon group having 2 to 4 carbon atoms.

$R^{31}$, $R^{32}$, $R^{33}$, and $R^{34}$ each independently preferably represent a hydrocarbon group having 1 to 10 carbon atoms, more preferably represent a hydrocarbon group having 1 to 8 carbon atoms, and even more preferably represent a hydrocarbon group having 1 to 3 carbon atoms.

$R^{35}$, $R^{36}$, $R^{37}$, and $R^{38}$ each independently preferably represent a hydrocarbon group having 1 to 20 carbon atoms, more preferably represent a hydrocarbon group having 1 to 16 carbon atoms, and even more preferably represent a hydrocarbon group having 1 to 12 carbon atoms. The hydrocarbon groups represented by $R^{35}$, $R^{36}$, $R^{37}$, and $R^{38}$ may be preferably substituted with an aryl group having 6 to 20 carbon atoms or $-S-R^{58}$. More preferably, these may be substituted with $-S-R^{58}$.

$R^{35}$, $R^{36}$, $R^{37}$, and $R^{38}$ each independently particularly preferably represent a hydrocarbon group having 1 to 12 carbon atoms substituted with $-S-R^{58}$, or a hydrocarbon group having 1 to 12 carbon atoms.

$L^{31}$, $L^{32}$, $L^{33}$, and $L^{34}$ each independently preferably represent -C(O)O-, or -OC(O)-.

$R^{58}$ preferably represents a hydrocarbon group having 1 to 10 carbon atoms and more preferably represents a hydrocarbon group having 1 to 8 carbon atoms.

The compound according to the embodiment of the present invention may form a salt.

Examples of the salt in a basic group include salts with mineral acids such as hydrochloric acid, hydrobromic acid, nitric acid, and sulfuric acid; salts with organic carboxylic acids such as formic acid, acetic acid, citric acid, oxalic acid, fumaric acid, maleic acid, succinic acid, malic acid, tartaric acid, aspartic acid, trichloroacetic acid, and trifluoroacetic acid; and salts with sulfonic acids such as methanesulfonic acid, benzenesulfonic acid, p-toluenesulfonic acid, mesitylenesulfonic acid, and naphthalenesulfonic acid.

Examples of the salt in an acidic group include salts with alkali metals such as sodium and potassium; salts with alkaline earth metals such as calcium and magnesium; ammonium salts; salts with nitrogen-containing organic bases such as trimethylamine, triethylamine, tributylamine, pyridine, N,N-dimethylaniline, N-methylpiperidine, N-methylmorpholine, diethylamine, dicyclohexylamine, procaine, dibenzylamine, N-benzyl-β-phenethylamine, 1-ephenamine, and N,N'-dibenzylethylenediamine; and the like.

Among the above-described salts, for example, pharmacologically acceptable salts are preferable.

Specifically, as the compound according to the embodiment of the present invention, for example, the compounds described in Examples 1 to 44 which will be described later are preferable. However, the present invention is not limited thereto.

The compounds described in Examples 1 to 44 are called Compounds 1 to 44 respectively.

Among the above-described compounds, the compounds 3, 8, 10, 11, 13, 14, 15 16, 17, 19, 20, 21, 22, 23, 24, 27, 28, 29, 32, 33, 34, 37, 38, 39, 42, 43, and 44 are preferable.

<Manufacturing method>

The manufacturing method of the compound according to the embodiment of the present invention is described.

The compound according to the embodiment of the present invention can be manufactured using known methods in combination. For example, the compound can be manufactured by the following manufacturing method.

[Manufacturing method 1]

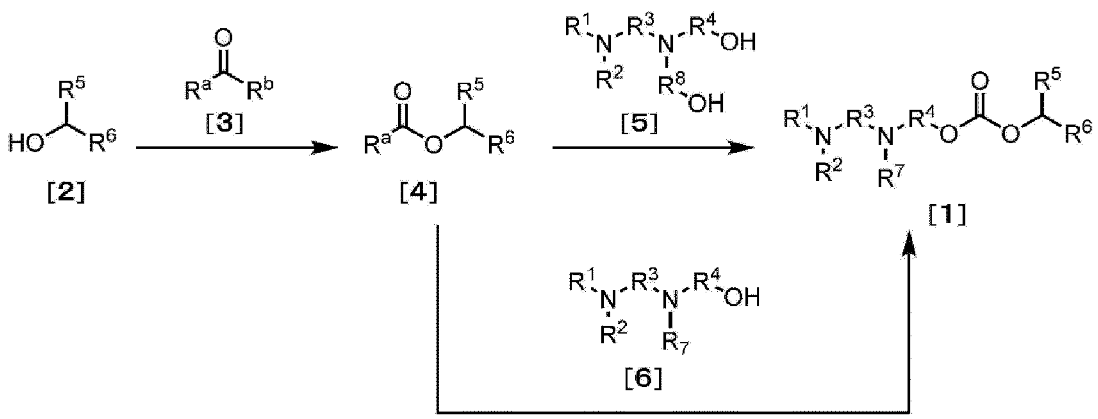

"In the formulae, $R^a$ and $R^b$ represent a leaving group; and $R^1$, $R^2$, $R^3$, $R^4$, $R^5$, $R^6$, $R^7$, and $R^8$ have the same meanings as those described above."

Examples of the leaving group include a chloro group, a fluoro group, a bromo group, a trichloromethoxy group, a 4-nitro-phenoxy group, a 2,4-dinitrophenoxy group, a 2,4,6-trichlorophenoxy group, a pentafluorophenoxy group, a 2,3,5,6-tetrafluorophenoxy group, an imidazolyl group, a triazolyl group, a 3,5-dioxo-4-methyl-1,2,4-oxadiazolidinyl group, an N-hydroxysuccinimidyl group, and the like.

(1-1)

As the compound represented by Formula [3], for example, 1,1'-carbonyldiimidazole, 4-nitrophenyl chloroformate, triphosgene, phosgene, and the like are known.

The compound represented by Formula [4] can be manufactured by reacting the compound represented by Formula [2] with the compound represented by Formula [3] in the presence or absence of a base.

The solvent used in this reaction is not particularly limited as long as the solvent does not affect the reaction. Examples of the solvent include halogenated hydrocarbons, ethers, esters, amides, nitriles, sulfoxides, and aromatic hydrocarbons. These solvents may be used by being mixed together.

Preferred examples of the solvent include ethers, and tetrahydrofuran is more preferable.

The used amount of the solvent is not particularly limited, but is only required to be 1 to 500 times (v/w) the amount of the compound represented by Formula [2].

Examples of the base used in this reaction include an inorganic base and an organic base. As the base, an organic base is preferable. Specifically, examples thereof include triethylamine, N,N-diisopropylethylamine, 4-methylmorpholine, pyridine, 1,8-diazabicyclo[5.4.0]-7-undecene, N,N-dimethylaminopyridine, and the like.

The used amount of the base is only required to be 1 to 50 times and preferably 1 to 10 times the molar amount of the compound represented by Formula [2].

The used amount of the compound represented by Formula [3] is not particularly limited, but is only required to be 0.3 to 10 times (v/w) the amount of the compound represented by Formula [2].

This reaction is required only to carry out at -30°C to 150°C, preferably at 0°C to 100°C for 5 minutes to 48 hours.

(1-2)

As the compound represented by Formula [5], for example, 2,2'-((2-diethylamino)ethyl)azanediyl)bis(ethan-1-ol), 2,2'-((2-dimethylamino)ethyl)azanediyl)bis(ethan-1-ol), 2,2'-((3-(diethylamino)propyl)azanediyl)bis(ethan-1-ol), and the like are known.

The compound represented by Formula [1] can be manufactured by reacting the compound represented by Formula [4] with the compound represented by Formula [5] in the presence of a base.

The solvent used in this reaction is not particularly limited as long as the solvent does not affect the reaction. Examples of the solvent include halogenated hydrocarbons, ethers, esters, amides, nitriles, sulfoxides, and aromatic hydrocarbons. These solvents may be used by being mixed together.

Preferred examples of the solvent include nitriles, and acetonitrile is more preferable.

The used amount of the solvent is not particularly limited, but is only required to be 1 to 500 times (v/w) the amount of the compound represented by Formula [4].

Examples of the base used in this reaction include an inorganic base and an organic base. Specifically, examples thereof include potassium carbonate, sodium carbonate, lithium carbonate, potassium phosphate, sodium phosphate, lithium phosphate, triethylamine, N,N-diisopropylethylamine, 4-methylmorpholine, pyridine, 1,8-diazabicyclo[5.4.0]-7-undecene, N,N-dimethylaminopyridine, and the like.

The used amount of the base is only required to be 1 to 50 times and preferably 1 to 10 times the molar amount of the compound represented by Formula [4].

The used amount of the compound represented by Formula [5] is not particularly limited, but is only required to be 0.1 to 10 times (v/w) the amount of the compound represented by Formula [4].

This reaction is required only to carry out at -30°C to 150°C, preferably at 0°C to 100°C for 5 minutes to 48 hours.

(1-3)

As the compound represented by Formula [5], for example, 2-((2-(diethylamino)ethyl)(ethyl)amino)ethan-1-ol, 2-((2-(diethylamino)ethyl)(isopropyl)amino)ethan-1-ol, and the like are known.

The compound represented by Formula [1] can be manufactured by reacting the compound represented by Formula [4] with the compound represented by Formula [6] in the presence of a base.

This reaction may be performed according to the manufacturing method (1-2).

[Manufacturing method 2]

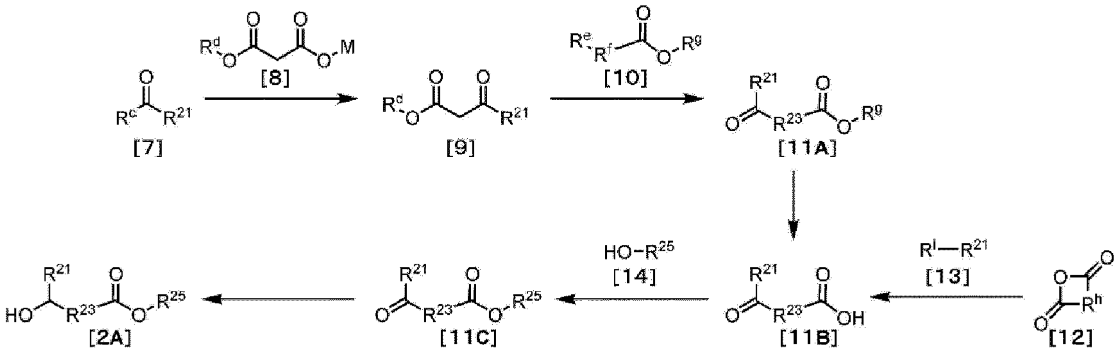

"In the formulae, $R^c$ and $R^e$ represent leaving groups; $R^d$ and $R^g$ represent hydrocarbon groups having 1 to 12 carbon atoms or hydrogen; $R^f$ represents a hydrocarbon group having 1 to 18 carbon atoms; $R^h$ represents a hydrocarbon group having 1 to 12 carbon atoms; M represents an alkali metal, an alkaline earth metal, or hydrogen; $R^i$ represents -MgCl, -MgBr, -MgI or -Li; and $R^{21}$, $R^{23}$, and $R^{25}$ have the same meanings as those described above."

Examples of the leaving group include a chloro group, a fluoro group, a bromo group, a trichloromethoxy group, a 4-nitro-phenoxy group, a 2,4-dinitrophenoxy group, a 2,4,6-trichlorophenoxy group, a pentafluorophenoxy group, a 2,3,5,6-tetrafluorophenoxy group, an imidazolyl group, a triazolyl group, a 3,5-dioxo-4-methyl-1,2,4-oxadiazolidinyl group, an N-hydroxysuccinimidyl group, and the like.

(2-1)

As the compound represented by Formula [8], for example, potassium ethyl malonate and the like are known.

The compound represented by Formula [9] can be manufactured by reacting the compound represented by Formula [7] with the compound represented by Formula [8] in the presence or absence of a base and in the presence or absence of anhydrous magnesium chloride.

The solvent used in this reaction is not particularly limited as long as the solvent does not affect the reaction. Examples of the solvent include halogenated hydrocarbons, ethers, esters, amides, nitriles, sulfoxides, and aromatic hydrocarbons. These solvents may be used by being mixed together.

Preferred examples of the solvent include nitriles, and acetonitrile is more preferable.

The used amount of the solvent is not particularly limited, but is only required to be 1 to 500 times (v/w) the amount of the compound represented by Formula [7].

Examples of the base used in this reaction include an inorganic base and an organic base. An organic base is preferable. Specifically, examples thereof include triethylamine, N,N-diisopropylethylamine, 4-methylmorpholine, pyridine, 1,8-diazabicyclo[5.4.0]-7-undecene, N,N-dimethylaminopyridine, and the like.

The used amount of the base is only required to be 1 to 50 times and preferably 1 to 10 times the molar amount of

the compound represented by Formula [7].

The used amount of the compound represented by Formula [8] is not particularly limited, but is only required to be 0.1 to 10 times (v/w) the amount of the compound represented by Formula [7].

This reaction is required only to carry out at -30°C to 150°C, preferably at 0°C to 100°C for 5 minutes to 48 hours.

(2-2)

As the compound represented by Formula [10], for example, ethyl 8-bromooctanoate and the like are known.

The compound represented by Formula [11A] can be manufactured by reacting the compound represented by Formula [9] with the compound represented by Formula [10] in the presence or absence of a base.

The solvent used in this reaction is not particularly limited as long as the solvent does not affect the reaction. Examples of the solvent include halogenated hydrocarbons, ethers, esters, amides, nitriles, sulfoxides, and aromatic hydrocarbons. These solvents may be used by being mixed together.

Preferred examples of the solvent include alcohols, and ethanol and methanol are more preferable.

The used amount of the solvent is not particularly limited, but is only required to be 1 to 500 times (v/w) the amount of the compound represented by Formula [9].

Examples of the base used in this reaction include an inorganic base and an organic base. Specifically, examples thereof include potassium hydroxide, sodium hydroxide, lithium hydroxide, potassium carbonate, sodium carbonate, lithium carbonate, potassium phosphate, sodium phosphate, lithium phosphate, triethylamine, N,N-diisopropylethylamine, 4-methylmorpholine, pyridine, 1,8-diazabicyclo[5.4.0]-7-undecene, N,N-dimethylaminopyridine, and the like.

The used amount of the base is only required to be 0.1 to 50 times and preferably 1 to 10 times the molar amount of the compound represented by Formula [9].

The used amount of the compound represented by Formula [8] is not particularly limited, but is only required to be 0.1 to 10 times (v/w) the amount of the compound represented by Formula [9].

This reaction is required only to carry out at -30°C to 150°C, preferably at 0°C to 100°C for 5 minutes to 48 hours.

(2-3)

The compound represented by Formula [11B] can be manufactured by hydrolysis reaction of the compound represented by Formula [11A] in the presence of a base or an acid.

The solvent used in this reaction is not particularly limited as long as it does not affect the reaction. Examples thereof include halogenated hydrocarbons, ethers, esters, amides, nitriles, sulfoxides, aromatic hydrocarbons, and water. These solvents may be used by being mixed together.

The used amount of the solvent is not particularly limited, but is only required to be 1 to 500 times (v/w) the amount of the compound represented by Formula [11A].

Examples of the base used in this reaction include an inorganic base and an organic base. An inorganic base is preferable. Specifically, examples thereof include potassium hydroxide, sodium hydroxide, lithium hydroxide, potassium carbonate, sodium carbonate, lithium carbonate, potassium phosphate, sodium phosphate, lithium phosphate, and the like.

Examples of the acid used in this reaction include an inorganic acid and an organic acid. An inorganic acid is preferable. Specifically, examples thereof include hydrochloric acid, hydrobromic acid, iodic acid, sulfuric acid, phosphoric acid, and the like.

The used amount of the base is only required to be 0.1 to 50 times and preferably 1 to 10 times the molar amount of the compound represented by Formula [11A].

The used amount of the acid is only required to be 0.01 to 50 times and preferably 1 to 10 times the molar amount of the compound represented by Formula [11A].

This reaction is required only to carry out at -30°C to 150°C, preferably at 0°C to 100°C for 5 minutes to 48 hours.

(2-4)

As the compound represented by Formula [13], for example, hexylmagnesium bromide and the like are known.

The compound represented by Formula [11B] can be manufactured by reacting the compound represented by Formula [12] with the compound represented by Formula [13].

The solvent used in this reaction is not particularly limited as long as the solvent does not affect the reaction. Examples of the solvent include halogenated hydrocarbons, ethers, amides, and aromatic hydrocarbons. These solvents may be used by being mixed together.

Preferred examples of the solvent include ethers, and tetrahydrofuran is more preferable.

The used amount of the solvent is not particularly limited, but is only required to be 1 to 500 times (v/w) the amount of the compound represented by Formula [12].

The used amount of the compound represented by Formula [13] is not particularly limited, but is only required to be 0.8 to 10 times (v/w) the amount of the compound represented by Formula [12].

This reaction is required only to carry out at -30°C to 150°C, preferably at 0°C to 100°C for 5 minutes to 48 hours.

(2-5)

As the compound represented by Formula [14], for example, 2-butyl-1-octanol, 2-pentyl-1-heptanol, and the like are

known.

The compound represented by Formula [11C] can be manufactured by reacting the compound represented by Formula [11B] with the compound represented by Formula [14] in the presence or absence of an acid, in the presence or absence of a condensing agent or an acid halide, and in the presence or absence of a base.

The solvent used in this reaction is not particularly limited as long as the solvent does not affect the reaction. Examples of the solvent include halogenated hydrocarbons, ethers, esters, amides, nitriles, sulfoxides, and aromatic hydrocarbons. These solvents may be used by being mixed together.

Preferred examples of the solvent include aromatic hydrocarbons and ethers, and toluene and tetrahydrofuran are more preferable.

The used amount of the solvent is not particularly limited, but is required to be 1 to 500 times (v/w) the amount of the compound represented by Formula [11B].

Examples of the acid used in this reaction include an inorganic acid and an organic acid. As the acid, sulfonic acids are preferable. Specifically, examples thereof include sulfuric acid, 4-toluenesulfonic acid, methanesulfonic acid, and the like.

Examples of the condensing agent used in this reaction include carbodiimides such as N,N'-dicyclohexylcarbodiimide and 1-ethyl-3-(3-dimethylaminopropyl)carbodiimide; carbonyls such as carbonyldiimidazole; acid azides such as diphenylphosphoryl azide; acid cyanides such as diethylphosphoryl cyanide; uroniums such as 2-ethoxy-1-ethoxycarbonyl-1,2-dihydroquinoline; O-benzotriazol-1-yl-1,1,3,3-tetramethyluronium=hexafluorophosphate; O-(7-azabenzotriazol-1-yl)-1,1,3,3-tetramethyluronium=hexafluorophosphate; and the like.

Examples of the acid halide used in this reaction include carboxylic acid halides such as acetyl chloride and trifluoroacetyl chloride; sulfonic acid halides such as methanesulfonyl chloride and tosyl chloride; chloroformic acid esters such as ethyl chloroformate and isobutyl chloroformate; and the like.

Examples of the base used in this reaction include an inorganic base and an organic base. As the base, an organic base is preferable. Specifically, examples thereof include triethylamine, N,N-diisopropylethylamine, 4-methylmorpholine, pyridine, 1,8-diazabicyclo[5.4.0]-7-undecene, N,N-dimethylaminopyridine, and the like.

The used amount of the base is only required to be 1 to 50 times and preferably 1 to 10 times the molar amount of the compound represented by Formula [11B].

The used amount of the compound represented by Formula [14] is not particularly limited, but is only required to be 0.8 to 10 times (v/w) the amount of the compound represented by Formula [11B].

This reaction is required only to carry out at -30°C to 150°C, preferably at 0°C to 100°C for 5 minutes to 48 hours.

(2-6)

The compound represented by Formula [2A] can be manufactured by reduction reaction of the compound represented by Formula [11C] in the presence of a reducing agent.

The solvent used in this reaction is not particularly limited as long as the solvent does not affect the reaction. Examples of the solvent include halogenated hydrocarbons, ethers, esters, amides, nitriles, alcohols, sulfoxides, aromatic hydrocarbons, and water. These solvents may be used by being mixed together.

The used amount of the solvent is not particularly limited, but is only required to be 1 to 500 times (v/w) the amount of the compound represented by Formula [11A].

Examples of the reducing agent used in this reaction include boron compounds such as sodium borohydride, and the like.

The used amount of the reducing agent is only required to be 0.1 to 50 times and preferably 1 to 10 times the molar amount of the compound represented by Formula [11A].

This reaction is required only to carry out at -30°C to 150°C, preferably at 0°C to 100°C for 5 minutes to 48 hours.

[Manufacturing method 3]

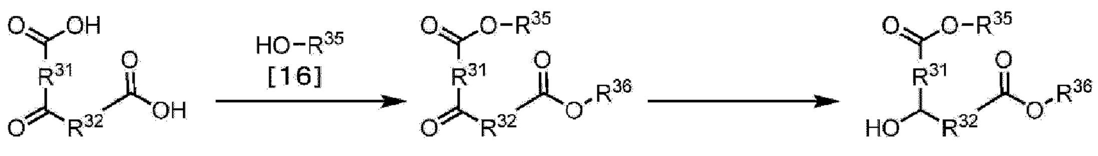


[15] [17] [2B]

"In the formulae, $R^{31}$, $R^{32}$, $R^{35}$, and $R^{36}$ have the same meanings as those described above."

(3-1)

As the compound represented by Formula [2A], for example, 1-heptanol, 2-(hexylthio)ethan-1-ol, 8-(methylthio)octan-1-ol, and the like are known.

The compound represented by Formula [17] can be manufactured by reacting the compound represented by Formula [15] with the compound represented by Formula [16] in the presence or absence of an acid, in the presence or absence of a condensing agent or an acid halide, and in the presence or absence of a base.

This reaction may be performed according to the manufacturing method (2-5).

(3-2)

The compound represented by Formula [2B] can be manufactured by reduction reaction of the compound represented by Formula [17] in the presence of a reducing agent.

This reaction may be performed according to the manufacturing method (2-6).

[Manufacturing method 4]

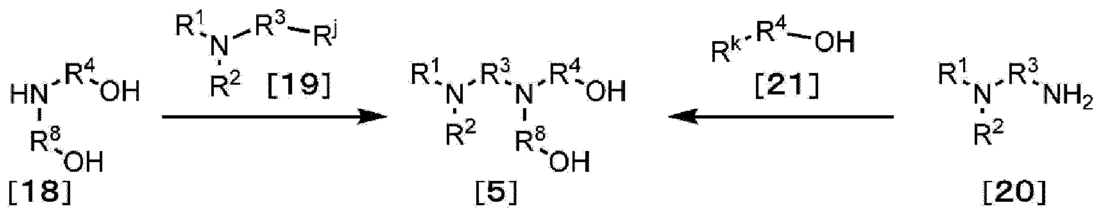

"In the formulae, $R^j$ and $R^k$ represent leaving groups; and $R^1$, $R^2$, $R^3$, $R^4$, and $R^8$ have the same meanings as described above."

Examples of the leaving group include a chloro group, a fluoro group, a bromo group, a trichloromethoxy group, a 4-nitro-phenoxy group, a 2,4-dinitrophenoxy group, a 2,4,6-trichlorophenoxy group, a pentafluorophenoxy group, a 2,3,5,6-tetrafluorophenoxy group, an imidazolyl group, a triazolyl group, a 3,5-dioxo-4-methyl-1,2,4-oxadiazolidinyl group, an N-hydroxysuccinimidyl group, and the like.

(4-1)

As the compound represented by Formula [19], for example, 2-chloro-N,N-diethylethan-1-amine, 3-chloro-N,N-diethylpropan-1-amine, 2-bromo-N,N-diethylethan-1-amine, and the like are known.

The compound represented by Formula [5] can be manufactured by reacting the compound represented by Formula [18] with the compound represented by Formula [19] in the presence or absence of a base.

The solvent used in this reaction is not particularly limited as long as the solvent does not affect the reaction. Examples of the solvent include alcohols, halogenated hydrocarbons, ethers, esters, amides, nitriles, sulfoxides, aromatic hydrocarbons, and water. These solvents may be used by being mixed together.

The used amount of the solvent is not particularly limited, but is only required to be 1 to 500 times (v/w) the amount of the compound represented by Formula [18].

Examples of the base used in this reaction include an inorganic base and an organic base. The used amount of the base is only required to be 1 to 10,000 times and preferably 1 to 5,000 times the molar amount of the compound represented by Formula [18].

The used amount of the compound represented by Formula [19] is not particularly limited, but is only required to be 1 to 10 times (v/w) the amount of the compound represented by Formula [18].

This reaction is required only to carry out at -30°C to 150°C, preferably at 0°C to 100°C for 5 minutes to 48 hours.

(4-2)

As the compound represented by Formula [20], for example, 2-bromo-1-ethanol, 3-bromo-1-propanol, and the like are known.

The compound represented by Formula [5] can be manufactured by reacting the compound represented by Formula [20] with the compound represented by Formula [21] in the presence or absence of a base.

The solvent used in this reaction is not particularly limited as long as the solvent does not affect the reaction. Examples of the solvent include alcohols, halogenated hydrocarbons, ethers, esters, amides, nitriles, sulfoxides, aromatic hydrocarbons, and water. These solvents may be used by being mixed together.

The used amount of the solvent is not particularly limited, but is only required to be 1 to 500 times (v/w) the amount of the compound represented by Formula [20].

Examples of the base used in this reaction include an inorganic base and an organic base. The used amount of the base is only required to be 1 to 10,000 times and preferably 1 to 5,000 times the molar amount of the compound represented by Formula [20].

The used amount of the compound represented by Formula [21] is not particularly limited, but is only required to

be 1 to 10 times (v/w) the amount of the compound represented by Formula [20].

This reaction is required only to carry out at -30°C to 150°C, preferably at 0°C to 100°C for 5 minutes to 48 hours.

In a case where the compounds used in the above-described manufacturing methods have isomers (for example, an optical isomer, a geometric isomer, a tautomer, and the like), these isomers can also be used.

In addition, in a case where the compounds are in the form of solvates, hydrates, and crystals of various shapes, these solvates, hydrates, and crystals of various shapes can also be used.

In a case where the compounds used in the above-described manufacturing methods have, for example, an amino group, a hydroxyl group, and a carboxyl group, these groups can be protected with ordinary protecting groups in advance, and these protecting groups can be eliminated by known methods after the reaction.

The compounds obtained by the above-described manufacturing methods can be induced into other compounds by being subjected to known reaction such as condensation, addition, oxidation, reduction, transition, substitution, halogenation, dehydration, or hydrolysis, or by being subjected to these reactions that are appropriately combined.

<Lipid particles>

In the present invention, lipid particles containing the compound or a salt thereof according to an embodiment of the present invention can be prepared. In preparing the lipid particles, in addition to the compound according to the embodiment of the present invention, it is possible to use at least one kind of lipid selected from the group consisting of a sterol and a lipid having a nonionic hydrophilic polymer chain. The lipid particles can further contain a neutral lipid. The lipid particles can further contain a nucleic acid.

In the lipid particles according to the embodiment of the present invention, the amount of the compound according to the embodiment of the present invention mixed in, with respect to the total mass of lipids, is preferably 20 mol% to 80 mol%, more preferably 35 mol% to 70 mol%, and even more preferably 40 mol% to 65 mol%.

<Sterol>

The lipid particles according to the embodiment of the present invention preferably contain a sterol. In a case where the lipid particles according to the embodiment of the present invention contain a sterol, the fluidity of the membrane can be reduced, and hence the lipid particle can be effectively stabilized.

The sterol is not particularly limited, and examples thereof include cholesterol, phytosterol (sitosterol, stigmasterol, fucosterol, spinasterol, brassicasterol, and the like) ergosterol, cholestanone, cholestenone, coprostanol, cholesteryl-2'-hydroxyethyl ether, cholesteryl-4'-hydroxybutyl ether, and the like. Among these, cholesterol is preferable.

In the lipid particles according to the embodiment of the present invention, the amount of the sterol mixed in, with respect to the total mass of lipids, is preferably 10 mol% to 60 mol%, more preferably 20 mol% to 55 mol%, and even more preferably 25 mol% to 50 mol%.

<Neutral lipid>

The lipid particles according to the embodiment of the present invention may contain a neutral lipid. The neutral lipid is not particularly limited, and examples thereof include phosphatidylcholine, phosphatidylethanolamine, sphingomyelin, ceramide, and the like. Among these, phosphatidylcholine is preferable. The neutral lipid may be a single neutral lipid or a combination of a plurality of different neutral lipids.

The phosphatidylcholine is not particularly limited, and examples thereof include soybean lecithin (SPC), hydrogenated soybean lecithin (HSPC), egg yolk lecithin (EPC), hydrogenated egg yolk lecithin (HEPC), dimyristoylphosphatidylcholine (DMPC), dipalmitoylphosphatidylcholine (DPPC), distearoylphosphatidylcholine (DSPC), 1-palmitoyl-2-oleoylphosphatidylcholine (POPC), dioleoylphosphatidylcholine (DOPC), and the like.

The phosphatidylethanolamine is not particularly limited, and examples thereof include dimyristoylphosphatidylethanolamine (DMPE), dipalmitoylphosphatidylethanolamine (DPPE), distearoylphosphatidylethanolamine (DSPE), dioleoylphosphatidylethanolamine (DOPE), dilinoleoylphosphatidylethanolamine (DLoPE), diphytanoylphosphatidylethanolamine (D(Phy)PE), 1-palmitoyl-2-oleoylphosphatidylethanolamine (POPE), ditetradecylphosphatidylethanolamine, dihexadecylphosphatidylethanolamine, dioctadecylphosphatidylethanolamine, diphytanylphosphatidylethanolamine, and the like.

The sphingomyelin is not particularly limited, and examples thereof include egg yolk-derived sphingomyelin, milk-derived sphingomyelin, and the like.

The ceramide is not particularly limited, and examples thereof include egg yolk-derived ceramide, milk-derived ceramide, and the like.

In the lipid particles according to the embodiment of the present invention, the amount of the neutral lipid mixed in is preferably 0 mol% or more and 55 mol% or less with respect to the total amount of the constituent lipid components.

<Lipid having nonionic hydrophilic polymer chain>

The lipid particles according to the embodiment of the present invention may contain a lipid having a nonionic hydrophilic polymer chain in an oil phase. In the present invention, in a case where the lipid particles contain the lipid having a nonionic hydrophilic polymer chain in an oil phase, the dispersion of the lipid particles can be effectively stabilized.

The nonionic hydrophilic polymer is not particularly limited, and examples thereof include a nonionic vinyl-based polymer, a nonionic polyamino acid, a nonionic polyester, a nonionic polyether, a nonionic natural polymer, a nonionic modified natural polymer, and a block polymer or a graft copolymer having two or more kinds of these polymers as constitutional units.

Among these nonionic hydrophilic polymers, a nonionic polyether, a nonionic polyester, a nonionic polyamino acid, or a nonionic synthetic polypeptide is preferable, a nonionic polyether or a nonionic polyester is more preferable, a nonionic polyether or a nonionic monoalkoxy polyether is even more preferable, and polyethylene glycol (hereinafter, polyethylene glycol will be also called PEG) is particularly preferable.

The lipid having a nonionic hydrophilic polymer chain is not particularly limited, and examples thereof include PEG-modified phosphoethanolamine, a diacylglycerol PEG derivative, a monoacylglycerol PEG derivative, a dialkylglycerol PEG derivative, a cholesterol PEG derivative, a ceramide PEG derivative, and the like. Among these, a monoacylglycerol PEG derivative or a diacylglycerol PEG derivative is preferable.

The weight-average molecular weight of the PEG chain of the nonionic hydrophilic polymer derivative is preferably 500 to 5,000 and more preferably 750 to 3,000.

The nonionic hydrophilic polymer chain may be branched or may have a substituent such as a hydroxymethyl group.

In the lipid particles according to the embodiment of the present invention, the amount of the lipid having a nonionic hydrophilic polymer chain mixed in with respect to the total amount of lipids is preferably 0.25 mol% to 12 mol%, more preferably 0.5 mol% to 6 mol%, and even more preferably 1 mol% to 3 mol%.

<Nucleic acid>

The lipid particles according to the embodiment of the present invention may contain a nucleic acid. Examples of the nucleic acid include a plasmid, single-stranded DNA, double-stranded DNA, small interfering RNA (siRNA), micro RNA (miRNA), mRNA, an antisense oligonucleotide (also referred to as ASO), ribozyme, an aptamer, saRNA, sgRNA, and the like. The lipid particles may contain any of these. In addition, the lipid particles may contain a modified nucleic acid. As the nucleic acid, RNA is particularly preferable, and RNA having a number of bases of 5 to 20,000 is preferable.

In the lipid particles according to the embodiment of the present invention, the mass ratio of the lipid to the nucleic acid is preferably 2 to 1,000, more preferably 3 to 500, even more preferably 5 to 200, and particularly preferably 5 to 100.

<Manufacturing method of lipid particles>

The manufacturing method of the lipid particles according to the embodiment of the present invention is described.

The manufacturing method of the lipid particles is not limited. For example, the lipid particles can be manufactured by a method in which all of the constituent components of the lipid particles or some of oil-soluble components of the lipid particles are dissolved in an organic solvent or the like such that an oil phase is formed, water-soluble components of the lipid particles are dissolved in water such that a water phase is formed, and the oil phase and the water phase are mixed together. A micromixer may be used for mixing, or an emulsification using an emulsifying machine such as a homogenizer, an ultrasonic emulsifying machine, a high-pressure injection emulsifying machine, or the like may be performed.

Alternatively, the lipid composition can also be manufactured by a method in which a lipid-containing solution is subjected to evaporation to dryness using an evaporator under reduced pressure or subjected to spray drying using a spray drier, so that a dried mixture containing a lipid is prepared, and then the mixture is added to an aqueous solvent and further emulsified using the aforementioned emulsifying machine or the like.

Examples of the manufacturing method of the lipid particles containing a nucleic acid include a method including

Step (a) of dissolving the constituent components of the lipid particles containing the compound according to an embodiment of the present invention in an organic solvent so as to obtain an oil phase,
Step (b) of mixing the oil phase obtained in Step (a) with a water phase containing a nucleic acid,
Step (c) of diluting the mixed solution containing the oil phase and the water phase obtained in Step (b) so as to obtain a dispersion liquid of nucleic acid lipid particles, and
Step (d) of removing the organic solvent from the dispersion liquid of the nucleic acid lipid particles obtained in Step (c).

In Step (a), the constituent components of the lipid particles containing the compound according to the embodiment of the present invention are dissolved in an organic solvent (an alcohol such as ethanol, an ester, or the like). The total lipid concentration is not particularly limited, but is generally 1 mmol/L to 100 mmol/L, preferably 5 mmol/L to 50 mmol/L, and more preferably 10 mmol/L to 30 mmol/L.

In Step (b), the water phase can be obtained by dissolving a nucleic acid (for example, siRNA, mRNA, an antisense nucleic acid, or the like) in water or a buffer solution. If necessary, a component such as an antioxidant can be added. The mixing ratio (volume ratio) of water phase:oil phase is preferably 5:1 to 1:1 and more preferably 4:1 to 2:1.

In Step (c), the mixed solution can be diluted with water or a buffer solution (for example, phosphate buffered saline (PBS)).

In Step (d), the method of removing the organic solvent from the dispersion liquid of nucleic acid lipid particles is not particularly limited, and a general method can be used. For example, the organic solvent can be removed by dialyzing the dispersion liquid with the phosphate buffered saline, a sucrose-Tris buffer solution, or the like.

If necessary, the lipid particles can be subjected to sizing. The method of sizing is not particularly limited, and the particle size can be reduced by using an extruder or the like.

In addition, the dispersion liquid containing the lipid particles according to the embodiment of the present invention can be frozen or freeze-dried by a general method.

<Lipid particles>

In the present invention, lipid particles mean particles composed of a lipid, and include a composition having any structure selected from a lipid aggregate composed of aggregated lipids, a micelle, and a liposome. The structure of the lipid particles is not limited to these as long as the lipid particles are a composition containing a lipid. The liposome has a lipid bilayer structure and has a water phase in the inside, and includes a liposome which has a single bilayer membrane, and a multilayer liposome which has multiple layers stacked together. The present invention may include any of these liposomes.

The form of the lipid particles can be checked by electron microscopy, structural analysis using X-rays, or the like. For example, by a method using Cryo transmission electron microscopy (CryoTEM method), it is possible to check, for example, whether or not a lipid particle is, such as a liposome, a bimolecular lipid membrane structure (lamella structure) and a structure having an inner water layer, and whether or not a lipid particle has a structure having an inner core with a high electron density and packed with constituent components including a lipid. The X-ray small angle scattering (SAXS) analysis also makes it possible to check whether or not a lipid particle has a bimolecular lipid membrane structure (lamella structure).

The particle size of the lipid particles according to the embodiment of the present invention is not particularly limited, and is preferably 10 to 1,000 nm, more preferably 30 to 500 nm, and even more preferably 50 to 250 nm. The particle size of the lipid particles can be measured by a general method (for example, a dynamic light scattering method, a laser diffraction method, or the like).

<Use of lipid particles>

As one example for utilizing the lipid particles in the present invention, a nucleic acid (for example, RNA or the like) can be introduced into a cell by introducing the lipid particles containing the nucleic acid into the cell. In addition, in a case where the lipid particles in the present invention contain a nucleic acid for a pharmaceutical use, the lipid particles can be administered to a living body as a nucleic acid drug.

In a case where the lipid particles according to the embodiment of the present invention are used as a nucleic acid drug, the lipid particles according to the embodiment of the present invention can be administered alone to a living body or administered to a living body by being mixed with a pharmaceutically acceptable dosing medium (for example, physiological saline, a phosphate buffer solution, or the like).

The concentration of the lipid particles in the mixture with a pharmaceutically acceptable carrier is not particularly limited, and can be set to 0.05% by mass to 90% by mass in general. In addition, other pharmaceutically acceptable additives, for example, a pH adjusting buffer and an osmotic pressure adjusting agent, may be added to the nucleic acid drug containing the lipid particles according to the embodiment of the present invention.

The route of administration for administering the nucleic acid drug containing the lipid particles according to the embodiment of the present invention is not particularly limited. The nucleic acid drug can be administered by any method. Examples of the administration method include oral administration and parenteral administration (intraarticular administration, intravenous administration, intraarterial administration, subcutaneous administration, intracutaneous administration, intravitreal administration, intraperitoneal administration, intramuscular administration, intravaginal administration, intravesical administration, intrathecal administration, pulmonary administration, rectal administration, colonic administration, buccal administration, nasal administration, intracisternal administration, inhalation, and the like). Parenteral administration is preferable. As the method of administration, intravenous injection, subcutaneous injection, intracutaneous injection, or intramuscular injection is preferable. The nucleic acid drug containing the lipid particles according to the embodiment of the present invention can also be administered by being directly injected into the affected area.

The dosage form of the lipid particles according to the embodiment of the present invention is not particularly limited. For oral administration, the lipid particles according to the embodiment of the present invention can be used in the form of tablets, troches, capsules, pills, suspension, syrup, and the like by being combined with an appropriate excipient. In addition, pharmaceutical preparation suitable for parenteral administration can appropriately contain an antioxidant, a buffer, a bacteriostat, and additives such as an isotonic sterile injection, a suspending agent, a solubilizer, a thickener, a stabilizer, or a preservative.

<Nucleic acid delivery carrier>

The lipid particles according to the embodiment of the present invention can retain a nucleic acid at a high encapsulation rate. Therefore, the lipid particles are extremely useful as a nucleic acid delivery carrier. According to the nucleic acid delivery carrier using the present invention, for example, by mixing the obtained lipid particles with a nucleic acid or the like and performing transfection in vitro or in vivo, the nucleic acid and the like can be introduced into cells. In addition, the nucleic acid delivery carrier using the present invention is also useful as a nucleic acid delivery carrier in nucleic acid drugs. That is, the lipid particles according to the embodiment of the present invention are useful as a composition for in vitro or in vivo (preferably in vivo) delivery of a nucleic acid.

Next, the present invention is described based on examples, but the present invention is not limited thereto.

Examples

Unless otherwise specified, in purification by column chromatography, the automatic purification device ISOLERA (Biotage AB, Inc.), the medium pressure preparative purification device Purif-espoir-2 (Shoko Science Co., Ltd.), or the medium pressure liquid chromatograph YFLC W-prep 2XY (Yamazen Corporation) was used.

Unless otherwise specified, as a carrier in silica gel column chromatography, Chromatorex Q-Pack SI 50 (FUJI SILYSIA CHEMICAL LTD.), or high flash column WO01, WO02, WO03, WO04, or WO05 (Yamazen Corporation) was used.

For NH silica gel, Chromatorex Q-Pack NH 60 (FUJI SILYSIA CHEMICAL LTD.) was used.

NMR spectra were measured using tetramethylsilane as an internal standard and using Bruker AV300 (manufactured by Bruker), Bruker AV400 (manufactured by Bruker), or AVNEO400 (manufactured by Bruker), and all $\delta$ values are indicated in terms of ppm.

MS spectra were measured using an ACQUITY SQD LC/MS System (manufactured by Waters Corporation).

clogP was calculated using ChemDraw Professional Version: 19.1.0.8 (manufactured by PerkinElmer, Inc.).

[Example 1]

(1)

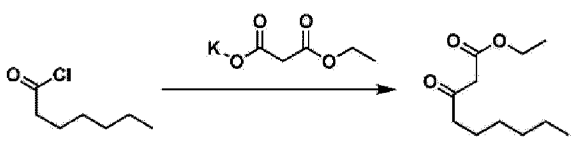

Triethylamine (75.0 mL) and anhydrous magnesium chloride (40.0 g) were added to a mixture of potassium monoethyl malonate (60.1 g) and acetonitrile (400 mL) under ice-cooling, and the mixture was stirred at room temperature for 2 hours. Heptanoyl chloride (25.0 g) was added dropwise to the reaction mixture under ice-cooling, and the mixture was stirred at room temperature for 2 hours. The solvent of the reaction mixture was distilled off under reduced pressure, toluene (200 mL) was added thereto, and the mixture was distilled off under reduced pressure, and then toluene (100 mL) was added thereto. A 15% aqueous hydrochloric acid solution (250 mL) was added dropwise to the obtained residue under ice-cooling, the organic layer was separated and washed with a 15% aqueous hydrochloric acid solution (76 mL) and then washed with water (75 mL), toluene (100 mL) was added thereto, and the mixture was distilled off under reduced pressure to obtain ethyl 3-oxononanoate (35.2 g).

$^1$H-NMR ($CDCl_3$) $\delta$: 4.20 (2H, q, J = 8.0 Hz), 3.43 (2H, s), 2.53 (2H, t, J = 8.0 Hz), 1.65-1.52 (2H, m), 1.26-1.21 (9H,

m), 0.88 (3H, t, 8.0 Hz).

(2)

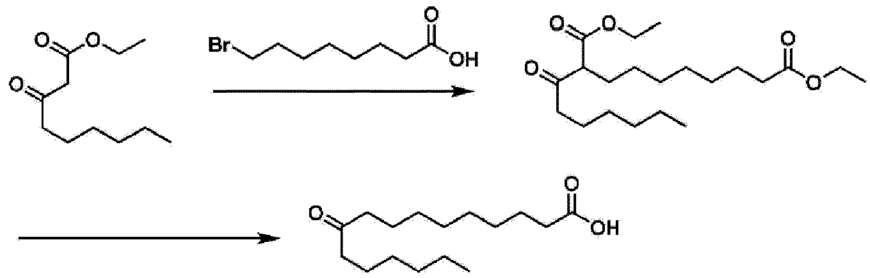

An ethanol solution (10 mL) of ethyl 3-oxononanoate (15.0 g) was added to a 20% sodium ethoxydo-ethanol solution (32 mL), then ethyl 8-bromooctanoate (18.9 g) was added dropwise thereto, and the mixture was stirred at 90°C for 4 hours. The reaction mixture was cooled to room temperature, then a 33% aqueous sodium hydroxide solution (22.5 mL) was added thereto, and the mixture was stirred at room temperature for 1 hour. A 15% aqueous hydrochloric acid solution (48 mL) was added to the reaction mixture, and the mixture was stirred at 60°C for 30 minutes. The reaction mixture was cooled to 40°C, then the organic layer was separated, and the solvent was distilled off under reduced pressure. Ethyl acetate and water were added to the obtained residue, the organic layer was separated, and the solvent was distilled off under reduced pressure. Hexane was added to the obtained residue, and the solid substance was collected by filtration, washed with hexane, and then dried under reduced pressure to obtain 10-oxohexadecanoic acid (9.1 g).

$^1$H-NMR ($CDCl_3$) δ: 2.45-2.28 (6H, m), 1.71-1.47 (6H, m), 1.40-1.20 (14H, m), 0.88 (3H, t, J = 8.0 Hz).

(3)

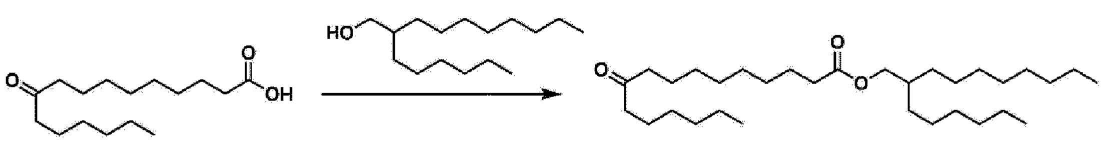

p-Toluenesulfonic acid (0.07 g) was added to a mixture of 10-oxohexadecanoic acid (2.0 g), 2-hexyl-1-decanol (1.8 g), and toluene (20 mL), and the mixture was stirred at 130°C for 3 hours. The reaction mixture was cooled to room temperature, then a 5% aqueous sodium hydrogen carbonate solution was added thereto, the organic layer was separated, washed with water, and dried over anhydrous magnesium sulfate, and the solvent was distilled off under reduced pressure to obtain 2-hexyldecyl 10-oxohexadecanoate (3.7 g).

$^1$H-NMR ($CDCl_3$) δ: 3.97 (2H, d, J = 5.6 Hz), 2.38 (4H, t, J = 7.6 Hz), 2.29 (2H, t, J = 7.6 Hz), 1.65-1.50 (7H, m), 1.35-1.20 (38H, m), 0.92-0.83 (9H, m).

(4)

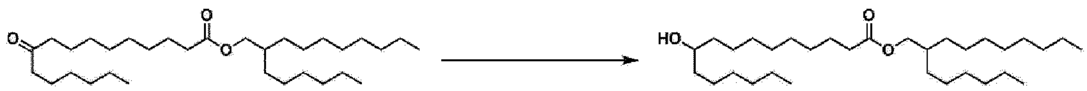

Sodium borohydride (0.42 g) was added to a mixture of 2-hexyldecyl 10-oxohexadecanoate (3.7 g) and toluene (18 mL), then methanol (3.7 mL) was added dropwise thereto under ice cooling, and the mixture was stirred at the same temperature for 3 hours. A 30% aqueous hydrochloric acid solution (18 mL) was added dropwise to the reaction mixture under ice-cooling, then the organic layer was separated, washed with water, dried over anhydrous magnesium sulfate, and distilled off under reduced pressure to obtain 2-hexyldecyl 10-hydroxyhexadecanoate (3.5 g).

$^1$H-NMR ($CDCl_3$) δ: 3.97 (2H, d, J = 6.0 Hz), 3.61-3.54 (1H, m), 2.30 (2H, t, J = 7.6 Hz), 1.65-1.56 (3H, m), 1.48-1.22 (46H, m), 0.92-0.83 (9H, m).

(5)

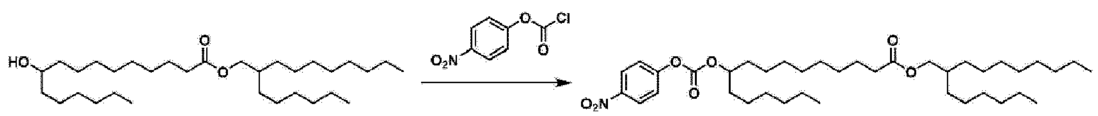

4-Nitrophenyl chloroformate (2.21 g) was added to a mixture of 2-hexyldecyl 10-hydroxyhexadecanoate (3.6 g), triethylamine (3.1 mL), and tetrahydrofuran (36 mL), and the mixture was stirred at room temperature for 4 hours. Water and ethyl acetate were added to the reaction mixture, the organic layer was separated, washed with water, and then dried over anhydrous sodium sulfate, and the solvent was distilled off under reduced pressure. The obtained residue was purified by silica gel column chromatography (ethyl acetate-hexane), thereby obtaining 2-hexyldecyl 10-(((4-nitrophenoxy)carbonyl)oxy)hexadecanoate (4.5 g) as a colorless oily substance.

$^1$H-NMR ($CDCl_3$) δ: 8.28 (2H, dd, J = 7.2 Hz, 2.1 Hz), 7.39 (2H, dd, J = 7.2 Hz, 2.1 Hz), 4.86-4.76 (1H, m), 3.97 (2H, d, J = 5.7 Hz), 2.30 (2H, t, J = 7.2 Hz), 1.74-1.20 (49H, m), 0.92-0.85 (9H, m).

(6)

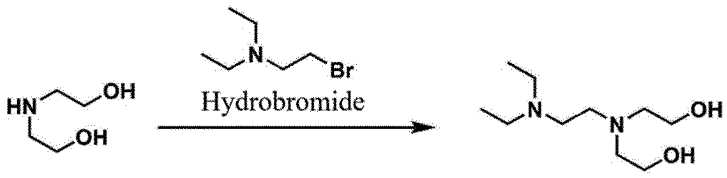


Potassium carbonate (7.9 g) was added to a mixture of 2,2'-azanediylbis(ethan-1-ol) (2.0 g), 2-bromo-N,N-diethylethan-1-amine hydrobromide (7.4 g), and ethanol (40 mL), and the mixture was stirred under reflux with heating for 8 hours. The reaction mixture was cooled to room temperature, the insoluble matters removed by filtration, and the solvent was distilled off under reduced pressure. The obtained residue was purified by silica gel column chromatography (ethyl acetate-hexane, NH silica gel), thereby obtaining 2,2'-((2-(diethylamino)ethyl)azanediyl)bis(ethan-1-ol) (2.3 g) as a light yellow oily substance.

$^1$H-NMR ($CDCl_3$) δ: 3.58 (4H, t, J = 5.4 Hz), 2.70 (4H, t, J = 5.4 Hz), 2.67-2.48 (8H, m), 1.04 (6H, t, J = 7.5 Hz).

MS m/z (M + H): 205.

(7)

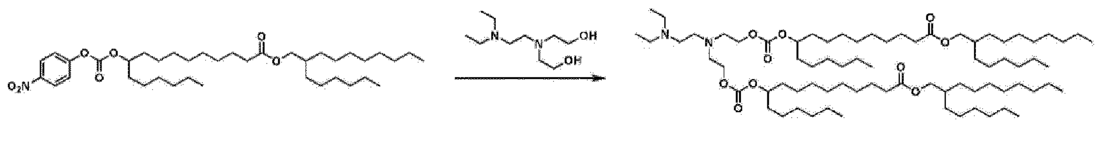

4-Dimethylaminopyridine (1.11 mg) was added to a mixture of 2-hexyldecyl 10-(((4-nitrophenoxy)carbonyl)oxy) hexadecanoate (2.00 g), 2,2'-((2-(diethylamino)ethyl)azanediyl)bis(ethan-1-ol) (1.85 g), triethylamine (1.27 mL), and tetrahydrofuran (20 mL), and the mixture was stirred at 80°C for 4 hours. The reaction mixture was cooled to room temperature, water and ethyl acetate were added thereto, the organic layer was separated, washed with a saturated aqueous sodium chloride solution, and then dried over anhydrous sodium sulfate, and the solvent was distilled off under reduced pressure. The obtained residue was purified by silica gel column chromatography (methanol-ethyl acetate) and silica gel column chromatography (ethyl acetate-hexane, NH silica gel), thereby obtaining bis(2-hexyldecyl) 16-(2-(diethylamino)ethyl)-10,22-dihexyl-12,20-dioxo-11,13,19,21-tetraoxa-16-azahentriacont anedioate (197 mg) as a colorless oily substance.

$^1$H-NMR ($CDCl_3$) δ: 4.71-4.59 (2H, m), 4.21-4.08 (4H, m), 3.97 (4H, d, J = 6.0 Hz), 2.84 (4H, t, J = 6.6 Hz), 2.71-2.44 (8H, m), 2.29 (4H, t, J = 7.8 Hz), 1.65-1.50 (14H, m), 1.35-1.20 (84H, m), 1.10-0.96 (6H, m), 0.92-0.84 (18H, m).

MS m/z (M + H): 1251.

clogP: 30.3764

[Example 2]

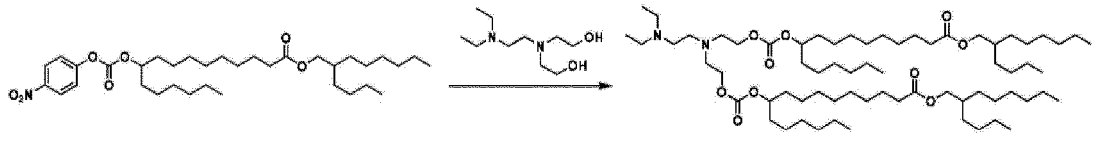

Bis(2-butyloctyl)

16-(2-(diethylamino)ethyl)-10,22-dihexyl-12,20-dioxo-11,13,19,21-tetraoxa-16-azahentriacont anedioate as a colorless oily substance was obtained by the same method as that in Example 1, except that 2-butyl-1-octanol was used instead of 2-hexyl-1-decanol in Example 1.
$^1$H-NMR ($CDCl_3$) δ: 4.72-4.58 (2H, m), 4.21-4.08 (4H, m), 3.97 (4H, d, J = 6.0 Hz), 2.84 (4H, t, J = 6.6 Hz), 2.73-2.43 (8H, m), 2.29 (4H, t, J = 7.8 Hz), 1.65-1.50 (14H, m), 1.36-1.20 (68H, m), 1.10-0.96 (6H, m), 0.92-0.84 (18H, m).
MS m/z (M + H): 1139.
clogP: 26.1444

[Example 3]

(1)

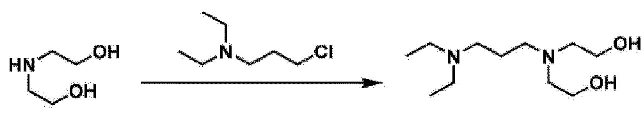

Potassium carbonate (4.3 g) was added to a mixture of 2,2'-azanediylbis(ethan-1-ol) (2.5 g), 3-chloro-N,N-diethylpropan-1-amine (4.6 g), and ethanol (25 mL), and the mixture was stirred under reflux with heating for 6 hours. The reaction mixture was cooled to room temperature, then insoluble matters were filtered off, and the solvent was distilled off under reduced pressure. The obtained residue was purified by silica gel column chromatography (ethyl acetate-hexane, NH silica gel), thereby obtaining 2,2'-((3-(diethylamino)propyl)azanediyl)bis(ethan-1-ol) (2.9 g) as a light yellow oily substance.
$^1$H-NMR ($CDCl_3$) δ: 3.62 (4H, t, J = 5.2 Hz), 2.26 (2H, t, J = 6.0 Hz), 2.61-2.49 (10H, m), 1.68-1.60 (2H, m), 1.04 (6H, t, J = 7.2 Hz).
MS m/z (M + H): 219.

(2)

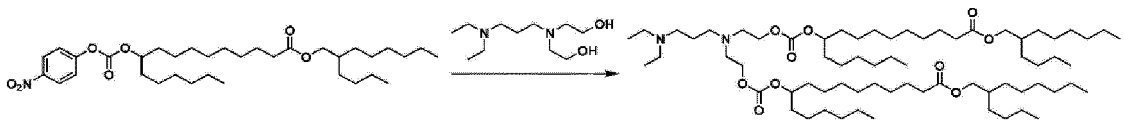

Bis(2-butyloctyl)

16-(3-(diethylamino)propyl)-10,22-dihexyl-12,20-dioxo-11,13,19,21-tetraoxa-16-azahentriaco ntanedioate as a colorless oily substance was obtained by the same method as that in Example 1, except that 2-butyl-1-octanol and 2,2'-((3-(diethylamino)propyl)azanediyl)bis(ethan-1-ol) were used instead of 2-hexyl-1-decanol and 2,2'-((2-(diethylamino)ethyl)azanediyl)bis(ethan-1-ol) in Example 1, respectively.
$^1$H-NMR ($CDCl_3$) δ: 4.72-4.60 (2H, m), 4.23-4.07 (4H, m), 3.97 (4H, d, J = 6.0 Hz), 2.81 (4H, t, J = 6.6 Hz), 2.72-2.37 (8H, m), 2.29 (4H, t, J = 7.8 Hz), 1.65-1.50 (16H, m), 1.35-1.20 (68H, m), 1.00 (6H, t, J = 7.2 Hz), 0.90-0.85 (18H, m).
MS m/z (M + H): 1153.
clogP: 26.389

[Example 4]

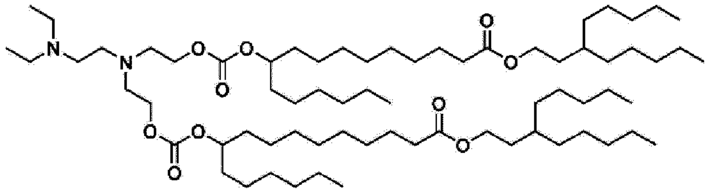

Bis(3-pentyloctyl)

16-(2-(diethylamino)ethyl)-10,22-dihexyl-12,20-dioxo-11,13,19,21-tetraoxa-16-azahentriacont anedioate as a colorless oily substance was obtained by the same method as that in Example 1, except that 3-pentyloctan-1-ol was used instead of 2-hexyl-1-decanol in Example 1.

$^1$H-NMR ($CDCl_3$) $\delta$:4.72-4.58 (2H, m), 4.21-4.11 (4H, m), 3.97 (4H, t, J = 7.2 Hz), 2.84 (4H, t, J = 6.6 Hz), 2.71-2.64 (2H, m), 2.56-2.46 (6H, m), 2.29 (4H, t, J = 7.8 Hz), 1.65-1.50 (16H, m), 1.36-1.20 (70H, m), 1.01 (6H, t, J = 7.2 Hz), 0.92-0.84 (18H, m).

MS m/z (M + H): 1167.

[Example 5]

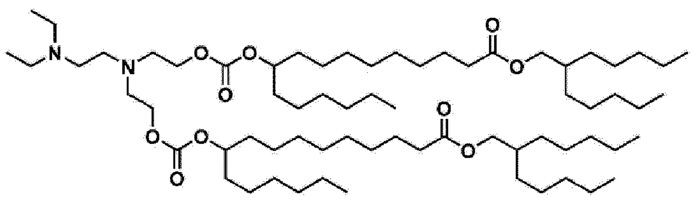

Bis(2-pentylheptyl)

16-(2-(diethylamino)ethyl)-10,22-dihexyl-12,20-dioxo-11,13,19,21-tetraoxa-16-azahentriacont anedioate as a colorless oily substance was obtained by the same method as that in Example 1, except that 2-pentylheptan-1-ol was used instead of 2-hexyl-1-decanol in Example 1.

$^1$H-NMR($CDCl_3$) $\delta$: 4.72-4.58 (2H, m), 4.21-4.08 (4H, m), 3.97 (4H, d, J = 6.0 Hz), 2.84 (4H, t, J = 6.6 Hz), 2.71-2.64 (2H, m), 2.56-2.46 (6H, m), 2.29 (4H, t, J = 7.8 Hz), 1.65-1.50 (14H, m), 1.36-1.20 (68H, m), 1.01 (6H, t, J = 7.2 Hz), 0.92-0.84 (18H, m).

MS m/z (M + H): 1139.
clogP: 26.1444

[Example 6]

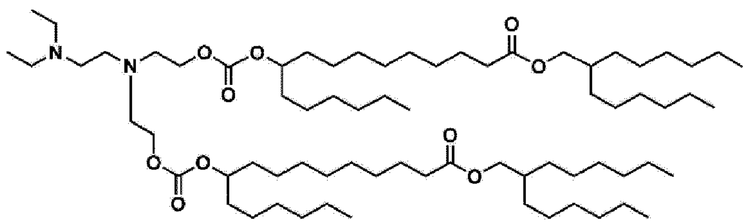

Bis(2-hexyloctyl)

16-(2-(diethylamino)ethyl)-10,22-dihexyl-12,20-dioxo-11,13,19,21-tetraoxa-16-azahentriacont anedioate as a colorless oily substance was obtained by the same method as that in Example 1, except that 2-hexyloctan-1-ol was used instead of 2-hexyl-1-decanol in Example 1.

$^1$H-NMR($CDCl_3$) $\delta$: 4.72-4.58 (2H, m), 4.21-4.08 (4H, m), 3.97 (4H, d, J = 6.0 Hz), 2.84 (4H, t, J = 6.6 Hz),

2.71-2.64 (2H, m), 2.56-2.46 (6H, m), 2.29 (4H, t, J = 7.8 Hz), 1.65-1.50 (14H, m), 1.36-1.20 (76H, m), 1.01 (6H, t, J = 7.2 Hz), 0.92-0.84 (18H, m).

MS m/z (M + H): 1195.

clogP: 28.2604

[Example 7]

(1)

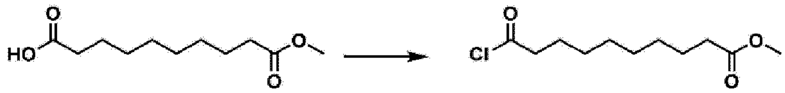

A mixture of 10-methoxy-10-oxodecanoic acid (47.6 g), thionyl chloride (47.6 mL), and N,N-dimethylformamide (0.1 mL) was stirred under reflux with heating for 1 hour. The solvent was distilled off under reduced pressure, thereby obtaining methyl 10-chloro-10-oxodecanoate (59.7 g) as a brown oily substance.

$^1$H-NMR ($CDCl_3$) $\delta$: 3.67 (3H, s), 2.88 (2H, t, J = 7.2 Hz), 2.30 (2H, t, J = 7.2 Hz), 1.75-1.57 (4H, m), 1.38-1.25 (8H, m).

(2)

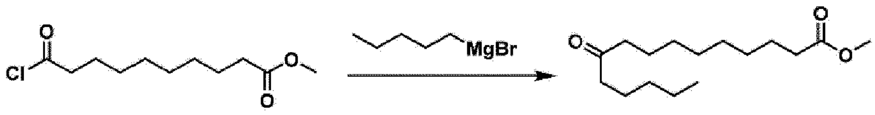

A 1.0 mol/L pentyl magnesium bromide-tetrahydrofuran solution (200 mL) was added dropwise to a tetrahydrofuran suspension (300 mL) of zinc (II) chloride (13.6 g) at -78°C, and the mixture was heated to 0°C and then stirred at the same temperature for 30 minutes. Tetrakis(triphenylphosphine)palladium(0) (2.9 g) was added to the reaction mixture under ice cooling, then methyl 10-chloro-10-oxodecanoate (24.9 g) was added dropwise thereto at the same temperature, and the reaction mixture was stirred at the same temperature for 1 hour. A 1.0 mol/L aqueous hydrochloric acid solution (100 mL), hexane (100 mL), and ethyl acetate (100 mL) were added to the reaction mixture, the organic layer was separated, washed with a saturated aqueous sodium chloride solution (100 mL), and then dried over anhydrous sodium sulfate, and the solvent was distilled off under reduced pressure. The obtained residue was purified by silica gel column chromatography (ethyl acetate-hexane), thereby obtaining methyl 10-oxopentadecanoate (19.8 g) as white solids.

$^1$H-NMR ($CDCl_3$) $\delta$: 3.67 (3H, s), 2.38 (4H, t, J = 7.2 Hz), 2.30 (2H, t, 7.2 Hz), 1.65-1.49 (6H, m), 1.35-1.20 (12H, m), 0.88 (3H, t, J = 7.2 Hz).

(3)

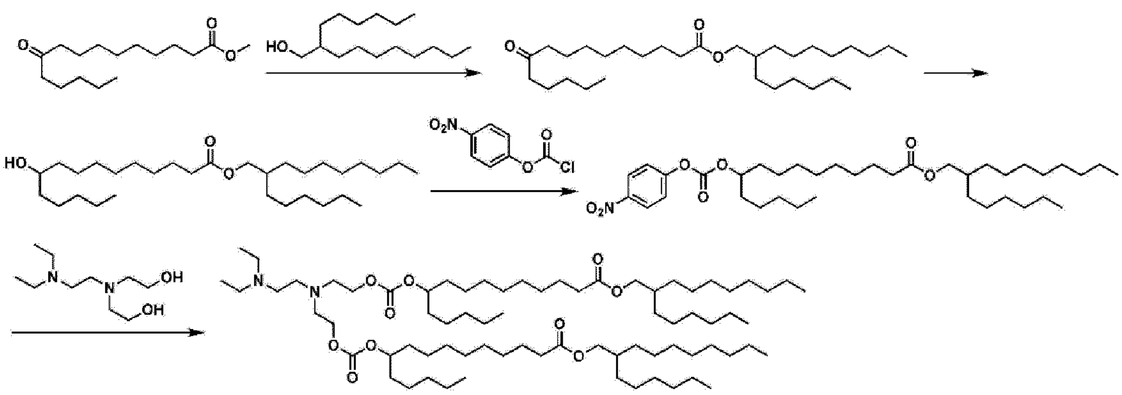

Tetraisopropyl orthotitanate (0.55 g) was added to a mixture of methyl 10-oxopentadecanoate (5.3 g) and 2-hexyldecan-1-ol (7.1 g), and the mixture was stirred at 110°C for 2 hours. The reaction mixture was cooled to room temperature, then water (0.5 mL) was added thereto, and the mixture was stirred at room temperature for 15 minutes. The

mixture was purified by silica gel column chromatography (ethyl acetate-hexane), thereby obtaining 2-hexyldecyl 10-oxopentadecanoate (9.2 g) as a colorless oily substance.

$^1$H-NMR ($CDCl_3$) δ: 3.97 (2H, d, J = 5.6 Hz), 2.38 (4H, t, J = 7.6 Hz), 2.29 (2H, t, J = 7.6 Hz), 1.65-1.50 (7H, m), 1.35-1.20 (36H, m), 0.92-0.83 (9H, m).

Sodium borohydride (1.0 g) was added to a mixture of 2-hexyldecyl 10-oxopentadecanoate (9.2 g), methanol (36 mL), and tetrahydrofuran (36 mL) under ice cooling, and the mixture was stirred at the same temperature for 1 hour. Water (80 mL), 1.0 mol/L aqueous hydrochloric acid solution (35 mL), and hexane (40 mL) were added to the reaction mixture, and the organic layer was separated. The organic layer was washed with a saturated aqueous sodium chloride solution (20 mL), dried over anhydrous magnesium sulfate, and then the solvent was distilled off under reduced pressure. The obtained residue was purified by silica gel column chromatography (ethyl acetate-hexane), thereby obtaining 2-hexyldecyl 10-hydroxypentadecanoate (7.2 g) as a colorless oily substance.

$^1$H-NMR ($CDCl_3$) δ: 3.97 (2H, d, J = 6.0 Hz), 3.61-3.54 (1H, m), 2.30 (2H, t, J = 7.6 Hz), 1.65-1.56 (3H, m), 1.48-1.22 (44H, m), 0.92-0.83 (9H, m).

4-Nitrophenyl chloroformate (1.8 g) was added to a mixture of 2-hexyldecyl 10-hydroxypentadecanoate (2.1 g), triethylamine (2.4 mL), and tetrahydrofuran (21 mL), and the mixture was stirred at room temperature for 4 hours. Water (60 mL) and hexane (60 mL) were added to the reaction mixture, the organic layer was separated, washed with water, and then dried over anhydrous sodium sulfate, and the solvent was distilled off under reduced pressure. The obtained residue was purified by silica gel column chromatography (ethyl acetate-hexane), thereby obtaining 2-hexyldecyl 10-(((4-nitrophenoxy)carbonyl)oxy)pentadecanoate (2.7 g) as a colorless oily substance.

$^1$H-NMR ($CDCl_3$) δ: 8.28 (2H, dd, J = 7.2 Hz, 2.1 Hz), 7.39 (2H, dd, J = 7.2 Hz, 2.1 Hz), 4.86-4.76 (1H, m), 3.97 (2H, d, J = 5.7 Hz), 2.30 (2H, t, J = 7.2 Hz), 1.74-1.20 (47H, m), 0.92-0.85 (9H, m).

4-Dimethylaminopyridine (1.13 g) was added to a mixture of 2-hexyldecyl 10-(((4-nitrophenoxy)carbonyl)oxy) pentadecanoate (2.00 g), 2,2'-((2-(diethylamino)ethyl)azanediyl)bis(ethan-1-ol) (1.89 g), triethylamine (1.29 mL), and tetrahydrofuran (10 mL), and the mixture was stirred at 80°C for 6 hours. The reaction mixture was cooled to room temperature, water and ethyl acetate were added thereto, the organic layer was separated, washed with a saturated aqueous sodium chloride solution, and then dried over anhydrous sodium sulfate, and the solvent was distilled off under reduced pressure. The obtained residue was purified by silica gel column chromatography (methanol-ethyl acetate) and silica gel column chromatography (ethyl acetate-hexane, NH silica gel), thereby obtaining bis(2-hexyldecyl) 16-(2-(diethylamino)ethyl)-12,20-dioxo-10,22-dipentyl-11,13,19,21-tetraoxa-16-azahentriacon tanedioate (0.12 g) as a colorless oily substance.

$^1$H-NMR($CDCl_3$) δ: 4.72-4.58 (2H, m), 4.21-4.08 (4H, m), 3.97 (4H, d, J = 6.0 Hz), 2.84 (4H, t, J = 6.6 Hz), 2.71-2.64 (2H, m), 2.56-2.46 (6H, m), 2.29 (4H, t, J = 7.8 Hz), 1.65-1.50 (14H, m), 1.36-1.20 (80H, m), 1.01 (6H, t, J = 7.2 Hz), 0.92-0.84 (18H, m).

MS m/z (M + H): 1223.

clogP: 29.3184

[Example 8]

(1)

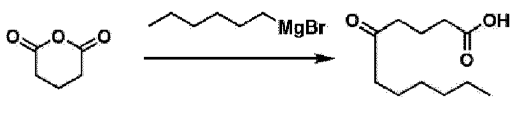


A 1.0 mol/L hexyl magnesium bromide-tetrahydrofuran solution (200 mL) was added dropwise to a tetrahydrofuran solution (273 mL) of glutaric anhydride (27.3 g) under ice cooling, and the mixture was stirred at the same temperature for 1 hour. A 2 mol/L aqueous hydrochloric acid solution (240 mL) was added to the reaction mixture under ice cooling, then ethyl acetate (270 mL) was added thereto, the organic layer was separated, washed with water and a saturated aqueous sodium chloride solution, and dried over anhydrous magnesium sulfate, and then the solvent was distilled off under reduced pressure. The obtained residue was purified by silica gel column chromatography (ethyl acetate-hexane), then hexane (10 mL) was added thereto, and solids were collected by filtration, washed with hexane, and then dried under reduced pressure, thereby obtaining 5-oxoundecanoic acid (16.0 g) as white solids.

$^1$H-NMR ($CDCl_3$) δ: 2.50 (2H, t, J = 7.2 Hz), 2.40 (4H, t, J = 7.2 Hz), 2.02-1.80 (2H, m), 1.63-1.48 (2H, m), 1.37-1.20 (6H, m), 0.88 (3H, t, J = 6.6 Hz).

(2)

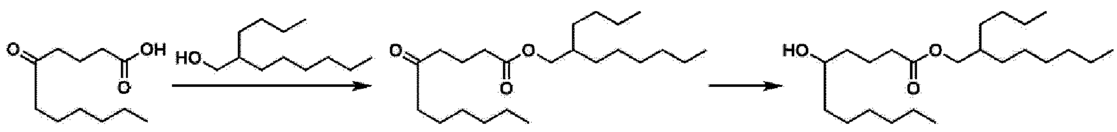

p-Toluenesulfonic acid (0.14 g) was added to a mixture of 5-oxoundecanoic acid (3,0 g), 2-butyloctan-1-ol (2.5 g), and toluene (6.0 mL), and the mixture was stirred at 100°C for 2 hours. The reaction mixture was cooled to room temperature and then purified by silica gel column chromatography (ethyl acetate-hexane), thereby obtaining 2-butyloctyl 5-oxoundecanoate (5.0 g) as a colorless oily substance.

$^1$H-NMR ($CDCl_3$) δ: 3.97 (2H, d, J = 5.1 Hz), 2.47 (2H, t, J = 7.2 Hz), 2.39 (2H, t, J = 7.2 Hz), 2.33 (2H, t, J = 7.2 Hz), 1.95-1.83 (2H, m), 1.66-1.49 (3H, m), 1.36-1.20 (22H, m), 0.92-0.82 (9H, m).

Sodium borohydride (0.90 g) was added to a mixture of 2-butyloctyl 5-oxoundecanoate (7.3 g), toluene (30 mL), and methanol (30 mL) under ice cooling, and the mixture was stirred at the same temperature for 1 hour. Water (30 mL), a 2.0 mol/L aqueous hydrochloric acid solution (30 mL), and ethyl acetate (30 mL) were added to the reaction mixture under ice cooling, and the organic layer was separated. The organic layer was washed with a saturated aqueous sodium chloride solution, and dried over anhydrous sodium sulfate, and the solvent was distilled off under reduced pressure. The obtained residue was purified by silica gel column chromatography (ethyl acetate-hexane), thereby obtaining 2-butyloctyl 5-hydroxyundecanoate (6.9 g) as a colorless oily substance.

$^1$H-NMR ($CDCl_3$) δ: 3.97 (2H, d, J = 5.7 Hz), 3.65-3.53 (1H, m), 2.35 (2H, t, J = 7.2 Hz), 1.87-1.20 (32H, m), 0.92-0.84 (9H, m).

(3)

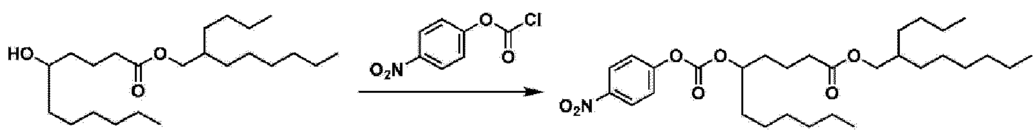

4-Nitrophenyl chloroformate (1.71 g) was added to a mixture of 2-butyloctyl 5-hydroxyundecanoate (1.62 g), triethylamine (2.38 mL), and tetrahydrofuran (16 mL), and the mixture was stirred at room temperature for 4 hours. Water and ethyl acetate were added to the reaction mixture, the organic layer was separated, washed with water, and then dried over anhydrous sodium sulfate, and the solvent was distilled off under reduced pressure. The obtained residue was purified by silica gel column chromatography (ethyl acetate-hexane), thereby obtaining 2-butyloctyl 5-(((4-nitrophenoxy)carbonyl)oxy)undecanoate (1.99 g) as a colorless oily substance.

$^1$H-NMR ($CDCl_3$) δ: 8.28 (2H, d, J = 9.3 Hz), 7.39 (2H, d, J = 9.3 Hz), 4.88-4.77 (1H, m), 3.99 (2H, d, J = 6.0 Hz), 2.41-2.31 (2H, m), 1.80-1.48 (7H, m), 1.44-1.20 (24H, m), 0.92-0.83 (9H, m).

(4)

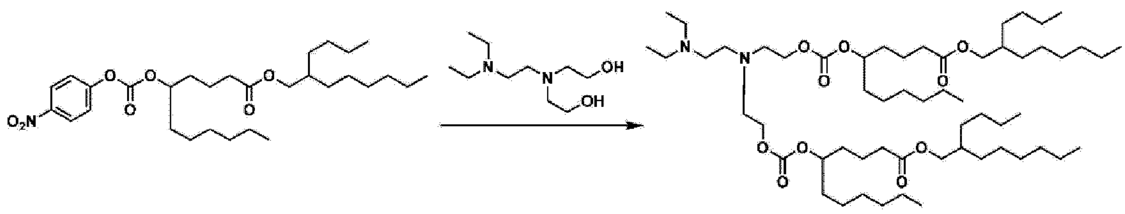

Bis(2-butyloctyl)

11-(2-(diethylamino)ethyl)-5,17-dihexyl-7,15-dioxo-6,8,14,16-tetraoxa-11-azahenicosanedioat e as a colorless oily substance was obtained by the same method as that in Example 1, except that 2-butyloctyl 5-(((4-nitrophenoxy)carbonyl)oxy)undecanoate was used instead of 2-hexyldecyl 10-(((4-nitrophenoxy)carbonyl)oxy)hexadecanoate in Example 1.

$^1$H-NMR($CDCl_3$) δ: 4.72-4.65 (2H, m), 4.22-4.09 (4H, m), 3.97 (4H, d, J = 6.0 Hz), 2.84 (4H, t, J = 6.4 Hz), 2.70-2.65 (2H, m), 2.55-2.48 (6H, m), 2.32 (4H, t, J = 7.2 Hz), 1.73-1.50 (14H, m), 1.33-1.20 (48H, m), 1.01 (6H, t, J = 7.2 Hz), 0.91-0.84 (18H, m).

MS m/z (M + H): 999.
clogP: 20.8544

[Example 9]

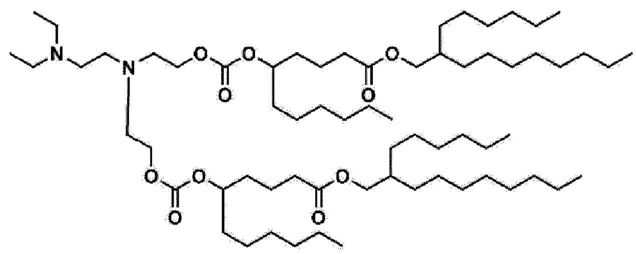

Bis(2-hexyldecyl)

11-(2-(diethylamino)ethyl)-5,17-dihexyl-7,15-dioxo-6,8,14,16-tetraoxa-11-azahenicosanedioat e as a colorless oily substance was obtained by the same method as that in Example 1, except that 2-hexyl-1-decanol was used instead of 2-butyl-1-octanol in Example 8.

$^1$H-NMR($CDCl_3$) δ: 4.72-4.65 (2H, m), 4.22-4.09 (4H, m), 3.97 (4H, d, J = 6.0 Hz), 2.84 (4H, t, J = 6.4 Hz), 2.70-2.65 (2H, m), 2.55-2.48 (6H, m), 2.32 (4H, t, J = 7.2 Hz), 1.73-1.50 (14H, m), 1.33-1.20 (64H, m), 1.01 (6H, t, J = 7.2 Hz), 0.91-0.84 (18H, m).

MS m/z (M + H): 1111.
clogP: 25.0864

[Example 10]

(1)

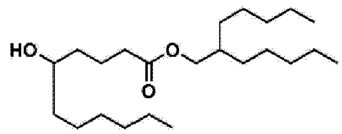

2-Pentylheptyl 5-hydroxyundecanoate as a colorless oily substance was obtained by the same method as that in (1) and (2) of Example 8, except that 2-pentyl-1-heptanol was used instead of 2-butyl-1-octanol in (1) and (2) of Example 8.

$^1$H-NMR ($CDCl_3$) δ: 3.98 (2H, d, J = 6.0 Hz), 3.63-3.57 (1H, m), 2.41-2.28 (2H, m), 1.84-1.22 (32H, m), 0.88 (9H, t, J = 7.2 Hz).

(2)

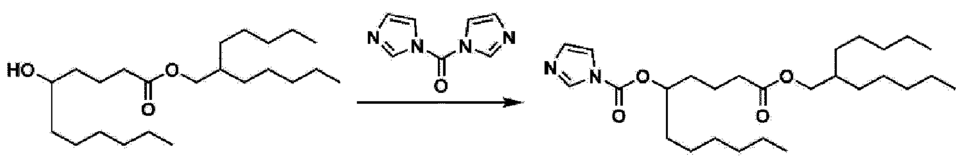

1,1'-Carbonyldiimidazole (0.33 g) was added to a tetrahydrofuran solution (5.0 mL) of 2-pentylheptyl 5-hydroxyundecanoate (0.50 g), and the mixture was stirred at room temperature for 30 hours. Water (10 mL) and hexane (20 mL) were added to the reaction mixture, the organic layer was separated, then washed with water and a saturated aqueous sodium chloride solution, and dried over anhydrous sodium sulfate, and then the solvent was distilled off under reduced pressure, thereby obtaining 1-oxo-1-((2-pentylheptyl)oxy)undecane-5-yl 1H-imidazole-1-carboxylate (0.64 g) as a light yellow oily substance.

$^1$H-NMR($CDCl_3$) δ: 8.14-8.12 (1H, m), 7.43-7.41 (1H, m), 7.08-7.06 (1H, m), 5.11-5.04 (1H, m), 3.97 (2H, d, J =

5.6 Hz), 2.38-2.32 (2H, m), 1.80-1.54 (7H, m), 1.40-1.22 (24H, m), 0.91-0.85 (9H, m).

(3)

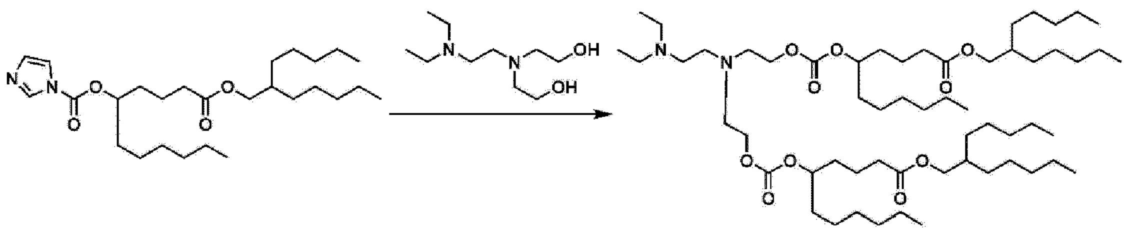

Potassium carbonate (0.33 g) was added to a mixture of 1-oxo-1-((2-pentylheptyl)oxy)undecane-5-yl 1H-imidazole-1-carboxylate (0.50 g), 2,2'-((2-(diethylamino)ethyl)azanediyl)bis(ethan-1-ol) (0.11 g), and acetonitrile (2.5 mL), and the mixture was stirred at 80°C for 2 hours. The reaction mixture was cooled to room temperature, then ethyl acetate (3 mL), hexane (3 mL), water (3 mL), and methanol (1 mL) were added thereto, and the organic layer was separated. The organic layer was washed with water and a saturated aqueous sodium chloride solution, and dried over anhydrous sodium sulfate, and the solvent was distilled off under reduced pressure. The obtained residue was purified by silica gel column chromatography (methanol-ethyl acetate), thereby obtaining bis(2-pentylheptyl) 11-(2-(diethylamino) ethyl)-5,17-dihexyl-7,15-dioxo-6,8,14,16-tetraoxa-11-azahenicosanedioat e (0.24 g) as a colorless oily substance.

$^1$H-NMR($CDCl_3$) $\delta$: 4.72-4.65 (2H, m), 4.22-4.09 (4H, m), 3.97 (4H, d, J = 6.0 Hz), 2.84 (4H, t, J = 6.4 Hz), 2.70-2.65 (2H, m), 2.55-2.48 (6H, m), 2.32 (4H, t, J = 7.2 Hz), 1.73-1.50 (14H, m), 1.33-1.20 (48H, m), 1.01 (6H, t, J = 7.2 Hz), 0.91-0.84 (18H, m).

MS m/z (M + H): 999.

clogP: 20.8544

[Example 11]

(1)

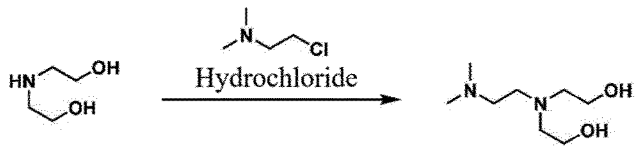


2,2'-((2-(Diethylamino)ethyl)azanediyl)bis(ethan-1-ol) as a colorless oily substance was obtained by the same method as that in (6) of Example 1, except that 2,2'-((2-(dimethylamino)ethyl)azanediyl)bis(ethan-1-ol) hydrochloride was used instead of 2-bromo-N,N-diethylethan-1-amine hydrobromide in (6) of Example 1.

$^1$H-NMR ($CDCl_3$) $\delta$: 3.57 (4H, t, J = 5.2 Hz), 2.71 (4H, t, J = 5.2 Hz), 2.64 (2H, t, J = 5.2 Hz), 2.41 (2H, t, J = 5.2 Hz), 2.61 (6H, s).

MS m/z (M + H): 177.

(2)

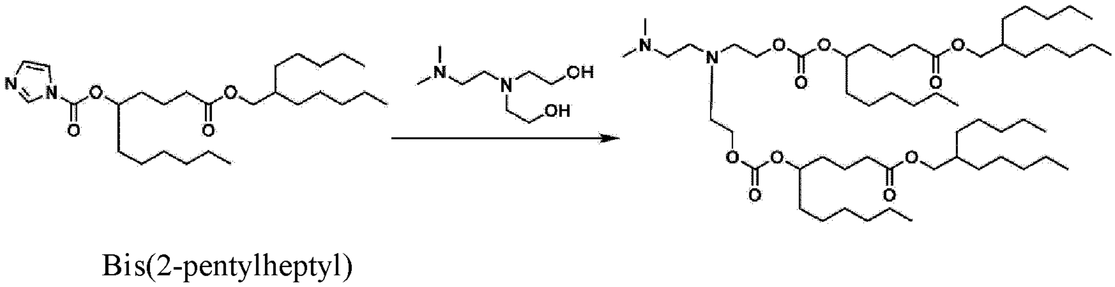

Bis(2-pentylheptyl)

11-(2-(dimethylamino)ethyl)-5,17-dihexyl-7,15-dioxo-6,8,14,16-tetraoxa-11-azahenicosanedio ate as a colorless oily substance was obtained by the same method as that in Example 10, except that 2,2'-((2-(dimethylamino)ethyl) azanediyl)bis(ethan-1-ol) was used instead of 2,2'-((2-(diethylamino)ethyl)azanediyl)bis(ethan-1-ol) in Example 10.
$^1$H-NMR ($CDCl_3$) δ: 4.72-4.65 (2H, m), 4.22-4.09 (4H, m), 3.97 (4H, d, J = 6.0 Hz), 2.85 (4H, t, J = 6.4 Hz), 2.77-2.65 (2H, m), 2.46-2.19 (12H, m) 1.73-1.50 (14H, m), 1.33-1.20 (48H, m), 0.91-0.84 (18H, m).
MS m/z (M + H): 970.
clogP: 19.7964

[Example 12]

(1)

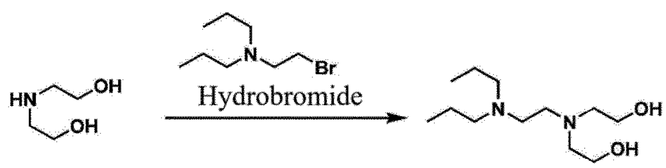


2,2'-((2-(Dimethylamino)ethyl)azanediyl)bis(ethan-1-ol) as a colorless oily substance was obtained by the same method as that in (6) of Example 1, except that N-(2-bromoethyl)-N-propylpropan-1-amine hydrobromide was used instead of 2-bromo-N,N-diethylethan-1-amine hydrobromide in (6) of Example 1.
$^1$H-NMR ($CDCl_3$) δ: 3.58 (4H, t, J = 5.4 Hz), 2.70 (4H, t, J = 5.4 Hz), 2.67-2.62 (2H, m), 2.54-2.48 (2H, m), 2.45-2.38 (4H, m), 1.57-1.43 (4H, m), 0.88 (6H, t, J = 7.2 Hz).
MS m/z (M + H): 233.

(2)

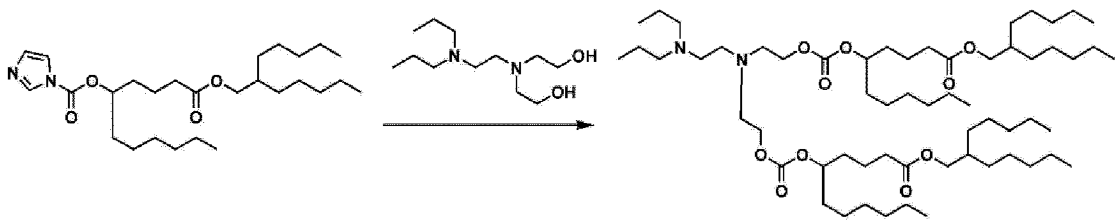

Bis(2-pentylheptyl)

11-(2-(dipropylamino)ethyl)-5,17-dihexyl-7,15-dioxo-6,8,14,16-tetraoxa-11-azahenicosanedio ate as a colorless oily substance was obtained by the same method as that in Example 10, except that 2,2'-((2-(dimethylamino)ethyl) azanediyl)bis(ethan-1-ol) was used instead of 2,2'-((2-(diethylamino)ethyl)azanediyl)bis(ethan-1-ol) in Example 10.
$^1$H-NMR ($CDCl_3$) δ: 4.72-4.65 (2H, m), 4.22-4.09 (4H, m), 3.97 (4H, d, J = 6.0 Hz), 2.83 (4H, t, J = 6.4 Hz), 2.69-2.62 (2H, m), 2.52-2.47 (2H, m), 2.39-2.29 (8H, m), 1.74-1.49 (14H, m), 1.49-1.37 (4H, m), 1.36-1.20 (48H, m), 0.91-0.84 (24H, m).
MS m/z (M + H): 1027.
clogP: 21.9124

[Example 13]

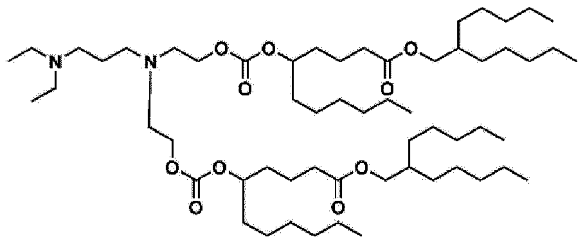

Bis(2-pentylheptyl)

11-(3-(diethylamino)propyl)-5,17-dihexyl-7,15-dioxo-6,8,14,16-tetraoxa-11-azahenicosanedio ate as a colorless oily substance was obtained by the same method as that in Example 10, except that 2,2'-((3-(diethylamino)propyl) azanediyl)bis(ethan-1-ol) was used instead of 2,2'-((2-(diethylamino)ethyl)azanediyl)bis(ethan-1-ol) in Example 10.
$^1$H-NMR($CDCl_3$) δ: 4.72-4.65 (2H, m), 4.22-4.09 (4H, m), 3.97 (4H, d, J = 6.0 Hz), 2.80 (4H, t, J = 6.4 Hz), 2.61-2.45 (6H, m), 2.44-2.37 (2H, m), 2.32 (4H, t, J = 7.2 Hz), 1.74-1.49 (16H, m), 1.35-1.21 (48H, m), 1.00 (6H, t, J = 7.2 Hz), 0.91-0.85 (18H, m).
MS m/z (M + H): 1013.
clogP: 21.099

[Example 14]

(1)

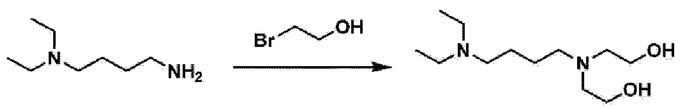

Potassium carbonate (1.5 g) was added to a mixture of N1,N1-diethylbutane-1,4-diamine (0.51 g), 2-bromoethan-1-ol (1.1 g), and acetonitrile (5 mL), and the mixture was stirred under reflux with heating for 2 hours. The reaction mixture was cooled to room temperature, then insoluble matters were filtered off, and the solvent was distilled off under reduced pressure. The obtained residue was purified by silica gel column chromatography (ethyl acetate-hexane, NH silica gel), thereby obtaining 2,2'-((4-(diethylamino)butyl)azanediyl)bis(ethan-1-ol) (0.18 g) as a light yellow oily substance.
$^1$H-NMR ($CDCl_3$) δ: 3.62 (4H, t, J = 5.2 Hz), 2.65 (4H, t, J = 5.2 Hz), 2.57-2.49 (6H, m), 2.44-2.39 (2H, m), 1.53-1.43 (4H, m), 1.02 (6H, t, J = 7.2 Hz).
MS m/z (M + H): 233.

(2)

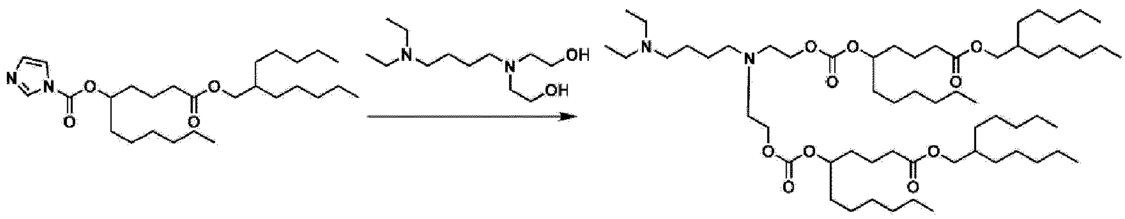

Bis(2-pentylheptyl)

11-(4-(diethylamino)butyl)-5,17-dihexyl-7,15-dioxo-6,8,14,16-tetraoxa-11-azahenicosanedioat e as a colorless oily substance was obtained by the same method as that in Example 10, except that 2,2'-((4-(diethylamino)butyl) azanediyl)bis(ethan-1-ol) was used instead of 2,2'-((2-(diethylamino)ethyl)azanediyl)bis(ethan-1-ol) in Example 10.
$^1$H-NMR ($CDCl_3$) δ: 4.72-4.65 (2H, m), 4.22-4.09 (4H, m), 3.97 (4H, d, J = 6.0 Hz), 2.80 (4H, t, J = 6.4 Hz), 2.58-2.47 (6H, m), 2.44-2.37 (2H, m), 2.32 (4H, t, J = 7.2 Hz), 1.74-1.49 (14H, m), 1.45-1.39 (4H, m), 1.35-1.21 (48H, m), 1.00 (6H, t, J = 7.2 Hz), 0.91-0.85 (18H, m).

MS m/z (M + H): 1027.
clogP: 20.68

[Example 15]

(1)

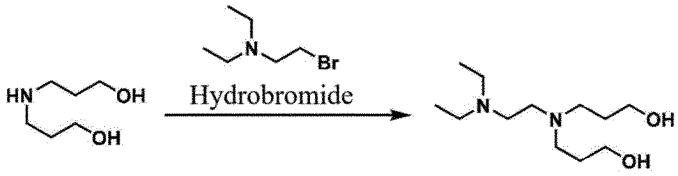


3,3'-((2-(Diethylamino)ethyl)azanediyl)bis(propan-1-ol) as a light yellow oily substance was obtained by the same method as that in (6) of Example 1, except that 3,3'-azanediylbis(propan-1-ol) was used instead of 2,2'-azanediylbis(ethan-1-ol) in (6) of Example 1.
$^1$H-NMR ($CDCl_3$) $\delta$: 3.74 (4H, t, J = 5.2 Hz), 2.61-2.51 (12H, m), 1.74-1.66 (4H, m), 1.04 (6H, t, J = 7.2 Hz).
MS m/z (M + H): 233.

(2)

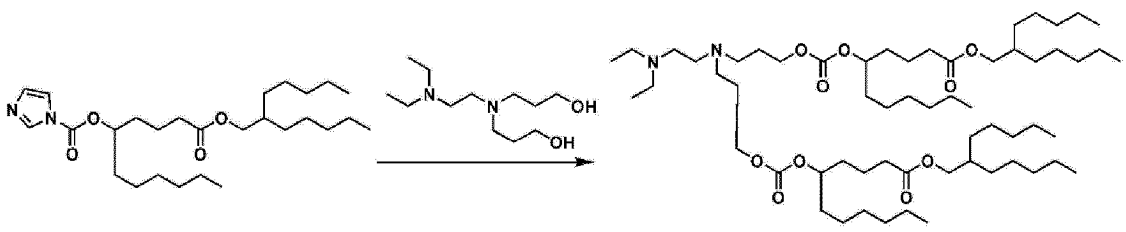

Bis(2-pentylheptyl)

12-(2-(diethylamino)ethyl)-5,19-dihexyl-7,17-dioxo-6,8,16,18-tetraoxa-12-azatricosanedioate as a colorless oily substance was obtained by the same method as that in Example 10, except that 3,3'-((2-(diethylamino)ethyl) azanediyl)bis(propan-1-ol) was used instead of 2,2'-((2-(diethylamino)ethyl)azanediyl)bis(ethan-1-ol) in Example 10.
$^1$H-NMR ($CDCl_3$) $\delta$: 4.72-4.65 (2H, m), 4.22-4.09 (4H, m), 3.97 (4H, d, J = 6.0 Hz), 2.56-2.46 (12H, m), 2.32 (4H, t, J = 7.2 Hz), 1.83-1.75 (4H, m), 1.73-1.50 (14H, m), 1.35-1.21 (48H, m), 1.01 (6H, t, J = 7.2 Hz), 0.91-0.84 (18H, m).
MS m/z (M + H): 1027.
clogP: 21.4444

[Example 16]

(1)

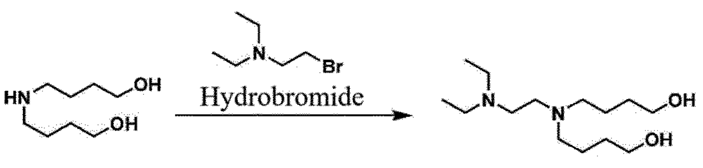


4,4'-((2-(Diethylamino)ethyl)azanediyl)bis(butan-1-ol) as a light yellow oily substance was obtained by the same method as that in (6) of Example 1, except that 4,4'-azanediylbis(butan-1-ol) was used instead of 2,2'-azanediylbis(ethan-1-ol) in (6) of Example 1.

$^{1}$H-NMR ($CDCl_3$) $\delta$: 3.62-3.58 (4H, m), 2.59-2.46 (12H, m), 1.66-1.59 (8H, m), 1.03 (6H, t, J = 7.2 Hz).
MS m/z (M + H): 261.

(2)

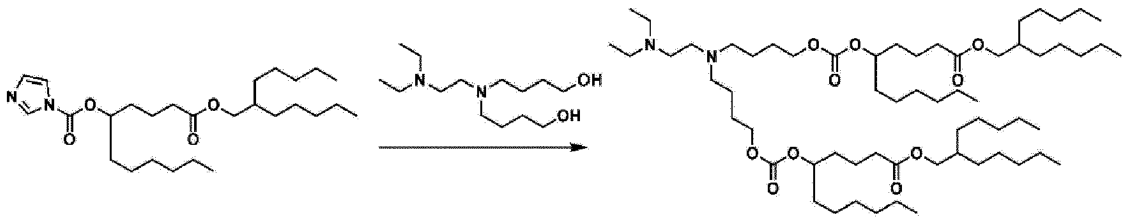

Bis(2-pentylheptyl)

13-(2-(diethylamino)ethyl)-5,21-dihexyl-7,19-dioxo-6,8,18,20-tetraoxa-13-azapentacosanedio ate as a colorless oily substance was obtained by the same method as that in Example 10, except that 4,4'-((2-(diethylamino)ethyl) azanediyl)bis(butan-1-ol) was used instead of 2,2'-((2-(diethylamino)ethyl)azanediyl)bis(ethan-1-ol) in Example 10.
$^{1}$H-NMR ($CDCl_3$) $\delta$:4.72-4.65 (2H, m), 4.22-4.09 (4H, m), 3.97 (4H, d, J = 6.0 Hz), 2.60-2.41 (12H, m), 2.32 (4H, t, J = 7.2 Hz), 1.75-1.45 (22H, m), 1.35-1.21 (48H, m), 1.03 (6H, t, J = 6.8 Hz), 0.91-0.84 (18H, m).
MS m/z (M + H): 1055.
clogP: 20.9424

[Example 17]

(1)

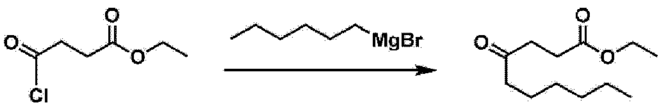

Ethyl 4-oxodecanoate as a colorless oily substance was obtained by the same method as that in (2) of Example 7, except that a 1.0 mol/L hexyl magnesium bromide-tetrahydrofuran solution and ethyl 4-chloro-4-oxobutanate were used instead of a 1.0 mol/L pentyl magnesium bromide-tetrahydrofuran solution and methyl 10-chloro-10-oxodecanoate in (2) of Example 7, respectively.
$^{1}$H-NMR ($CDCl_3$) $\delta$: 4.13 (2H, q, J = 6.8 Hz), 2.72 (2H, t, J = 7.2 Hz), 2.57 (2H, t, J = 7.2 Hz), 2.45 (2H, t, J = 7.2 Hz), 1.62-1.51 (2H, m), 1.33-1.23 (9H, m), 0.88 (3H, t, J = 6.8 Hz).

(2)

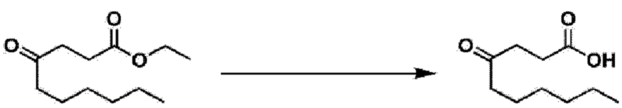

A 7.0 mol/L aqueous potassium hydroxide solution was added to a mixture of ethyl 4-oxodecanoate (2.0 g), tetrahydrofuran (4.0 mL), and ethanol (2.0 mL), and the mixture was stirred at 40°C for 45 minutes. The reaction mixture was cooled to the room temperature, then a 20% aqueous potassium hydrogen sulfate solution (15 mL), ethyl acetate (10 mL), and water (10 mL) were added thereto, the organic layer was separated and dried over anhydrous sodium sulfate, and then the solvent was distilled off under reduced pressure. Hexane (6 mL) was added to the obtained residue under ice cooling, and the solid substance was collected by filtration, washed with ice cooled hexane, and then dried under reduced pressure, thereby obtaining 4-oxodecanoic acid (1.5 g) as white solids.
$^{1}$H-NMR ($CDCl_3$) $\delta$: 2.75-2.68 (2H, m), 2.66-2.61 (2H, m), 2.45 (2H, t, J = 7.2 Hz), 1.63-1.54 (2H, m), 1.34-1.23 (6H, m), 0.88 (3H, t, J = 6.8 Hz).

(3)

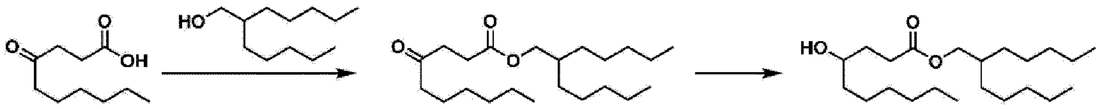

2-Pentylheptyl 4-hydroxydecanoate as a colorless oily substance was obtained by the same method as that in (2) of Example 8, except that 4-oxoundecanoate and 2-pentylpentan-1-ol were used instead of 5-oxoundecanoate and 2-butyloctan-1-ol in (2) of Example 8, respectively.

$^1$H-NMR ($CDCl_3$) δ: 3.97 (2H, d, J = 6.0 Hz), 3.65-3.58 (1H, m), 2.49-2.43 (2H, m), 1.89-1.56 (7H, m), 1.49-1.23 (22H, m), 0.89 (9H, t, J = 6.8 Hz).

(4)

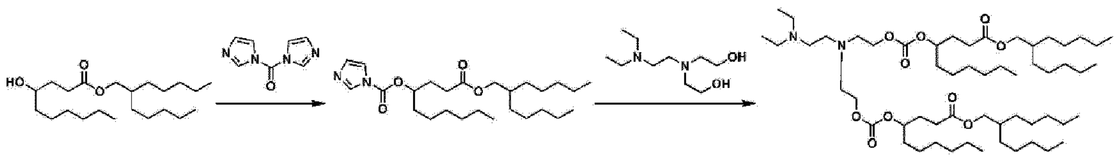

Bis(2-pentylheptyl)

10-(2-(diethylamino)ethyl)-4,16-dihexyl-6,14-dioxo-5,7,13,15-tetraoxa-10-azanonadecanedioa te as a colorless oily substance was obtained by the same method as that in (2) and (3) of Example 10, except that 2-pentylheptyl 4-hydroxydecanoate was used instead of 2-pentylheptyl 5-hydroxyundecanoate in (2) and (3) of Example 10.

$^1$H-NMR ($CDCl_3$) δ: 4.75-4.68 (2H, m), 4.23-4.09 (4H, m), 3.97 (4H, d, J = 6.0 Hz), 2.84 (4H, t, J = 6.4 Hz), 2.71-2.64 (2H, m), 2.55-2.48 (6H, m), 2.45-2.29 (4H, m), 2.01-1.81 (4H, m), 1.67-1.48 (6H, m), 1.36-1.20 (48H, m), 1.01 (6H, t, J = 7.2 Hz), 0.91-0.84 (18H, m).

MS m/z (M + H): 970.
clogP: 20.9884

[Example 18]

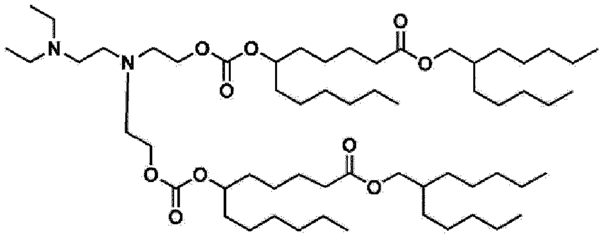

Bis(2-pentylheptyl)

12-(2-(diethylamino)ethyl)-6,18-dihexyl-8,16-dioxo-7,9,15,17-tetraoxa-12-azatricosanedioate as a colorless oily substance was obtained by the same method as that in Example 17, except that methyl 6-chloro-6-oxohexanoate was used instead of 4-chloro-4-oxobutanoate in Example 17.

$^1$H-NMR($CDCl_3$) δ: 4.71-4.63 (2H, m), 4.22-4.09 (4H, m), 3.97 (4H, d, J = 6.0 Hz), 2.84 (4H, t, J = 6.4 Hz), 2.70-2.65 (2H, m), 2.55-2.48 (6H, m), 2.30 (4H, t, J = 7.2 Hz), 1.73-1.51 (14H, m), 1.46-1.22 (52H, m), 1.01 (6H, t, J = 7.2 Hz), 0.91-0.84 (18H, m).

MS m/z (M + H): 1027.
clogP: 21.9124

[Example 19]

(1)

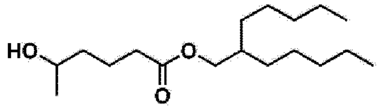

2-Pentylheptyl 5-hydroxyhexanoate as a colorless oily substance was obtained by the same method as that in (2) of Example 8, except that 5-oxohexanoic acid was used instead of 5-oxoundecanoic acid in (2) of Example 8.

$^1$H-NMR ($CDCl_3$) δ: 3.98 (2H, d, J = 6.0 Hz), 3.85-3.76 (1H, m), 2.41-2.86 (2H, m), 1.81-1.54 (3H, m), 1.51-1.43 (3H, m), 1.31-1.21 (16H, m), 1.20 (3H, d, J = 6.0 Hz), 0.89 (6H, t, J = 6.8 Hz).

(2)

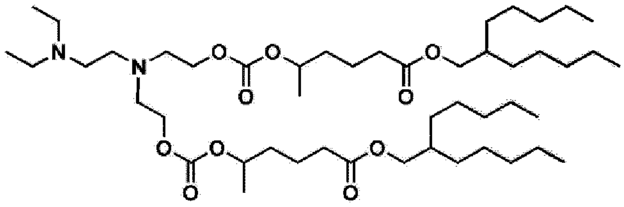

Bis(2-pentylheptyl)

11-(2-(diethylamino)ethyl)-5,17-dimethyl-7,15-dioxo-6,8,14,16-tetraoxa-11-azahenicosanedio ate as a colorless oily substance was obtained by the same method as that in (2) and (3) of Example 10, except that 2-pentylheptyl 5-hydroxyhexanoate was used instead of 2-pentylheptyl 5-hydroxyundecanoate in (2) and (3) of Example 10.

$^1$H-NMR ($CDCl_3$) δ: 4.78-4.71 (2H, m), 4.21-4.10 (4H, m), 3.97 (4H, d, J = 5.6 Hz), 2.84 (4H, t, J = 6.4 Hz), 2.69-2.65 (2H, m), 2.55-2.49 (6H, m), 2.34-2.30 (4H, m), 1.75-1.56 (10H, m), 1.39-1.20 (38H, m), 1.01 (6H, t, J = 7.2 Hz), 0.90-0.86 (12H, m).

MS m/z (M + H): 858.
clogP: 15.5644

[Example 20]

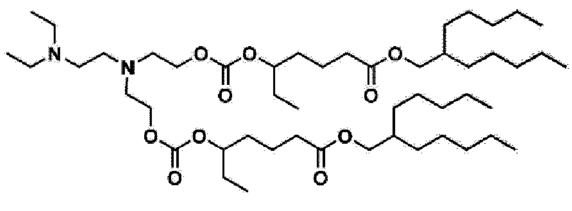

Bis(2-pentylheptyl) 11 -(2-(diethylamino)ethyl)-5,17-diethyl-7,15-dioxo-6,8,14,16-tetraoxa-11 -azahenicosanedioate as a colorless oily substance was obtained by the same method as that in Example 10, except that a 1.0 mol/L ethyl magnesium bromide-tetrahydrofuran solution was used instead of a 1.0 mol/L hexyl magnesium bromide-tetrahydrofuran solution in Example 10.

$^1$H-NMR ($CDCl_3$) δ: 4.67-4.61 (2H, m), 4.22-4.10 (4H, m), 3.97 (4H, d, J = 5.6 Hz), 2.84 (4H, t, J = 6.4 Hz), 2.69-2.65 (2H, m), 2.55-2.49 (6H, m), 2.34-2.31 (4H, m), 1.74-1.55 (14H, m), 1.34-1.20 (32H, m), 1.01 (6H, t, J = 7.2 Hz), 0.94-0.87 (18H, m).

MS m/z (M + H): 886.
clogP: 16.6224

[Example 21]

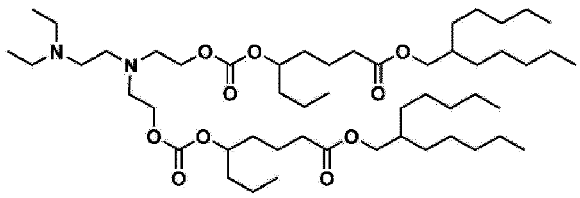

Bis(2-pentylheptyl)

11-(2-(diethylamino)ethyl)-7,15-dioxo-5,17-dipropyl-6,8,14,16-tetraoxa-11-azahenicosanedioa te as a colorless oily substance was obtained by the same method as that in Example 10, except that a 2.0 mol/L propyl magnesium bromide-tetrahydrofuran solution was used instead of a 1.0 mol/L hexyl magnesium bromide-tetrahydrofuran solution in Example 10.

$^1$H-NMR ($CDCl_3$) δ: 4.74-4.67 (2H, m), 4.22-4.10 (4H, m), 3.97 (4H, d, J = 5.6 Hz), 2.83 (4H, t, J = 6.4 Hz), 2.69-2.65 (2H, m), 2.55-2.49 (6H, m), 2.34-2.30 (4H, m), 1.74-1.48 (14H, m), 1.43-1.20 (36H, m), 1.01 (6H, t, J = 7.2 Hz), 0.93-0.87 (18H, m).

MS m/z (M + H): 914.
clogP: 17.6804

[Example 22]

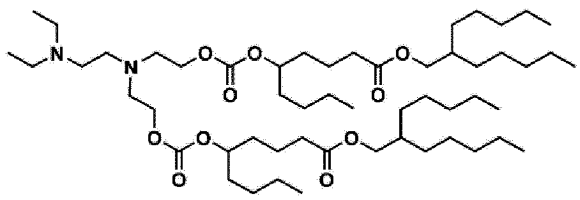

Bis(2-pentylheptyl)

5,17-dibutyl-11-(2-(diethylamino)ethyl)-7,15-dioxo-6,8,14,16-tetraoxa-11-azahenicosanedioat e as a colorless oily substance was obtained by the same method as that in Example 10, except that a 2.0 mol/L butyl magnesium chloride-tetrahydrofuran solution was used instead of a 1.0 mol/L hexyl magnesium bromide-tetrahydrofuran solution in Example 10.

$^1$H-NMR($CDCl_3$) δ: 4.72-4.65 (2H, m), 4.22-4.09 (4H, m), 3.97 (4H, d, J = 6.0 Hz), 2.84 (4H, t, J = 6.4 Hz), 2.70-2.65 (2H, m), 2.55-2.48 (6H, m), 2.32 (4H, t, J = 7.2 Hz), 1.73-1.50 (14H, m), 1.33-1.20 (40H, m), 1.01 (6H, t, J = 7.2 Hz), 0.91-0.84 (18H, m).

MS m/z (M + H): 942.
clogP: 18.7384

[Example 23]

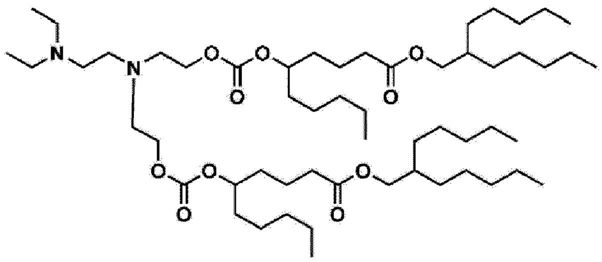

Bis(2-pentylheptyl)

11-(2-(diethylamino)ethyl)-7,15-dioxo-5,17-dipentyl-6,8,14,16-tetraoxa-11-azahenicosanedioa te as a colorless oily substance was obtained by the same method as that in Example 10, except that a 2.0 mol/L butyl magnesium chloride-tetrahydrofuran solution was used instead of a 1.0 mol/L hexyl magnesium bromide-tetrahydrofuran solution in Example 10.

$^1$H-NMR($CDCl_3$) $\delta$: 4.72-4.65 (2H, m), 4.22-4.09 (4H, m), 3.97 (4H, d, J = 6.0 Hz), 2.84 (4H, t, J = 6.4 Hz), 2.70-2.65 (2H, m), 2.56-2.48 (6H, m), 2.32 (4H, t, J = 7.2 Hz), 1.73-1.50 (14H, m), 1.33-1.20 (44H, m), 1.01 (6H, t, J = 7.2 Hz), 0.91-0.84 (18H, m).

MS m/z (M + H): 970.
clogP: 19.7964

[Example 24]

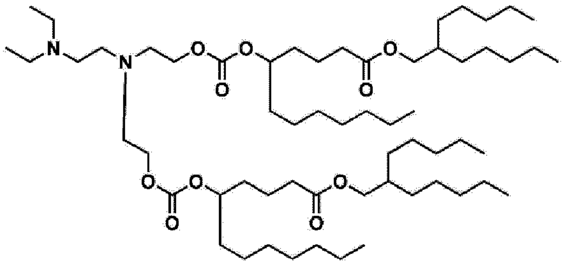

Bis(2-pentylheptyl)

11-(2-(diethylamino)ethyl)-5,17-diheptyl-7,15-dioxo-6,8,14,16-tetraoxa-11-azahenicosanedioa te as a colorless oily substance was obtained by the same method as that in Example 10, except that a 1.0 mol/L heptyl magnesium bromide-tetrahydrofuran solution was used instead of a 1.0 mol/L hexyl magnesium bromide-tetrahydrofuran solution in Example 10.

$^1$H-NMR($CDCl_3$) $\delta$: 4.72-4.65 (2H, m), 4.22-4.09 (4H, m), 3.97 (4H, d, J = 6.0 Hz), 2.84 (4H, t, J = 6.4 Hz), 2.70-2.65 (2H, m), 2.56-2.48 (6H, m), 2.32 (4H, t, J = 7.2 Hz), 1.73-1.50 (14H, m), 1.33-1.20 (52H, m), 1.01 (6H, t, J = 7.2 Hz), 0.91-0.84 (18H, m).

MS m/z (M + H): 1027.
clogP: 21.9124

[Example 25]

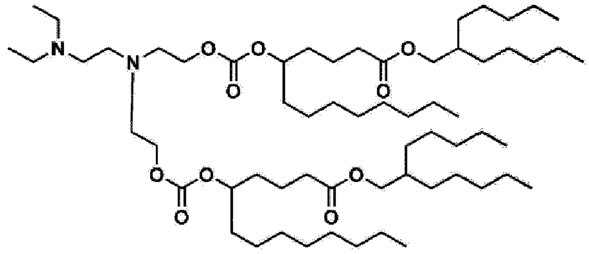

Bis(2-pentylheptyl)

11-(2-(diethylamino)ethyl)-5,17-dioctyl-7,15-dioxo-6,8,14,16-tetraoxa-11-azahenicosanedioat e as a colorless oily substance was obtained by the same method as that in Example 10, except that a 2.0 mol/L octyl magnesium bromide-diethyl ether solution was used instead of a 1.0 mol/L hexyl magnesium bromide-tetrahydrofuran solution in Example 10.

$^1$H-NMR($CDCl_3$) $\delta$: 4.72-4.65 (2H, m), 4.22-4.09 (4H, m), 3.97 (4H, d, J = 6.0 Hz), 2.84 (4H, t, J = 6.4 Hz), 2.70-2.65 (2H, m), 2.56-2.48 (6H, m), 2.32 (4H, t, J = 7.2 Hz), 1.73-1.50 (14H, m), 1.33-1.20 (56H, m), 1.01 (6H, t, J = 7.2 Hz), 0.91-0.84 (18H, m).

MS m/z (M + H): 1055.
clogP: 22.9704

[Example 26]

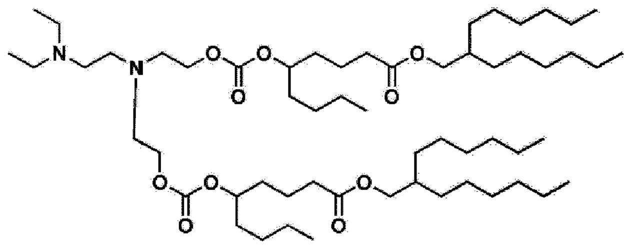

Bis(2-hexyloctyl)

5,17-dibutyl-11-(2-(diethylamino)ethyl)-7,15-dioxo-6,8,14,16-tetraoxa-11-azahenicosanedioat e as a colorless oily substance was obtained by the same method as that in Example 22, except that 2-hexyloctan-1-ol was used instead of 2-pentylheptan-1-ol in Example 22.

$^1$H-NMR($CDCl_3$) $\delta$: 4.72-4.65 (2H, m), 4.22-4.09 (4H, m), 3.97 (4H, d, J = 6.0 Hz), 2.84 (4H, t, J = 6.4 Hz), 2.70-2.65 (2H, m), 2.55-2.48 (6H, m), 2.32 (4H, t, J = 7.2 Hz), 1.73-1.50 (14H, m), 1.33-1.20 (48H, m), 1.01 (6H, t, J = 7.2 Hz), 0.91-0.84 (18H, m).

MS m/z (M + H): 999.
clogP: 20.8544

[Example 27]

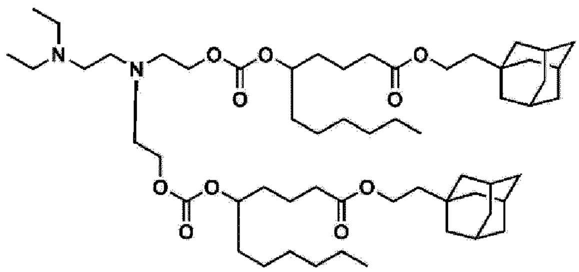

Bis (2-(((3r,5r,7r)-adamantan-1-yl)ethyl)11-(2-(diethylamino)ethyl)-5,17-dihexyl-7,15-dioxo-6,8,1 4,16-tetraoxa-11-azahenicosanedioate as a colorless oily substance was obtained by the same method as that in Example 10, except that 2-(1-adamantyl)ethanol was used instead of 2-pentylheptan-1-ol in Example 10.

$^1$H-NMR($CDCl_3$) δ: 4.72-4.65 (2H, m), 4.22-4.09 (8H, m), 2.84 (4H, t, J = 6.4 Hz), 2.70-2.65 (2H, m), 2.55-2.48 (6H, m), 2.30 (4H, t, J = 7.2 Hz), 1.97-1.92 (6H, m), 1.74-1.48 (36H, m), 1.44-1.22 (20H, m), 1.01 (6H, t, J = 7.2 Hz), 0.88 (6H, t, J = 6.4 Hz).

MS m/z (M + H): 986.

clogP: 19.1704

[Example 28]

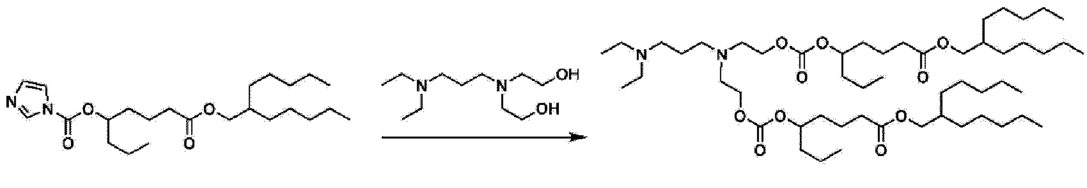

Bis(2-pentylheptyl)

11-(3-(diethylamino)propyl)-7,15-dioxo-5,17-dipropyl-6,8,14,16-tetraoxa-11-azahenicosanedi oate as a colorless oily substance was obtained by the same method as that in Example 21, except that 2,2'-((3-(diethylamino)propyl) azanediyl)bis(ethan-1-ol) was used instead of 2,2'-((2-(diethylamino)ethyl)azanediyl)bis(ethan-1-ol) in Example 21.

$^1$H-NMR ($CDCl_3$) δ: 4.73-4.67 (2H, m), 4.22-4.10 (4H, m), 3.97 (4H, d, J = 5.6 Hz), 2.80 (4H, t, J = 6.4 Hz), 2.58-2.47 (6H, m), 2.43-2.39 (2H, m), 2.34-2.30 (4H, m), 1.74-1.48 (16H, m), 1.43-1.20 (36H, m), 1.01 (6H, t, J = 7.2 Hz), 0.94-0.87 (18H, m).

MS m/z (M + H): 928.

clogP: 17.925

[Example 29]

(1)

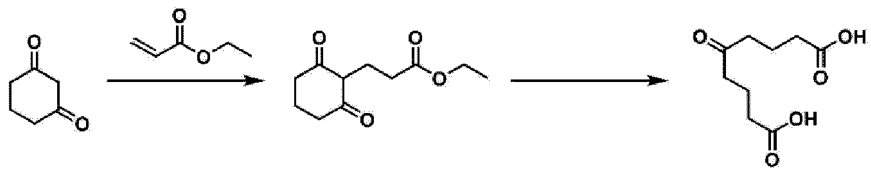

Ethyl acrylate (24.0 mL) was added to a mixture of cyclohexane-1,3-dione (16.5 g), potassium carbonate (20.3 g), benzyltriethylammonium chloride (33.5 g), and dimethyl sulfoxide (165 mL), and the mixture was stirred at 60°C for 4 hours. The reaction mixture was cooled to room temperature, then an N-acetyl-L-cysteine (14.4 g) was added thereto, and the mixture was stirred at room temperature for 1 hour. Ethyl acetate (165 mL) and a 20% aqueous potassium hydrogen sulfate solution (330 mL) were added to the reaction mixture, and the organic layer was separated. The organic layer was washed with a 20% aqueous potassium hydrogen sulfate solution (100 mL) twice, then washed with water (100 mL), and dried over anhydrous magnesium sulfate, and then the solvent was distilled off under reduced pressure. Ethyl acetate (30 mL) and hexane (60 mL) were added to the obtained residue, and the mixture was stirred at room temperature for 20 minutes. The solid substance was collected by filtration, washed with a 33% ethyl acetate-hexane solution, and dried under reduced pressure, thereby obtaining ethyl 3-(2,6-dioxocyclohexyl)propanoate (20.2 g) as light yellow solids.

$^1$H-NMR ($CDCl_3$) δ: 9.57 (1H, s), 4.18 (2H, q, J = 7.2 Hz), 2.58-2.44 (6H, m), 2.32 (2H, t, J = 6.8 Hz), 1.95-1.87 (2H, m), 1.27 (3H, t, J = 6.8 Hz).

MS m/z (M + H): 213.

A 10% aqueous hydrochloric acid solution (200 mL) was added to ethyl 3-(2,6-dioxocyclohexyl)propanoate (20.2

g), and the mixture was stirred at 100°C for 4 hours. The reaction mixture was cooled to room temperature, then ethyl acetate (200 mL) was added thereto, and the organic layer was separated. Water layer was extracted three times with ethyl acetate (50 mL), the organic layer was added to the extracted solution, the mixture was dried over anhydrous sodium sulfate, and then the solvent was distilled off under reduced pressure. Ethyl acetate (20 mL) and hexane (40 mL) were added to the obtained residue, and solids were collected by filtration, washed with a 33% ethyl acetate-hexane solution, then dried under reduced pressure, thereby obtaining 5-oxononanedioic acid (9.1 g) as light yellow solids.

$^1$H-NMR ($CDCl_3$) δ: 2.43 (4H, t, J = 7.2 Hz), 2.19 (4H, t, J = 7.2 Hz), 1.66 (4H, quin, J = 7.2 Hz).

MS m/z (M - H): 201.

(2)

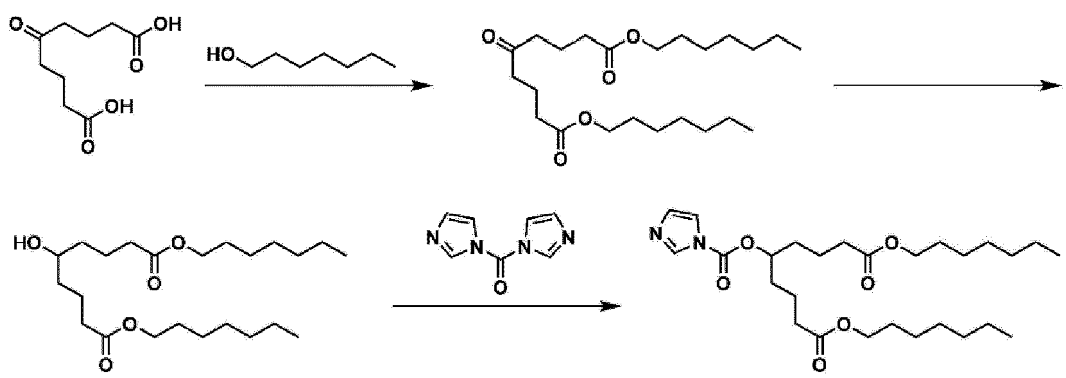

p-Toluenesulfonic acid (0.023 g) was added to a mixture of 5-oxononanedioic acid (0.500 g), heptan-1-ol (0.517 g), and toluene (1.0 mL), and the mixture was stirred at 100°C for 2 hours. The reaction mixture was cooled to room temperature and then purified by silica gel column chromatography (ethyl acetate-hexane), thereby obtaining diheptyl 5-oxononanedioate (0.746 g) as a colorless oily substance.

$^1$H-NMR ($CDCl_3$) δ: 4.06 (4H, d, J = 6.8 Hz), 2.47 (4H, t, J = 7.2 Hz), 2.32 (4H, t, J = 7.2 Hz), 1.93-1.85 (4H, m), 1.66-1.56 (4H, m), 1.36-1.24 (16H, m), 0.89 (6H, t, J = 6.8 Hz).

Sodium borohydride (0.085 g) was added to a mixture of diheptyl 5-oxononanedioate (0.746 g), toluene (3.0 mL), and methanol (3.0 mL) under ice cooling, and the mixture was stirred at the same temperature for 1 hour. Water (3.0 mL), a 1.0 mol/L aqueous hydrochloric acid solution (3.0 mL), and ethyl acetate were added to the reaction mixture under ice cooling, and the organic layer was separated. The organic layer was washed with a saturated aqueous sodium chloride solution, and dried over anhydrous sodium sulfate, and the solvent was distilled off under reduced pressure. The obtained residue was purified by silica gel column chromatography (ethyl acetate-hexane), thereby obtaining diheptyl 5-hydroxynonandioate (0.656 g) as a colorless oily substance.

$^1$H-NMR ($CDCl_3$) δ: 4.06 (4H, d, J = 6.8 Hz), 3.65-3.56 (1H, m), 2.39-2.27 (4H, m), 1.83-1.39 (12H, m), 1.36-1.23 (16H, m), 0.88 (6H, t, J = 6.8 Hz).

1,1'-Carbonyldiimidazole (0.398 g) was added to a tetrahydrofuran solution (4.0 mL) of diheptyl 5-hydroxynonandioate (0.656 g), and the mixture was stirred at room temperature for 3 hours. Water (4 mL) and hexane (4 mL) were added to the reaction mixture, the organic layer was separated, then washed with water and a saturated aqueous sodium chloride solution, and dried over anhydrous sodium sulfate, and then the solvent was distilled off under reduced pressure, thereby obtaining diheptyl 5-((1H-imidazole-1-carbonyl)oxy)nonanedioate (0.82 g) as a light yellow oily substance.

$^1$H-NMR ($CDCl_3$) δ: 8.14-8.12 (1H, m), 7.43-7.41 (1H, m), 7.08-7.06 (1H, m), 5.12-5.05 (1H, m), 4.06 (4H, d, J = 6.4 Hz), 2.35 (4H, t, J = 6.4 Hz), 1.80-1.54 (12H, m), 1.35-1.23 (16H, m), 0.88 (6H, t, J = 7.2 Hz).

(3)

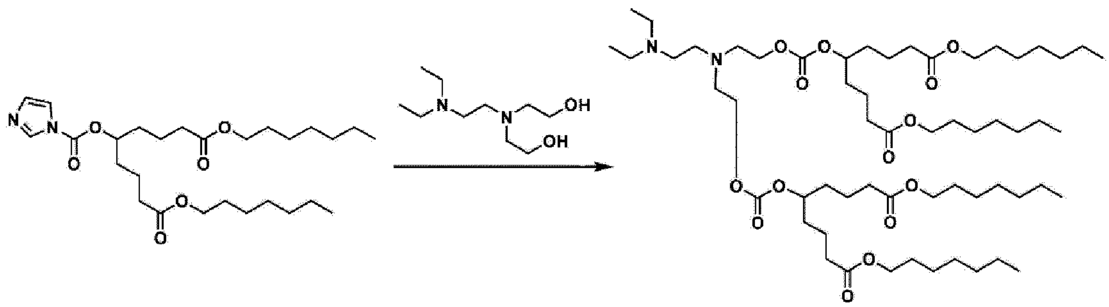

Potassium carbonate (0.45 g) was added to a mixture of diheptyl 5-((1H-imidazole-1-carbonyl)oxy)nonanedioate (0.82 g), 2,2'-((2-(diethylamino)ethyl)azanediyl)bis(ethan-1-ol) (0.17 g), and acetonitrile (4.0 mL), and the mixture was stirred at 80°C for 2 hours. The reaction mixture was cooled to room temperature, then ethyl acetate (4 mL) and water (4 mL) were added thereto, the organic layer was separated, and the solvent was distilled off under reduced pressure. Ethyl acetate (4 mL), hexane (4 mL), and water (4 mL) were added to the obtained residue, and the organic layer was separated. The organic layer was dried over anhydrous sodium sulfate, and then the solvent was distilled off under reduced pressure. The obtained residue was purified by silica gel column chromatography (methanol-ethyl acetate-hexane), thereby obtaining diheptyl 11-(2-(diethylamino)ethyl)-5,17-bis(4-(heptyloxy)-4-oxobutyl)-7,15-dioxo-6,8,14,16-tetraoxa-11-azahenicosanedioate (0.39 g).

$^1$H-NMR ($CDCl_3$) $\delta$: 4.74-4.66 (2H, m), 4.15 (4H, t, J = 6.0 Hz), 4.05 (8H, t, J = 6.8 Hz), 2.84 (4H, t, J = 6.4 Hz), 2.70-2.64 (2H, m), 2.55-2.48 (6H, m), 2.34-2.28 (8H, m), 1.72-1.54 (24H, m), 1.36-1.24 (32H, m), 1.01 (6H, t, J = 7.2 Hz), 0.88 (12H, t, J = 6.8 Hz).

MS m/z (M + H): 1059.

clogP: 16.9164

[Example 30]

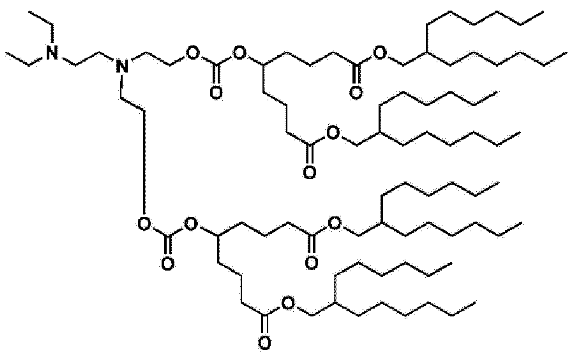

Bis(2-hexyloctyl)

11-(2-(diethylamino)ethyl)-5,17-bis(4-((2-hexyloctyl)oxy)-4-oxobutyl)-7,15-dioxo-6,8,14,16-t etraoxa-11-azahenicosanedioate as a colorless oily substance was obtained by the same method as that in Example 29, except that 2-hexyloctan-1-ol was used instead of heptan-1-ol in Example 29.

$^1$H-NMR ($CDCl_3$) $\delta$: 4.75-4.66 (2H, m), 4.15 (4H, t, J = 6.0 Hz), 3.96 (8H, d, J = 5.6 Hz), 2.83 (4H, t, J = 6.4 Hz), 2.70-2.64 (2H, m), 2.55-2.48 (6H, m), 2.34-2.28 (8H, m), 1.73-1.56 (20H, m), 1.36-1.24 (80H, m), 1.01 (6H, t, J = 7.2 Hz), 0.88 (24H, t, J = 6.8 Hz).

MS m/z (M + H): 1451.

clogP: 31.2084

[Example 31]

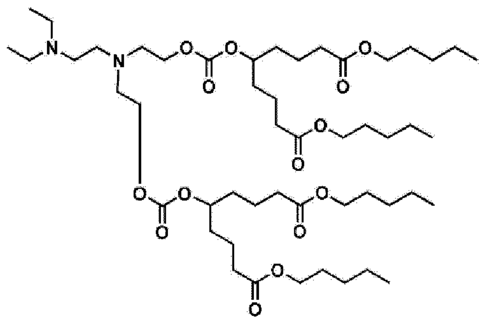

Dipentyl

11-(2-(diethylamino)ethyl)-7,15-dioxo-5,17-bis(4-oxo-4-(pentyloxy)butyl)-6,8,14,16-tetraoxa-11-azahenicosanedioate as a colorless oily substance was obtained by the same method as that in Example 29, except that pentan-1-ol was used instead of heptan-1-ol in Example 29.
$^1$H-NMR ($CDCl_3$) $\delta$: 4.74-4.66 (2H, m), 4.15 (4H, t, J = 6.0 Hz), 4.05 (8H, t, J = 6.8 Hz), 2.84 (4H, t, J = 6.4 Hz), 2.70-2.64 (2H, m), 2.55-2.48 (6H, m), 2.34-2.28 (8H, m), 1.72-1.54 (24H, m), 1.36-1.24 (16H, m), 1.01 (6H, t, J = 7.2 Hz), 0.88 (12H, t, J = 6.8 Hz).
MS m/z (M + H): 946.
clogP: 12.6844

[Example 32]

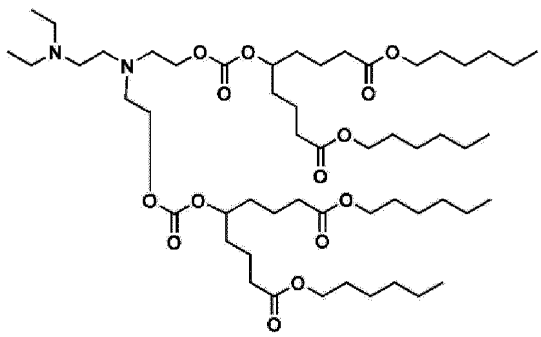

Dihexyl

11-(2-(diethylamino)ethyl)-5,17-bis(4-(hexyloxy)-4-oxobutyl)-7,15-dioxo-6,8,14,16-tetraoxa-11-azahenicosanedioate as a colorless oily substance was obtained by the same method as that in Example 29, except that hexane-1-ol was used instead of heptan-1-ol in Example 29.
$^1$H-NMR ($CDCl_3$) $\delta$: 4.74-4.66 (2H, m), 4.15 (4H, t, J = 6.0 Hz), 4.05 (8H, t, J = 6.8 Hz), 2.84 (4H, t, J = 6.4 Hz), 2.70-2.64 (2H, m), 2.55-2.48 (6H, m), 2.34-2.28 (8H, m), 1.72-1.54 (24H, m), 1.36-1.24 (24H, m), 1.01 (6H, t, J = 7.2 Hz), 0.88 (12H, t, J = 6.8 Hz).
MS m/z (M + H): 1002.
clogP: 14.8004

[Example 33]

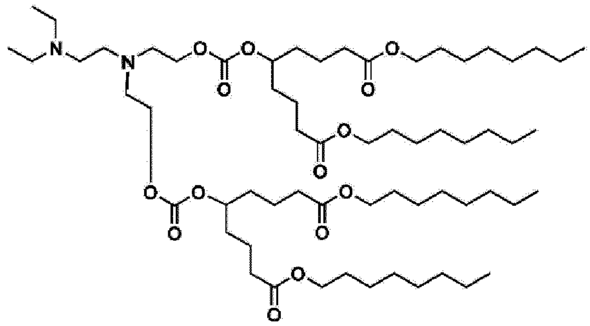

Dioctyl

11-(2-(diethylamino)ethyl)-5,17-bis(4-(octyloxy)-4-oxobutyl)-7,15-dioxo-6,8,14,16-tetraoxa-1 1-azahenicosanedioate as a colorless oily substance was obtained by the same method as that in Example 29, except that octan-1-ol was used instead of heptan-1-ol in Example 29.

$^1$H-NMR ($CDCl_3$) δ: 4.74-4.66 (2H, m), 4.15 (4H, t, J = 6.0 Hz), 4.05 (8H, t, J = 6.8 Hz), 2.84 (4H, t, J = 6.4 Hz), 2.70-2.64 (2H, m), 2.55-2.48 (6H, m), 2.34-2.28 (8H, m), 1.72-1.54 (24H, m), 1.36-1.24 (40H, m), 1.01 (6H, t, J = 7.2 Hz), 0.88 (12H, t, J = 6.8 Hz).

MS m/z (M + H): 1115.

clogP: 19.0324

[Example 34]

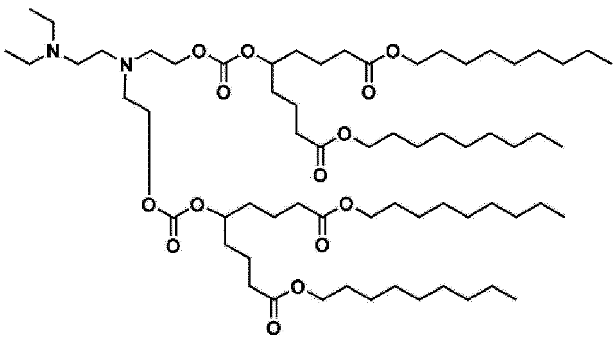

Dinonyl

11-(2-(diethylamino)ethyl)-5,17-bis(4-(nonyloxy)-4-oxobutyl)-7,15-dioxo-6,8,14,16-tetraoxa-11-azahenicosanedioate as a colorless oily substance was obtained by the same method as that in Example 29, except that nonan-1-ol was used instead of heptan-1-ol in Example 29.

$^1$H-NMR ($CDCl_3$) δ: 4.74-4.66 (2H, m), 4.15 (4H, t, J = 6.0 Hz), 4.05 (8H, t, J = 6.8 Hz), 2.84 (4H, t, J = 6.4 Hz), 2.70-2.64 (2H, m), 2.55-2.48 (6H, m), 2.34-2.28 (8H, m), 1.72-1.54 (24H, m), 1.36-1.24 (48H, m), 1.01 (6H, t, J = 7.2 Hz), 0.88 (12H, t, J = 6.8 Hz).

MS m/z (M + H): 1171.

clogP: 21.1484

[Example 35]

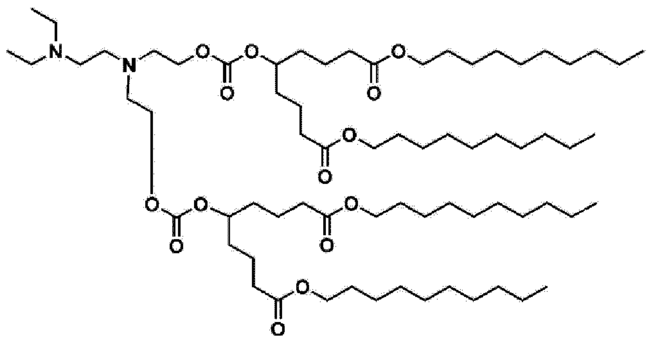

Didecyl

5,17-bis(4-(decyloxy)-4-oxobutyl)-11-(2-(diethylamino)ethyl)-7,15-dioxo-6,8,14,16-tetraoxa-1 1-azahenicosanedioate as a colorless oily substance was obtained by the same method as that in Example 29, except that decan-1-ol was used instead of heptan-1-ol in Example 29.

$^1$H-NMR ($CDCl_3$) δ: 4.74-4.66 (2H, m), 4.15 (4H, t, J = 6.0 Hz), 4.05 (8H, t, J = 6.8 Hz), 2.84 (4H, t, J = 6.4 Hz), 2.70-2.64 (2H, m), 2.55-2.48 (6H, m), 2.34-2.28 (8H, m), 1.72-1.54 (24H, m), 1.36-1.24 (56H, m), 1.01 (6H, t, J = 7.2 Hz), 0.88 (12H, t, J = 6.8 Hz).

MS m/z (M + H): 1227.

clogP: 23.2644

[Example 36]

(1)

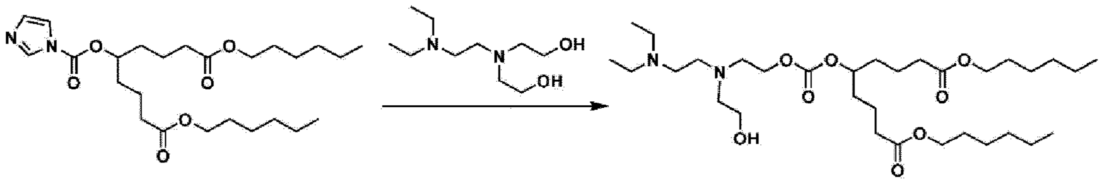

Potassium carbonate (0.339 g) was added to a mixture of dihexyl 5-((1H-imidazole-1-carbonyl)oxy)nonanedioate (0.691 g), 2,2'-((2-(diethylamino)ethyl)azanediyl)bis(ethan-1-ol) (0.234 g), and acetonitrile (3.0 mL), and the mixture was stirred at 80°C for 1.5 hours. The reaction mixture was cooled to the room temperature, then ethyl acetate (4.0 mL) and water (4.0 mL) were added thereto, the organic layer was separated and dried over anhydrous sodium sulfate, and then the solvent was distilled off under reduced pressure. The obtained residue was purified by silica gel column chromatography (methanol-ethyl acetate-hexane), thereby obtaining dihexyl 5-(((2-((2-(diethylamino)ethyl)(2-hydroxyethyl)amino)ethoxy)carbonyl)oxy)nonanedioate (0.212 g).

$^1$H-NMR ($CDCl_3$) δ: 4.73-4.66 (1H, m), 4.20 (2H, t, J = 6.0 Hz), 4.06 (4H, t, J = 6.8 Hz), 3.54 (2H, t, J = 5.2 Hz), 2.88 (2H, t, J = 6.2 Hz), 2.71-2.66 (4H, m), 2.58-2.47 (6H, m), 2.33-2.29 (4H, m), 1.74-1.55 (13H, m), 1.36-1.27 (12H, m), 1.03 (6H, t, J = 7.0 Hz), 0.91-0.87 (6H, m).

MS m/z (M + H): 604.

(2)

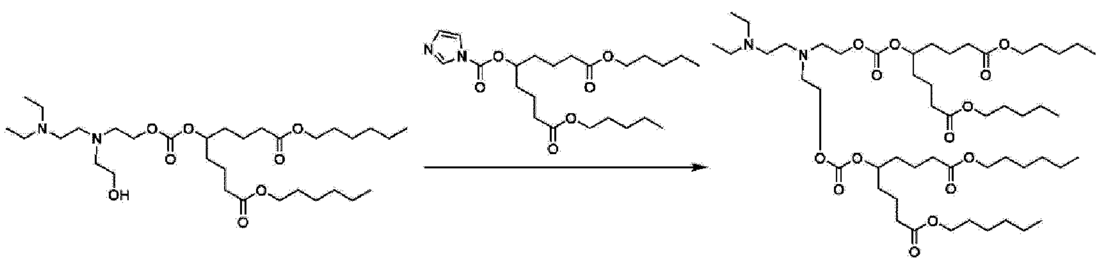

Potassium carbonate (0.830 g) was added to a mixture of dihexyl 5-(((2-((2-diethylamino)ethyl)(2-hydroxyethyl) amino)ethoxy)carbonyl)oxy)nonanedioate (0.106 g), dipentyl 5-((1H-imidazole-1-carbonyl)oxy)nonanedioate (0.093 g), and acetonitrile (1.0 mL), and the mixture was stirred at 80°C for 2 hours. The reaction mixture was cooled to the room temperature, then ethyl acetate (4 mL) and water (4 mL) were added thereto, the organic layer was separated and dried over anhydrous sodium sulfate, and then the solvent was distilled off under reduced pressure. The obtained residue was purified by silica gel column chromatography (methanol-ethyl acetate-hexane), thereby obtaining 1-hexyl 21-pentyl 11-(2-(diethylamino)ethyl)-5-(4-(hexyloxy)-4-oxobutyl)-7,15-dioxo-17-(4-oxo-4-(pentyloxy)b utyl)-6,8,14,16-tetraoxa-11-azahenicosanedioate (0.110 g).

$^1$H-NMR($CDCl_3$) $\delta$: 4.73-4.66 (2H, m), 4.15 (4H, t, J = 6.8 Hz), 4.05 (8H, t, J = 6.6 Hz), 2.83 (4H, t, J = 6.4 Hz), 2.69-2.65 (2H, m), 2.54-2.49 (6H, m), 2.33-2.29 (8H, m), 1.74-1.55 (24H, m), 1.38-1.25 (20H, m), 1.01 (6H, t, J = 7.2 Hz), 0.93-0.87 (12H, m).

MS m/z (M + H): 974.
clogP: 13.7424

[Example 37]

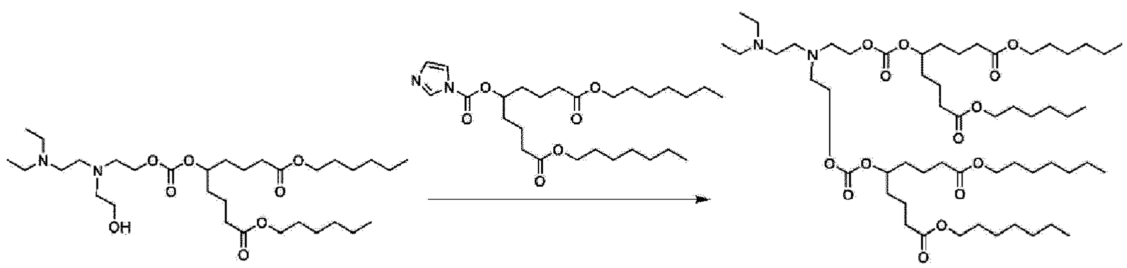

1-Heptyl 21-hexyl 11-(2-(diethylamino)ethyl)-5-(4-(heptyloxy)-4-oxobutyl)-17-(4-(hexyloxy)-4-oxobutyl)-7,15-d ioxo-6,8,14,16-tetraoxa-11-azahenicosanedioate as a colorless oily substance was obtained by the same method as that in Example 36, except that diheptyl 5-((1H-imidazole-1-carbonyl)oxy)nonanedioate was used instead of dipentyl 5-((1H-imidazole-1-carbonyl)oxy)nonanedioate in Example 36.

$^1$H-NMR($CDCl_3$) $\delta$: 4.73-4.66 (2H, m), 4.16 (4H, t, J = 6.8 Hz), 4.05 (8H, t, J = 6.6 Hz), 2.83 (4H, t, J = 6.0 Hz), 2.70-2.66 (2H, m), 2.55-2.49 (6H, m), 2.33-2.29 (8H, m), 1.73-1.56 (24H, m), 1.38-1.23 (28H, m), 1.01 (6H, t, J = 7.2 Hz), 0.91-0.86 (12H, m).

MS m/z (M + H): 1030.
clogP: 15.8584

[Example 38]

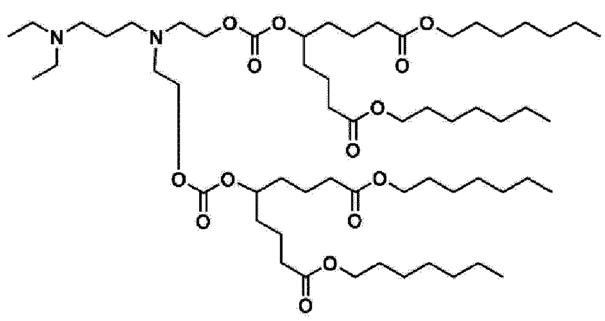

Diheptyl

11-(3-(diethylamino)propyl)-5,17-bis(4-(heptyloxy)-4-oxobutyl)-7,15-dioxo-6,8,14,16-tetraox a-11-azahenicosanedioate as a colorless oily substance was obtained by the same method as that in Example 29, except that 2,2'-((3-(diethylamino)propyl)azanediyl)bis(ethan-1-ol) was used instead of 2,2'-((2-(diethylamino)ethyl)azanediyl)bis(ethan-1-ol) in Example 29.

$^1$H-NMR($CDCl_3$) δ: 4.73-4.66 (2H, m), 4.15 (4H, t, J = 6.8 Hz), 4.05 (8H, t, J = 6.6 Hz), 2.80 (4H, t, J = 6.4 Hz), 2.58-2.47 (6H, m), 2.42-2.39 (2H, m), 2.33-2.29 (8H, m), 1.74-1.57 (26H, m), 1.35-1.21 (32H, m), 1.00 (6H, t, J = 7.2 Hz), 0.90-0.86 (12H, m).

MS m/z (M + H): 1073.
clogP: 17.161

[Example 39]

(1)

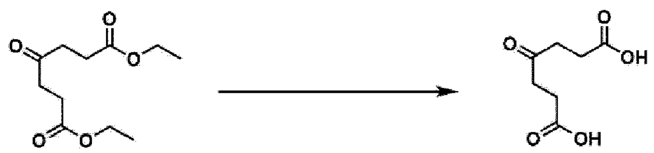

A 20% aqueous potassium hydroxide solution (14 g) was added to a mixture of diethyl 4-oxoheptandioate (5.0 g), tetrahydrofuran (10 mL), and ethanol (10 mL), and the mixture was stirred at room temperature for 30 minutes. A 30% aqueous hydrochloric acid solution (10 mL) and ethyl acetate (10 mL) were added to the reaction mixture, the organic layer was separated and dried over anhydrous sodium sulfate, and then the solvent was distilled off under reduced pressure, thereby obtaining 4-oxoheptanedioic acid (4.3 g) as light yellow solids.

$^1$H-NMR ($CDCl_3$) δ: 2.82-2.74 (4H, m), 2.68-2.58 (4H, m).

MS m/z (M - H): 173.

(2)

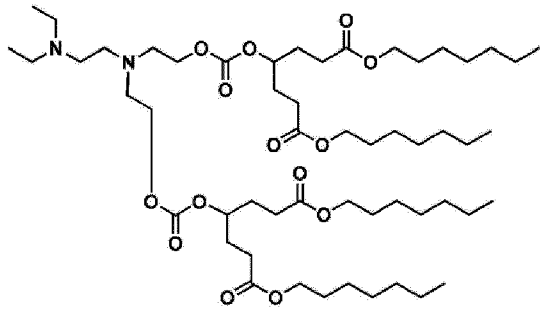

Diheptyl

10-(2-(diethylamino)ethyl)-4,16-bis(3-(heptyloxy)-3-oxopropyl)-6,14-dioxo-5,7,13,15-tetraox a-10-azanonadecanedioate as a colorless oily substance was obtained by the same method as that in (2) and (3) of Example 29, except that 4-oxoheptanedioic acid was used instead of 5-oxononanedioic acid in (2) and (3) of Example 29.

$^1$H-NMR ($CDCl_3$) δ: 4.80-4.73 (2H, m), 4.16 (4H, t, J = 6.0 Hz), 4.06 (8H, t, J = 6.8 Hz), 2.84 (4H, t, J = 6.4 Hz), 2.70-2.64 (2H, m), 2.55-2.48 (6H, m), 2.44-2.31 (8H, m), 2.00-1.85 (8H, m), 1.66-1.56 (8H, m), 1.36-1.24 (32H, m), 1.01 (6H, t, J = 7.2 Hz), 0.88 (12H, t, J = 6.8 Hz).

MS m/z (M + H): 1002.
clogP: 17.1844

[Example 40]

(1)

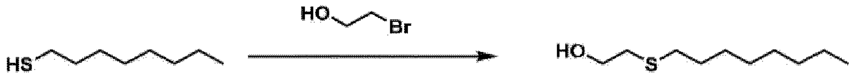

Potassium carbonate (9.4 g) was added to a mixture of octane-1-thiol (5.0 g), 2-bromoethane-1-ol (4.7 g), and acetonitrile (25 mL), and the mixture was stirred at 60°C for 4 hours. The reaction mixture was cooled to room temperature, then water (25 mL) and hexane (25 mL) were added thereto, and the organic layer was separated, and washed with a saturated aqueous sodium chloride solution. The organic layer was dried over anhydrous sodium sulfate, and then the solvent was distilled off under reduced pressure. The obtained residue was purified by silica gel column chromatography (ethyl acetate-hexane), thereby obtaining 2-(octylthio)ethan-1-ol (6.1 g).

$^{1}$H-NMR ($CDCl_3$) δ: 3.72 (2H, q, J = 6.0 Hz), 2.73 (2H, t, J = 6.0 Hz), 2.52 (2H, t, J = 7.2 Hz), 2.23 (1H, t, J = 6.0 Hz), 1.63-1.54 (2H, m), 1.43-1.21 (10H, m), 0.88 (3H, t, J = 6.8 Hz).

(2)

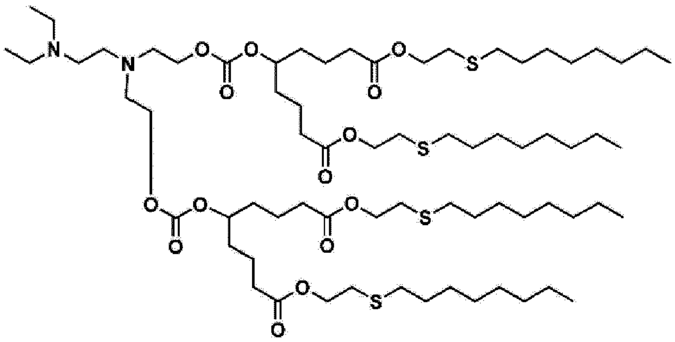

Bis(2-(octylthio)ethyl)

11-(2-(diethylamino)ethyl)-5,17-bis(4-(2-(octylthio)ethoxy)-4-oxobutyl)-7,15-dioxo-6,8,14,16 -tetraoxa-11-azahenicosanedioate as a colorless oily substance was obtained by the same method as that in (2) and (3) of Example 29, except that 2-(octylthio)ethan-1-ol was used instead of heptan-1-ol in (2) and (3) of Example 29.

$^{1}$H-NMR ($CDCl_3$) δ: 4.74-4.66 (2H, m), 4.21 (8H, t, J = 6.8 Hz), 4.16 (4H, t, J = 6.4 Hz), 2.84 (4H, t, J = 6.4 Hz), 2.75-2.65 (10H, m), 2.57-2.48 (14H, m), 2.36-2.31 (8H, m), 1.72-1.54 (16H, m), 1.42-1.23 (48H, m), 1.01 (6H, t, J = 7.2 Hz), 0.88 (12H, t, J = 6.8 Hz).

MS m/z (M + H): 1354.
clogP: 21.954

[Example 41]

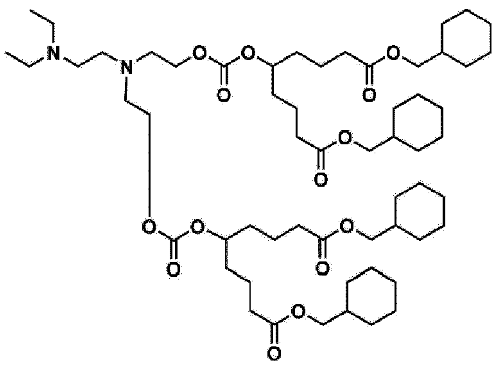

Bis(cyclohexylmethyl)

5,17-bis(4-(cyclohexylmethoxy)-4-oxobutyl)-11-(2-(diethylamino)ethyl)-7,15-dioxo-6,8,14,16 -tetraoxa-11-azahenicosanedioate as a colorless oily substance was obtained by the same method as that in Example 29, except that cyclohexylmethanol was used instead of heptan-1-ol in Example 29.
$^1$H-NMR ($CDCl_3$) $\delta$: 4.73-4.66 (2H, m), 4.16 (4H, t, J = 6.4 Hz), 3.87 (8H, d, J = 6.8 Hz), 2.84 (4H, t, J = 6.4 Hz), 2.70-2.64 (2H, m), 2.57-2.46 (6H, m), 2.34-2.30 (8H, m), 1.76-1.55 (40H, m), 1.30-0.90 (26H, m).
MS m/z (M + H): 1050.
clogP: 14.8204

[Example 42]

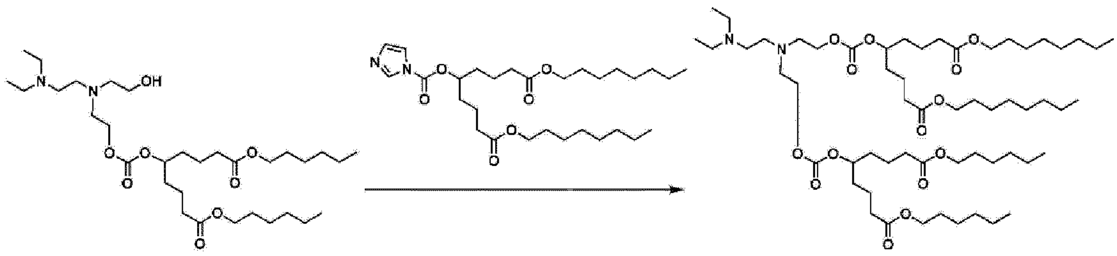

1-Hexyl 21-octyl 11-(2-(diethylamino)ethyl)-5-(4-(hexyloxy)-4-oxobutyl)-17-(4-(octyloxy)-4-oxobutyl)-7,15-dioxo-6,8,14,16-tetraoxa-11-azahenicosanedioate as a colorless oily substance was obtained by the same method as that in (2) of Example 36, except that dihexyl 5-((1H-imidazole-1-carbonyl)oxy)nonanedioate was used instead of dipentyl 5-((1H-imidazole-1-carbonyl)oxy)nonanedioate in (2) of Example 36.
$^1$H-NMR ($CDCl_3$) $\delta$: 4.73-4.66 (2H, m), 4.16 (4H, t, J = 6.4 Hz), 4.07-4.03 (8H, m), 2.84 (4H, t, J = 6.4 Hz), 2.70-2.66 (2H, m), 2.56-2.50 (6H, m), 2.33-2.29 (8H, m), 1.73-1.54 (24H, m), 1.37-1.21 (32H, m), 1.02 (6H, t, J = 7.2 Hz), 0.91-0.86 (12H, m).
MS m/z (M + H): 1059.
clogP: 16.9164

[Example 43]

(1)

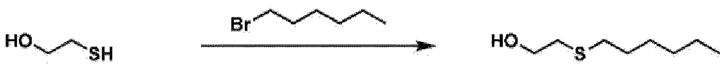

1-Bromohexane (5.2 g) was added to a mixture of 2-mercaptoethanol (3.0 g), potassium hydroxide (2.6 g), and ethanol (100 mL), the mixture was stirred at room temperature for 2 hours, and then the solvent was distilled off under reduced pressure. Ethyl acetate (150 mL) and water (200 mL) were added to the obtained residue, and the organic layer was separated and washed with water (100 mL) and a saturated aqueous sodium chloride solution. The organic layer was dried over anhydrous sodium sulfate, and the solvent was distilled off under reduced pressure, thereby obtaining 2-(hexylthio)ethan-1-ol (6.2 g).
$^1$H-NMR ($CDCl_3$) $\delta$: 3.72 (2H, dt, J = 6.0, 6.0 Hz), 2.73 (2H, t, J = 6.0 Hz), 2.52 (2H, t, J = 7.6 Hz), 2.22-2.15 (1H, m), 1.63-1.53 (2H, m), 1.42-1.22 (6H, m), 0.89 (3H, t, J = 6.8 Hz).

(2)

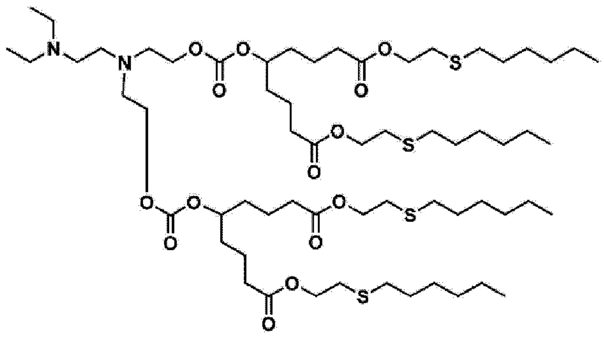

Bis(2-(hexylthio)ethyl)

11-(2-(diethylamino)ethyl)-5,17-bis(4-(2-(hexylthio)ethoxy)-4-oxobutyl)-7,15-dioxo-6,8,14,16 -tetraoxa-11-azahenicosanedioate as a colorless oily substance was obtained by the same method as that in (2) and (3) of Example 29, except that 2-(hexylthio)ethan-1-ol was used instead of heptan-1-ol in (2) and (3) of Example 29.

$^1$H-NMR ($CDCl_3$) δ: 4.72-4.66 (2H, m), 4.21 (8H, t, J = 7.0 Hz), 4.16 (4H, t, J = 6.2 Hz), 2.84 (4H, t, J = 6.4 Hz), 2.73 (8H, t, J = 6.8 Hz), 2.69-2.65 (2H, m), 2.57-2.49 (14H, m), 2.36-2.32 (8H, m), 1.75-1.54 (24H, m), 1.42-1.23 (24H, m), 1.01 (6H, t, J = 7.2 Hz), 0.91-0.87 (12H, m).

MS m/z (M + H): 1242.
clogP: 17.722

[Example 44]

(1)

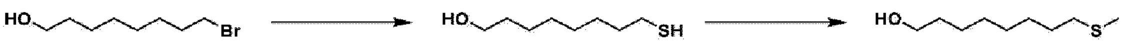

A 70% aqueous sodium hydrogen sulfide solution (13.4 g) was added to a N-methylpyrrolidone solution (25 mL) of 8-bromooctan-1-ol (5.0 g) under ice cooling, and the mixture was stirred at room temperature for 1 hour. Water (100 mL), ethyl acetate (50 mL), and hexane (50 mL) were added to the reaction mixture, and the organic layer was separated and washed with water (100 mL) and a saturated aqueous sodium chloride solution. The organic layer was dried over anhydrous sodium sulfate, and the solvent was distilled off under reduced pressure, thereby obtaining 8-mercaptooctan-1-ol (3.8 g).

$^1$H-NMR ($CDCl_3$) δ: 3.68-3.61 (2H, m), 2.53 (2H, dt, J = 7.6, 7.6 Hz), 1.71-1.53 (5H, m), 1.42-1.20 (9H, m).

Methyl p-toluenesulfonate (2.2 g) was added to a mixture of 8-mercaptooctan-1-ol (1.9 g), potassium hydroxide (0.72 g), and ethanol (60 mL), the mixture was stirred at room temperature for 1 hour, and then the solvent was distilled off under reduced pressure. Ethyl acetate (150 mL) and water (100 mL) were added to the obtained residue, and the organic layer was separated and washed with water (100 mL) and a saturated aqueous sodium chloride solution. The organic layer was dried over anhydrous sodium sulfate, and the solvent was distilled off under reduced pressure, thereby obtaining 8-(methylthio)octan-1-ol (1.3 g).

$^1$H-NMR ($CDCl_3$) δ: 3.67-3.62 (2H, m), 2.49 (2H, t, J = 7.4 Hz), 2.10 (3H, s), 1.63-1.53 (4H, m), 1.42-1.20 (9H, m).

(2)

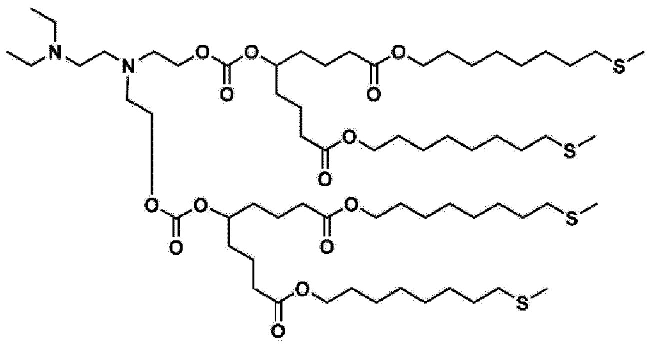

Bis(8-(methylthio)octyl)

11-(2-(diethylamino)ethyl)-5,17-bis(4-((8-(methylthio)octyl)oxy)-4-oxobutyl)-7,15-dioxo-6,8, 14,16-tetraoxa-11-azahenicosanedioate as a colorless oily substance was obtained by the same method as that in (2) and (3) of Example 29, except that 8-(methylthio)octan-1-ol was used instead of heptan-1-ol in (2) and (3) of Example 29.

$^1$H-NMR ($CDCl_3$) $\delta$: 4.72-4.66 (2H, m), 4.16 (4H, t, J = 6.4 Hz), 4.05 (8H, t, J = 6.6 Hz), 2.84 (4H, t, J = 6.4 Hz), 2.69-2.65 (2H, m), 2.55-2.46 (14H, m), 2.33-2.29 (8H, m), 2.10 (12H, s), 1.72-1.56 (32H, m), 1.42-1.31 (32H, m), 1.01 (6H, t, J = 7.0 Hz).

MS m/z (M + H): 1298.

clogP: 17.5084

The structures of the compounds of Comparative Example 1 and Comparative Example 2 are shown below.

Comparative Example 1:

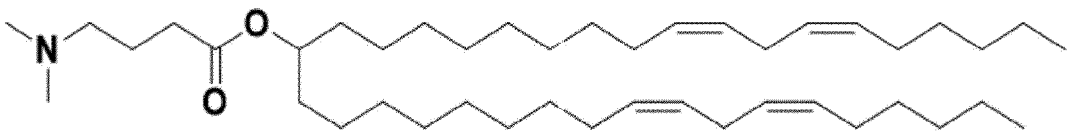

Comparative Example 2:

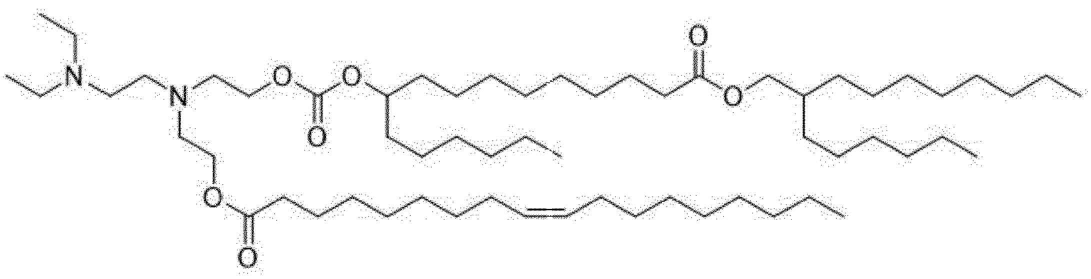

Test Example 1: Preparation of mRNA-encapsulating lipid particles and measurement of reporter protein expression rate in mice

<Preparation of EPO mRNA-encapsulating lipid particles>

The compounds described in Table 1, the neutral lipid, cholesterol (product name: Cholesterol HP; NIPPON FINE CHEMICAL CO., LTD.), and 1,2-dimyristoyl-rac-glycero-3-(methylpolyoxyethylene 2000) (hereinafter, called DMG-PEG2000) (product name: SUNBRIGHT(R) GM-020; NOF corporation) were dissolved in ethanol at a molar ratio in Table 1 such that the total lipid concentration became 20 mmol/L, thereby obtaining an oil phase.

As the neutral lipid, 1,2-distearoyl-sn-glycero-3-phosphocholine (product name: COATSOME (R) MC-8080;

NOF corporation) (hereinafter, DSPC), or L-α-dioleoyl phosphatidylethanolamine (Hereinafter, DOPE) (product name: COATSOME (R) ME-8181; NOF corporation), or 1,2-dioleoyl-sn-glycero-3-phosphocholine (hereinafter, DOPC) (product name: COATSOME (R) MC-8181; NOF corporation) was used.

EPO mRNA (product name: CleanCap EPO mRNA (5moU); TriLink) was diluted with 50 mmol/L citrate buffer having a pH 4 such that the weight ratio of total lipid concentration to mRNA concentration is about 16:1 to 64:1, thereby obtaining a water phase. Then, the water phase and the oil phase were mixed together using NanoAssemblr (Precision NanoSystems) such that the volume ratio of water phase:oil phase was 3:1, and the mixed solution was 1.5-fold diluted with phosphate buffered saline (PBS), thereby obtaining a dispersion liquid of mRNA lipid particles. The dispersion liquid was dialyzed with a 10% aqueous sucrose solution using a dialysis cassette (Slide-A-Lyzer G2, MWCO: 10 kD, Thermo Fisher Scientific) to remove ethanol, thereby obtaining EPO mRNA-encapsulating lipid particles.

<Measurement of particle size>

The particle size of the mRNA-encapsulating lipid particles was measured by 10-fold diluting the dispersion liquid of lipid particle with phosphate buffered saline (PBS) using Zeta-potential & Particle size Analyzer ELS-Z2 (Otsuka Electronics Co., Ltd.). The results are shown in Table 1.

<Evaluation of encapsulation rate of mRNA>

(Quantification of total mRNA concentration)

15 to 30 μL of a 3 mol/L aqueous sodium acetate solution and 4.5 to 9 μL of glycogen were added to 30 to 60 μL of the lipid particles retaining mRNA, and 0.75 to 1.5 mL of ethanol was added thereto, so that only the nucleic acids were precipitated while the lipid was dissolved. Then, the supernatant was removed by centrifugation. The precipitates were air-dried for 15 minutes or longer, then water was added thereto to redissolve the precipitates, and the concentration thereof was measured using Nanodrop NF1000 (Thermo Fisher Scientific), thereby quantifying the total mRNA concentration.

(Quantification of mRNA concentration in outer water phase)

The mRNA concentration was quantified using a Quant-iT RiboGreen RNA Assay Kit (Thermo Fisher Scientific) according to the protocol. First, a 20× TE buffer included in the above-described kit was diluted with water, thereby obtaining a 1× TE buffer. TE represents Tris/EDTA (ethylenediaminetetraacetic acid). In order to quantify only mRNA in the outer water phase, the dispersion liquid of lipid particles retaining mRNA was 10,000-fold diluted with the 1× TE buffer. 100 μL of the 10,000-fold diluted dispersion liquid of lipid particles was put into a 96-well plate, then 100 μL of a RiboGreen reagent (reagent included in the Quanti-iT Ribogreen RNA Assay Kit described above) 2,000-fold diluted with the 1× TE buffer was added to a sample, and fluorescence (excitation wavelength: 485 nm, fluorescence wavelength: 535 nm) was measured using a plate reader Infinite F200 (TECAN), thereby quantifying the mRNA concentration in the outer water phase.

(Calculation of encapsulation rate)

By using the quantification results of the total mRNA concentration and the mRNA concentration in the outer water phase that were obtained through the above steps, the mRNA encapsulation rate of the mRNA lipid particles was calculated according to the following Equation. The results are shown in Table 1.

mRNA encapsulation rate (%) = (total mRNA concentration-mRNA concentration in outer water phase)/total mRNA concentration × 100

<Measurement of EPO enzyme activity>

The dispersion liquid of mRNA lipid particles prepared in above-described <Preparation of EPO mRNA-encapsulating lipid particles> was tail-vein intravenously administered to C57BL/6J mice at a mRNA dosage of 0.1 mg/kg. 20 to 24 hours after the administration, blood was collected from the caudal vena cava, thereby obtaining blood plasma. The human EPO enzyme activity was quantified by using the obtained blood plasma and ab119522 Erythropoietin (EPO) Human Elisa Kit (Abcam.plc). The quantitative value is described as a relative amount of EPO protein in a case where Comparative Example 1 is 1.

The results are shown in Table 1.

[Table 1]

| Data of particle size, nucleic acid encapsulation rate, and quantification of EPO protein of nucleic acid-encapsulating lipid particles | | | | | | | | | |
|---|---|---|---|---|---|---|---|---|---|
| Compound used | Aminolipid composition (mol%) | Neutral lipid | Neutral lipid composition (mol%) | Cholesterol composition (mol%) | DMG-PEG2000 composition (mol%) | Lipid/nucleic acid (wt/wt) | Particle size (nm) | Nucleic acid encapsulation rate (%) | Relative amount of EPO protein (with respect to Comparative Example 1) |
| Comparative Example 1 | 50 | DSPC | 10 | 38.5 | 1.5 | 20 | 89.0 | 93.4 | 1 |
| Comparative Example 2 | 50 | DOPE | 10 | 38.5 | 1.5 | 64 | 88.8 | 100 | 0.74 |
| Example 3 | 50 | DOPE | 10 | 38.5 | 1.5 | 64 | 81.2 | 97.8 | 1.92 |
| Example 8 | 50 | DOPE | 10 | 38.5 | 1.5 | 64 | 81.3 | 98.0 | 2.27 |
| Example 10 | 50 | DOPE | 10 | 38.5 | 1.5 | 64 | 88.0 | 100.0 | 2.39 |
| Example 11 | 50 | DOPE | 10 | 38.5 | 1.5 | 64 | 73.2 | 98.6 | 2.70 |
| Example 13 | 50 | DOPE | 10 | 38.5 | 1.5 | 64 | 79.5 | 98.5 | 5.09 |
| Example 14 | 50 | DOPE | 10 | 38.5 | 1.5 | 64 | 75.9 | 98.8 | 3.85 |
| Example 15 | 50 | DOPE | 10 | 38.5 | 1.5 | 64 | 76.8 | 97.5 | 1.92 |
| Example 16 | 50 | DOPE | 10 | 38.5 | 1.5 | 64 | 59.4 | 97.3 | 2.53 |
| Example 17 | 50 | DOPE | 10 | 38.5 | 1.5 | 64 | 81.7 | 96.9 | 2.93 |
| Example 19 | 50 | DOPE | 10 | 38.5 | 1.5 | 64 | 147.0 | 100.0 | 1.26 |
| Example 20 | 50 | DOPE | 10 | 38.5 | 1.5 | 64 | 133.4 | 100.0 | 3.22 |
| Example 21 | 50 | DOPE | 10 | 38.5 | 1.5 | 64 | 109.4 | 99.9 | 4.66 |
| Example 22 | 50 | DOPE | 10 | 38.5 | 1.5 | 64 | 78.3 | 98.6 | 4.69 |
| Example 23 | 50 | DOPE | 10 | 38.5 | 1.5 | 64 | 76.8 | 97.5 | 3.60 |
| Example 24 | 50 | DOPE | 10 | 38.5 | 1.5 | 64 | 80.6 | 97.9 | 1.46 |
| Example 27 | 50 | DOPE | 10 | 38.5 | 1.5 | 64 | 101.5 | 99.8 | 3.60 |
| Example 28 | 45 | DOPE | 10 | 43.5 | 1.5 | 32 | 115.7 | 98.8 | 1.23 |
| Example 28 | 45 | - | - | 53.5 | 1.5 | 32 | 155.2 | 99.2 | 1.47 |
| Example 28 | 45 | DOPC | 10 | 43.5 | 1.5 | 32 | 82.1 | 99.2 | 3.54 |
| Example 29 | 50 | DOPE | 10 | 38.5 | 1.5 | 64 | 108.7 | 97.1 | 9.90 |

(continued)

| Data of particle size, nucleic acid encapsulation rate, and quantification of EPO protein of nucleic acid-encapsulating lipid particles | | | | | | | | | |
|---|---|---|---|---|---|---|---|---|---|
| Compound used | Aminolipid composition (mol%) | Neutral lipid | Neutral lipid composition (mol%) | Cholesterol composition (mol%) | DMG-PEG2000 composition (mol%) | Lipid/nucleic acid (wt/wt) | Particle size (nm) | Nucleic acid encapsulation rate (%) | Relative amount of EPO protein (with respect to Comparative Example 1) |
| Example 32 | 50 | DOPE | 10 | 38.5 | 1.5 | 64 | 165.3 | 95.5 | 4.03 |
| Example 33 | 50 | DOPE | 10 | 38.5 | 1.5 | 64 | 132.8 | 88.8 | 2.70 |
| Example 34 | 50 | DOPE | 10 | 38.5 | 1.5 | 64 | 99.2 | 100.0 | 1.20 |
| Example 37 | 50 | DOPE | 10 | 38.5 | 1.5 | 64 | 136.1 | 99.0 | 5.18 |
| Example 38 | 50 | DOPE | 10 | 38.5 | 1.5 | 64 | 101.4 | 100.0 | 2.13 |
| Example 39 | 50 | DOPE | 10 | 38.5 | 1.5 | 64 | 147.2 | 93.8 | 8.03 |
| Example 42 | 45 | DOPE | 10 | 43.5 | 1.5 | 64 | 128.2 | 98.9 | 4.28 |
| Example 43 | 45 | DOPE | 10 | 43.5 | 1.5 | 64 | 155.0 | 95.7 | 3.12 |
| Example 44 | 45 | DOPE | 10 | 43.5 | 1.5 | 64 | 201.9 | 81.2 | 1.39 |

In comparison with the nucleic acid lipid composition in Comparative Examples, the nucleic acid lipid composition according to the embodiment of the present invention showed a higher EPO protein expression rate.

Test Example 2: Preparation of mRNA-encapsulating lipid particles and measurement of reporter protein expression rate in mice

<Preparation of FLuc mRNA-encapsulating lipid particles>

The compounds described in Table 2, the neutral lipid, cholesterol (product name: Cholesterol HP; NIPPON FINE CHEMICAL CO., LTD.), and 1,2-dimyristoyl-rac-glycero-3-(methylpolyoxyethylene 2000) (hereinafter, called DMG-PEG2000) were dissolved in ethanol at a molar ratio in Table 2 such that the total lipid concentration became 20 mmol/L, thereby obtaining an oil phase.

As the neutral lipid, 1,2-distearoyl-sn-glycero-3-phosphocholine (product name: COATSOME (R) MC-8080; NOF corporation) was used in a case of Comparative Example 1, and L-α-dioleoylphosphatidylethanolamine (product name: COATSOME (R) MC-8181; NOF corporation) was used in a case other than Comparative Example 1.

FLuc mRNA (product name: CleanCap FLuc mRNA (5 moU); TriLink) was diluted with 50 mmol/L citrate buffer having a pH 4 such that the weight ratio of total lipid concentration to mRNA concentration is about 19:1 to 64:1, obtaining a water phase. Then, the water phase and the oil phase were mixed together using NanoAssemblr (Precision NanoSystems) such that the volume ratio of water phase:oil phase was 3:1, and the mixed solution was 1.5-fold diluted with phosphate buffered saline (PBS), thereby obtaining a dispersion liquid of mRNA lipid particles. The dispersion liquid was dialyzed with a 10% aqueous sucrose solution using a dialysis cassette (Slide-A-Lyzer G2, MWCO: 10 kD, Thermo Fisher Scientific) to remove ethanol, thereby obtaining FLuc mRNA-encapsulating lipid particles.

<Measurement of particle size>

The particle size of the mRNA-encapsulating lipid particles was measured by 10-fold diluting the dispersion liquid of lipid particle with phosphate buffered saline (PBS) using Zeta-potential & Particle size Analyzer ELS-Z2 (Otsuka Electronics Co., Ltd.). The results are shown in Table 2.

<Evaluation of encapsulation rate of mRNA>

(Quantification of total mRNA concentration)

15 to 30 μL of a 3 mol/L aqueous sodium acetate solution and 4.5 to 9 μL of glycogen were added to 30 to 60 μL of the lipid particles retaining mRNA, and 0.75 to 1.5 mL of ethanol was added thereto, so that only the nucleic acids were precipitated while the lipid was dissolved. Then, the supernatant was removed by centrifugation. The precipitates were air-dried for 15 minutes or longer, then water was added thereto to redissolve the precipitates, and the concentration thereof was measured using Nanodrop NF1000 (Thermo Fisher Scientific), thereby quantifying the total mRNA concentration.

(Quantification of mRNA concentration in outer water phase)

The mRNA concentration was quantified using a Quant-iT RiboGreen RNA Assay Kit (Thermo Fisher Scientific) according to the protocol. First, a 20× TE buffer included in the above-described kit was diluted with water, thereby obtaining a 1× TE buffer. TE represents Tris/EDTA (ethylenediaminetetraacetic acid). In order to quantify only mRNA in the outer water phase, the dispersion liquid of lipid particles retaining mRNA was 10,000-fold diluted with the 1× TE buffer. 100 μL of the 10,000-fold diluted dispersion liquid of lipid particles was put into a 96-well plate, then 100 μL of a RiboGreen reagent (reagent included in the Quanti-iT Ribogreen RNA Assay Kit described above) 2,000-fold diluted with the 1× TE buffer was added to a sample, and fluorescence (excitation wavelength: 485 nm, fluorescence wavelength: 535 nm) was measured using a plate reader Infinite F200 (TECAN), thereby quantifying the mRNA concentration in the outer water phase.

(Calculation of encapsulation rate)

By using the quantification results of the total mRNA concentration and the mRNA concentration in the outer water phase that were obtained through the above steps, the mRNA encapsulation rate of the mRNA lipid particles was calculated according to the following Equation. The results are shown in Table 2.

mRNA encapsulation rate (%) = (total mRNA concentration-mRNA concentration in outer water phase)/total mRNA concentration × 100

<Luciferase luminescence measurement>

The dispersion liquid of mRNA lipid particles prepared in above <Preparation of FLuc mRNA-encapsulating lipid particles> was singly administered to the rectus femoris from the dorsal side of ICR mice at a mRNA dosage of 1 μg. 5 hours and 50 minutes after administration, D-luciferin potassium (Fujifilm Wako Pure Chemical Corporation) was intraperitoneally administered at a dosage of 150 mg/kg, and 6 hours after administration, luminescence was measured using IVIS Imaging System (PerkinElmer, Inc.) in the prone position under isoflurane gas anesthesia. The ROI was set such that the entire lower limbs on the administered side were included, and the amount of luminescence (Photons/Sec) was quantified using Living Image Software (PerkinElmer, Inc.). Luciferase [P/S] in Table 2 indicates photons/sec (light intensity).

The results are shown in Table 2.

[Table 2]

| Data of particle size, nucleic acid encapsulation rate, and amount of Luc luminescence of nucleic acid-encapsulating lipid particles | | | | | | | | | |
|---|---|---|---|---|---|---|---|---|---|
| Compound used | Aminolipid composition (mol%) | Neutral lipid | Neutral lipid composition (mol%) | Cholesterol composition (mol%) | DMG-PEG2 000 composition (mol%) | Lipid/nucleic acid (wt/wt) | Particle size (nm) | Nucleic acid encapsulation rate (%) | Amount of luminescence Luciferase [P/S] |
| Example 13 | 45 | DOPE | 10 | 43.5 | 1.5 | 32 | 83.4 | 99.1 | 4.38.E+08 |
| Example 21 | 45 | DOPE | 10 | 43.5 | 1.5 | 32 | 100.9 | 98.4 | 6.19.E+08 |
| Example 22 | 45 | DOPE | 10 | 43.5 | 1.5 | 32 | 95 | 98.7 | 4.23.E+08 |
| Example 28 | 45 | DOPE | 10 | 43.5 | 1.5 | 32 | 82.9 | 100.0 | 1.64.E+09 |
| Example 28 | 45 | DOPC | 10 | 43.5 | 1.5 | 32 | 104.3 | 99.1 | 6.54.E+08 |
| Example 28 | 45 | - | 10 | 53.5 | 1.5 | 32 | 164.6 | 99.1 | 1.86.E+08 |
| Example 29 | 45 | DOPE | 10 | 43.5 | 1.5 | 32 | 129 | 95.0 | 4.23.E+08 |
| Example 29 | 50 | DOPE | 10 | 38.5 | 1.5 | 64 | 95.1 | 100.0 | 8.48.E+08 |
| Example 29 | 40 | DOPE | 10 | 48.5 | 1.5 | 64 | 104.8 | 100.0 | 5.05.E+08 |
| Example 38 | 45 | DOPE | 10 | 43.5 | 1.5 | 32 | 116 | 99.7 | 5.98.E+08 |
| Example 39 | 45 | DOPE | 10 | 43.5 | 1.5 | 32 | 141.1 | 90.1 | 8.07.E+08 |
| Example 43 | 45 | DOPE | 10 | 43.5 | 1.5 | 32 | 144 | 100.0 | 1.10.E+09 |
| Example 44 | 45 | DOPE | 10 | 43.5 | 1.5 | 32 | 170.5 | 100.0 | 7.65.E+08 |

The nucleic acid lipid composition according to the embodiment of the present invention showed a higher reporter protein expression rate.

(PTEN antisense oligonucleotide information)

Phosphatase and Tensin Homolog Deleted from Chromosome 10 (PTEN) is an enzyme that catalyzes a dephosphorylation reaction of phosphatidylinositol 3,4,5-triphosphate, which is an inositol phospholipid.

An antisense oligonucleotide nucleic acid (PTEN ASO) for a PTEN protein was purchased from Hokkaido System Science Co., Ltd. PTEN ASO is an oligonucleotide consisting of 20 bases, in which nucleotides are connected by a phosphodiester bond, and the sequence of which is described below.

5(m)^t(m)^g(m)^5(m)^t(m)^a^g^5c^5c^t^5c^t^g^g^a^t(m)^t(m)^t(m)^g(m)^a(m)

Here, small letters (a, g, and t) represent adenine, guanine, and thymidine, respectively, and (m) represents 2'-MOE modification.

5(m) represents 2'-MOE 5-Me cytosine, t(m) represents 2'-MOE thymidine, g(m) represents 2'-MOE guanine, a(m) represents 2'-MOE adenine, 5c represents 5-methylcytosine and ^ represents phosphorothioate.

2'-MOE represents 2'-O-methoxyethyl.

(Preparation of PTEN ASO-LNP)

The first lipid, phospholipid, cholesterol, and polyethylene glycol lipid (PEG lipid) shown in Table 3 were dissolved in ethanol at the molar ratio shown in Table 3 such that the total lipid concentration was 20 mmol/L to obtain the oil phase.

As the neutral lipid, 1,2-Distearoyl-sn-glycero-3-phosphocholine (DSPC, product name: COATSOME MC-8080, NOF corporation) was used. As the cholesterol, cholesterol (product name: Cholesterol HP; Nippon Seika Co., Ltd.) was used. As the PEG lipid, monostearyl PEG (product name: Polyethylene Glycol Monostearate (4E.O.), FUJIFILM Wako Pure Chemical Corporation) was used.

The structure of monostearyl PEG (also referred to mono PEG) is shown below.

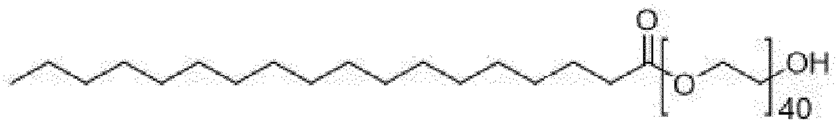

5 mg of PTEN ASO was dissolved in 1 mL of sterile water and diluted with a 10 mmol/L acetate buffer having a pH of 4 such that the nucleic acid concentration was 54.6 μmol/L, to obtain a water phase. Then the water phase and the oil phase were mixed together with a micromixer (see JP5288254B) using a syringe pump such that the volume ratio of water phase:oil phase was 3:1, and the mixed solution was two-fold diluted with a phosphate buffered physiological saline (PBS), thereby obtaining a dispersion of nucleic acid lipid particles.

Table 3 also shows the molar ratios of the first lipid, the phospholipid, the sterol, and the PEG lipid in the lipid composition, and the mass ratio of the nucleic acid to the total lipid at the time of mixing.

[Table 3]

| Particle size and nucleic acid encapsulation rate of nucleic acid-encapsulating lipid particles | | | | | | | | | |
|---|---|---|---|---|---|---|---|---|---|
| Compound used | Aminolipid composition (mol%) | Neutral lipid | PEG lipid | Neutral lipid composition (mol%) | Cholesterol composition (mol%) | PEG lipid composition (mol%) | Lipid/nucleic acid (wt/wt) | Particle size (nm) | Nucleic acid encapsulation rate (%) |
| Example 8 | 50 | DSPC | monostearyl PEG | 20 | 28.5 | 1.5 | 11.8 | 87.7 | 93 |
| Example 13 | 50 | DSPC | monostearyl PEG | 20 | 28.5 | 1.5 | 11.8 | 60.4 | 97 |
| Example 21 | 50 | DSPC | monostearyl PEG | 20 | 28.5 | 1.5 | 11.8 | 81.5 | 96 |
| Example 29 | 50 | DSPC | monostearyl PEG | 20 | 28.5 | 1.5 | 11.8 | 104.3 | 92 |
| Example 37 | 50 | DSPC | monostearyl PEG | 20 | 28.5 | 1.5 | 11.8 | 58.6 | 92 |

<Measurement of particle size>

The particle size and polydispersion index of the lipid particles were measured by 10-fold diluting the dispersion liquid of lipid particle with phosphate buffered physiological saline (PBS) using Zeta-potential & Particle size Analyzer ELS-Z2 (Otsuka Electronics Co., Ltd.). The measurement results are shown in Table 3.

<Evaluation of encapsulation rate of PTEN ASO>

(Quantification of total nucleic acid concentration)

30 μL of 3 mol/L aqueous sodium acetate solution and 9 μL of glycogen were added to 60 μL of the lipid particles retaining nucleic acids, and then 1.5 mL of ethanol was added thereto, so that only the nucleic acids were precipitated while the lipid was dissolved. Then, the supernatant was removed by centrifugation. The precipitates were air-dried for 15 minutes or longer, then water was added thereto to redissolve the precipitates, and the concentration thereof was measured using Nanodrop ND1000 (Thermo Fisher Scientific), thereby quantifying the total nucleic acid concentration.

(Quantification of nucleic acid concentration in outer water phase)

The mRNA concentration was quantified using a Quant-iT RiboGreen RNA Assay Kit (Thermo Fisher Scientific) according to the protocol. First, a 20× TE buffer included in the above-described kit was diluted with water, thereby obtaining a 1× TE buffer. TE represents Tris/EDTA (ethylenediaminetetraacetic acid). In order to quantify only the nucleic acid in the outer water phase, the dispersion liquid of lipid particles retaining nucleic acids was 10,000-fold diluted with the 1× TE buffer.

100 μL of the 10,000-fold diluted dispersion liquid of lipid particles was put into a 96-well plate, then 100 μL of a RiboGreen reagent (reagent included in the Quanti-iT Ribogreen RNA Assay Kit described above) 2,000-fold diluted with the 1× TE buffer was added to a sample, and fluorescence (excitation wavelength: 485 nm, fluorescence wavelength: 535 nm) was measured using a plate reader Infinite F200 (TECAN), thereby quantifying the nucleic acid concentration in the outer water phase.

(Calculation of encapsulation rate)

By using the quantification results of the total nucleic acid concentration and the nucleic acid concentration in the outer water phase that were obtained through the above steps, the nucleic acid encapsulation rate of the nucleic acid lipid particles was calculated according to the following Equation.

Nucleic acid encapsulation rate (%) = (total nucleic acid concentration - nucleic acid concentration in outer water phase)/total nucleic acid concentration × 100

The calculation results are shown in Table 3.

<Evaluation of PTEN mRNA knockdown in vitro>

(Cells used for evaluation)

In an in vitro test using A431 cells (American Type Culture Collection), E-MEM (gibco), fetal bovine serum (FBS) (gibco), Penicillin-streptomycin (gibco), and non-essential amino acid (NEAA) (FUJIFILM Wako Pure Chemical Corporation) were mixed at a ratio of 88:10:1:1 and the mixture was used as a culture medium.

In an in vitro test using SH-SY5Y cells (American Type Culture Collection), E-MEM (gibco), Ham's F12 (gibco), FBS (gibco), Penicillin-streptomycin (gibco), and NEAA (FUJIFILM Wako Pure Chemical Corporation) were mixed at a ratio of 41.5:41.5:15:1:1 and the mixture was used as a culture medium.

(Quantification of PTEN mRNA by PCR)

The measurement of PTEN protein mRNA was performed according to the protocol of TaqMan (register trademark) Fast Advanced Cells-to-CT™ Kit (Thermo Fisher Scientific). To A431 cells or SH-SY5Y cells, a dispersion liquid of nucleic acid lipid particles prepared to have a final concentration of 500 nmol/L as an ASO concentration, Naked ASO or PBS was added. After exposure for 24 hours under the control of 37°C and 5% $CO_2$, the culture supernatant was

removed, and the mixture was washed once with PBS at 4°C. After removing the PBS, a lysis solution was added at 50 μL/well, and the mixture was stood still for 5 minutes at room temperature to obtain a cell lysate. For the cell lysate, TaqMan (register trademark) Fast Advanced Cells-to-CT™ Kit (Thermo Fisher scientific), and Hs02621230_s1, FAM/MGB (Thermo Fisher Scientific) and Human GAPDH Endogenous Control (VIC (register trademark)/MGB, Thermo Fisher Scientific) as a PCR reaction reagent were used to perform reverse transcription and PCR reaction.

The PTEN mRNA level of each sample was calculated by the ΔΔCt method. Specifically, the Ct value of GAPDH is subtracted from the Ct value of PTEN to calculate the ΔCt value of each sample. The average value of the ΔCt values of the PBS treatment group was subtracted from the calculated ΔCt value to calculate the ΔΔCt value. For each ΔΔCt value, a relative value with respect to the Naked ASO to be compared was taken as a PTEN mRNA relative value. The calculation results are shown in Table 4.

[Table 4]

| PTEN knockdown relative value with respect to Naked ASO | |
|---|---|
| | SH-SY5Y cells |
| Example 8 | 0.65 |
| Example 13 | 0.34 |
| Example 21 | 0.33 |
| Example 29 | 0.80 |
| Example 37 | 0.83 |

## Claims

1. A compound represented by Formula (1) or a salt thereof,

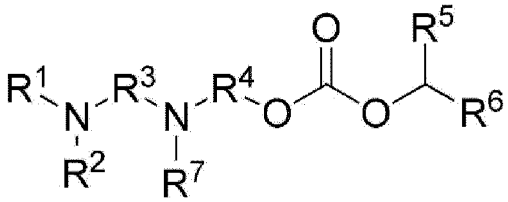

(1)

in the formula,
$R^1$ and $R^2$ each independently represent a hydrocarbon group having 1 to 18 carbon atoms, and $R^3$ represents a hydrocarbon group having 2 to 8 carbon atoms, where the hydrocarbon groups represented by $R^1$, $R^2$, and $R^3$ may be substituted with one or more substituents selected from -OH, -COOH, $-NR^{51}R^{52}$, $-OC(O)O-R^{53}$, $-C(O)O-R^{54}$, $-OC(O)-R^{55}$, and $-O-R^{56}$,
$R^4$ represents a hydrocarbon group having 1 to 8 carbon atoms,
$R^5$ and $R^6$ each independently represent a hydrocarbon group having 1 to 8 carbon atoms or $-R^8-L^1-R^9$, excluding a case that both $R^5$ and $R^6$ are hydrocarbon groups having 1 to 8 carbon atoms,
$R^7$ represents $-R^{10}-L^2-R^{11}-L^3-R^{12}$,
$R^{51}$ and $R^{52}$ each independently represent a hydrocarbon group having 1 to 8 carbon atoms,
$R^{53}$, $R^{54}$, $R^{55}$, and $R^{56}$ each independently represent a hydrocarbon group having 1 to 24 carbon atoms,
the hydrocarbon groups represented by $R^{53}$, $R^{54}$, $R^{55}$, and $R^{56}$ may be substituted with an aryl group having 6 to 20 carbon atoms or $-S-R^{58}$,
the aryl group having 6 to 20 carbon atoms may be substituted with -OH, -COOH, $-NR^{51}R^{52}$, $-OC(O)O-R^{53}$, $-C(O)O-R^{54}$, $-OC(O)-R^{55}$, $-O-R^{56}$, or -(hydrocarbon group having 1 to 12 carbon atoms)-$R^{57}$,
$R^{58}$ represents a hydrocarbon group having 1 to 12 carbon atoms,
$R^{57}$ represents -OH, -COOH, $-NR^{61}R^{62}$, $-OC(O)O-R^{63}$, $-C(O)O-R^{64}$, $-OC(O)-R^{65}$, or $-O-R^{66}$,
$R^{61}$ and $R^{62}$ each independently represent a hydrocarbon group having 1 to 8 carbon atoms,
$R^{63}$, $R^{64}$, $R^{65}$, and $R^{66}$ each independently represent a hydrocarbon group having 1 to 24 carbon atoms,
the hydrocarbon groups represented by $R^{63}$, $R^{64}$, $R^{65}$, and $R^{66}$ may be substituted with an aryl group having 6 to 20 carbon atoms or $-S-R^{68}$,

the aryl group having 6 to 20 carbon atoms may be substituted with -OH, -COOH, $-NR^{61}R^{62}$, $-OC(O)O-R^{63}$, $-C(O)O-R^{64}$, $-OC(O)-R^{65}$, $-O-R^{66}$, or -(hydrocarbon group having 1 to 12 carbon atoms)-$R^{67}$,
$R^{68}$ represents a hydrocarbon group having 1 to 12 carbon atoms,
$L^1$, $L^2$, and $L^3$ each independently represent -OC(O)O-, -C(O)O-, -OC(O)-, or -O-,
$R^8$ represents a hydrocarbon group having 1 to 12 carbon atoms,
$R^9$ represents a hydrocarbon group having 1 to 24 carbon atoms,
$R^{10}$ represents a hydrocarbon group having 1 to 8 carbon atoms,
$R^{11}$ represents a hydrocarbon group having 1 to 24 carbon atoms,
$R^{12}$ represents a hydrocarbon group having 1 to 24 carbon atoms,
the hydrocarbon groups represented by $R^9$ and $R^{12}$ may be substituted with an aryl group, $-OC(O)O-R^{53}$, $-C(O)O-R^{54}$, $-OC(O)-R^{55}$, or $-S-R^{58}$, where definitions of $R^{53}$, $R^{54}$, $R^{55}$, and $R^{58}$ are as described above, and
the hydrocarbon group represented by $R^{11}$ may be substituted with $-OC(O)O-R^{53}$ $-C(O)O-R^{54}$, or $-OC(O)-R^{55}$, where the definitions of $R^{53}$, $R^{54}$, and $R^{55}$ are as described above.

**2.** The compound or a salt thereof according to claim 1,

wherein $R^1$ and $R^2$ each independently represent a hydrocarbon group having 1 to 3 carbon atoms, and $R^3$ represents a hydrocarbon group having 2 to 4 carbon atoms, where the hydrocarbon groups represented by $R^1$, $R^2$, and $R^3$ may be substituted with -OH,
$R^4$ represents a hydrocarbon group having 1 to 8 carbon atoms,
$R^5$ and $R^6$ each independently represent a hydrocarbon group having 1 to 8 carbon atoms or $-R^8-L^1-R^9$, excluding a case that both $R^5$ and $R^6$ are hydrocarbon groups having 1 to 8 carbon atoms,
$R^7$ represents $-R^{10}-L^2-R^{11}-L^3-R^{12}$,
$L^1$ and $L^3$ each independently represent -C(O)O- or -OC(O)-,
$L^2$ represents -OC(O)O-, -C(O)O-, or -OC(O)-,
$R^8$ represents a hydrocarbon group having 1 to 8 carbon atoms,
$R^9$ represents a hydrocarbon group having 1 to 16 carbon atoms,
$R^{10}$ represents a hydrocarbon group having 1 to 8 carbon atoms,
$R^{11}$ represents a hydrocarbon group having 1 to 9 carbon atoms,
$R^{12}$ represents a hydrocarbon group having 1 to 16 carbon atoms,
the hydrocarbon groups represented by $R^9$ and $R^{12}$ may be substituted with an aryl group or $-S-R^{58}$,
$R^{58}$ represents a hydrocarbon group having 1 to 8 carbon atoms,
the hydrocarbon group represented by $R^{11}$ may be substituted with $-C(O)O-R^{55}$ or $-OC(O)-R^{56}$,
$R^{55}$ and $R^{56}$ each independently represent a hydrocarbon group having 1 to 16 carbon atoms, and
the hydrocarbon groups represented by $R^{55}$ and $R^{56}$ may be substituted with an aryl group having 6 to 20 carbon atoms or $-S-R^{58}$, and definition of $R^{58}$ is as described above.

**3.** The compound or a salt thereof according to claim 1, which is a compound represented by Formula (1-1) or a salt thereof,

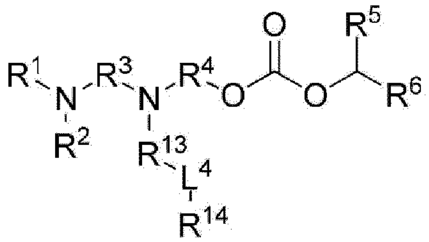

(1－1)

in the formula,
$R^1$ and $R^2$ each independently represent a hydrocarbon group having 1 to 18 carbon atoms, and $R^3$ represents a hydrocarbon group having 2 to 8 carbon atoms, where the hydrocarbon groups represented by $R^1$, $R^2$, and $R^3$ may be substituted with -OH, -COOH, $-NR^{51}R^{52}$, $-OC(O)O-R^{53}$, $-C(O)O-R^{54}$, $-OC(O)-R^{55}$, or $-O-R^{56}$,
$R^4$ represents a hydrocarbon group having 1 to 8 carbon atoms,
$R^5$ and $R^6$ each independently represent a hydrocarbon group having 1 to 8 carbon atoms or $-R^8-L^1-R^9$, excluding a case that both $R^5$ and $R^6$ are hydrocarbon groups having 1 to 8 carbon atoms,
$L^1$ represents -OC(O)O-, -C(O)O-, -OC(O)-, or -O-,

$R^8$ represents a hydrocarbon group having 1 to 12 carbon atoms,
$R^9$ represents a hydrocarbon group having 1 to 24 carbon atoms, where the hydrocarbon group represented by $R^9$ may be substituted with an aryl group, -OC(O)O-$R^{53}$, -C(O)O-$R^{54}$, -OC(O)-$R^{55}$, or -S-$R^{58}$,
$R^{51}$ and $R^{52}$ each independently represent a hydrocarbon group having 1 to 8 carbon atoms,
$R^{53}$, $R^{54}$, $R^{55}$, and $R^{56}$ each independently represent a hydrocarbon group having 1 to 24 carbon atoms,
the hydrocarbon groups represented by $R^{53}$, $R^{54}$, $R^{55}$, and $R^{56}$ may be substituted with an aryl group having 6 to 20 carbon atoms or -S-$R^{58}$,
the aryl group having 6 to 20 carbon atoms may be substituted with -OH, -COOH, -N$R^{51}R^{52}$, -OC(O)O-$R^{53}$, -C(O)O-$R^{54}$, -OC(O)-$R^{55}$, -O-$R^{56}$, or -(hydrocarbon group having 1 to 12 carbon atoms)-$R^{57}$,
$R^{58}$ represents a hydrocarbon group having 1 to 12 carbon atoms,
$R^{57}$ represents -OH, -COOH, -N$R^{51}R^{52}$, -OC(O)O-$R^{53}$, -C(O)O-$R^{54}$, -OC(O)-$R^{55}$, or -O-$R^{56}$,
$R^{13}$ represents a hydrocarbon group having 1 to 8 carbon atoms,
$R^{14}$ represents -$R^{15}$-$L^5$-$R^{16}$, where $R^{15}$ represents a hydrocarbon group having 1 to 24 carbon atoms, $L^5$ represents -OC(O)O-, -C(O)O-, -OC(O)-, or -O-, and $R^{16}$ represents a hydrocarbon group having 1 to 24 carbon atoms,
the hydrocarbon group having 1 to 24 carbon atoms represented by $R^{15}$ may be substituted with -OC(O)O-$R^{53}$, -C(O)O-$R^{54}$, or -OC(O)-$R^{55}$, where definitions of $R^{53}$, $R^{54}$, and $R^{55}$ are as described above, and
the hydrocarbon group having 1 to 24 carbon atoms represented by $R^{16}$ may be substituted with an aryl group having 6 to 20 carbon atoms, -OC(O)O-$R^{53}$, -C(O)O-$R^{54}$, -OC(O)-$R^{55}$ or -S-$R^{58}$, where the definitions of $R^{53}$, $R^{54}$, $R^{55}$, and $R^{58}$ are as described above.

**4.** The compound and a salt thereof according to claim 3,

wherein in Formula (1-1),
$R^1$ and $R^2$ each independently represent a hydrocarbon group having 1 to 3 carbon atoms, and $R^3$ represents a hydrocarbon group having 2 to 4 carbon atoms, where the hydrocarbon groups represented by $R^1$, $R^2$, and $R^3$ may be substituted with -OH,
$R^4$ represents a hydrocarbon group having 1 to 8 carbon atoms,
$R^5$ and $R^6$ each independently represent a hydrocarbon group having 1 to 8 carbon atoms or -$R^8$-$L^1$-$R^9$, excluding a case that both $R^5$ and $R^6$ are hydrocarbon groups having 1 to 8 carbon atoms,
$L^1$ represents -C(O)O- or -OC(O)-,
$R^8$ represents a hydrocarbon group having 1 to 8 carbon atoms,
$R^9$ represents a hydrocarbon group having 1 to 18 carbon atoms, and the hydrocarbon group represented by $R^9$ may be substituted with an aryl group having 6 to 20 carbon atoms or -S-$R^{58}$,
$R^{58}$ represents a hydrocarbon group having 1 to 8 carbon atoms,
$R^{13}$ represents a hydrocarbon group having 1 to 8 carbon atoms,
$R^{14}$ represents -$R^{15}$-$L^5$-$R^{16}$, where $R^{15}$ represents a hydrocarbon group having 1 to 18 carbon atoms, $L^5$ represents -OC(O)O-, and $R^{16}$ represents a hydrocarbon group having 1 to 18 carbon atoms,
the hydrocarbon group having 1 to 18 carbon atoms represented by $R^{15}$ may be substituted with -C(O)O-$R^{55}$ or -OC(O)-$R^{56}$,
$R^{55}$ and $R^{56}$ each independently represent a hydrocarbon group having 1 to 16 carbon atoms, and the hydrocarbon groups represented by $R^{55}$ and $R^{56}$ may be substituted with an aryl group having 6 to 20 carbon atoms or -S-$R^{58}$, where the definition of $R^{58}$ is as described above, and
the hydrocarbon group having 1 to 18 carbon atoms represented by $R^{16}$ may be substituted with an aryl group or -S-$R^{58}$, where the definition of $R^{58}$ is as described above.

**5.** The compound or a salt thereof according to claim 1, which is a compound represented by Formula (1-2) or a salt thereof,

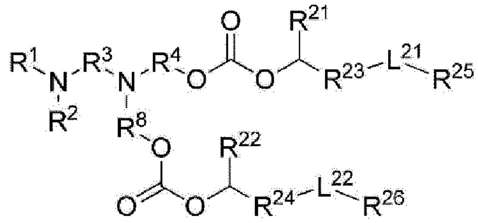

(1-2)

in the formula,
$R^1$ and $R^2$ each independently represent a hydrocarbon group having 1 to 18 carbon atoms, and $R^3$ represents a hydrocarbon group having 2 to 8 carbon atoms, where the hydrocarbon groups represented by $R^1$, $R^2$, and $R^3$ may be substituted with -OH, -COOH, -$NR^{51}R^{52}$, -OC(O)O-$R^{53}$, -C(O)O-$R^{54}$, -OC(O)-$R^{55}$, or -O-$R^{56}$,
$R^4$ and $R^8$ each independently represent a hydrocarbon group having 1 to 8 carbon atoms,
$R^{21}$ and $R^{22}$ each independently represent a hydrocarbon group having 1 to 18 carbon atoms,
$R^{23}$ and $R^{24}$ each independently represent a hydrocarbon group having 1 to 12 carbon atoms,
$R^{25}$ and $R^{26}$ each independently represent a hydrocarbon group having 1 to 24 carbon atoms,
$L^{21}$ and $L^{22}$ each independently represent -OC(O)O-, -C(O)O-, -OC(O)-, or -O-,
the hydrocarbon groups represented by $R^{25}$ and $R^{26}$ may be substituted with an aryl group having 6 to 20 carbon atoms, -OC(O)O-$R^{53}$, -C(O)O-$R^{54}$, -OC(O)-$R^{55}$, or -S-$R^{58}$,
$R^{51}$ and $R^{52}$ each independently represent a hydrocarbon group having 1 to 8 carbon atoms,
$R^{53}$, $R^{54}$, $R^{55}$, and $R^{56}$ each independently represent a hydrocarbon group having 1 to 18 carbon atoms,
the aryl group having 6 to 20 carbon atoms may be substituted with -OH, -COOH, -$NR^{51}R^{52}$, -OC(O)O-$R^{53}$, -C(O)O-$R^{54}$, -OC(O)-$R^{55}$, -O-$R^{56}$, or -(hydrocarbon group having 1 to 12 carbon atoms)-$R^{57}$,
$R^{57}$ represents -OH, -COOH, -$NR^{51}R^{52}$, -OC(O)O-$R^{53}$, -C(O)O-$R^{54}$, -OC(O)-$R^{55}$, or -O-$R^{56}$, and
$R^{58}$ represents a hydrocarbon group having 1 to 12 carbon atoms.

**6.** The compound and a salt thereof according to claim 5,

wherein in Formula (1-2),
$R^1$ and $R^2$ each independently represent a hydrocarbon group having 1 to 3 carbon atoms, where the hydrocarbon groups represented by $R^1$ and $R^2$ may be substituted with -OH,
$R^3$ represents a hydrocarbon group having 2 to 4 carbon atoms,
$R^4$ and $R^8$ each independently represent a hydrocarbon group having 1 to 8 carbon atoms,
$R^{21}$ and $R^{22}$ each independently represent a hydrocarbon group having 1 to 8 carbon atoms,
$R^{23}$ and $R^{24}$ each independently represent a hydrocarbon group having 1 to 8 carbon atoms,
$R^{25}$ and $R^{26}$ each independently represent a hydrocarbon group having 1 to 16 carbon atoms, and
$L^{21}$ and $L^{22}$ each independently represent -C(O)O- or -OC(O)-, or
wherein in Formula (1-2),
$R^1$ and $R^2$ each independently represent a hydrocarbon group having 1 to 3 carbon atoms,
$R^3$ represents a hydrocarbon group having 2 to 4 carbon atoms,
$R^4$ and $R^8$ each independently represent a hydrocarbon group having 1 to 8 carbon atoms,
$R^{21}$ and $R^{22}$ each independently represent a hydrocarbon group having 1 to 6 carbon atoms,
$R^{23}$ and $R^{24}$ each independently represent a hydrocarbon group having 1 to 8 carbon atoms,
$R^{25}$ and $R^{26}$ each independently represent a hydrocarbon group having 1 to 12 carbon atoms, and
$L^{21}$ and $L^{22}$ each independently represent -C(O)O- or -OC(O)-.

**7.** The compound or a salt thereof according to claim 1, which is a compound represented by Formula (1-3) or a salt thereof,

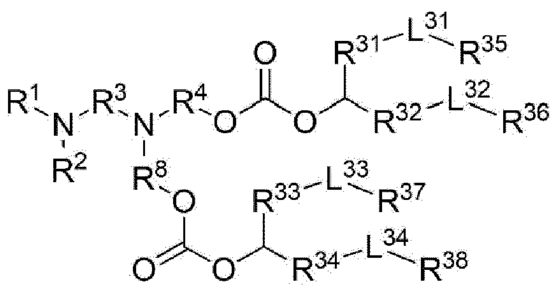

(1-3)

in the formula,
$R^1$ and $R^2$ each independently represent a hydrocarbon group having 1 to 18 carbon atoms, and $R^3$ represents a hydrocarbon group having 2 to 8 carbon atoms, where the hydrocarbon groups represented by $R^1$, $R^2$, and $R^3$ may be substituted with -OH, -COOH, -$NR^{51}R^{52}$, -OC(O)O-$R^{53}$, -C(O)O-$R^{54}$, -OC(O)-$R^{55}$, or -O-$R^{56}$,
$R^4$ and $R^8$ each independently represent a hydrocarbon group having 1 to 8 carbon atoms,
$R^{31}$, $R^{32}$, $R^{33}$, and $R^{34}$ each independently represent a hydrocarbon group having 1 to 12 carbon atoms,

$R^{35}$, $R^{36}$, $R^{37}$, and $R^{38}$ each independently represent a hydrocarbon group having 1 to 24 carbon atoms, $L^{31}$, $L^{32}$, $L^{33}$, and $L^{34}$ each independently represent -OC(O)O-, -C(O)O-, -OC(O)-, or -O-, the hydrocarbon groups represented by $R^{35}$, $R^{36}$, $R^{37}$, and $R^{38}$ may be substituted with an aryl group having 6 to 20 carbon atoms, -OC(O)O-$R^{53}$, -C(O)O-$R^{54}$, -OC(O)-$R^{55}$, or -S-$R^{58}$,
$R^{51}$ and $R^{52}$ each independently represent a hydrocarbon group having 1 to 8 carbon atoms,
$R^{53}$, $R^{54}$, $R^{55}$, and $R^{56}$ each independently represent a hydrocarbon group having 1 to 18 carbon atoms,
the aryl group having 6 to 20 carbon atoms may be substituted with -OH, -COOH, -N$R^{51}R^{52}$, -OC(O)O-$R^{53}$, -C(O)O-$R^{54}$, -OC(O)-$R^{55}$, -O-$R^{56}$, or -(hydrocarbon group having 1 to 12 carbon atoms)-$R^{57}$,
$R^{57}$ represents -OH, -COOH, -N$R^{51}R^{52}$, -OC(O)O-$R^{53}$, -C(O)O-$R^{54}$, -OC(O)-$R^{55}$, or -O-$R^{56}$, and
$R^{58}$ represents a hydrocarbon group having 1 to 12 carbon atoms.

**8.** The compound and a salt thereof according to claim 7,

wherein in Formula (1-3),
$R^1$ and $R^2$ each independently represent a hydrocarbon group having 1 to 3 carbon atoms, where the hydrocarbon groups represented by $R^1$ and $R^2$ may be substituted with -OH,
$R^3$ represents a hydrocarbon group having 2 to 4 carbon atoms,
$R^4$ and $R^8$ each independently represent a hydrocarbon group having 1 to 8 carbon atoms,
$R^{31}$, $R^{32}$, $R^{33}$, and $R^{34}$ each independently represent a hydrocarbon group having 1 to 8 carbon atoms,
$R^{35}$, $R^{36}$, $R^{37}$, and $R^{38}$ each independently represent a hydrocarbon group having 1 to 16 carbon atoms,
$L^{31}$, $L^{32}$, $L^{33}$, and $L^{34}$ each independently represent -C(O)O- or -OC(O)-,
the hydrocarbon groups represented by $R^{35}$, $R^{36}$, $R^{37}$, and $R^{38}$ may be substituted with an aryl group having 6 to 20 carbon atoms or -S-$R^{58}$, and
$R^{58}$ represents a hydrocarbon group having 1 to 8 carbon atoms, or
wherein in Formula (1-3),
$R^1$ and $R^2$ each independently represent a hydrocarbon group having 1 to 3 carbon atoms,
$R^3$ represents a hydrocarbon group having 2 to 4 carbon atoms,
$R^4$ and $R^8$ each independently represent a hydrocarbon group having 1 to 8 carbon atoms,
$R^{31}$, $R^{32}$, $R^{33}$, and $R^{34}$ each independently represent a hydrocarbon group having 1 to 3 carbon atoms,
$R^{35}$, $R^{36}$, $R^{37}$, and $R^{38}$ each independently represent a hydrocarbon group having 1 to 12 carbon atoms,
$L^{31}$, $L^{32}$, $L^{33}$, and $L^{34}$ each independently represent -C(O)O- or -OC(O)-,
the hydrocarbon groups represented by $R^{35}$, $R^{36}$, $R^{37}$, and $R^{38}$ may be substituted with -S-$R^{58}$, and
$R^{58}$ represents a hydrocarbon group having 1 to 8 carbon atoms, or
wherein in Formula (1-3),
$R^1$ and $R^2$ each independently represent a hydrocarbon group having 1 to 3 carbon atoms,
$R^3$ represents a hydrocarbon group having 2 to 4 carbon atoms,
$R^4$ and $R^8$ each independently represent a hydrocarbon group having 1 to 8 carbon atoms,
$R^{31}$, $R^{32}$, $R^{33}$, and $R^{34}$ each independently represent a hydrocarbon group having 1 to 3 carbon atoms,
$R^{35}$, $R^{36}$, $R^{37}$, and $R^{38}$ each independently represent a hydrocarbon group having 1 to 12 carbon atoms substituted with -S-$R^{58}$, where $R^{58}$ represents a hydrocarbon group having 1 to 8 carbon atoms, and
$L^{31}$, $L^{32}$, $L^{33}$, and $L^{34}$ each independently represent -C(O)O- or -OC(O)-, or
wherein in Formula (1-3),
$R^1$ and $R^2$ each independently represent a hydrocarbon group having 1 to 3 carbon atoms,
$R^3$ represents a hydrocarbon group having 2 to 4 carbon atoms,
$R^4$ and $R^8$ each independently represent a hydrocarbon group having 1 to 8 carbon atoms,
$R^{31}$, $R^{32}$, $R^{33}$, and $R^{34}$ each independently represent a hydrocarbon group having 1 to 3 carbon atoms,
$R^{35}$, $R^{36}$, $R^{37}$, and $R^{38}$ each independently represent a hydrocarbon group having 1 to 12 carbon atoms, and
$L^{31}$, $L^{32}$, $L^{33}$, and $L^{34}$ each independently represent -C(O)O- or -OC(O)-.

**9.** The compound or a salt thereof according to claim 1, wherein the compound is selected from the following formulae, or salts thereof,

Bis(2-butyloctyl) 16-(3-(diethylamino)propyl)-10,22-dihexyl-12,20-dioxo-11,13,19,21-tetraoxa-16-azahentriacontanedioate:

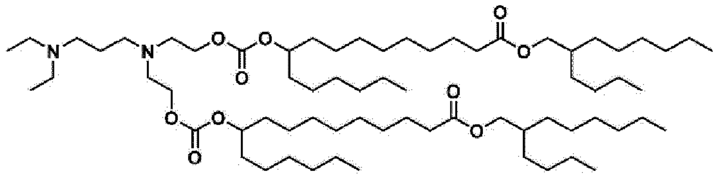

Bis(2-butyloctyl)

11-(2-(diethylamino)ethyl)-5,17-dihexyl-7,15-dioxo-6,8,14,16-tetraoxa-11-azahenicosanedioat e:

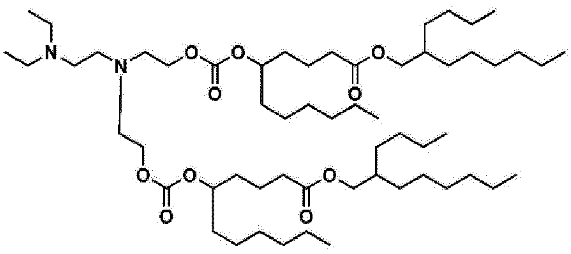

Bis(2-pentylheptyl)

11-(2-(diethylamino)ethyl)-5,17-dihexyl-7,15-dioxo-6,8,14,16-tetraoxa-11-azahenicosanedioat e:

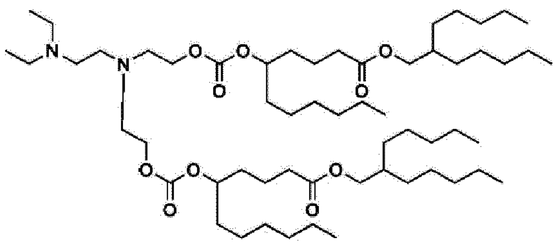

Bis(2-pentylheptyl)

11-(2-(dimethylamino)ethyl)-5,17-dihexyl-7,15-dioxo-6,8,14,16-tetraoxa-11-azahenicosanedio ate:

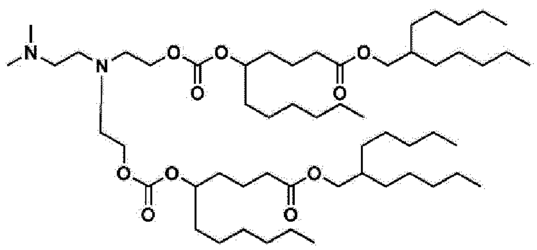

Bis(2-pentylheptyl)

11-(3-(diethylamino)propyl)-5,17-dihexyl-7,15-dioxo-6,8,14,16-tetraoxa-11-azahenicosanedio ate:

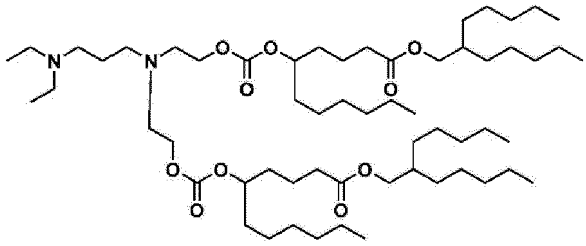

Bis(2-pentylheptyl)

11-(4-(diethylamino)butyl)-5,17-dihexyl-7,15-dioxo-6,8,14,16-tetraoxa-11-azahenicosanedioat e:

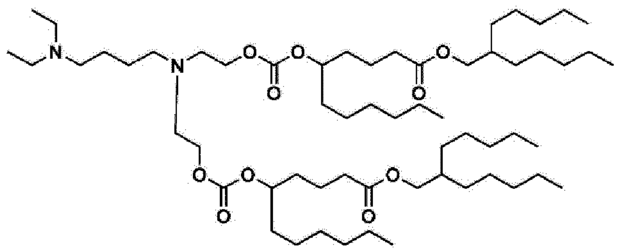

Bis(2-pentylheptyl)

12-(2-(diethylamino)ethyl)-5,19-dihexyl-7,17-dioxo-6,8,16,18-tetraoxa-12-azatricosanedioate:

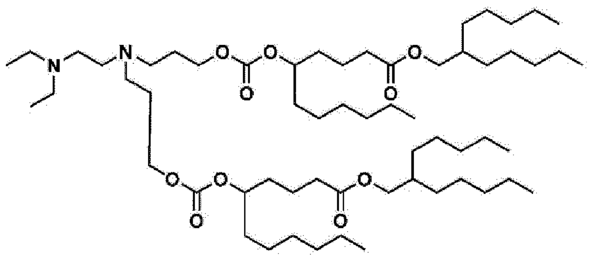

Bis(2-pentylheptyl)

13-(2-(diethylamino)ethyl)-5,21-dihexyl-7,19-dioxo-6,8,18,20-tetraoxa-13-azapentacosanedio ate:

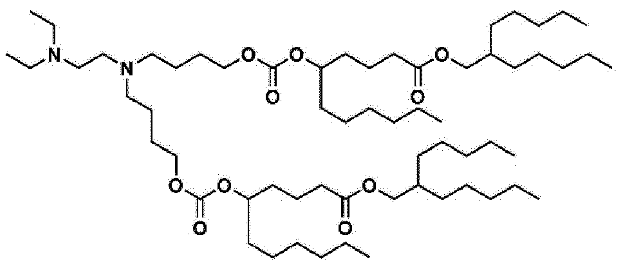

Bis(2-pentylheptyl)

10-(2-(diethylamino)ethyl)-4,16-dihexyl-6,14-dioxo-5,7,13,15-tetraoxa-10-azanonadecanedioa te:

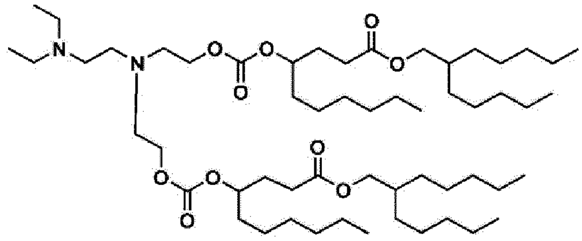

Bis(2-pentylheptyl)

11-(2-(diethylamino)ethyl)-5,17-dimethyl-7,15-dioxo-6,8,14,16-tetraoxa-11-azahenicosanedio ate:

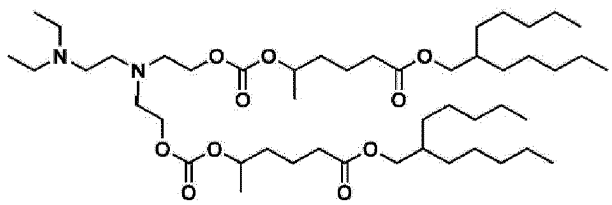

Bis(2-pentylheptyl)

11-(2-(diethylamino)ethyl)-5,17-diethyl-7,15-dioxo-6,8,14,16-tetraoxa-11-azahenicosanedioat e:

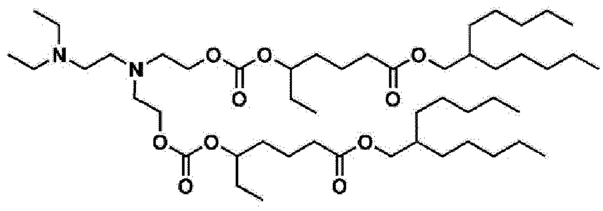

Bis(2-pentylheptyl)

11-(2-(diethylamino)ethyl)-7,15-dioxo-5,17-dipropyl-6,8,14,16-tetraoxa-11-azahenicosanedioa te:

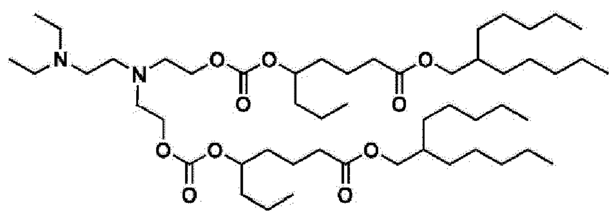

Bis(2-pentylheptyl)

5,17-dibutyl-11-(2-(diethylamino)ethyl)-7,15-dioxo-6,8,14,16-tetraoxa-11-azahenicosanedioat e:

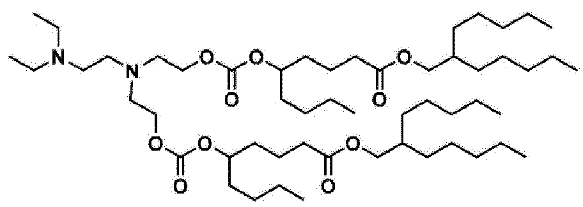

Bis(2-pentylheptyl)

11-(2-(diethylamino)ethyl)-7,15-dioxo-5,17-dipentyl-6,8,14,16-tetraoxa-11-azahenicosanedioa te:

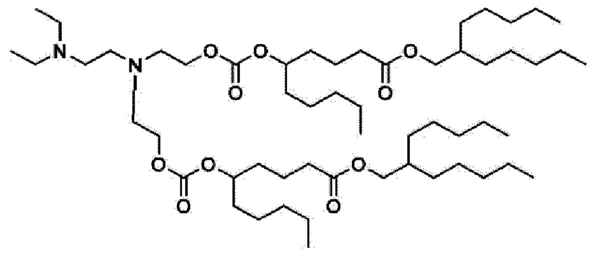

Bis(2-pentylheptyl)

11-(2-(diethylamino)ethyl)-5,17-diheptyl-7,15-dioxo-6,8,14,16-tetraoxa-11-azahenicosanedioa te:

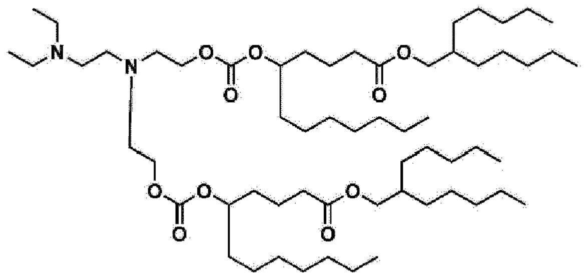

Bis

(2-(((3r,5r,7r)-adamantan-1-yl)ethyl)11-(2-(diethylamino)ethyl)-5,17-dihexyl-7,15-dioxo-6,8,1 4,16-tetraoxa-11-azahenicosanedioate;

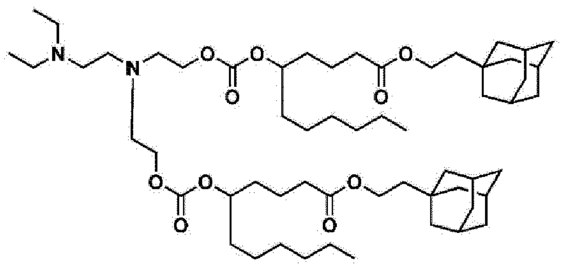

Bis(2-pentylheptyl)

11-(3-(diethylamino)propyl)-7,15-dioxo-5,17-dipropyl-6,8,14,16-tetraoxa-11-azahenicosanedi oate:

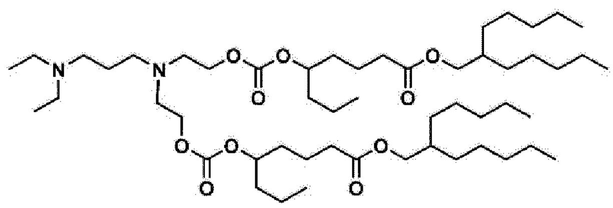

Diheptyl

11-(2-(diethylamino)ethyl)-5,17-bis(4-(heptyloxy)-4-oxobutyl)-7,15-dioxo-6,8,14, 16-tetraoxa-11-azahenicosanedioate:

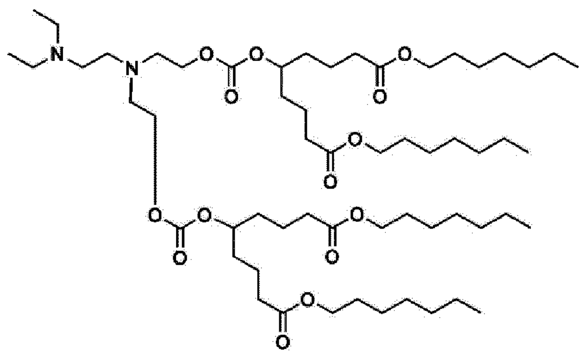

Dihexyl

11-(2-(diethylamino)ethyl)-5,17-bis(4-(hexyloxy)-4-oxobutyl)-7,15-dioxo-6,8,14,16-tetraoxa-11-azahenicosanedioate:

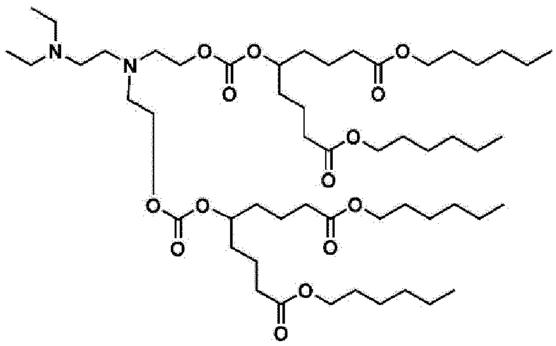

Dioctyl

11-(2-(diethylamino)ethyl)-5,17-bis(4-(octyloxy)-4-oxobutyl)-7,15-dioxo-6,8,14,16-tetraoxa-1 1-azahenicosanedioate:

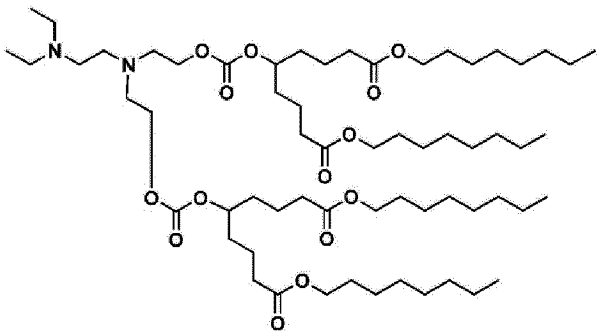

Dinonyl

11-(2-(diethylamino)ethyl)-5,17-bis(4-(nonyloxy)-4-oxobutyl)-7,15-dioxo-6,8,14,16-tetraoxa-11-azahenicosanedioate:

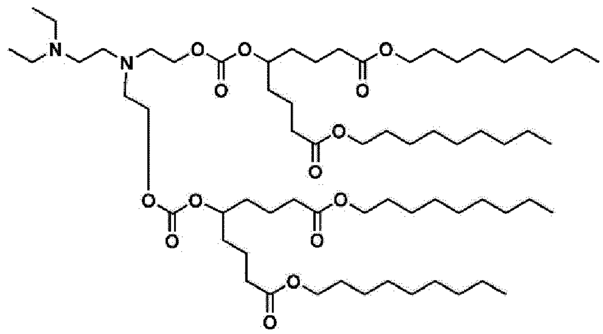

1-Heptyl 21-hexyl

11-(2-(diethylamino)ethyl)-5-(4-(heptyloxy)-4-oxobutyl)-17-(4-(hexyloxy)-4-oxobutyl)-7,15-d ioxo-6,8,14,16-tetraoxa-11-azahenicosanedioate:

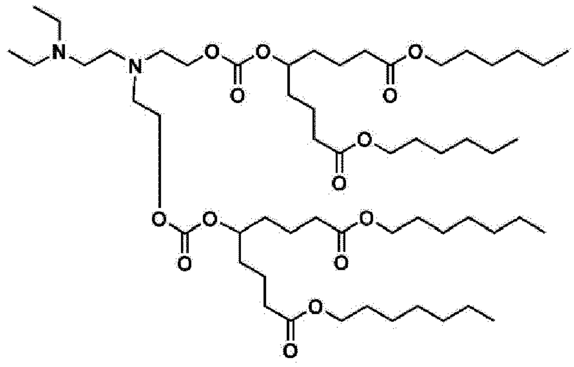

Diheptyl

11-(3-(diethylamino)propyl)-5,17-bis(4-(heptyloxy)-4-oxobutyl)-7,15-dioxo-6,8,14,16-tetraox a-11-azahenicosanedioate:

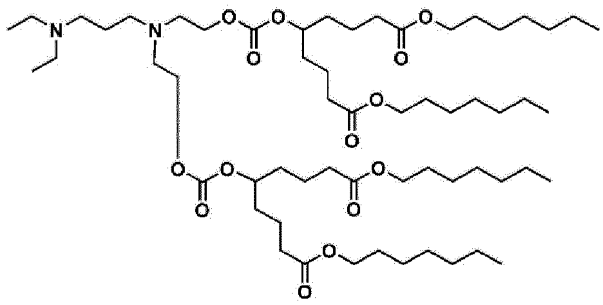

Diheptyl

10-(2-(diethylamino)ethyl)-4,16-bis(3-(heptyloxy)-3-oxopropyl)-6,14-dioxo-5,7,13,15-tetraox a-10-azanonadecanedioate:

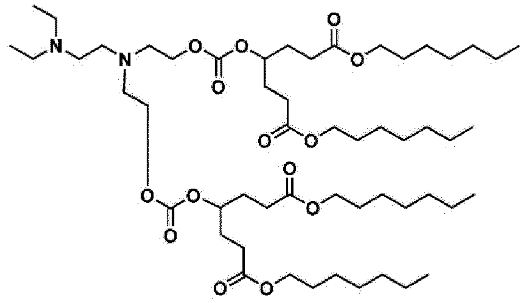

1-Hexyl 21-octyl

11-(2-(diethylamino)ethyl)-5-(4-(hexyloxy)-4-oxobutyl)-17-(4-(octyloxy)-4-oxobutyl)-7,15-di oxo-6,8,14,16-tetraoxa-11-azahenicosanedioate:

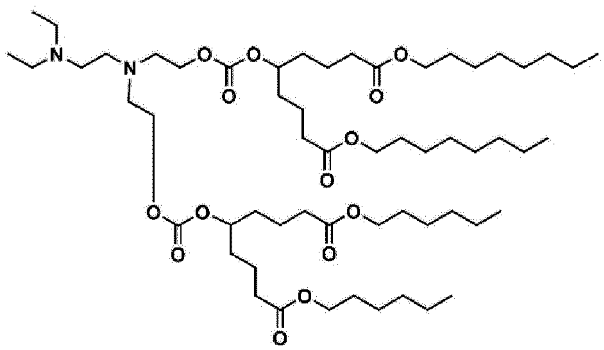

Bis(2-(hexylthio)ethyl)

11-(2-(diethylamino)ethyl)-5,17-bis(4-(2-(hexylthio)ethoxy)-4-oxobutyl)-7,15-dioxo-6,8,14,16 -tetraoxa-11-azahenicosanedioate:

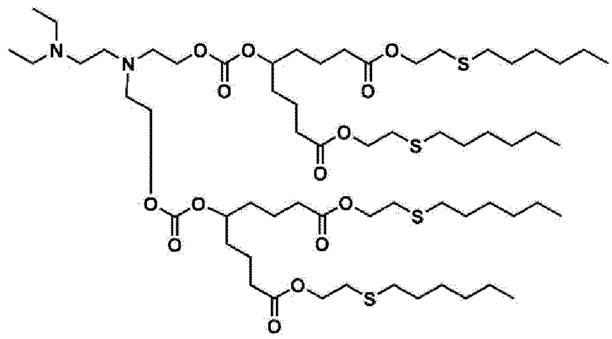

Bis(8-(methylthio)octyl)

11-(2-(diethylamino)ethyl)-5,17-bis(4-((8-(methylthio)octyl)oxy)-4-oxobutyl)-7,15-dioxo-6,8, 14,16-tetraoxa-11-azahenicosanedioate:

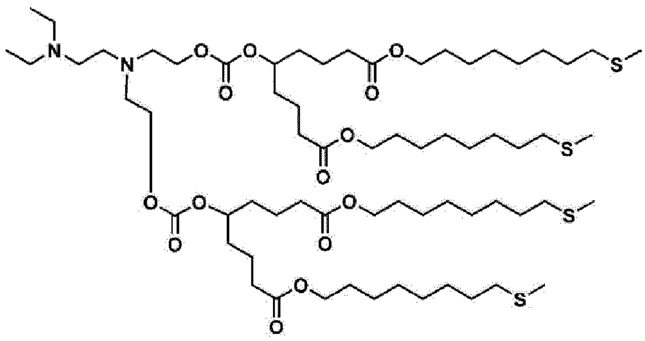

.

**10.** Lipid particles comprising:

the compound or a salt thereof according to any one of claims 1 to 9; and
a lipid.

**11.** The lipid particles according to claim 10,
wherein the lipid is at least one kind of lipid selected from the group consisting of a sterol and a lipid having a nonionic hydrophilic polymer chain.

**12.** The lipid particles according to claim 10 or 11, further comprising:
a neutral lipid.

**13.** The lipid particles according to any one of claims 10 to 12, further comprising:
a nucleic acid.

**14.** The lipid particles according to claim 13,
wherein the nucleic acid includes a nucleic acid having 50 or more bases.

**15.** A pharmaceutical composition comprising:
the lipid particles according to any one of claims 10 to 14 as an active ingredient.

## Patentansprüche

**1.** Verbindung, die durch Formel (1) dargestellt wird, oder Salz davon,

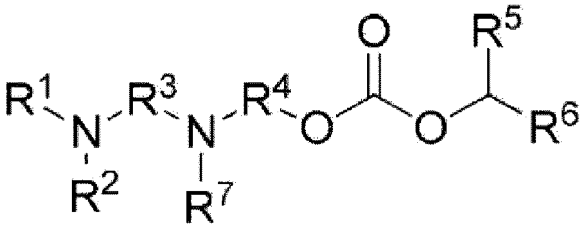

(1)

wobei in der Formel
$R^1$ und $R^2$ jeweils unabhängig voneinander eine Kohlenwasserstoffgruppe mit 1 bis 18 Kohlenstoffatomen darstellen und $R^3$ eine Kohlenwasserstoffgruppe mit 2 bis 8 Kohlenstoffatomen darstellt, wobei die durch $R^1$, $R^2$ und $R^3$ dargestellten Kohlenwasserstoffgruppen mit einem oder mehr Substituenten, die aus -OH, -COOH, -$NR^{51}R^{52}$, -OC(O)O-$R^{53}$, -C(O)O-$R^{54}$, -OC(O)-$R^{55}$ und -O-$R^{56}$ ausgewählt werden, substituiert sein können,
$R^4$ eine Kohlenwasserstoffgruppe mit 1 bis 8 Kohlenstoffatomen darstellt,
$R^5$ und $R^6$ jeweils unabhängig voneinander eine Kohlenwasserstoffgruppe mit 1 bis 8 Kohlenstoffatomen oder -$R^8$-$L^1$-$R^9$ darstellen, mit Ausnahme eines Falls, in dem sowohl $R^5$ als auch $R^6$ Kohlenwasserstoffgruppen mit 1 bis 8 Kohlenstoffatomen sind,
$R^7$ -$R^{10}$-$L^2$-$R^{11}$-$L^3$-$R^{12}$ darstellt,
$R^{51}$ und $R^{52}$ jeweils unabhängig voneinander eine Kohlenwasserstoffgruppe mit 1 bis 8 Kohlenstoffatomen

darstellen,
$R^{53}$, $R^{54}$, $R^{55}$ und $R^{56}$ jeweils unabhängig voneinander eine Kohlenwasserstoffgruppe mit 1 bis 24 Kohlenstoffatomen darstellen,
die durch $R^{53}$, $R^{54}$, $R^{55}$ und $R^{56}$ dargestellten Kohlenwasserstoffgruppen mit einer Arylgruppe mit 6 bis 20 Kohlenstoffatomen oder -S-$R^{58}$ substituiert sein können,
die Arylgruppe mit 6 bis 20 Kohlenstoffatomen mit -OH, -COOH, -N$R^{51}R^{52}$, -OC(O)O-$R^{53}$, -C(O)O-$R^{54}$, -OC(O)-$R^{55}$, -O-$R^{56}$ oder -(Kohlenwasserstoffgruppe mit 1 bis 12 Kohlenstoffatomen)-$R^{57}$ substituiert sein kann,
$R^{58}$ eine Kohlenwasserstoffgruppe mit 1 bis 12 Kohlenstoffatomen darstellt,
$R^{57}$ -OH, -COOH, -N$R^{61}R^{62}$, -OC(O)O-$R^{63}$, -C(O)O-$R^{64}$, -OC(O)-$R^{65}$ oder -O-$R^{66}$ darstellt,
$R^{61}$ und $R^{62}$ jeweils unabhängig voneinander eine Kohlenwasserstoffgruppe mit 1 bis 8 Kohlenstoffatomen darstellen,
$R^{63}$, $R^{64}$, $R^{65}$ und $R^{66}$ jeweils unabhängig voneinander eine Kohlenwasserstoffgruppe mit 1 bis 24 Kohlenstoffatomen darstellen,
die durch $R^{63}$, $R^{64}$, $R^{65}$ und $R^{66}$ dargestellten Kohlenwasserstoffgruppen mit einer Arylgruppe mit 6 bis 20 Kohlenstoffatomen oder -S-$R^{68}$ substituiert sein können,
die Arylgruppe mit 6 bis 20 Kohlenstoffatomen mit -OH, -COOH, -N$R^{61}R^{62}$, -OC(O)O-$R^{63}$, -C(O)O-$R^{64}$, -OC(O)-$R^{65}$, -O-$R^{66}$ oder -(Kohlenwasserstoffgruppe mit 1 bis 12 Kohlenstoffatomen)-$R^{67}$ substituiert sein kann,
$R^{68}$ eine Kohlenwasserstoffgruppe mit 1 bis 12 Kohlenstoffatomen darstellt, $L^1$, $L^2$ und $L^3$ jeweils unabhängig voneinander -OC(O)O-, -C(O)O-, -OC(O)- oder -O-darstellen,
$R^8$ eine Kohlenwasserstoffgruppe mit 1 bis 12 Kohlenstoffatomen darstellt,
$R^9$ eine Kohlenwasserstoffgruppe mit 1 bis 24 Kohlenstoffatomen darstellt,
$R^{10}$ eine Kohlenwasserstoffgruppe mit 1 bis 8 Kohlenstoffatomen darstellt,
$R^{11}$ eine Kohlenwasserstoffgruppe mit 1 bis 24 Kohlenstoffatomen darstellt,
$R^{12}$ eine Kohlenwasserstoffgruppe mit 1 bis 24 Kohlenstoffatomen darstellt,
die durch $R^9$ und $R^{12}$ dargestellten Kohlenwasserstoffgruppen mit einer Arylgruppe, - OC(O)O-$R^{53}$, -C(O)O-$R^{54}$, -OC(O)-$R^{55}$ oder -S-$R^{58}$ substituiert sein können, wobei Definitionen von $R^{53}$, $R^{54}$, $R^{55}$ und $R^{58}$ wie oben beschrieben sind, und
die durch $R^{11}$ dargestellte Kohlenwasserstoffgruppe mit -OC(O)O-$R^{53}$, -C(O)O-$R^{54}$, - OC(O)-$R^{55}$ substituiert sein kann, wobei die Definitionen von $R^{53}$, $R^{54}$ und $R^{55}$ wie oben beschrieben sind.

**2.** Verbindung oder Salz davon nach Anspruch 1,

wobei $R^1$ und $R^2$ jeweils unabhängig voneinander eine Kohlenwasserstoffgruppe mit 1 bis 3 Kohlenstoffatomen darstellen und $R^3$ eine Kohlenwasserstoffgruppe mit 2 bis 4 Kohlenstoffatomen darstellt, wobei die durch $R^1$, $R^2$ und $R^3$ dargestellten Kohlenwasserstoffgruppen mit -OH substituiert sein können,
$R^4$ eine Kohlenwasserstoffgruppe mit 1 bis 8 Kohlenstoffatomen darstellt,
$R^5$ und $R^6$ jeweils unabhängig voneinander eine Kohlenwasserstoffgruppe mit 1 bis 8 Kohlenstoffatomen oder -$R^8$-$L^1$-$R^9$ darstellen, mit Ausnahme eines Falls, in dem sowohl $R^5$ als auch $R^6$ Kohlenwasserstoffgruppen mit 1 bis 8 Kohlenstoffatomen sind, $R^7$ -$R^{10}$-$L^2$-$R^{11}$-$L^3$-$R^{12}$ darstellt,
$L^1$ und $L^3$ jeweils unabhängig voneinander -C(O)O- oder -OC(O)- darstellen,
$L^2$ -OC(O)O-, -C(O)O- oder -OC(O)- darstellt,
$R^8$ eine Kohlenwasserstoffgruppe mit 1 bis 8 Kohlenstoffatomen darstellt,
$R^9$ eine Kohlenwasserstoffgruppe mit 1 bis 16 Kohlenstoffatomen darstellt,
$R^{10}$ eine Kohlenwasserstoffgruppe mit 1 bis 8 Kohlenstoffatomen darstellt,
$R^{11}$ eine Kohlenwasserstoffgruppe mit 1 bis 9 Kohlenstoffatomen darstellt,
$R^{12}$ eine Kohlenwasserstoffgruppe mit 1 bis 16 Kohlenstoffatomen darstellt,
die durch $R^9$ und $R^{12}$ dargestellten Kohlenwasserstoffgruppen mit einer Arylgruppe oder -S-$R^{58}$ substituiert sein können,
$R^{58}$ eine Kohlenwasserstoffgruppe mit 1 bis 8 Kohlenstoffatomen darstellt,
die durch $R^{11}$ dargestellte Kohlenwasserstoffgruppe mit -C(O)O-$R^{55}$ oder -OC(O)-$R^{56}$ substituiert sein kann,
$R^{55}$ und $R^{56}$ jeweils unabhängig voneinander eine Kohlenwasserstoffgruppe mit 1 bis 16 Kohlenstoffatomen darstellen, und
die durch $R^{55}$ und $R^{56}$ dargestellten Kohlenwasserstoffgruppen mit einer Arylgruppe mit 6 bis 20 Kohlenstoffatomen oder -S-$R^{58}$ substituiert sein können, und Definition von $R^{58}$ wie oben beschrieben ist.

**3.** Verbindung oder Salz davon nach Anspruch 1, die eine Verbindung, die durch Formel (1-1) dargestellt wird, oder ein Salz davon ist,

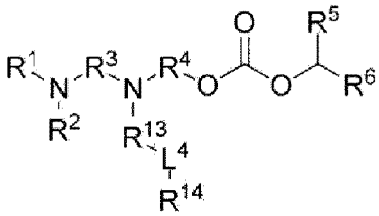

(1 − 1)

wobei in der Formel

$R^1$ und $R^2$ jeweils unabhängig voneinander eine Kohlenwasserstoffgruppe mit 1 bis 18 Kohlenstoffatomen darstellen und $R^3$ eine Kohlenwasserstoffgruppe mit 2 bis 8 Kohlenstoffatomen darstellt, wobei die durch $R^1$, $R^2$ und $R^3$ dargestellten Kohlenwasserstoffgruppen mit -OH, -COOH, $-NR^{51}R^{52}$, $-OC(O)O-R^{53}$, $-C(O)O-R^{54}$, $-OC(O)-R^{55}$ oder $-O-R^{56}$ substituiert sein können,

$R^4$ eine Kohlenwasserstoffgruppe mit 1 bis 8 Kohlenstoffatomen darstellt,

$R^5$ und $R^6$ jeweils unabhängig voneinander eine Kohlenwasserstoffgruppe mit 1 bis 8 Kohlenstoffatomen oder $-R^8-L^1-R^9$ darstellen, mit Ausnahme eines Falls, in dem sowohl $R^5$ als auch $R^6$ Kohlenwasserstoffgruppen mit 1 bis 8 Kohlenstoffatomen sind,

$L^1$ -OC(O)O-, -C(O)O-, -OC(O)- oder -O- darstellt,

$R^8$ eine Kohlenwasserstoffgruppe mit 1 bis 12 Kohlenstoffatomen darstellt,

$R^9$ eine Kohlenwasserstoffgruppe mit 1 bis 24 Kohlenstoffatomen darstellt, wobei die durch $R^9$ dargestellte Kohlenwasserstoffgruppe mit einer Arylgruppe, $-OC(O)O-R^{53}$, $- C(O)O-R^{54}$, $-OC(O)-R^{55}$ oder $-S-R^{58}$ substituiert sein kann,

$R^{51}$ und $R^{52}$ jeweils unabhängig voneinander eine Kohlenwasserstoffgruppe mit 1 bis 8 Kohlenstoffatomen darstellen,

$R^{53}$, $R^{54}$, $R^{55}$ und $R^{56}$ jeweils unabhängig voneinander eine Kohlenwasserstoffgruppe mit 1 bis 24 Kohlenstoffatomen darstellen,

die durch $R^{53}$, $R^{54}$, $R^{55}$ und $R^{56}$ dargestellten Kohlenwasserstoffgruppen mit einer Arylgruppe mit 6 bis 20 Kohlenstoffatomen oder $-S-R^{58}$ substituiert sein können,

die Arylgruppe mit 6 bis 20 Kohlenstoffatomen mit -OH, -COOH, $-NR^{51}R^{52}$, $-OC(O)O-R^{53}$, $-C(O)O-R^{54}$, $-OC(O)-R^{55}$, $-O-R^{56}$ oder -(Kohlenwasserstoffgruppe mit 1 bis 12 Kohlenstoffatomen)-$R^{57}$ substituiert sein kann,

$R^{58}$ eine Kohlenwasserstoffgruppe mit 1 bis 12 Kohlenstoffatomen darstellt,

$R^{57}$ -OH, -COOH, $-NR^{51}R^{52}$, $-OC(O)O-R^{53}$, $-C(O)O-R^{54}$, $-OC(O)-R^{55}$ oder $-O-R^{56}$ darstellt,

$R^{13}$ eine Kohlenwasserstoffgruppe mit 1 bis 8 Kohlenstoffatomen darstellt,

$R^{14}$ $-R^{15}-L^5-R^{16}$ darstellt, wobei $R^{15}$ eine Kohlenwasserstoffgruppe mit 1 bis 24 Kohlenstoffatomen darstellt, $L^5$ -OC(O)O-, -C(O)O-, -OC(O)- oder -O- darstellt und $R^{16}$ eine Kohlenwasserstoffgruppe mit 1 bis 24 Kohlenstoffatomen darstellt,

die durch $R^{15}$ dargestellte Kohlenwasserstoffgruppe mit 1 bis 24 Kohlenstoffatomen mit $-OC(O)O-R^{53}$, $-C(O)O-R^{54}$ oder $-OC(O)-R^{55}$ substituiert sein kann, wobei Definitionen von $R^{53}$, $R^{54}$ und $R^{55}$ wie oben beschrieben sind, und

die durch $R^{16}$ dargestellte Kohlenwasserstoffgruppe mit 1 bis 24 Kohlenstoffatomen mit einer Arylgruppe mit 6 bis 20 Kohlenstoffatomen, $-OC(O)O-R^{53}$, $-C(O)O-R^{54}$, $- OC(O)-R^{55}$ oder $-S-R^{58}$ substituiert sein kann, wobei die Definitionen von $R^{53}$, $R^{54}$, $R^{55}$ und $R^{58}$ wie oben beschrieben sind.

**4.** Verbindung und Salz davon nach Anspruch 3,

wobei in Formel (1-1)

$R^1$ und $R^2$ jeweils unabhängig voneinander eine Kohlenwasserstoffgruppe mit 1 bis 3 Kohlenstoffatomen darstellen und $R^3$ eine Kohlenwasserstoffgruppe mit 2 bis 4 Kohlenstoffatomen darstellt, wobei die durch $R^1$, $R^2$ und $R^3$ dargestellten Kohlenwasserstoffgruppen mit -OH substituiert sein können,

$R^4$ eine Kohlenwasserstoffgruppe mit 1 bis 8 Kohlenstoffatomen darstellt,

$R^5$ und $R^6$ jeweils unabhängig voneinander eine Kohlenwasserstoffgruppe mit 1 bis 8 Kohlenstoffatomen oder $-R^8-L^1-R^9$ darstellen, mit Ausnahme eines Falls, in dem sowohl $R^5$ als auch $R^6$ Kohlenwasserstoffgruppen mit 1 bis 8 Kohlenstoffatomen sind, $L^1$ -C(O)O- oder -OC(O)- darstellt,

$R^8$ eine Kohlenwasserstoffgruppe mit 1 bis 8 Kohlenstoffatomen darstellt,

$R^9$ eine Kohlenwasserstoffgruppe mit 1 bis 18 Kohlenstoffatomen darstellt und die durch $R^9$ dargestellte Kohlenwasserstoffgruppe mit einer Arylgruppe mit 6 bis 20 Kohlenstoffatomen oder $-S-R^{58}$ substituiert sein kann,

$R^{58}$ eine Kohlenwasserstoffgruppe mit 1 bis 8 Kohlenstoffatomen darstellt,

$R^{13}$ eine Kohlenwasserstoffgruppe mit 1 bis 8 Kohlenstoffatomen darstellt,
$R^{14}$ -$R^{15}$-$L^5$-$R^{16}$ darstellt, wobei $R^{15}$ eine Kohlenwasserstoffgruppe mit 1 bis 18 Kohlenstoffatomen darstellt, $L^5$ -OC(O)O- darstellt und $R^{16}$ eine Kohlenwasserstoffgruppe mit 1 bis 18 Kohlenstoffatomen darstellt,
die durch $R^{15}$ dargestellte Kohlenwasserstoffgruppe mit 1 bis 18 Kohlenstoffatomen mit -C(O)O-$R^{55}$ oder -OC(O)-$R^{56}$ substituiert sein kann,
$R^{55}$ und $R^{56}$ jeweils unabhängig voneinander eine Kohlenwasserstoffgruppe mit 1 bis 16 Kohlenstoffatomen darstellen und die durch $R^{55}$ und $R^{56}$ dargestellten Kohlenwasserstoffgruppen mit einer Arylgruppe mit 6 bis 20 Kohlenstoffatomen oder - S-$R^{58}$ substituiert sein können, wobei die Definition von $R^{58}$ wie oben beschrieben ist, und
die durch $R^{16}$ dargestellte Kohlenwasserstoffgruppe mit 1 bis 18 Kohlenstoffatomen mit einer Arylgruppe oder -S-$R^{58}$ substituiert sein kann, wobei die Definition von $R^{58}$ wie oben beschrieben ist.

**5.** Verbindung oder Salz davon nach Anspruch 1, die eine Verbindung, die durch Formel (1-2) dargestellt wird, oder ein Salz davon ist,

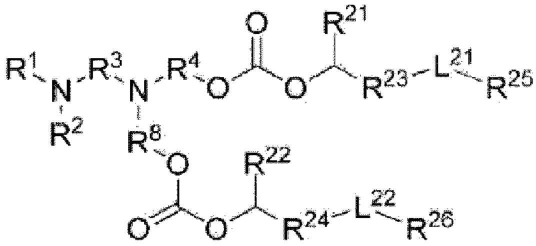

(1 − 2)

wobei in der Formel
$R^1$ und $R^2$ jeweils unabhängig voneinander eine Kohlenwasserstoffgruppe mit 1 bis 18 Kohlenstoffatomen darstellen und $R^3$ eine Kohlenwasserstoffgruppe mit 2 bis 8 Kohlenstoffatomen darstellt, wobei die durch $R^1$, $R^2$ und $R^3$ dargestellten Kohlenwasserstoffgruppen mit -OH, -COOH, -$NR^{51}R^{52}$, -OC(O)O-$R^{53}$, -C(O)O-$R^{54}$, -OC(O)-$R^{55}$ oder -O-$R^{56}$ substituiert sein können,
$R^4$ und $R^8$ jeweils unabhängig voneinander eine Kohlenwasserstoffgruppe mit 1 bis 8 Kohlenstoffatomen darstellen,
$R^{21}$ und $R^{22}$ jeweils unabhängig voneinander eine Kohlenwasserstoffgruppe mit 1 bis 18 Kohlenstoffatomen darstellen,
$R^{23}$ und $R^{24}$ jeweils unabhängig voneinander eine Kohlenwasserstoffgruppe mit 1 bis 12 Kohlenstoffatomen darstellen,
$R^{25}$ und $R^{26}$ jeweils unabhängig voneinander eine Kohlenwasserstoffgruppe mit 1 bis 24 Kohlenstoffatomen darstellen,
$L^{21}$ und $L^{22}$ jeweils unabhängig voneinander -OC(O)O-, -C(O)O-, -OC(O)- oder -O-darstellen,
die durch $R^{25}$ und $R^{26}$ dargestellten Kohlenwasserstoffgruppen mit einer Arylgruppe mit 6 bis 20 Kohlenstoffatomen, -OC(O)O-$R^{53}$, -C(O)O-$R^{54}$, -OC(O)-$R^{55}$ oder -S-$R^{58}$ substituiert sein können,
$R^{51}$ und $R^{52}$ jeweils unabhängig voneinander eine Kohlenwasserstoffgruppe mit 1 bis 8 Kohlenstoffatomen darstellen,
$R^{53}$, $R^{54}$, $R^{55}$ und $R^{56}$ jeweils unabhängig voneinander eine Kohlenwasserstoffgruppe mit 1 bis 18 Kohlenstoffatomen darstellen,
die Arylgruppe mit 6 bis 20 Kohlenstoffatomen mit -OH, -COOH, -$NR^{51}R^{52}$, -OC(O)O-$R^{53}$, -C(O)O-$R^{54}$, -OC(O)-$R^{55}$, -O-$R^{56}$ oder -(Kohlenwasserstoffgruppe mit 1 bis 12 Kohlenstoffatomen)-$R^{57}$ substituiert sein kann,
$R^{57}$ -OH, -COOH, -$NR^{51}R^{52}$, -OC(O)O-$R^{53}$, -C(O)O-$R^{54}$, -OC(O)-$R^{55}$ oder -O-$R^{56}$ darstellt, und
$R^{58}$ eine Kohlenwasserstoffgruppe mit 1 bis 12 Kohlenstoffatomen darstellt.

**6.** Verbindung und Salz davon nach Anspruch 5,

wobei in Formel (1-2)
$R^1$ und $R^2$ jeweils unabhängig voneinander eine Kohlenwasserstoffgruppe mit 1 bis 3 Kohlenstoffatomen darstellen, wobei die durch $R^1$ und $R^2$ dargestellten Kohlenwasserstoffgruppen mit -OH substituiert sein können,
$R^3$ eine Kohlenwasserstoffgruppe mit 2 bis 4 Kohlenstoffatomen darstellt,
$R^4$ und $R^8$ jeweils unabhängig voneinander eine Kohlenwasserstoffgruppe mit 1 bis 8 Kohlenstoffatomen darstellen,

$R^{21}$ und $R^{22}$ jeweils unabhängig voneinander eine Kohlenwasserstoffgruppe mit 1 bis 8 Kohlenstoffatomen darstellen,
$R^{23}$ und $R^{24}$ jeweils unabhängig voneinander eine Kohlenwasserstoffgruppe mit 1 bis 8 Kohlenstoffatomen darstellen,
$R^{25}$ und $R^{26}$ jeweils unabhängig voneinander eine Kohlenwasserstoffgruppe mit 1 bis 16 Kohlenstoffatomen darstellen, und
$L^{21}$ und $L^{22}$ jeweils unabhängig voneinander -C(O)O- oder -OC(O)- darstellen, oder wobei in Formel (1-2)
$R^1$ und $R^2$ jeweils unabhängig voneinander eine Kohlenwasserstoffgruppe mit 1 bis 3 Kohlenstoffatomen darstellen,
$R^3$ eine Kohlenwasserstoffgruppe mit 2 bis 4 Kohlenstoffatomen darstellt,
$R^4$ und $R^8$ jeweils unabhängig voneinander eine Kohlenwasserstoffgruppe mit 1 bis 8 Kohlenstoffatomen darstellen,
$R^{21}$ und $R^{22}$ jeweils unabhängig voneinander eine Kohlenwasserstoffgruppe mit 1 bis 6 Kohlenstoffatomen darstellen,
$R^{23}$ und $R^{24}$ jeweils unabhängig voneinander eine Kohlenwasserstoffgruppe mit 1 bis 8 Kohlenstoffatomen darstellen,
$R^{25}$ und $R^{26}$ jeweils unabhängig voneinander eine Kohlenwasserstoffgruppe mit 1 bis 12 Kohlenstoffatomen darstellen, und
$L^{21}$ und $L^{22}$ jeweils unabhängig voneinander -C(O)O- oder -OC(O)- darstellen.

**7.** Verbindung oder Salz davon nach Anspruch 1, die eine Verbindung, die durch Formel (1-3) dargestellt wird, oder ein Salz davon ist,

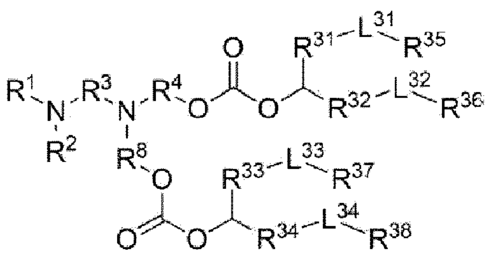
(1 − 3)

wobei in der Formel
$R^1$ und $R^2$ jeweils unabhängig voneinander eine Kohlenwasserstoffgruppe mit 1 bis 18 Kohlenstoffatomen darstellen und $R^3$ eine Kohlenwasserstoffgruppe mit 2 bis 8 Kohlenstoffatomen darstellt, wobei die durch $R^1$, $R^2$ und $R^3$ dargestellten Kohlenwasserstoffgruppen mit -OH, -COOH, -$NR^{51}R^{52}$, -OC(O)O-$R^{53}$, -C(O)O-$R^{54}$, -OC(O)-$R^{55}$ oder -O-$R^{56}$ substituiert sein können,
$R^4$ und $R^8$ jeweils unabhängig voneinander eine Kohlenwasserstoffgruppe mit 1 bis 8 Kohlenstoffatomen darstellen,
$R^{31}$, $R^{32}$, $R^{33}$ und $R^{34}$ jeweils unabhängig voneinander eine Kohlenwasserstoffgruppe mit 1 bis 12 Kohlenstoffatomen darstellen,
$R^{35}$, $R^{36}$, $R^{37}$ und $R^{38}$ jeweils unabhängig voneinander eine Kohlenwasserstoffgruppe mit 1 bis 24 Kohlenstoffatomen darstellen,
$L^{31}$, $L^{32}$, $L^{33}$ und $L^{34}$ jeweils unabhängig voneinander -OC(O)O-, -C(O)O-, -OC(O)- oder -O- darstellen,
die durch $R^{35}$, $R^{36}$, $R^{37}$ und $R^{38}$ dargestellten Kohlenwasserstoffgruppen mit einer Arylgruppe mit 6 bis 20 Kohlenstoffatomen, -OC(O)O-$R^{53}$, -C(O)O-$R^{54}$, -OC(O)-$R^{55}$ oder -S-$R^{58}$ substituiert sein können,
$R^{51}$ und $R^{52}$ jeweils unabhängig voneinander eine Kohlenwasserstoffgruppe mit 1 bis 8 Kohlenstoffatomen darstellen,
$R^{53}$, $R^{54}$, $R^{55}$ und $R^{56}$ jeweils unabhängig voneinander eine Kohlenwasserstoffgruppe mit 1 bis 18 Kohlenstoffatomen darstellen,
die Arylgruppe mit 6 bis 20 Kohlenstoffatomen mit -OH, -COOH, -$NR^{51}R^{52}$, -OC(O)O-$R^{53}$, -C(O)O-$R^{54}$, -OC(O)-$R^{55}$, -O-$R^{56}$ oder -(Kohlenwasserstoffgruppe mit 1 bis 12 Kohlenstoffatomen)-$R^{57}$ substituiert sein kann,
$R^{57}$-OH, -COOH, -$NR^{51}R^{52}$, -OC(O)O-$R^{53}$, -C(O)O-$R^{54}$, -OC(O)-$R^{55}$ oder -O-$R^{56}$ darstellt, und
$R^{58}$ eine Kohlenwasserstoffgruppe mit 1 bis 12 Kohlenstoffatomen darstellt.

**8.** Verbindung und Salz davon nach Anspruch 7,

wobei in Formel (1-3)
$R^1$ und $R^2$ jeweils unabhängig voneinander eine Kohlenwasserstoffgruppe mit 1 bis 3 Kohlenstoffatomen darstellen, wobei die durch $R^1$ und $R^2$ dargestellten Kohlenwasserstoffgruppen mit -OH substituiert sein können,
$R^3$ eine Kohlenwasserstoffgruppe mit 2 bis 4 Kohlenstoffatomen darstellt,
$R^4$ und $R^8$ jeweils unabhängig voneinander eine Kohlenwasserstoffgruppe mit 1 bis 8 Kohlenstoffatomen darstellen,
$R^{31}$, $R^{32}$, $R^{33}$ und $R^{34}$ jeweils unabhängig voneinander eine Kohlenwasserstoffgruppe mit 1 bis 8 Kohlenstoffatomen darstellen,
$R^{35}$, $R^{36}$, $R^{37}$ und $R^{38}$ jeweils unabhängig voneinander eine Kohlenwasserstoffgruppe mit 1 bis 16 Kohlenstoffatomen darstellen,
$L^{31}$, $L^{32}$, $L^{33}$ und $L^{34}$ jeweils unabhängig voneinander -C(O)O- oder -OC(O)- darstellen, die durch $R^{35}$, $R^{36}$, $R^{37}$ und $R^{38}$ dargestellten Kohlenwasserstoffgruppen mit einer Arylgruppe mit 6 bis 20 Kohlenstoffatomen oder -S-$R^{58}$ substituiert sein können, und
$R^{58}$ eine Kohlenwasserstoffgruppe mit 1 bis 8 Kohlenstoffatomen darstellt, oder wobei in Formel (1-3)
$R^1$ und $R^2$ jeweils unabhängig voneinander eine Kohlenwasserstoffgruppe mit 1 bis 3 Kohlenstoffatomen darstellen,
$R^3$ eine Kohlenwasserstoffgruppe mit 2 bis 4 Kohlenstoffatomen darstellt,
$R^4$ und $R^8$ jeweils unabhängig voneinander eine Kohlenwasserstoffgruppe mit 1 bis 8 Kohlenstoffatomen darstellen,
$R^{31}$, $R^{32}$, $R^{33}$ und $R^{34}$ jeweils unabhängig voneinander eine Kohlenwasserstoffgruppe mit 1 bis 3 Kohlenstoffatomen darstellen,
$R^{35}$, $R^{36}$, $R^{37}$ und $R^{38}$ jeweils unabhängig voneinander eine Kohlenwasserstoffgruppe mit 1 bis 12 Kohlenstoffatomen darstellen,
$L^{31}$, $L^{32}$, $L^{33}$ und $L^{34}$ jeweils unabhängig voneinander -C(O)O- oder -OC(O)- darstellen, die durch $R^{35}$, $R^{36}$, $R^{37}$ und $R^{38}$ dargestellten Kohlenwasserstoffgruppen mit -S-$R^{58}$ substituiert sein können, und
$R^{58}$ eine Kohlenwasserstoffgruppe mit 1 bis 8 Kohlenstoffatomen darstellt, oder wobei in Formel (1-3)
$R^1$ und $R^2$ jeweils unabhängig voneinander eine Kohlenwasserstoffgruppe mit 1 bis 3 Kohlenstoffatomen darstellen,
$R^3$ eine Kohlenwasserstoffgruppe mit 2 bis 4 Kohlenstoffatomen darstellt,
$R^4$ und $R^8$ jeweils unabhängig voneinander eine Kohlenwasserstoffgruppe mit 1 bis 8 Kohlenstoffatomen darstellen,
$R^{31}$, $R^{32}$, $R^{33}$ und $R^{34}$ jeweils unabhängig voneinander eine Kohlenwasserstoffgruppe mit 1 bis 3 Kohlenstoffatomen darstellen,
$R^{35}$, $R^{36}$, $R^{37}$ und $R^{38}$ jeweils unabhängig voneinander eine Kohlenwasserstoffgruppe mit 1 bis 12 Kohlenstoffatomen, die mit -S-$R^{58}$ substituiert ist, darstellen, wobei $R^{58}$ eine Kohlenwasserstoffgruppe mit 1 bis 8 Kohlenstoffatomen darstellt, und
$L^{31}$, $L^{32}$, $L^{33}$ und $L^{34}$ jeweils unabhängig voneinander -C(O)O- oder -OC(O)- darstellen, oder
wobei in Formel (1-3)
$R^1$ und $R^2$ jeweils unabhängig voneinander eine Kohlenwasserstoffgruppe mit 1 bis 3 Kohlenstoffatomen darstellen,
$R^3$ eine Kohlenwasserstoffgruppe mit 2 bis 4 Kohlenstoffatomen darstellt,
$R^4$ und $R^8$ jeweils unabhängig voneinander eine Kohlenwasserstoffgruppe mit 1 bis 8 Kohlenstoffatomen darstellen,
$R^{31}$, $R^{32}$, $R^{33}$ und $R^{34}$ jeweils unabhängig voneinander eine Kohlenwasserstoffgruppe mit 1 bis 3 Kohlenstoffatomen darstellen,
$R^{35}$, $R^{36}$, $R^{37}$ und $R^{38}$ jeweils unabhängig voneinander eine Kohlenwasserstoffgruppe mit 1 bis 12 Kohlenstoffatomen darstellen, und
$L^{31}$, $L^{32}$, $L^{33}$ und $L^{34}$ jeweils unabhängig voneinander -C(O)O- oder -OC(O)- darstellen.

**9.** Verbindung oder Salz davon nach Anspruch 1, wobei die Verbindung aus den folgenden Formeln oder Salzen davon ausgewählt wird,

Bis(2-butyloctyl)-16-(3-(diethylamino)propyl)-10,22-dihexyl-12,20-dioxo-11,13,19,21-tetraoxa-16-azahentriacontandioat:

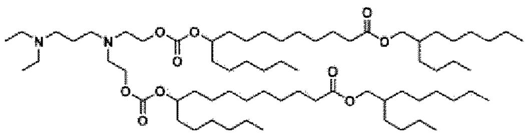

Bis(2-butyloctyl)-11-(2-(diethylamino)ethyl)-5,17-dihexyl-7,15-dioxo-6,8,14,16-tetraoxa-11-azahenicosandioat:

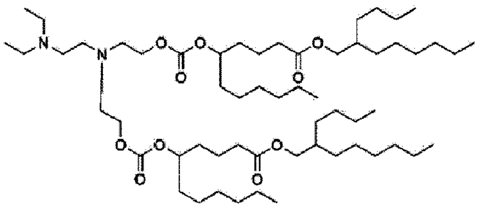

Bis(2-pentylheptyl)-11-(2-(diethylamino)ethyl)-5,17-dihexyl-7,15-dioxo-6,8,14,16-tetraoxa-11-azahenicosandioat:

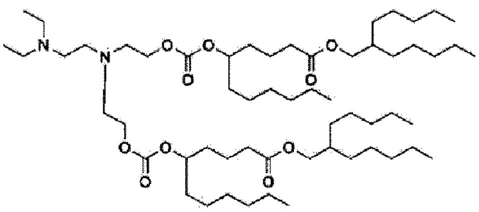

Bis(2-pentylheptyl)-11-(2-(dimethylamino)ethyl)-5,17-dihexyl-7,15-dioxo-6,8,14,16-tetraoxa-11-azahenicosandioat:

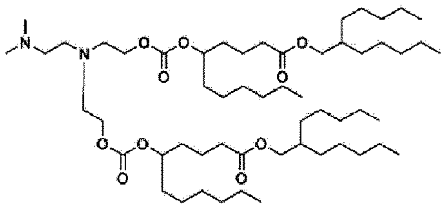

Bis(2-pentylheptyl)-11-(3-(diethylamino)propyl)-5,17-dihexyl-7,15-dioxo-6,8,14,16-tetraoxa-11-azahenicosandioat:

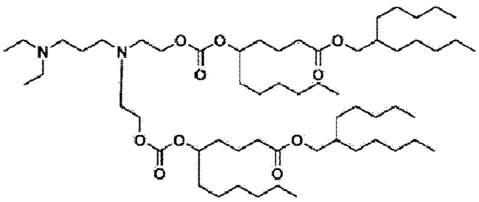

Bis(2-pentylheptyl)-11-(4-(diethylamino)butyl)-5,17-dihexyl-7,15-dioxo-6,8,14,16-tetraoxa-11-azahenicosandioat:

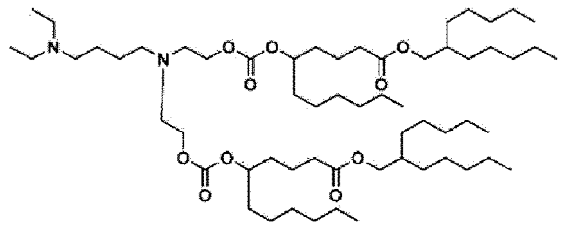

Bis(2-pentylheptyl)-12-(2-(diethylamino)ethyl)-5,19-dihexyl-7,17-dioxo-6,8,16,18-tetraoxa-12-azatricosan-dioat:

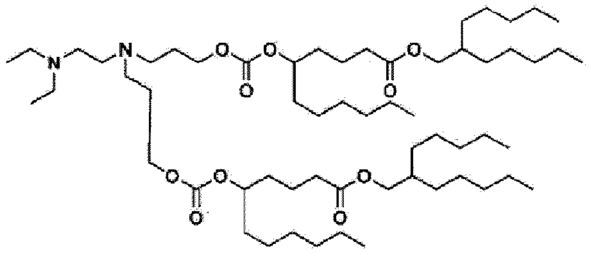

Bis(2-pentylheptyl)-13-(2-(diethylamino)ethyl)-5,21-dihexyl-7,19-dioxo-6,8,18,20-tetraoxa-13-azapentacosan-dioat:

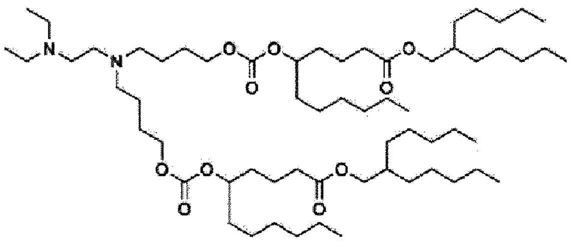

Bis(2-pentylheptyl)-10-(2-(diethylamino)ethyl)-4,16-dihexyl-6,14-dioxo-5,7,13,15-tetraoxa-10-azanonadecan-dioat:

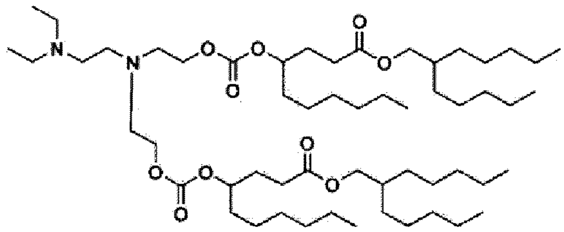

Bis(2-pentylheptyl)-11-(2-(diethylamino)ethyl)-5,17-dimethyl-7,15-dioxo-6,8,14,16-tetraoxa-11-azahenicosan-dioat:

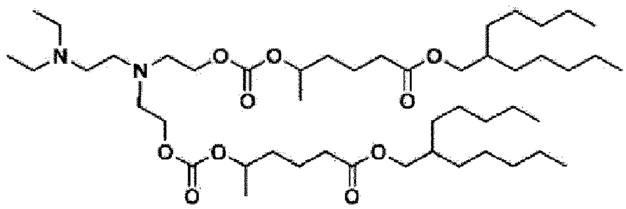

Bis(2-pentylheptyl)-11-(2-(diethylamino)ethyl)-5,17-diethyl-7,15-dioxo-6,8,14,16-tetraoxa-11-azahenicosan-dioat:

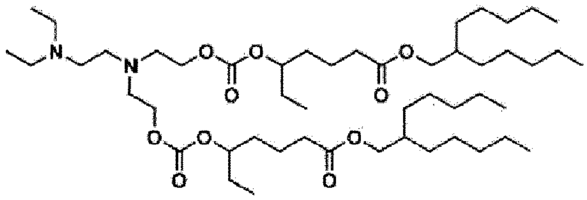

Bis(2-pentylheptyl)-11-(2-(diethylamino)ethyl)-7,15-dioxo-5,17-dipropyl-6,8,14,16-tetraoxa-11-azahenicosandioat:

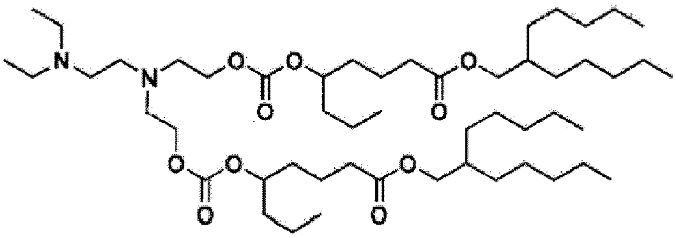

Bis(2-pentylheptyl)-5,17-dibutyl-11-(2-(diethylamino)ethyl)-7,15-dioxo-6,8,14,16-tetraoxa-11-azahenicosandioat:

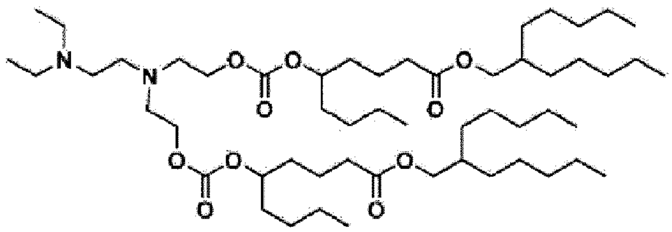

Bis(2-pentylheptyl)-11-(2-(diethylamino)ethyl)-7,15-dioxo-5,17-dipentyl-6,8,14,16-tetraoxa-11-azahenicosandioat:

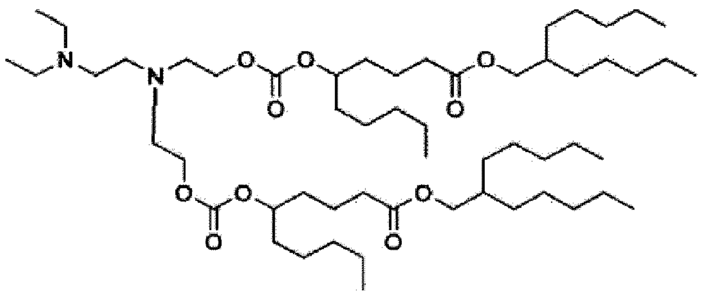

Bis(2-pentylheptyl)-11-(2-(diethylamino)ethyl)-5,17-diheptyl-7,15-dioxo-6,8,14,16-tetraoxa-11-azahenicosandioat:

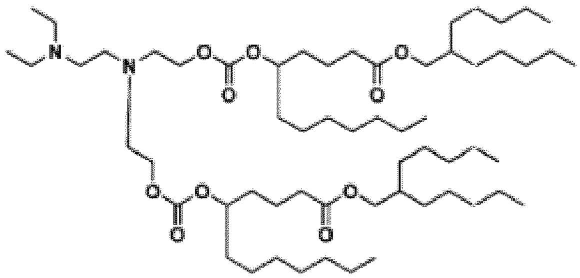

Bis(2-(((3r,5r,7r)-adamantan-1-yl)ethyl)-11-(2-(diethylamino)ethyl)-5,17-dihexyl-7,15-dioxo-6,8,14,16-tetra-

oxa-11-azahenicosandioat;

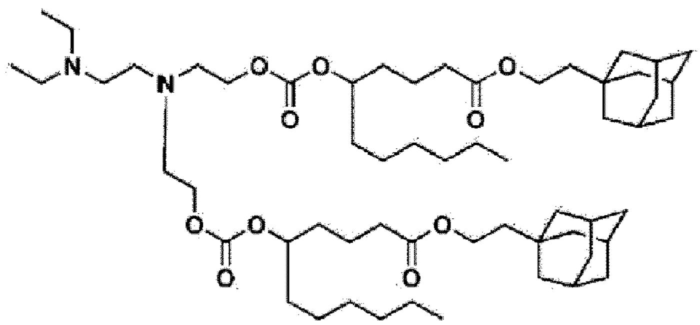

Bis(2-pentylheptyl)-11-(3-(diethylamino)propyl)-7,15-dioxo-5,17-dipropyl-6,8,14,16-tetraoxa-11-azahenicosandioat:

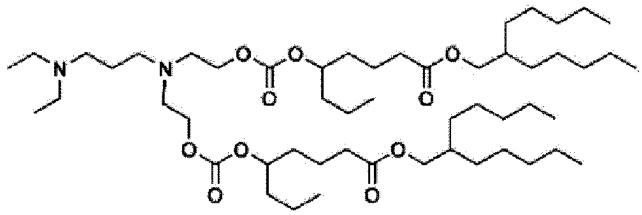

Diheptyl-11-(2-(diethylamino)ethyl)-5,17-bis(4-(heptyloxy)-4-oxobutyl)-7,15-dioxo-6,8,14,16-tetraoxa-11-azahenicosandioat:

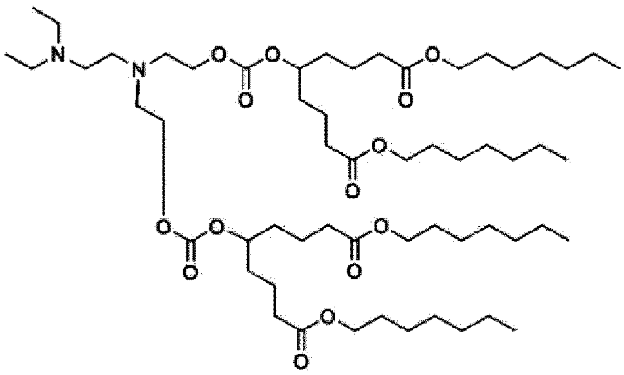

Dihexyl-11-(2-(diethylamino)ethyl)-5,17-bis(4-(hexyloxy)-4-oxobutyl)-7,15-dioxo-6,8,14,16-tetraoxa-11-azahenicosandioat:

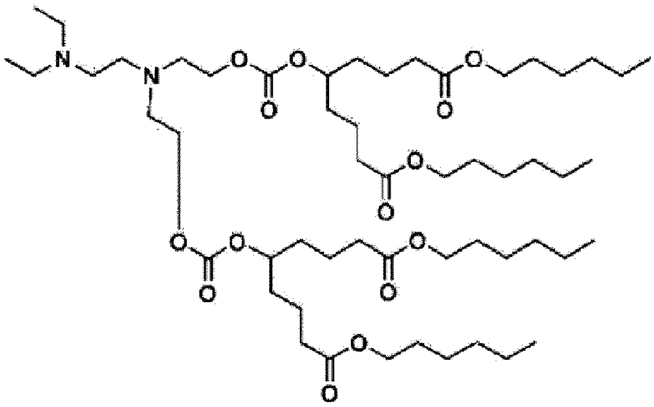

Dioctyl-11-(2-(diethylamino)ethyl)-5,17-bis(4-(octyloxy)-4-oxobutyl)-7,15-dioxo-6,8,14,16-tetraoxa-11-azahe-

nicosandioat:

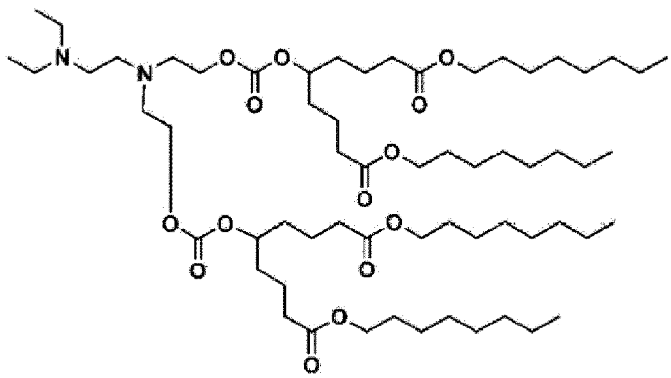

Dinonyl-11-(2-(diethylamino)ethyl)-5,17-bis(4-(nonyloxy)-4-oxobutyl)-7,15-dioxo-6,8,14,16-tetraoxa-11-azahenicosandioat:

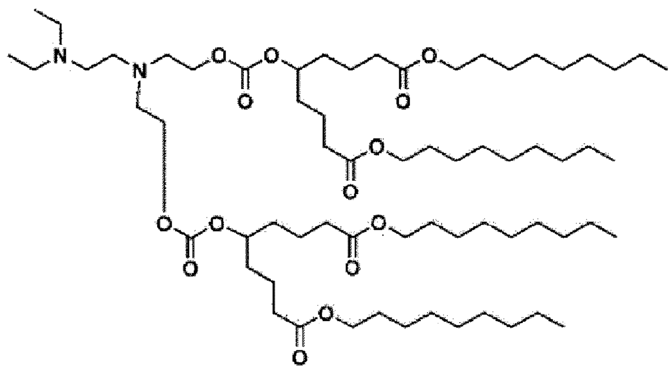

1-Heptyl-21-hexyl-11-(2-(diethylamino)ethyl)-5-(4-(heptyloxy)-4-oxobutyl)-17-(4-(hexyloxy)-4-oxobutyl)-7,15-dioxo-6,8,14,16-tetraoxa-11-azahenicosandioat:

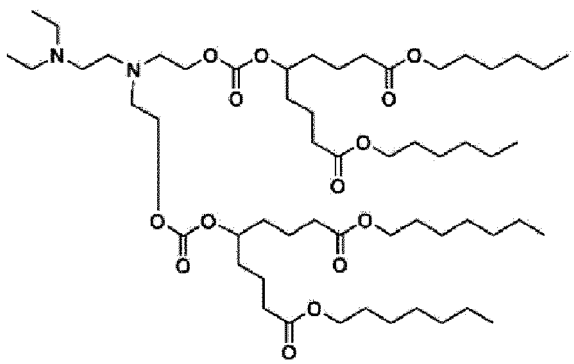

Diheptyl-11-(3-(diethylamino)propyl)-5,17-bis(4-(heptyloxy)-4-oxobutyl)-7,15-dioxo-6,8,14,16-tetraoxa-11-azahenicosandioat:

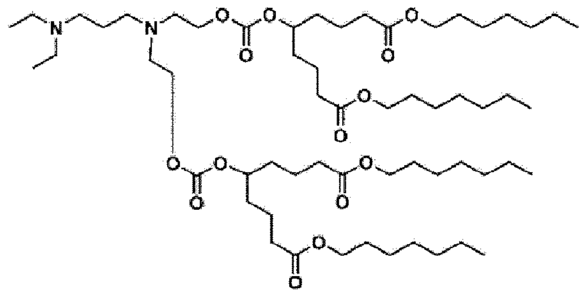

Diheptyl-10-(2-(diethylamino)ethyl)-4,16-bis(3-(heptyloxy)-3-oxopropyl)-6,14-dioxo-5,7,13,15-tetraoxa-10-azanonadecandioat:

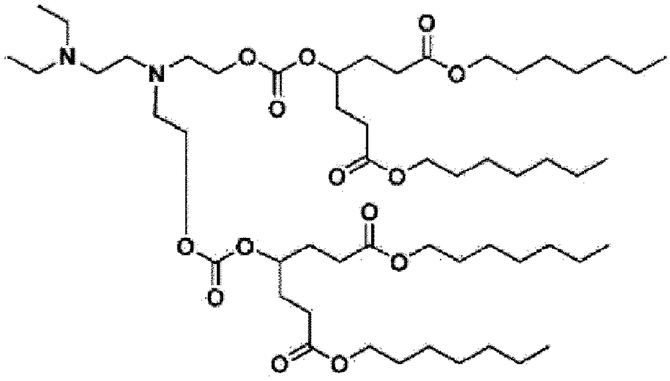

1-Hexyl-21-octyl-11-(2-(diethylamino)ethyl)-5-(4-(hexyloxy)-4-oxobutyl)-17-(4-(octyloxy)-4-oxobutyl)-7,15-dioxo-6,8,14,16-tetraoxa-11-azahenicosandioat:

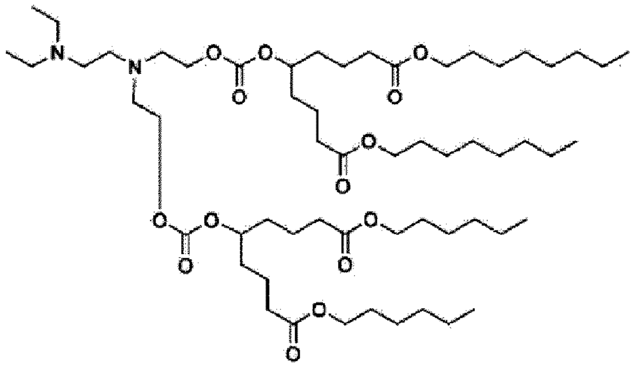

Bis(2-(hexylthio)ethyl)-11-(2-(diethylamino)ethyl)-5,17-bis(4-(2-(hexylthio)ethoxy)-4-oxobutyl)-7,15-dioxo-6,8,14,16-tetraoxa-11-azahenicosandioat:

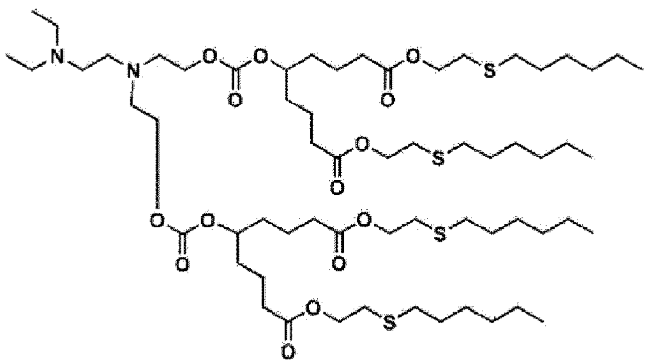

Bis(8-(methylthio)octyl)-11-(2-(diethylamino)ethyl)-5,17-bis(4-((8-(methylthio)octyl)oxy)-4-oxobutyl)-7,15-dioxo-6,8,14,16-tetraoxa-11-azahenicosandioat:

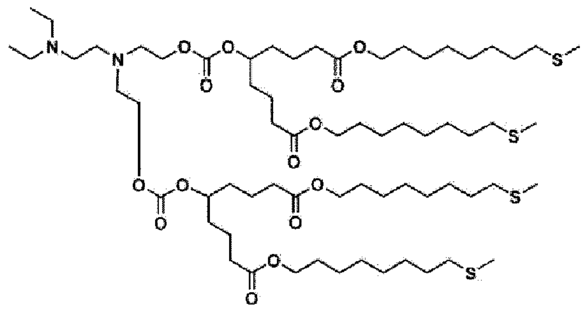

10. Lipidpartikel, umfassend:

die Verbindung oder ein Salz davon nach einem der Ansprüche 1 bis 9; und
ein Lipid.

11. Lipidpartikel nach Anspruch 10,
wobei das Lipid mindestens eine Art von Lipid ist, die aus der Gruppe, die aus einem Sterol und einem Lipid mit einer nichtionischen hydrophilen Polymerkette besteht, ausgewählt wird.

12. Lipidpartikel nach Anspruch 10 oder 11, ferner umfassend:
ein neutrales Lipid.

13. Lipidpartikel nach einem der Ansprüche 10 bis 12, ferner umfassend:
eine Nukleinsäure.

14. Lipidpartikel nach Anspruch 13,
wobei die Nukleinsäure eine Nukleinsäure mit 50 oder mehr Basen enthält.

15. Pharmazeutische Zusammensetzung, umfassend:
die Lipidpartikel nach einem der Ansprüche 10 bis 14 als einen Wirkstoff.

## Revendications

1. Composé représenté par Formule (1) ou un sel de celui-ci,

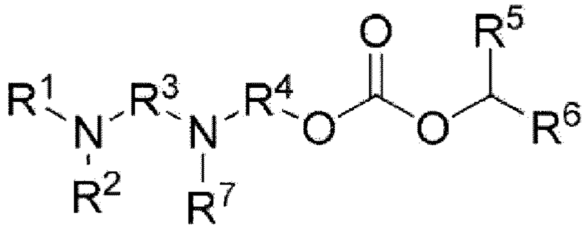

(1)

dans la formule,

$R^1$ et $R^2$ représentent chacun indépendamment un groupe hydrocarboné ayant de 1 à 18 atomes de carbone, et $R^3$ représente un groupe hydrocarboné ayant de 2 à 8 atomes de carbone, où les groupes hydrocarbonés représentés par $R^1$, $R^2$ et $R^3$ peuvent être substitués par un ou plusieurs substituants choisis parmi -OH, -COOH, $-NR^{51}R^{52}$, - OC(O)O-$R^{53}$, -C(O)O-$R^{54}$, -OC(O)-$R^{55}$ et -O-$R^{56}$,
$R^4$ représente un groupe hydrocarboné ayant de 1 à 8 atomes de carbone,
$R^5$ et $R^6$ représentent chacun indépendamment un groupe hydrocarboné ayant de 1 à 8 atomes de carbone ou

$-R^8-L^1-R^9$, à l'exclusion d'un cas où $R^5$ et $R^6$ sont tous deux des groupes hydrocarbonés ayant de 1 à 8 atomes de carbone,
$R^7$ représente $-R^{10}-L^2-R^{11}-L^3-R^{12}$,
$R^{51}$ et $R^{52}$ représentent chacun indépendamment un groupe hydrocarboné ayant de 1 à 8 atomes de carbone,
$R^{53}$, $R^{54}$, $R^{55}$ et $R^{56}$ représentent chacun indépendamment un groupe hydrocarboné ayant de 1 à 24 atomes de carbone,
les groupes hydrocarbonés représentés par $R^{53}$, $R^{54}$, $R^{55}$ et $R^{56}$ peuvent être substitués par un groupe aryle ayant de 6 à 20 atomes de carbone ou $-S-R^{58}$,
le groupe aryle ayant de 6 à 20 atomes de carbone peut être substitué par -OH, -COOH, $-NR^{51}R^{52}$, $-OC(O)O-R^{53}$, $-C(O)O-R^{54}$, $-OC(O)-R^{55}$, $-O-R^{56}$ ou -(groupe hydrocarboné ayant de 1 à 12 atomes de carbone)-$R^{57}$,
$R^{58}$ représente un groupe hydrocarboné ayant de 1 à 12 atomes de carbone,
$R^{57}$ représente -OH, -COOH, $-NR^{61}R^{62}$, $-OC(O)O-R^{63}$, $-C(O)O-R^{64}$, $-OC(O)-R^{65}$ ou $-OR^{66}$,
$R^{61}$ et $R^{62}$ représentent chacun indépendamment un groupe hydrocarboné ayant de 1 à 8 atomes de carbone,
$R^{63}$, $R^{64}$, $R^{65}$ et $R^{66}$ représentent chacun indépendamment un groupe hydrocarboné ayant de 1 à 24 atomes de carbone,
les groupes hydrocarbonés représentés par $R^{63}$, $R^{64}$, $R^{65}$ et $R^{66}$ peuvent être substitués par un groupe aryle ayant de 6 à 20 atomes de carbone ou $-S-R^{68}$,
le groupe aryle ayant de 6 à 20 atomes de carbone peut être substitué par -OH, -COOH, $-NR^{61}R^{62}$, $-OC(O)O-R^{63}$, $-C(O)O-R^{64}$, $-OC(O)-R^{65}$, $-O-R^{66}$ ou -(groupe hydrocarboné ayant de 1 à 12 atomes de carbone)-$R^{67}$,
$R^{68}$ représente un groupe hydrocarboné ayant de 1 à 12 atomes de carbone,
$L^1$, $L^2$ et $L^3$ représentent chacun indépendamment -OC(O)O-, -C(O)O-, -OC(O)**-** ou - **O-,**
$R^8$ représente un groupe hydrocarboné ayant de 1 à 12 atomes de carbone,
$R^9$ représente un groupe hydrocarboné ayant de 1 à 24 atomes de carbone,
$R^{10}$ représente un groupe hydrocarboné ayant de 1 à 8 atomes de carbone,
$R^{11}$ représente un groupe hydrocarboné ayant de 1 à 24 atomes de carbone,
$R^{12}$ représente un groupe hydrocarboné ayant de 1 à 24 atomes de carbone,
les groupes hydrocarbonés représentés par $R^9$ et $R^{12}$ peuvent être substitués par un groupe aryle, $-OC(O)O-R^{53}$, $-C(O)O-R^{54}$, $-OC(O)-R^{55}$ ou $-S-R^{58}$, où les définitions de $R^{53}$, $R^{54}$, $R^{55}$ et $R^{58}$ sont telles que décrites ci-dessus, et le groupe hydrocarboné représenté par $R^{11}$ peut être substitué par $-OC(O)O-R^{53}$, $-C(O)O-R^{54}$, $-OC(O)-R^{55}$, où les définitions de $R^{53}$, $R^{54}$ et $R^{55}$ sont telles que décrites ci-dessus.

**2.** Composé ou un sel de celui-ci selon la revendication 1,

dans lequel $R^1$ et $R^2$ représentent chacun indépendamment un groupe hydrocarboné ayant de 1 à 3 atomes de carbone, et $R^3$ représente un groupe hydrocarboné ayant de 2 à 4 atomes de carbone, où les groupes hydrocarbonés représentés par $R^1$, $R^2$ et $R^3$ peuvent être substitués par -OH,
$R^4$ représente un groupe hydrocarboné ayant de 1 à 8 atomes de carbone,
$R^5$ et $R^6$ représentent chacun indépendamment un groupe hydrocarboné ayant de 1 à 8 atomes de carbone ou $-R^8-L^1-R^9$, à l'exclusion d'un cas où $R^5$ et $R^6$ sont tous deux des groupes hydrocarbonés ayant de 1 à 8 atomes de carbone,
$R^7$ représente $-R^{10}-L^2-R^{11}-L^3-R^{12}$,
$L^1$ et $L^3$ représentent chacun indépendamment -C(O)O- ou -OC(O)-,
$L^2$ représente -OC(O)O-, -C(O)O- ou -OC(O)-,
$R^8$ représente un groupe hydrocarboné ayant de 1 à 8 atomes de carbone,
$R^9$ représente un groupe hydrocarboné ayant de 1 à 16 atomes de carbone,
$R^{10}$ représente un groupe hydrocarboné ayant de 1 à 8 atomes de carbone,
$R^{11}$ représente un groupe hydrocarboné ayant de 1 à 9 atomes de carbone,
$R^{12}$ représente un groupe hydrocarboné ayant de 1 à 16 atomes de carbone,
les groupes hydrocarbonés représentés par $R^9$ et $R^{12}$ peuvent être substitués par un groupe aryle ou $-S-R^{58}$,
$R^{58}$ représente un groupe hydrocarboné ayant de 1 à 8 atomes de carbone,
le groupe hydrocarboné représenté par $R^{11}$ peut être substitué par $-C(O)O-R^{55}$ ou $-OC(O)-R^{56}$,
$R^{55}$ et $R^{56}$ représentent chacun indépendamment un groupe hydrocarboné ayant de 1 à 16 atomes de carbone, et
les groupes hydrocarbonés représentés par $R^{55}$ et $R^{56}$ peuvent être substitués par un groupe aryle ayant de 6 à 20 atomes de carbone ou $-S-R^{58}$, et la définition de $R^{51}$ est telle que décrite ci-dessus.

**3.** Composé ou un sel de celui-ci selon la revendication 1, qui est un composé représenté par Formule (1-1) ou un sel de celui-ci,

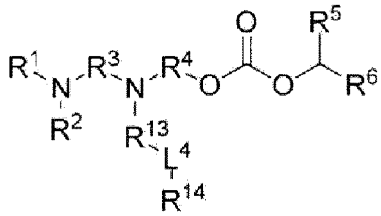

(1-1)

dans la formule,

$R^1$ et $R^2$ représentent chacun indépendamment un groupe hydrocarboné ayant de 1 à 18 atomes de carbone, et $R^3$ représente un groupe hydrocarboné ayant de 2 à 8 atomes de carbone, où les groupes hydrocarbonés représentés par $R^1$, $R^2$ et $R^3$ peuvent être substitués par -OH, -COOH, $-NR^{51}R^{52}$, $-OC(O)O-R^{53}$, $-C(O)O-R^{54}$, $-OC(O)-R^{55}$ ou $-OR^{56}$,

$R^4$ représente un groupe hydrocarboné ayant de 1 à 8 atomes de carbone,

$R^5$ et $R^6$ représentent chacun indépendamment un groupe hydrocarboné ayant de 1 à 8 atomes de carbone ou $-R^8-L^1-R^9$, à l'exclusion d'un cas où $R^5$ et $R^6$ sont tous deux des groupes hydrocarbonés ayant de 1 à 8 atomes de carbone,

$L^1$ représente -OC(O)O-, -C(O)O-, -OC(O)- ou -O-,

$R^8$ représente un groupe hydrocarboné ayant de 1 à 12 atomes de carbone,

$R^9$ représente un groupe hydrocarboné ayant de 1 à 24 atomes de carbone, où le groupe hydrocarboné représenté par $R^9$ peut être substitué par un groupe aryle, $-OC(O)O-R^{53}$, $-C(O)O-R^{54}$, $-OC(O)-R^{55}$ ou $-S-R^{58}$,

$R^{51}$ et $R^{52}$ représentent chacun indépendamment un groupe hydrocarboné ayant de 1 à 8 atomes de carbone,

$R^{53}$, $R^{54}$, $R^{55}$ et $R^{56}$ représentent chacun indépendamment un groupe hydrocarboné ayant de 1 à 24 atomes de carbone,

les groupes hydrocarbonés représentés par $R^{53}$, $R^{54}$, $R^{55}$ et $R^{56}$ peuvent être substitués par un groupe aryle ayant de 6 à 20 atomes de carbone ou $-S-R^{58}$,

le groupe aryle ayant de 6 à 20 atomes de carbone peut être substitué par -OH, -COOH, $-NR^{51}R^{52}$, $-OC(O)O-R^{53}$, $-C(O)O-R^{54}$, $-OC(O)-R^{55}$, $-O-R^{56}$ ou -(groupe hydrocarboné ayant de 1 à 12 atomes de carbone)-$R^{57}$,

$R^{58}$ représente un groupe hydrocarboné ayant de 1 à 12 atomes de carbone,

$R^{57}$ représente -OH, -COOH, $-NR^{51}R^{52}$, $-OC(O)O-R^{53}$, $-C(O)O-R^{54}$, $-OC(O)-R^{55}$ ou $-OR^{56}$,

$R^{13}$ représente un groupe hydrocarboné ayant de 1 à 8 atomes de carbone,

$R^{14}$ représente $-R^{15}-L^5-R^{16}$, où $R^{15}$ représente un groupe hydrocarboné ayant de 1 à 24 atomes de carbone, $L^5$ représente -OC(O)O-, -C(O)O-, -OC(O)- ou -O-, et $R^{16}$ représente un groupe hydrocarboné ayant de 1 à 24 atomes de carbone,

le groupe hydrocarboné ayant de 1 à 24 atomes de carbone représenté par $R^{15}$ peut être substitué par $-OC(O)O-R^{53}$, $-C(O)O-R^{54}$ ou $-OC(O)-R^{55}$, où les définitions de $R^{53}$, $R^{54}$ et $R^{55}$ sont telles que décrites ci-dessus, et

le groupe hydrocarboné ayant de 1 à 24 atomes de carbone représenté par $R^{16}$ peut être substitué par un groupe aryle ayant de 6 à 20 atomes de carbone, $-OC(O)O-R^{53}$, $- C(O)O-R^{54}$, $-OC(O)-R^{55}$ ou $-S-R^{58}$, où les définitions de $R^{53}$, $R^{54}$, $R^{55}$ et $R^{58}$ sont telles que décrites ci-dessus.

**4.** Composé et un sel de celui-ci selon la revendication 3,

dans lequel dans Formule (1-1),

$R^1$ et $R^2$ représentent chacun indépendamment un groupe hydrocarboné ayant de 1 à 3 atomes de carbone, et $R^3$ représente un groupe hydrocarboné ayant de 2 à 4 atomes de carbone, où les groupes hydrocarbonés représentés par $R^1$, $R^2$ et $R^3$ peuvent être substitués par -OH,

$R^4$ représente un groupe hydrocarboné ayant de 1 à 8 atomes de carbone,

$R^5$ et $R^6$ représentent chacun indépendamment un groupe hydrocarboné ayant de 1 à 8 atomes de carbone ou $-R^8-L^1-R^9$, à l'exclusion d'un cas où $R^5$ et $R^6$ sont tous deux des groupes hydrocarbonés ayant de 1 à 8 atomes de carbone,

$L^1$ représente -C(O)O- ou -OC(O)-,

$R^8$ représente un groupe hydrocarboné ayant de 1 à 8 atomes de carbone,

$R^9$ représente un groupe hydrocarboné ayant de 1 à 18 atomes de carbone, et le groupe hydrocarboné représenté par $R^9$ peut être substitué par un groupe aryle ayant de 6 à 20 atomes de carbone ou $-S-R^{58}$,

$R^{58}$ représente un groupe hydrocarboné ayant de 1 à 8 atomes de carbone,

$R^{13}$ représente un groupe hydrocarboné ayant de 1 à 8 atomes de carbone,

$R^{14}$ représente $-R^{15}-L^5-R^{16}$, où $R^{15}$ représente un groupe hydrocarboné ayant de 1 à 18 atomes de carbone, $L^5$

représente -OC(O)O-, et $R^{16}$ représente un groupe hydrocarboné ayant de 1 à 18 atomes de carbone,
le groupe hydrocarboné ayant de 1 à 18 atomes de carbone représenté par $R^{15}$ peut être substitué par -C(O)O-$R^{55}$ ou -OC(O)-$R^{56}$,
$R^{55}$ et $R^{56}$ représentent chacun indépendamment un groupe hydrocarboné ayant de 1 à 16 atomes de carbone, et les groupes hydrocarbonés représentés par $R^{55}$ et $R^{56}$ peuvent être substitués par un groupe aryle ayant de 6 à 20 atomes de carbone ou -S-$R^{58}$, où la définition de $R^{58}$ est telle que décrite ci-dessus, et
le groupe hydrocarboné ayant de 1 à 18 atomes de carbone représenté par $R^{16}$ peut être substitué par un groupe aryle ou -S-$R^{58}$, où la définition de $R^{58}$ est telle que décrite ci-dessus.

**5.** Composé ou un sel de celui-ci selon la revendication 1, qui est un composé représenté par Formule (1-2) ou un sel de celui-ci,

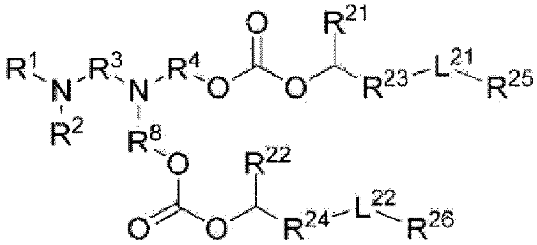

(1-2)

dans la formule,
$R^1$ et $R^2$ représentent chacun indépendamment un groupe hydrocarboné ayant de 1 à 18 atomes de carbone, et $R^3$ représente un groupe hydrocarboné ayant de 2 à 8 atomes de carbone, où les groupes hydrocarbonés représentés par $R^1$, $R^2$ et $R^3$ peuvent être substitués par -OH, -COOH, -NR$^{51}$R$^{52}$, -OC(O)O-$R^{53}$, -C(O)O-$R^{54}$, -OC(O)-$R^{55}$ ou -OR$^{56}$,
$R^4$ et $R^8$ représentent chacun indépendamment un groupe hydrocarboné ayant de 1 à 8 atomes de carbone,
$R^{21}$ et $R^{22}$ représentent chacun indépendamment un groupe hydrocarboné ayant de 1 à 18 atomes de carbone,
$R^{23}$ et $R^{24}$ représentent chacun indépendamment un groupe hydrocarboné ayant de 1 à 12 atomes de carbone,
$R^{25}$ et $R^{26}$ représentent chacun indépendamment un groupe hydrocarboné ayant de 1 à 24 atomes de carbone,
$L^{21}$ et $L^{22}$ représentent chacun indépendamment -OC(O)O-, -C(O)O-, -OC(O)- ou -O-, les groupes hydrocarbonés représentés par $R^{25}$ et $R^{26}$ peuvent être substitués par un groupe aryle ayant 6 à 20 atomes de carbone, -OC(O)O-$R^{53}$, -C(O)O-$R^{54}$, -OC(O)-$R^{55}$ ou -S-$R^{58}$,
$R^{51}$ et $R^{52}$ représentent chacun indépendamment un groupe hydrocarboné ayant de 1 à 8 atomes de carbone,
$R^{53}$, $R^{54}$, $R^{55}$ et $R^{56}$ représentent chacun indépendamment un groupe hydrocarboné ayant de 1 à 18 atomes de carbone,
le groupe aryle ayant de 6 à 20 atomes de carbone peut être substitué par -OH, -COOH, -NR$^{51}$R$^{52}$, -OC(O)O-$R^{53}$, -C(O)O-$R^{54}$, -OC(O)-$R^{55}$, -O-$R^{56}$ ou -(groupe hydrocarboné ayant de 1 à 12 atomes de carbone)-$R^{57}$,
$R^{57}$ représente -OH, -COOH, -NR$^{51}$R$^{52}$, -OC(O)O-$R^{53}$, -C(O)O-$R^{54}$, -OC(O)-$R^{55}$ ou -OR$^{56}$, et
$R^{58}$ représente un groupe hydrocarboné ayant de 1 à 12 atomes de carbone.

**6.** Composé et un sel de celui-ci selon la revendication 5,

dans lequel dans Formule (1-2),
$R^1$ et $R^2$ représentent chacun indépendamment un groupe hydrocarboné ayant de 1 à 3 atomes de carbone, où les groupes hydrocarbonés représentés par $R^1$ et $R^2$ peuvent être substitués par -OH,
$R^3$ représente un groupe hydrocarboné ayant de 2 à 4 atomes de carbone,
$R^4$ et $R^8$ représentent chacun indépendamment un groupe hydrocarboné ayant de 1 à 8 atomes de carbone,
$R^{21}$ et $R^{22}$ représentent chacun indépendamment un groupe hydrocarboné ayant de 1 à 8 atomes de carbone,
$R^{23}$ et $R^{24}$ représentent chacun indépendamment un groupe hydrocarboné ayant de 1 à 8 atomes de carbone,
$R^{25}$ et $R^{26}$ représentent chacun indépendamment un groupe hydrocarboné ayant de 1 à 16 atomes de carbone, et
$L^{21}$ et $L^{22}$ représentent chacun indépendamment -C(O)O- ou -OC(O)-, ou
dans lequel dans Formule (1-2),
$R^1$ et $R^2$ représentent chacun indépendamment un groupe hydrocarboné ayant de 1 à 3 atomes de carbone,
$R^3$ représente un groupe hydrocarboné ayant de 2 à 4 atomes de carbone,
$R^4$ et $R^8$ représentent chacun indépendamment un groupe hydrocarboné ayant de 1 à 8 atomes de carbone,

$R^{21}$ et $R^{22}$ représentent chacun indépendamment un groupe hydrocarboné ayant de 1 à 6 atomes de carbone, $R^{23}$ et $R^{24}$ représentent chacun indépendamment un groupe hydrocarboné ayant de 1 à 8 atomes de carbone, $R^{25}$ et $R^{26}$ représentent chacun indépendamment un groupe hydrocarboné ayant de 1 à 12 atomes de carbone, et
$L^{21}$ et $L^{22}$ représentent chacun indépendamment -C(O)O- ou -OC(O)-.

**7.** Composé ou un sel de celui-ci selon la revendication 1, qui est un composé représenté par Formule (1-3) ou un sel de celui-ci,

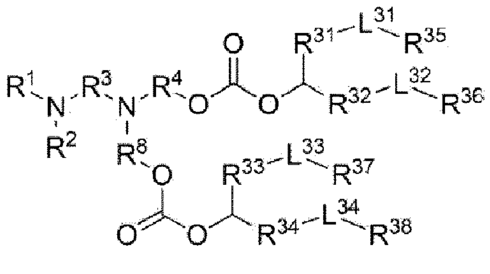
(1 − 3)

dans la formule,
$R^1$ et $R^2$ représentent chacun indépendamment un groupe hydrocarboné ayant de 1 à 18 atomes de carbone, et $R^3$ représente un groupe hydrocarboné ayant de 2 à 8 atomes de carbone, où les groupes hydrocarbonés représentés par $R^1$, $R^2$ et $R^3$ peuvent être substitués par -OH, -COOH, $-NR^{51}R^{52}$, $-OC(O)O-R^{53}$, $-C(O)O-R^{54}$, $-OC(O)-R^{55}$ ou $-OR^{56}$,
$R^4$ et $R^8$ représentent chacun indépendamment un groupe hydrocarboné ayant de 1 à 8 atomes de carbone,
$R^{31}$, $R^{32}$, $R^{33}$ et $R^{34}$ représentent chacun indépendamment un groupe hydrocarboné ayant de 1 à 12 atomes de carbone,
$R^{35}$, $R^{36}$, $R^{37}$ et $R^{38}$ représentent chacun indépendamment un groupe hydrocarboné ayant de 1 à 24 atomes de carbone,
$L^{31}$, $L^{32}$, $L^{33}$ et $L^{34}$ représentent chacun indépendamment -OC(O)O-, -C(O)O-, -OC(O)- ou -O-,
les groupes hydrocarbonés représentés par $R^{35}$, $R^{36}$, $R^{37}$ et $R^{31}$ peuvent être substitués par un groupe aryle ayant 6 à 20 atomes de carbone, $-OC(O)O-R^{53}$, $-C(O)O-R^{54}$, - $OC(O)-R^{55}$ ou $-S-R^{58}$,
$R^{51}$ et $R^{52}$ représentent chacun indépendamment un groupe hydrocarboné ayant de 1 à 8 atomes de carbone,
$R^{53}$, $R^{54}$, $R^{55}$ et $R^{56}$ représentent chacun indépendamment un groupe hydrocarboné ayant de 1 à 18 atomes de carbone,
le groupe aryle ayant de 6 à 20 atomes de carbone peut être substitué par -OH, -COOH, $-NR^{51}R^{52}$, $-OC(O)O-R^{53}$, $-C(O)O-R^{54}$, $-OC(O)-R^{55}$, $-O-R^{56}$ ou -(groupe hydrocarboné ayant de 1 à 12 atomes de carbone)-$R^{57}$,
$R^{57}$ représente -OH, -COOH, $-NR^{51}R^{52}$, $-OC(O)O-R^{53}$, $-C(O)O-R^{54}$, $-OC(O)-R^{55}$ ou $-OR^{56}$, et
$R^{58}$ représente un groupe hydrocarboné ayant de 1 à 12 atomes de carbone.

**8.** Composé et un sel de celui-ci selon la revendication 7,

dans lequel dans Formule (1-3),
$R^1$ et $R^2$ représentent chacun indépendamment un groupe hydrocarboné ayant de 1 à 3 atomes de carbone, où les groupes hydrocarbonés représentés par $R^1$ et $R^2$ peuvent être substitués par -OH,
$R^3$ représente un groupe hydrocarboné ayant de 2 à 4 atomes de carbone,
$R^4$ et $R^8$ représentent chacun indépendamment un groupe hydrocarboné ayant de 1 à 8 atomes de carbone,
$R^{31}$, $R^{32}$, $R^{33}$ et $R^{34}$ représentent chacun indépendamment un groupe hydrocarboné ayant de 1 à 8 atomes de carbone,
$R^{35}$, $R^{36}$, $R^{37}$ et $R^{38}$ représentent chacun indépendamment un groupe hydrocarboné ayant de 1 à 16 atomes de carbone,
$L^{31}$, $L^{32}$, $L^{33}$ et $L^{34}$ représentent chacun indépendamment -C(O)O- ou -OC(O)-, les groupes hydrocarbonés représentés par $R^{35}$, $R^{36}$, $R^{37}$ et $R^{38}$ peuvent être substitués par un groupe aryle ayant 6 à 20 atomes de carbone ou $-S-R^{58}$, et
$R^{58}$ représente un groupe hydrocarboné ayant de 1 à 8 atomes de carbone, ou dans lequel dans Formule (1-3),
$R^1$ et $R^2$ représentent chacun indépendamment un groupe hydrocarboné ayant de 1 à 3 atomes de carbone,
$R^3$ représente un groupe hydrocarboné ayant de 2 à 4 atomes de carbone,
$R^4$ et $R^8$ représentent chacun indépendamment un groupe hydrocarboné ayant de 1 à 8 atomes de carbone,

$R^{31}$, $R^{32}$, $R^{33}$ et $R^{34}$ représentent chacun indépendamment un groupe hydrocarboné ayant de 1 à 3 atomes de carbone,
$R^{35}$, $R^{36}$, $R^{37}$ et $R^{38}$ représentent chacun indépendamment un groupe hydrocarboné ayant de 1 à 12 atomes de carbone,
$L^{31}$, $L^{32}$, $L^{33}$ et $L^{34}$ représentent chacun indépendamment -C(O)O- ou -OC(O)-,
les groupes hydrocarbonés représentés par $R^{35}$, $R^{36}$, $R^{37}$ et $R^{38}$ peuvent être substitués par -S-$R^{58}$, et
$R^{58}$ représente un groupe hydrocarboné ayant de 1 à 8 atomes de carbone, ou
dans lequel dans Formule (1-3),
$R^1$ et $R^2$ représentent chacun indépendamment un groupe hydrocarboné ayant de 1 à 3 atomes de carbone,
$R^3$ représente un groupe hydrocarboné ayant de 2 à 4 atomes de carbone,
$R^4$ et $R^8$ représentent chacun indépendamment un groupe hydrocarboné ayant de 1 à 8 atomes de carbone,
$R^{31}$, $R^{32}$, $R^{33}$ et $R^{34}$ représentent chacun indépendamment un groupe hydrocarboné ayant de 1 à 3 atomes de carbone,
$R^{35}$, $R^{36}$, $R^{37}$ et $R^{38}$ représentent chacun indépendamment un groupe hydrocarboné ayant de 1 à 12 atomes de carbone substitué par -S-$R^{58}$, où $R^{58}$ représente un groupe hydrocarboné ayant de 1 à 8 atomes de carbone, et
$L^{31}$, $L^{32}$, $L^{33}$ et $L^{34}$ représentent chacun indépendamment -C(O)O- ou -OC(O)-, ou
dans lequel dans Formule (1-3),
$R^1$ et $R^2$ représentent chacun indépendamment un groupe hydrocarboné ayant de 1 à 3 atomes de carbone,
$R^3$ représente un groupe hydrocarboné ayant de 2 à 4 atomes de carbone,
$R^4$ et $R^8$ représentent chacun indépendamment un groupe hydrocarboné ayant de 1 à 8 atomes de carbone,
$R^{31}$, $R^{32}$, $R^{33}$ et $R^{34}$ représentent chacun indépendamment un groupe hydrocarboné ayant de 1 à 3 atomes de carbone,
$R^{35}$, $R^{36}$, $R^{37}$ et $R^{38}$ représentent chacun indépendamment un groupe hydrocarboné ayant de 1 à 12 atomes de carbone, et
$L^{31}$, $L^{32}$, $L^{33}$ et $L^{34}$ représentent chacun indépendamment -C(O)O- ou -OC(O)-.

**9.** Composé ou un sel de celui-ci selon la revendication 1, dans lequel le composé est choisi parmi les formules suivantes, ou des sels de celles-ci,

Bis(2-butyloctyl)16-(3-(diéthylamino)propyl)-10,22-dihexyl-12,20-dioxo-11,13,19,21-tétraoxa-16-azahentriacontanedioate :

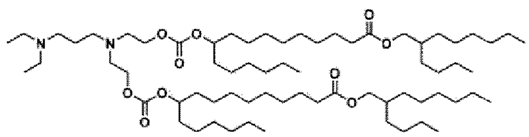

Bis(2-butyloctyl)11-(2-(diéthylamino)éthyl)-5,17-dihexyl-7,15-dioxo-6,8,14,16-tétraoxa-11-azahenicosanedioate :

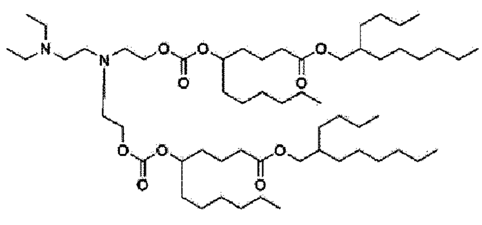

Bis(2-pentylheptyl)11-(2-(diéthylamino)éthyl)-5,17-dihexyl-7,15-dioxo-6,8,14,16-tétraoxa-11-azahenicosanedioate :

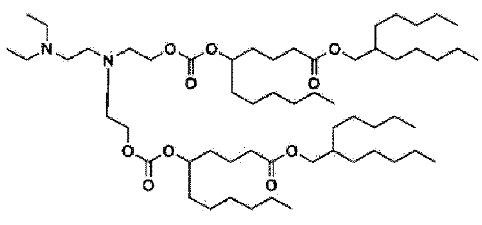

Bis(2-pentylheptyl)11-(2-(diméthylamino)éthyl)-5,17-dihexyl-7,15-dioxo-6,8,14,16-tétraoxa-11-azahenicosanedioate :

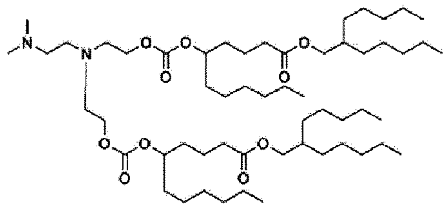

Bis(2-pentylheptyl)11-(3-(diéthylamino)propyl)-5,17-dihexyl-7,15-dioxo-6,8,14,16-tétraoxa-11-azahenicosanedioate :

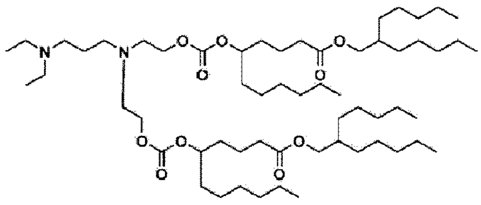

Bis(2-pentylheptyl)11-(4-(diéthylamino)butyl)-5,17-dihexyl-7,15-dioxo-6,8,14,16-tétraoxa-11-azahenicosanedioate :

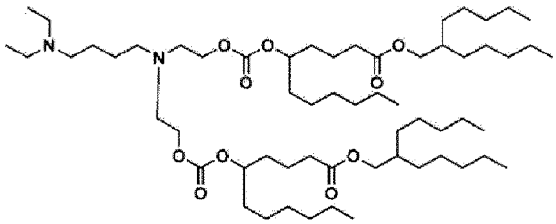

Bis(2-pentylheptyl)12-(2-(diéthylamino)éthyl)-5,19-dihexyl-7,17-dioxo-6,8,16,18-tétraoxa-12-azatricosanedioate :

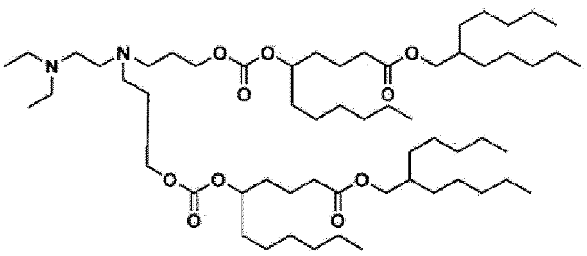

Bis(2-pentylheptyl)13-(2-(diéthylamino)éthyl)-5,21-dihexyl-7,19-dioxo-6,8,18,20-tétraoxa-13-azapentacosanedioate :

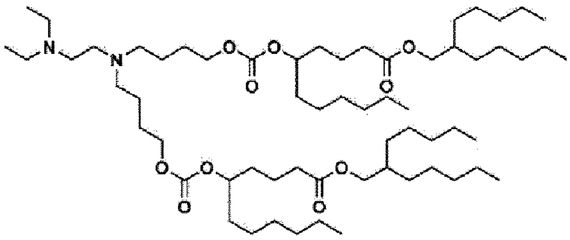

Bis(2-pentylheptyl)10-(2-(diéthylamino)éthyl)-4,16-dihexyl-6,14-dioxo-5,7,13,15-tétraoxa-10-azanonadecanedioate :

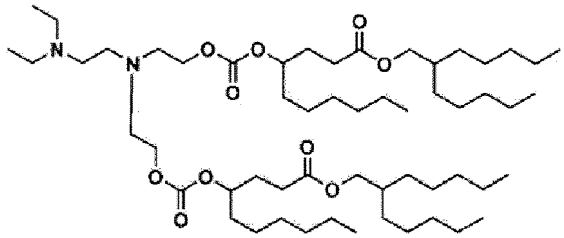

Bis(2-pentylheptyl)11-(2-(diéthylamino)éthyl)-5,17-diméthyl-7,15-dioxo-6,8,14,16-tétraoxa-11-azahenicosanedioate :

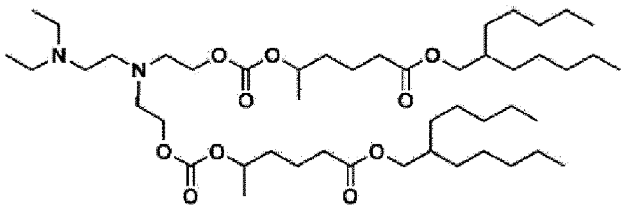

Bis(2-pentylheptyl)11-(2-(diéthylamino)éthyl)-5,17-diéthyl-7,15-dioxo-6,8,14,16-tétraoxa-11-azahenicosanedioate :

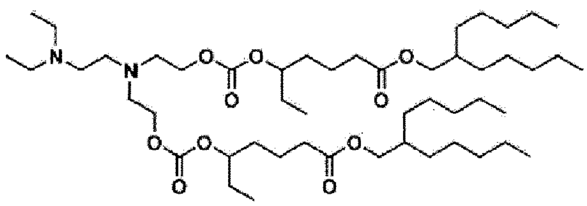

Bis(2-pentylheptyl)11-(2-(diéthylamino)éthyl)-7,15-dioxo-5,17-dipropyl-6,8,14,16-tétraoxa-11-azahenicosanedioate :

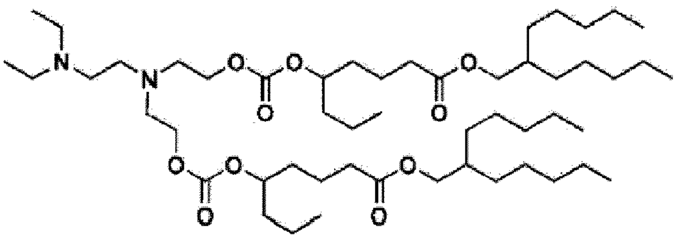

Bis(2-pentylheptyl)5,17-dibutyl-11-(2-(diéthylamino)éthyl)-7,15-dioxo-6,8,14,16-tétraoxa-11-azahenicosanedioate :

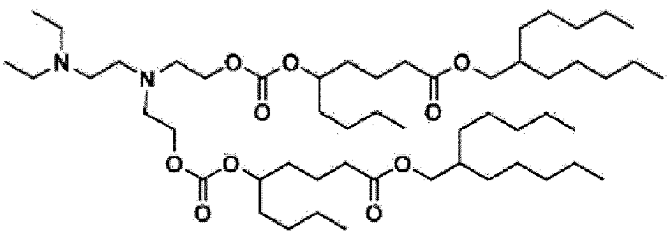

Bis(2-pentylheptyl)11-(2-(diéthylamino)éthyl)-7,15-dioxo-5,17-dipentyl-6,8,14,16-tétraoxa-11-azahenicosanedioate :

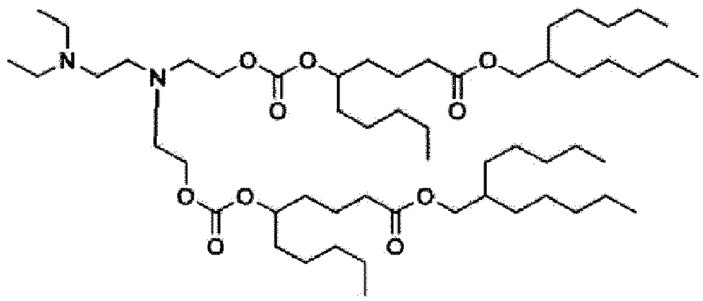

Bis(2-pentylheptyl)11-(2-(diéthylamino)éthyl)-5,17-diheptyl-7,15-dioxo-6,8,14,16-tétraoxa-11-azahenicosanedioate :

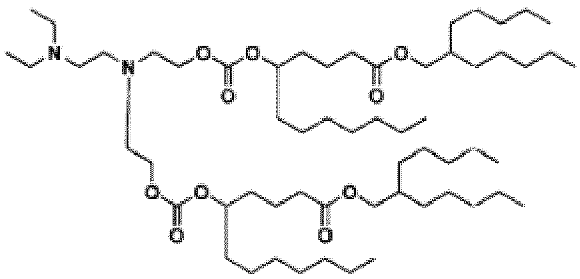

Bis(2-(((3r,5r,7r)-adamantan-1-yl)éthyl)11-(2-(diéthylamino)éthyl)-5,17-dihexyl-7,15-dioxo-6,8,14,16-tétraoxa-11-azahéicosanedioate ;

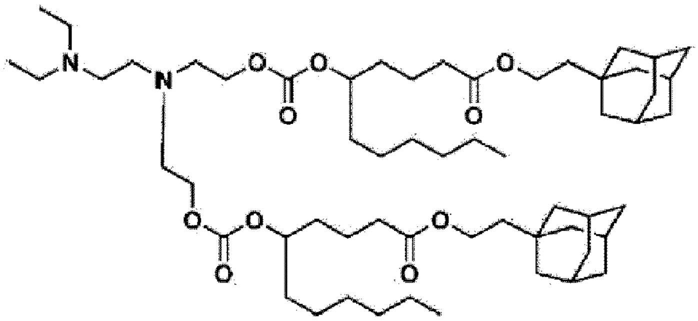

Bis(2-pentylheptyl)11-(3-(diéthylamino)propyl)-7,15-dioxo-5,17-dipropyl-6,8,14,16-tétraoxa-11-azahenicosanedioate :

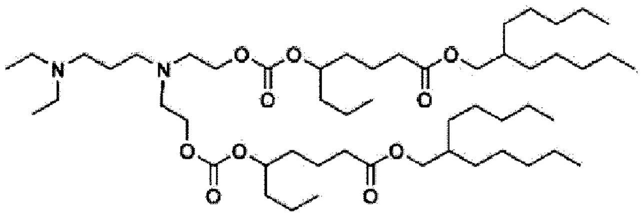

Diheptyl11-(2-(diéthylamino)éthyl)-5,17-bis(4-(heptyloxy)-4-oxobutyl)-7,15-dioxo-6,8,14,16-tétraoxa-11-azahenicosanedioate :

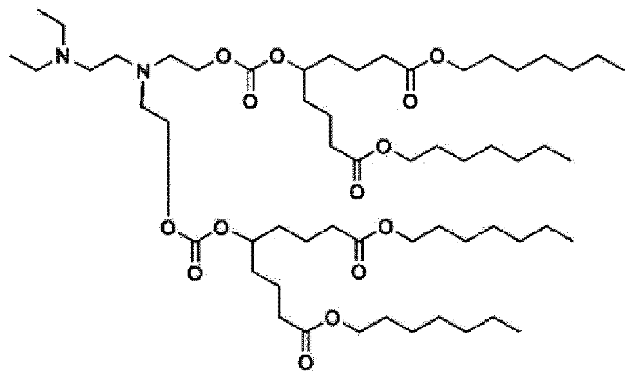

Dihexyl11-(2-(diéthylamino)éthyl)-5,17-bis(4-(hexyloxy)-4-oxobutyl)-7,15-dioxo-6,8,14,16-tétraoxa-11-azahenicosanedioate :

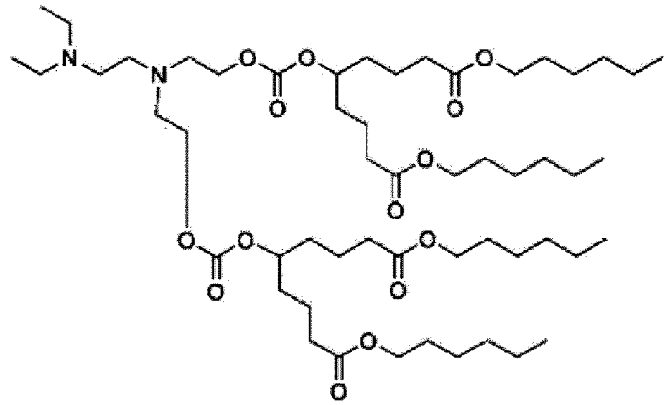

Dioctyl11-(2-(diéthylamino)éthyl)-5,17-bis(4-(octyloxy)-4-oxobutyl)-7,15-dioxo-6,8,14,16-tétraoxa-11-azahenicosanedioate :

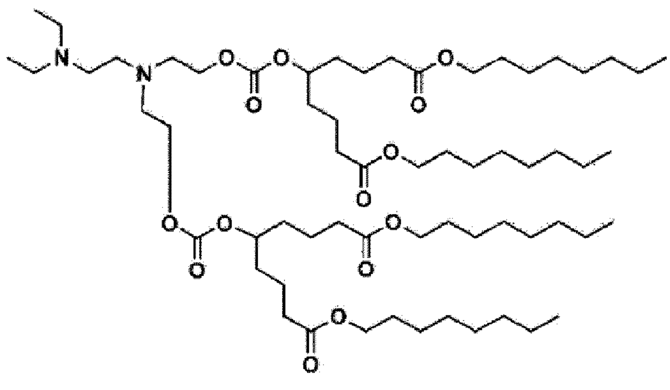

Dinonyl11-(2-(diéthylamino)éthyl)-5,17-bis(4-(nonyloxy)-4-oxobutyl)-7,15-dioxo-6,8,14,16-tétraoxa-11-azahenicosanedioate :

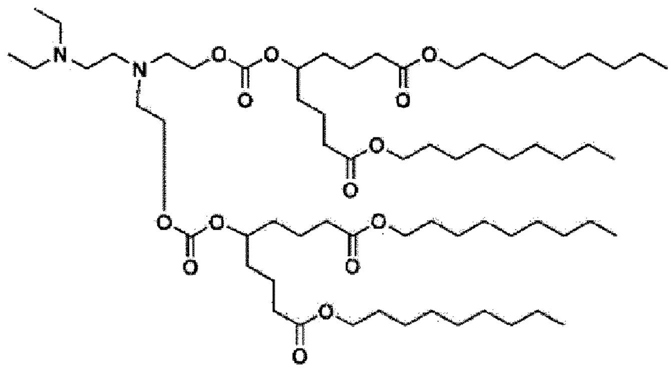

1-Heptyl21-hexyl11-(2-(diéthylamino)éthyl)-5-(4-(heptyloxy)-4-oxobutyl)-17-(4-(hexyloxy)-4-oxobutyl)-7,15-dioxo-6,8,14,16-tétraoxa-11-azahenicosanedioate :

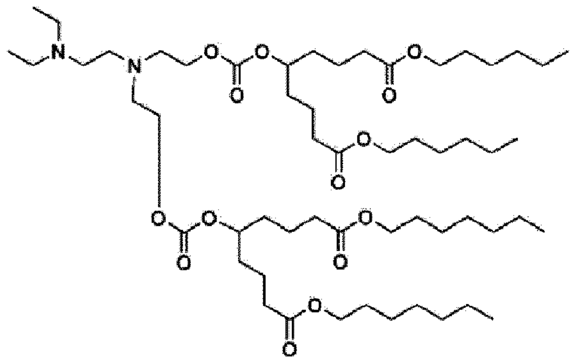

Diheptyl11-(3-(diéthylamino)propyl)-5,17-bis(4-(heptyloxy)-4-oxobutyl)-7,15-dioxo-6,8,14,16-tétraoxa-11-aza-henicosanedioate :

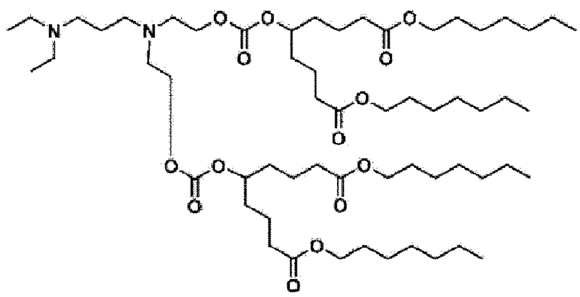

Diheptyl10-(2-(diéthylamino)éthyl)-4,16-bis(3-(heptyloxy)-3-oxopropyl)-6,14-dioxo-5,7,13,15-tétraoxa-10-aza-nonadecanedioate :

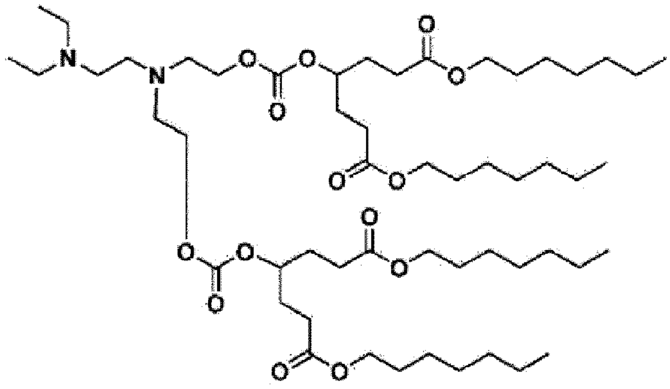

1-Hexyl21-octyl11-(2-(diéthylamino)éthyl)-5-(4-(hexyloxy)-4-oxobutyl)-17-(4-(octyloxy)-4-oxobutyl)-7,15-dioxo-6,8,14,16-tétraoxa-11-azahenicosanedioate :

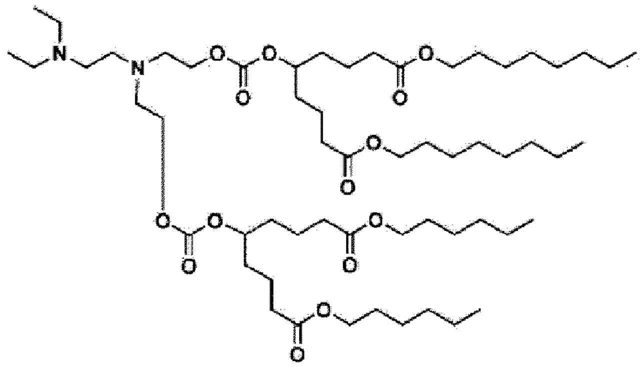

Bis(2-(hexylthio)éthyl)11-(2-(diéthylamino)éthyl)-5,17-bis(4-(2-(hexylthio)éthoxy)-4-oxobutyl)-7,15-dioxo-6,8,14,16-tétraoxa-11-azahenicosanedioate :

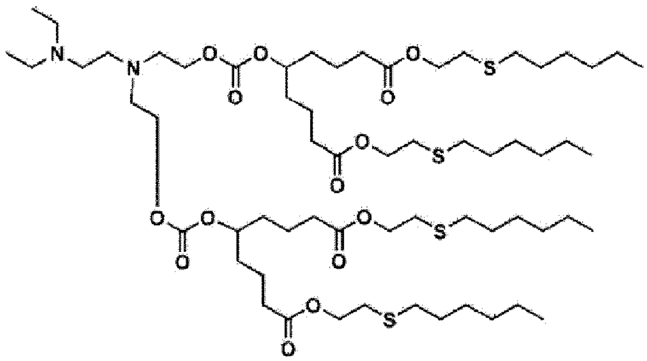

Bis(8-(méthylthio)octyl) 11-(2-(diéthylamino)éthyl)-5,17-bis(4-((8-(méthylthio)octyl)oxy)-4-oxobutyl)-7,15-dioxo-6,8,14,16-tétraoxa-11-azahenicosanedioate :

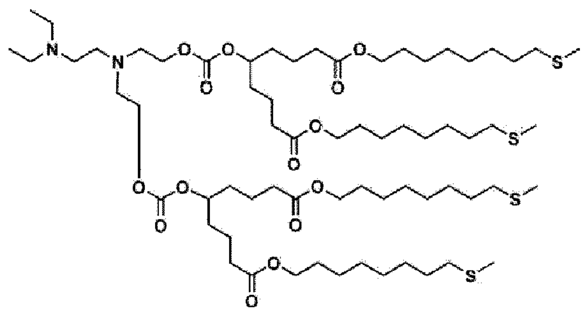

**10.** Particules lipidiques comprenant :

le composé ou un sel de celui-ci selon l'une quelconque des revendications 1 à 9 ; et
un lipide.

**11.** Particules lipidiques selon la revendication 10,
dans lesquelles le lipide est au moins un type de lipide choisi parmi le groupe constitué d'un stérol et d'un lipide ayant une chaîne polymère hydrophile non ionique.

**12.** Particules lipidiques selon la revendication 10 ou la revendication 11, comprenant en outre :
un lipide neutre.

**13.** Particules lipidiques selon l'une quelconque des revendications 10 à 12, comprenant en outre : un acide nucléique.

**14.** Particules lipidiques selon la revendication 13, dans lesquelles l'acide nucléique inclut un acide nucléique ayant 50 bases ou plus.

**15.** Composition pharmaceutique comprenant : les particules lipidiques selon l'une quelconque des revendications 10 à 14 en tant qu'ingrédient actif.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010054401 A
- WO 2019235635 A
- JP 5288254 B